# EUROPEAN PATENT APPLICATION

(11) **EP 3 342 421 A1**
(43) Date of publication of application: **04.07.2018**
(21) Application number: 16306822.4
(22) Date of filing: 27.12.2016
(51) Int. Cl.: A61K 39/00, A61K 39/02, A61K 39/10

(54) **IMMUNOGENIC COMPOSITION COMPRISING CYAA-DERIVED POLYPEPTIDE PROMOTING A TH1/TH17-ORIENTED IMMUNE RESPONSE**

(71) Applicant: GENTICEL, 31670 Labege (FR); Serum Institute of India Private Limited, Pune 411 028 MAH (IN)
(72) Inventor: PALMANTIER, Rémi, 65230 Guizerix (FR); MISSERI, Yolande, 31280 Drémil-Lafage (FR); DIVEU-SADER, Caroline, 75006 Paris (FR); GAIROLA, Sunil, Hadapsar Pune 411028 (IN); GAUTAM, Manish, Panchkula Haryana 134109 (IN); RAO, Harish, Hadapsar Pune 411013 (IN); SHALIGRAM, Umesh, Pune 411009 (IN)
(74) Representative: Desaix, Anne

(57) **Abstract**

The invention relates to the use of a polypeptide derived from the adenylate cyclase of a *Bordetella sp*.(CyaA-derived polypeptide) by deletion of a segment of at least 93 amino acid residues, in particular a polypeptide derived from CyaA of *Bordetella pertussis,* as an immunomodifying antigen of the TH1/TH17-oriented immune response in an immunogenic composition. The invention relates to a vaccine candidate comprising such CyaA-derived polypeptide, either in an acellular immunogenic composition for active immunization against a condition causally related to the infection of a host by *Bordetella sp.* or in a combination composition encompassing said acellular immunogenic composition.

## Description

The invention relates to the use of a polypeptide derived from the adenylate cyclase of a *Bordetella sp.* (CyaA-derived polypeptide), in particular of *Bordetella pertussis,* as an agent promoting the TH1/TH17-oriented immune response in an immunogenic composition. In particular, the invention relates to an immunogenic composition comprising said CyaA-derived polypeptide for the preparation of an acellular candidate vaccine against a condition causally related to the infection by a *Bordetella sp.* or for the preparation of a combination candidate vaccine encompassing the latter in association with other active ingredients for active immunization.

Whooping cough (pertussis) is a vaccine-preventable disease caused by the bacterium *Bordetella pertussis.* The first pertussis vaccines were developed during the 30's and implemented in the developed countries during the 40's and 50's (1). These were whole cell vaccines (WCV or wP) composed of chemically killed bacteria and combined with diphtheria and tetanus toxoids (DTwP) in the late 40's. Between 1950 and 1960, large-scale vaccination with the wP vaccine was followed by a dramatic reduction (>90%) of incidence and mortality of pertussis in industrialized world. However, wP vaccines were reactogenic causing seizures, neurological diseases and other side effects (2).

To avoid those side effects, a second generation of pertussis vaccines, acellular pertussis vaccines (aP) was developed in the early 1980s. The aP vaccines included from 1 to 5 purified antigens from *Bordetella pertussis,* e.g. pertussis toxin (PT) and adhesins such as filamentous hemagglutinine (FHA), pertactin (PRN) and fimbrial agglutinogens (AG). aP vaccines have been used in Japan since 1981 (3) and have been recommended for boosters in the United States since 1991 (4). Because of their safety profile, many developed countries switched to the use of aP for the entire vaccination course at the end of the 1990's and the early 2000's (5).

Since the introduction of aP vaccines, the scientific community has observed a resurgence of whooping cough cases (5-8). In 2008, estimates from WHO suggest that about 16 million cases of pertussis occurred worldwide, and that about 195 000 children died from this disease (9).

aP vaccines have been described to be protective against clinical symptoms of pertussis but not against colonization and transmission of the pathogen in contrast with WCV (14). This observation is supported by a distinct immune response induced by both vaccine types. aP vaccination induces a TH2-oriented immune response whereas WCV induces a TH1 and Th17-oriented immune response (15). This was recently supported by an experiment in a baboon model, where a correlation was made between the absence of a good TH1 response in aP vaccination, the shorter duration of protection and the maintenance of the bacteria since it can colonize lungs of vaccinated animals without inducing the disease (14).

Several studies indicate that aP-induced immunity is shorter in duration than wP- or infection induced immunity, resulting in an increased susceptibility to the infection for those vaccinated with aP compared to those vaccinated with WCV (10, 11). Children that had DTaP as primary vaccination and subsequent doses are more prone to disease that those who received DTwP vaccine as illustrated by a study in the US where after the fifth dose of DTaP, the odds of acquiring pertussis increased by an average of 42% per year (12). This rapid waning immunity has also been described when using aP as boosters (13). Based on a large population of persons aged 8-20 years with a high attack rate due to pertussis outbreaks in the early 2010s in the US and UK, Witt et al showed that there was an 8.57 relative risk (RR) of pertussis (P < .0001) for patients who received only aP vaccines in contrast to those with at least one dose of wP during their pertussis vaccination history (14). In addition, the administration of 1 or more wP doses markedly augmented significantly the durability of immunity from subsequent aP doses.

The difference in the protection and duration may indeed result from the different types of immune responses that are induced after wP and aP. For instance, aP and wP vaccination induce functionally different T cell responses to *pertussis.* Noteworthy, the Th1 or Th2 bias induced by wP or aP primary vaccination, respectively, become fixed and are unchanged upon boosting with Tdap (Bancroft et al, 2016) or even further enhanced in the case of the aP-induced Th2 response (Ryan et al, 2000). Similar observations have been made in mice. These works have evaluated different types of adjuvants (Allen et Mills, Expert Review of Vaccines, 13(10)2014) showing that protection to *B*. *pertussis* challenge was improved in the presence of Th1-oriented adjuvants. In these challenges the injection routes of the vaccines are in majority intra-peritoneal sometimes sub-cutaneous or intra-nasal. The same authors presented at the 11^{th} International Bordetella symposium (April, 5^{th}-8^{th}, 2016 Buenos Aires, Argentina; http://bordetella2016.com.ar/en/images/Programa-Bordetella-Symposium.pdf) results with a prime boost setting where the protection against a B. pertussis challenge of animals immunized with aP+ LP1569 (LP1569 is a TLR2 agonist used as adjuvant prone to induce Th1 or Th17 or both immune responses (TH1/TH17)) and boosted with the same product was obtained in trachea and lungs whereas a prime with aP+alum and a boost with aP+LP1569 was not able to confer this protection. The authors concluded that despite the presence of the Th1-type adjuvant in the boost vaccine the immune response and consequent protection was equivalent to the group vaccinated with aP+alum and boosted with aP+alum.

From these observations, it is understood that the type of immune response induced against the antigens of an acellular *Pertussis* vaccine is one critical factor for the clinical efficacy of the protection of the host, in particular for a sustained protection over time. The identification of agents that may influence the orientation of the immune response toward a TH1 response has been looked for several years and interest has been accordingly directed to properties of one of *Pertussis* proteins, i.e., adenylate cyclase.

The Adenylate Cyclase of *Bordetella pertussis* (CyaA) is a toxin-hemolysin, a protein responsible for immune neutralization of the host's immune system since it targets professional immune cells by binding with high affinity to the CD11b/CD18 integrin receptor which is expressed on innate immune cells, including Antigen-presenting cells (APCs) as macrophages and dendritic cells (DC) (16). Within cells, CyaA is able to suppress host antibactericidal activity thereby promoting bacterial colonization and persistence (17-20). Accordingly detoxified or non-toxic CyaA was preferred in order to assess its properties in relation to immune response.

Different works with detoxified or native CyaA have shown several properties of the molecule. Conclusions and administration protocols of published experiments are disclosed hereafter.
- MacDonald-Fyall et al. (21) describe the adjuvant activity of the detoxified CyaA but this adjuvant activity seems to be lost against Pertussis Toxin (PT) when CyaA is added to the vaccine;
- Cheung et al. (22) describe no significant variation of the total level of IgG antibody responses to PT, FHA and PRN in mice immunized at day 0 and day 28 with an aP vaccine + different forms of CyaA (proCyaA, detoxified CyaA and native CyaA). They describe significant greater levels of IgG2a (TH1 response) to PRN in those mice vaccinated with acellular pertussis vaccine + detoxified CyaA. They also show an increase in aP-specific IFNg response following 2 immunizations with DTaP 1/8 containing 1/8 of the human dose of Aluminium (i.e. 0.0625mg Al⁺⁺⁺/injection) in the presence of detoxified CyaA compared to the same DTaP in the absence of CyaA.
- Dunne et al., (23) describe the capacity of CyaA to promote an IL-1β-mediated Th17 immune response protective in a respiratory challenge model with WT *B*. *pertussis* in mice. They show that CyaA co-injected with KLH, in mice with no addition of conventional vaccine adjuvant such as Aluminum salts induces a specific KLH-TH17 response.
- Dadaglio et al. (24), describe CyaA as an important Toll like receptor (TLR) 4-signaling factor in the induction of protective responses against *B*. *pertussis* through its induction of Th1 T-cells and cytotoxic T lymphocytes (CTL) responses independently of lipopolysaccharide (LPS);

| **Mouse immunization with** | | | **Administration route** | **Adjuvant** | **Vaccination schedule** | **Main results** | | **Reference** |
|---|---|---|---|---|---|---|---|---|
| 1- | PT (120ng), FHA (25ng), PRN (10µg) (per dose) +/-CyaA or CyaA* (15µg/dose) [1] | | Intra-peritoneal | No adj. | Prime | - | Induction of CyaA and CyaA* neutralizing Ab. | MacDonald-Fyall et al., 2004 |
| | | | | | | - | Co-administration with CyaA*: increase of Ab response against PT, FHA and PRN but inclusion of PTd with CyaA* reduces adjuvant effect of CyaA* on Ab response to other Ag. | |
| 2- | PTd [2], FHA, PRN each at 10µg/ dose +/- CyaA or CyaA* (15µg/dose) | | | | | | | |
| | | | | | | - | CyaA*+ Ag activates macrophages (NO production) | |
| | | | | | | - | CyaA* and CyaA when mixed with B. pertussis Ag increase IFNg production | |
| 1- | CyaA + Al(OH)₃ | | Intra-peritoneal | Aluminum hydroxide (Al(OH)₃) | Day 0 and day 28 | - | No significant influence of total IgG antibody response to PT, FHA and PRN | Cheung et al., 2006 |
| 2- | CyaA + 1/8 DTap [3] Infanrix™, GSK) | | | | | | | |
| | | | | | | - | Dtap+CyaA*: increased IgG2a to PRN | |
| 3- | DTap | | | | | - | CyaA*+Dtap: CyaA* appeared to promote a mixed Th1/Th2 response to B. pertussis antigens (compared to CyaA+Dtap and different pro-CyaA forms): increased production of IL5, II6, GM-CSF and IFN-g from spleen cells and NO from macrophages. | |
| 1- | KLH (5µg/mouse) +/-CyaA (1µg/mouse) | | Sub-cutaneous | PBS | Day 0 | - | CyaA induces production of IL-1beta. | Dunne et al., 2010 |
| | | | | | | - | CyaA activates the inflammasome independently of CyaA cytotoxic activity. | |
| 2- | CyaA alone | | | | | | | |
| | | | | | - | CyaA+KLH: CyaA drives KLH-specific IL-17 production *in vivo.* | | |
| | | | | | - | CyaA promotes the induction of *B*. *pertussis-*specific IL-17 production. | | |
| 1- | PT and FHA +Alum | Intra-peritoneal | Alum or LP1569 | -Day 0 and week 4 | - | Immunization with PT/FHA + Alum didn't confer the appropriate lung protection compared to an immunization with PT/FHA + LP1569 | | Dunne et al., 2015 |
| 2- | PT and FHA + LP1569 | | | | | | | |
| 3- | PT+FHA+PRN +/- LP1569 | | | - Day 0 and week 5 | | | | |
| | | | | | - | PT/FHA/PRN + Alum induce a TH2 immune response compared to PT/FHA + LP1569 (Figure 7) | | |
| | 1- CyaA-Tyr | Intravenous | No adjuvant | D0 | | - | highly purified CyaA-Tyr is able to generate efficient CD8+ T cell responses in vivo in the absence of any adjuvant | Dadaglio et al, 2014 |
| | 2- CyaA-OVA | | | | | | | |
| | 3- CyaA-A488 | | | | | | | |
| | | | | | | - | the efficiency of CyaA-Tyr in priming CD8+ T cells is due to its ability to activate cDC | |
| | | | | | | - | CyaA triggers DC activation through the TLR4/TRIF pathway | |
| | | | | | | - | the activation of CD8+ T cells by CyaA is independent of both the inflammasome and IL-1b | |

| | | | | | | | | |
|---|---|---|---|---|---|---|---|---|
| [1] Two Adenylate cyclases were tested, an enzymatically active form named CyaA and an enzymatically inactivated form named CyaA*. [2] PTd: detoxified pertussis toxin [3] one eigth of a single human dose of commercially available diphtheria, tetanus and acellular pertussis vaccine from GSK (25 µg dPT, 25µg FHA, 8µg PRN, 30IU of diphtheria toxoid and 40 IU of tetanus toxoid with 0.5mg of Al(OH)₃ per single human dose. | | | | | | | | |

The experiments reported in the literature, involving the use of CyaA or detoxified CyaA in immunogenic compositions provide contrasted results regarding the influence of CyaA or detoxified CyaA proteins and do not allow to direct further searches in a clear direction as many parameters likely influence the elicitation of an immune response of a type favorable to a strong and lasting protection. Needs for design of new vaccine candidates and suitable administration settings in order to achieve protection thus remain.

### Summary of the invention

Beyond selection of a CyaA polypeptide that might be effective in eliciting or in promoting a response to the antigens of a *Pertussis* vaccine in the context of its administration to a host, the inventors have observed that results achieved in the prior art (such as results disclosed in Cheung et al) could not be reproduced in conditions where the TH2 adjuvant (alum) was in concentration similar to its concentration in doses of human vaccine and also in conditions wherein the animals are primed with TH2 polarizing Acellular Pertussis based vaccines. The inventors have however elucidated appropriate conditions for assessment of the immune response of administered acellular *Pertussis* vaccines, including in the context of combination vaccines encompassing aP and shown that non-toxic CyaA may be a successful immunomodifier for the design of vaccination settings suitable for administration routes used in human hosts.From their experimental results, the inventors have for the first time shown that a non-toxic CyaA, such as the GTL003 used herein provides better anti-CyaA-specific antibody titres, IFN gamma and IL-17 responses when comprised in a vaccine candidate with respect to whole cell vaccine as well as acellular pertussis vaccine that does not contain such non-toxic CyaA polypeptide. Accordingly using a non-toxic CyaA polypeptide will favorably influence protective responses against infection by *Bordetella* strains in the mucosa and the lungs, including for immune response required for a sustained protection.

The inventors have accordingly determined the influence of a non-toxic CyaA, in particular of a CyaA-derived polypeptide which is non-toxic as a result of a deletion of an amino acid segment of the sequence of the native CyaA protein and have assessed such non-toxic CyaA-derived polypeptide in conditions that appear close to administration conditions of available human aP vaccines. Based on results obtained they have been able to devise new compositions comprising detoxified CyaA, in particular recombinant genetically detoxified CyaA-derived polypeptides as described in WO 2014/016310 (and named hereafter GTL003) which have been associated with acellular vaccine antigens of *Bordetella pertussis* in the context of a combination Tdap vaccine adjuvanted with an adjuvant prone to induce primarily TH2 immune responses such as aluminum hydroxide at the human dose of the Tdap vaccine (0.39mg Al⁺⁺⁺ / injection). Unexpectedly, despite the presence of this TH2 adjuvant, the addition of GTL003 to the Tdap vaccine allowed to obtain an antigen-specific cytokine response bias toward TH1/TH17 profilefollowing injection(s) after the first injection administered in mice.

The inventors have accordingly shown that GTL003, as an illustrative example of non-toxic CyaA, when present in an immunogenic composition containing a TH2 type aluminum adjuvant has the previously unknown capacity to induce a cytokine profile of the TH1 / TH17 type, favorable for the generation of effector and memory T-lymphocytes resulting in TH1 IgG isotypes production and the induction of a more protective mucosal immunity, which are key factors in anti-bacterial infection. This crucial orientation of the immune response and possible additional effect on the level of antibodies raised against the antigens of *Pertussis* is an unexpected result that enable to develop a new generation of aP vaccines including a Tdap vaccine that may especially be used for the second and further immunizations when a CyaA-derived polypeptide such as GTL003 promotes a response suitable for higher and long-lasting protection.

The invention is directed to the use of a non-toxic CyaA, in particular a CyaA-derived polypeptide, for the manufacture of an immunogenic composition comprising active ingredients of the immune response of a combination vaccine, wherein said non-toxic CyaA, in particular CyaA-derived polypeptide is provided as an active ingredient of the protective immune response against diseases or conditions causally related to infection by *Bordetella,* in particular *Bordetella pertussis,* and/or is provided as an immunomodulatory agent or immunomodifier triggering the immune response for preventive protection against at least one of the active ingredients of the combination vaccine (preferably against all the active ingredients of the combination vaccine) toward a TH1- and/or a TH17- oriented immune response.

Such a non-toxic CyaA is a protein or a polypeptide that has lost its enzymatic adenylate cyclase activity. Non-toxic CyaA have been disclosed in the art, and may accordingly result from chemical or genetic modifications of the native protein of *Bordetella.*

In particular, it is a CyaA-derived polypeptide which is non cytotoxic polypeptide and is derived from the adenylate cyclase protein (CyaA-derived polypeptide) of a *Bordetella* strain, said polypeptide being **(i)** a polypeptide the amino acid sequence of which is obtained from CyaA of Bordetella pertussis and contains a deletion of a continuous segment of at least 93 amino acid residues from position 227 to position 321 in the amino acid sequence of the native CyaA of *Bordetella pertussis* or **(ii)** a polypeptide which is a variant of the polypeptide in (i) the amino acid sequence of said variant polypeptide being obtained from CyaA of *Bordetella parapertussis, Bordetella bronchiseptica* or *Bordetella hinzii,* and containing the deletion of a continuous segment of at least 93 amino acid residues defined by the positions in the native CyaA sequence of *Bordetella parapertussis, Bordetella bronchiseptica* or *Bordetella hinzii* matching those in CyaA of said continuous segment of at least 93 amino acid residues of *Bordetella pertussis.*

In a particular embodiment of the invention the immunogenic composition is chosen in the group of:
a) an acellular immunogenic composition suitable for the protection against a condition causally related to the infection by a *Bordetella* strain, in particular *Bordetella pertussis* in a human host, or
b) a combination immunogenic composition comprising said acellular immunogenic composition suitable for the protection against a condition causally related to the infection by a *Bordetella* strain, in particular *Bordetella pertussis* in a human host
wherein said acellular immunogenic composition comprises:
- a non-toxic polypeptide, in particular a non-cytotoxic polypeptide, derived from the adenylate cyclase protein (CyaA-derived polypeptide) of a *Bordetella* strain, said polypeptide being in a particular embodiment **(i)** a polypeptide the amino acid sequence of which is obtained from CyaA of *Bordetella pertussis* and contains a deletion of a continuous segment of at least 93 amino acid residues from position 227 to position 321 in the amino acid sequence of the native CyaA of *Bordetella pertussis* or **(ii)** a polypeptide which is a variant of the polypeptide in (i) in particular a variant the amino acid sequence of which is obtained from CyaA of *Bordetella parapertussis, Bordetella bronchiseptica* or *Bordetella hinzii,* and containing the deletion of a continuous segment of at least 93 amino acid residues defined by the positions in the native CyaA sequence of *Bordetella parapertussis, Bordetella bronchiseptica* or *Bordetella hinzii* matching positions 227 and 321 in CyaA of said continuous segment of at least 93 amino acid residues of *Bordetella pertussis* and,
- optionally an adjuvant of the TH1 immune response or an adjuvant of the TH2 immune response or a combination of both

In a particular aspect of the invention, the immunogenic composition is an acellular immunogenic composition suitable for the protection against a condition causally related to the infection by a *Bordetella* strain, in particular *Bordetella pertussis,* in a human which comprises:
- at least two antigens from a *Bordetella* strain wherein one antigen is **(i)** a non-toxic polypeptide, in particular a non-cytotoxic polypeptide, derived from the adenylate cyclase protein (CyaA-derived polypeptide) of a *Bordetella* strain said polypeptide being **(i.1)** either a polypeptide the amino acid sequence of which is obtained from CyaA of *Bordetella pertussis* and contains a deletion of a continuous segment of at least 93 amino acid residues from position 227 to position 321 in the amino acid sequence of the native CyaA of *Bordetella pertussis,* **(i.2)** or a polypeptide which is a variant of the polypeptide in (i.1) the amino acid sequence of said variant polypeptide being obtained from CyaA of *Bordetella parapertussis, Bordetella bronchiseptica* or *Bordetella hinzii,* and containing the deletion of a continuous segment of at least 93 amino acid residues defined by the positions in the native CyaA sequence of *Bordetella parapertussis, Bordetella bronchiseptica* or *Bordetella hinzii* matching positions 227 and 321 in CyaA of said continuous segment of at least 93 amino acid residues of *Bordetella pertussis,* and wherein further antigen(s) is (are) selected in the group of **(ii)** *Pertussis* toxoid (PT), **(iii)** Filamentous Haemagglutinin (FHA), **(iv)** Pertactin (PRN) and **(v)** Fimbriae (Fim),
   wherein a protective immune response against the *Bordetella* strain is raised against said antigens and,
- optionally an adjuvant of the TH1 immune response or an adjuvant of the TH2 immune response or a combination of both.

In another particular embodiment of the invention a combination immunogenic composition as herein defined further comprises antigens as active ingredients for the elicitation of an immune protection against determined pathogens which are **(vi)** at least one antigen of *Clostridium tetani* consisting of the Tetanus toxoid, **(vii)** an antigen of *Corynebacterium* complex consisting of the diphtheria toxoid, and optionally **(viii)** further antigens of different pathogen(s).

Additional antigens may further be included in the immunogenic composition to especially increase the valence of the immune response and accordingly a combination immunogenic composition may further comprise antigens as active ingredients for the elicitation of an immune protection against determined pathogens which are selected in the group of Hepatitis B surface antigen (HBs), inactivated poliovirus (IPV) of one or several virus strains, *Haemophilus influenza* type b polysaccharide.

Combination antigens originating from different pathogens are conventionally included in vaccine compositions administered to human hosts and should benefit from the particular properties of the CyaA-derived polypeptide as described herein to trigger the response toward a TH1 and/or TH17-oriented response even in the presence of the TH2 conventional adjuvants.

In a particular embodiment the immunogenic composition the antigens of *Bordetella sp.* are selected in the group of *Pertussis* toxoid (PT), Filamentous Haemagglutinin (FHA), Pertactin (PRN) and Fimbriae (Fim) are independently of each other from *Bordetella pertussis, Bordetella parapertussis, Bordetella bronchiseptica* or *Bordetella hinzii,* and are preferably from B. *pertussis,* and preferably are all from the same *Bordetella* strain.

A particular combination immunogenic composition according to the invention is such that one dose of 0.5ml of the immunogenic composition contains:
- *Bordetella* toxin in detoxified form (in particular either genetically or chemically detoxified), in particular *Pertussis* toxoid: 1 to 50 micrograms;
- Filamentous Haemagglutinin: 1 to 50 µg;
- Pertactin: 1 to 20µg;
- Optionally, Fimbriae: 2 to 25 µg;
- Tetanus toxoid: at least 0.25 and less than 25 Lf;
- Diphteria toxoid: at least 0.25 and less than 50Lf;
   and optionally,
- Hepatitis B surface antigen: 5µg to 25 µg
- inactivated poliovirus: from 5 to 45 D-antigen unit, in particular from 8 to 40 D-antigen unit per strain;
- *Haemophilus influenza* type b polysaccharide: 1 to 20 µg

Advantageously, a particular combination composition is such that one dose of 0.5ml of the immunogenic composition comprises:
- *Bordetella* toxin as a detoxified form, in particular *Bordetella* toxoid in particular *Pertussis* toxoid: 8µg;
- Filamentous Haemagglutinin: 8µg;
- Pertactin: 2.5µg;
- Tetanus toxoid: 5Lf;
- Diphteria toxoid : 2.5Lf.

In a specific embodiment the immunogenic composition, in particular the combination immunogenic composition, is further characterized in that one dose of 0.5ml of the immunogenic composition comprises 2.5 to 600 µg of the CyaA-derived polypeptide, in particular 2.5 to 500µg, 2.5 to 400µg, 2.5 to 300µg, 2.5 to 200µg or 2.5 to 100µg and preferably comprises 25µg of the CyaA-derived polypeptide of *Bordetella pertussis.*

Having observed the immunomodifier function of the CyaA-derived polypeptide in accordance with the invention, the quantitative composition of the immunogenic composition may be adapted in particular for use of the composition such as when using it as a further dose in a multiple-dose setting or as a booster composition for a prime/boost vaccination schedule.

The presence of the CyaA-derived polypeptide in the immunogenic composition function has been shown both to elicit antibodies against said CyaA-derived polypeptide and to trigger orientation of the immune response toward TH1/TH17 response, in particular encompassing production of IL-17 when the composition is an acellular immunogenic composition consisting of active ingredients which comprise antigens of *Bordetella sp.* This should be in favor of the protection against whooping cough but also against various conditions causally related to the infection of the host by a *Bordetella sp*., in particular by *Bordetella pertussis,* such as protection against the transmission of the bacteria by the host to another individual or protection against the colonization of the airways of the infected host by the bacteria.

The immunogenic composition according to the invention may comprise adjuvant of several categories, including adjuvants of the TH2 response. Such adjuvants may be selected in the following groups:
- An adjuvant of the TH2 response which is an aluminum salt, in particular an aluminum hydroxide, aluminum hydroxyphosphate sulfate or aluminum phosphate,
- Adjuvant of TH1 response selected in the group of MPL-containing adjuvant, in particular AS15, AS01B, AS01D, or AS01E (provided by GSK); CpG-containing adjuvant, in particular AS15, QS21-containing adjuvant (provided by GSK), in particular AS01B, D, and E or AS15, immune stimulating complexes (ISCOMs), IC31-containing adjuvant, chitosan-containing adjuvant, liposomal formulation-based adjuvant, in particular AS01B D, E or AS15 or ISCOMs, lipid-based emulsions such as MF59,
- An adjuvant inducing a TH1 response which is or contains a ligand of a toll-like receptor selected among:
   - a ligand of toll-like receptor 4 (TLR-4), in particular monophosphoryl lipid A (MPL) or Glucopyranosyl Lipid A (GLA) or,
   - a ligand of toll-like receptor 9 (TLR-9), in particular a synthetic oligodeoxynucleotides (ODNs) containing unmethylated CpG motifs and more particularly HBsAg-1018 or,
   - a ligand of toll-like receptor 3 (TLR-3), in particular a synthetic analog of double-stranded RNA (dsRNA) and more particularly a composition containing Poly(I:C) such as polyinosinic-polycytidylic acid plus poly-L-lysine double-stranded RNA in the presence of carboxymethylcellulose (Poly (ICLC).or,
   - a ligand of toll-like receptor 2 (TLR-2), in particular synthetic lipopeptides or a recombinant lipoprotein and,
   - a ligand of toll-like receptor 5 (TLR-5), in particular a recombinant bacterial flagellin.

In an aspect of the immunogenic composition of the invention, the CyaA-derived polypeptide of *Bordetella* consists of:
- a segment or a fragment of *Bordetella pertussis* CyaA protein as set forth in SEQ ID No. 2, the sequence of said segment or fragment beginning with the first residue of SEQ ID No.2 and ending with a residue located from position 183 to position 227 of SEQ ID No.2 or a polypeptide variant obtained from *Bordetella parapertussis, Bordetella bronchiseptica* or *Bordetella hinzii* and having at least 95% identity in amino acid residues with the segment or fragment consisting of residues 1 to 183 or 1 to 227 in SEQ ID No.2, fused to
- a segment or a fragment of *Bordetella* pertussis CyaA protein as set forth in SEQ ID No. 2, the sequence of said fragment beginning with a residue located from position 321 to position 387 of SEQ ID No.2 and ending with the last residue of SEQ ID No.2 or a polypeptide variant obtained from *Bordetella parapertussis, Bordetella bronchiseptica* or *Bordetella hinzii,* and having at least 95% identity in amino acid residues with the segment or fragment consisting of residues 321 to final residue or 387 to final residue in SEQ ID No.2.

The invention also concerns a medicinal composition for administration to a human host which comprises an immunogenic composition according to any one of the embodiments disclosed herein and which is provided as an administration form selected among a powder, a powder and solution for reconstitution, a liquid, in particular a suspension or a solution, a lyophilized component and a combination of such administration forms.

In a particular embodiment, one dose of the medicinal composition for administration to a human host contains 0.25 ml to 1 ml of liquid, in particular of reconstituted product.

Various administration routes are available for administration to the host, including injection, preferably intramuscular injection.

The invention also concerns a method for preparing an immunogenic composition (also designated vaccine for the purpose of this disclosure) comprising as active ingredients for protection against determined pathogens or condition causally related to such pathogens, antigens of said pathogens and furthermore a polypeptide derived from the adenylate cyclase protein (CyaA-derived polypeptide) of a *Bordetella* strain as defined herein, wherein the method comprises the step(s) of:
a) providing antigens selected in the group of **(i)** at least one of the antigens from *Bordetella* selected in the group of *Pertussis* toxin in detoxified form (either genetically or chemically) (PT), Filamentous Haemagglutinin (FHA), Pertactin (PRN) and Fimbriae (Fim) and preferably the first three antigens or all of these antigens, **(ii)** at least one antigen of *Clostridium tetani* consisting of the Tetanus toxin in detoxified form (either genetically or chemically), **(iii)** an antigen of *Corynebacterium* bacteria complex consisting of the diphtheria toxin in detoxified form (either genetically or chemically), and **(iv)** the CyaA-derived polypeptide as defined in any one of the embodiments disclosed herein and optionally **(v)** further antigens of different pathogen(s) as active ingredients for the elicitation of an immune response against said determined pathogens formulated as one or several component(s) and,
b) admixing said component(s) with one or more compounds that enhance the immune response when the vaccine is administered to a host such as adjuvant(s) of the TH2- oriented immune response, adjuvant of the TH1-oriented immune response, or a combination of such adjuvants,
wherein steps a) and b) are optionally carried out as a single step.

According to an embodiment of the invention, an immunogenic composition as described in the present application is for use in active immunization of a human host, in particular for use for the protection of a human host, against diphtheria, tetanus and against a condition causally related to *pertussis* infection, and optionally against hepatitis B, poliomyelitis and/or disease caused by *Haemophilus influenza* type b.

When the immunogenic composition is an acellular immunogenic composition containing antigens of the *Bordetella sp*., it is for use in active immunization of a human host, in particular for use for the protection of a human host, against a condition causally related to *pertussis* infection. As mentioned above the protection achieved may be against whooping cough but also against various conditions causally related to the infection of the host by a *Bordetella sp*., in particular by *Bordetella pertussis,* such as protection against the persistence of the bacteria, against the transmission of the bacteria by the host to another individual or protection against the colonization of the airways of the infected host by the bacteria.

The invention also relates to a method of administration of the immunogenic composition to a human host, for active immunization for protection against the pathogens providing the antigens of said composition or for protection against a condition causally related to the infection by such pathogen(s).

Due to its properties resulting from the association of various antigens including the CyaA-derived polypeptide, the immunogenic composition may be used in various administration schemes corresponding to various requirements of protection of the host which are presently not fulfilled by the available products.

In a particular embodiment, the immunogenic composition or the medicinal composition disclosed herein may be for use as a dose for administration after a first dose of a vaccine composition, in particular after a first dose of an immunogenic composition a first dose which does not comprise the CyaA-derived polypeptide described herein. Doses administered after the first one are either priming doses of a multiple-dose setting wherein said further priming doses are usually administered a few weeks or less than 6 months after the first or after the immediately preceding dose. Doses administered after the first one may alternatively or also be boosting dose(s) i.e., doses usually administered more than 6 months or up to several years after the first dose or the last dose of the multiple priming doses.

In a particular embodiment, the first dose which is different from the dose comprising the immunogenic composition of the invention is different in its antigenic contents and especially does not contain a non-toxic CyaA derived polypeptide and in particular does not contain a CyaA-derived polypeptide as described herein. Such different first dose may also be different in antigen concentration, in particular it may comprise higher concentrations of the antigens than the immunogenic composition of the invention.

In particular, the immunogenic composition or the medicinal composition disclosed herein may be for use as a dose for administration after a first dose, in particular (i) as a second or as a further dose of a multiple-dose setting or (ii) as a booster dose in individuals who previously received a first vaccination dose or primary vaccination with a monovalent or a combination vaccine against at least one of the diseases selected in the group of tetanus, diphtheria and a condition causally related to infection by a *Bordetella* strain, in particular *Bordetella pertussis,* and optionally hepatitis B, poliomyelitis and disease caused by *Haemophilus influenza* type b.

In a particular embodiment, a booster dose is different in antigenic composition and optionally in antigen concentration from the first or from the previously administered dose(s), in particular because said first or previous dose(s) is(are) devoid of non-toxic CyaA-derived polypeptide as described herein.

In a particular embodiment, a booster dose may be indicated for use as a booster dose in a prime/boost vaccination schedule wherein the host is a child above 4 years of age, an adolescent over 11 years of age or an adult, in particular an elderly person, a pregnant woman or a host who is a relative close to a pregnant woman (such as recommended in the "cocooning strategy" defined by the WHO), said host having previously received primary vaccination with a different acellular vaccine against a condition causally related to infection by *Bordetella* strain, in particular *Bordetella pertussis* either administered as a monovalent *pertussis* vaccine or a combination vaccine against whopping cough.

In another particular embodiment, the host is a child at birth or later, in particular until 18 months of age.

### Detailed description of the invention

The expression ***"protection"*** applied to the immunogenic composition (or to its active ingredients eliciting an immune response) relates to and defines the ability of said immunogenic composition to elicit an immune response in a host, in particular a human host, to whom said composition has been administered for active immunization, wherein such immune response encompasses (i) a specific response to all or part of the antigens of the composition, (ii) comprises the expression of cytokines of the immune system and antibodies, in particular neutralising antibodies. As a consequence an immune response is effective against infection by the pathogen(s) providing the antigens of the composition or against a condition causally related to this pathogen. In a particular embodiment of the invention, the protective response is a preventive response such as one enabled by prophylactic vaccines. In another embodiment of the invention, the protection if therapeutic, i.e., it alleviates or prevents the pathological outcomes of the infection. Accordingly, the elicited immune response prevents the onset or the development of the disease or condition caused by the pathogen(s) against which the active immunization is sought, in particular prevents invasive stage of the pathogen or prevents its transmission to other hosts or prevents at least clinical symptoms of the disease from developing. Advantageously, the protection may also be prophylactic vaccination of the host.

The indication of the immunogenic composition to provide protection encompasses an indication to provide protection against a condition causally related to infection of a human host by a *Bordetella* strain, in particular *B*. *pertussis* or against other strains of Bordetella such as *Bordetella parapertussis, Bordetella bronchiseptica* or *Bordetella hinzii.* The ability to elicit protection, in particular preventive protection, is based on the demonstration of immunogenicity of the combination of the antigens originating from *Bordetella* contained in the composition and on the comparison of the response elicited using said antigens with that raised with conventional acellular vaccines (designated aP) against whooping cough or with whole cell vaccines (designated WCV) against whooping cough. In particular, the immune response triggered by the immune composition against *Bordetella,* in particular against *B. pertussis* encompasses a TH1- and/or TH17- oriented immune response favourable to long duration protection of the individual.

A particular aspect of the protection combination is that, in addition to protection against the whooping cough, the protection triggered by the antigens of *Bordetella* may extend to prevention of the invasive infection, prevention of the transmission or colonization of the airways of the host by the bacteria. The capability of the immunogenic composition to trigger a response which is TH1- and/or TH2-oriented may be a factor of the ability of the composition to extend the immune response beyond prevention of whooping cough as emphasized above.

Particular embodiments of the CyaA-derived polypeptide used in order to carry out the invention are described hereafter with respect to the structure of the polypeptide.

As mentioned above, the CyaA-derived polypeptide may be defined with respect to the sequence of SEQ ID NO: 2 which represents the amino acid sequence of the wild type CyaA protein of *Bordetella pertussis.* In addition, a polynucleotide encoding the CyaA as set forth in SEQ ID NO: 2, is as set forth in SEQ ID NO:1 and may be used in order to define fragments that express the CyaA-derived polypeptide used in the immunogenic composition of the invention. Another particular embodiment of a polynucleotide, encoding the CyaA as set forth in SEQ ID NO: 2, is a modified version of sequence SEQ ID NO:1, such as a sequence obtained by silent nucleotide mutations, *i.e.,* by modifications which do not result in a change to the amino acid of SEQ ID NO :2. A particular modified version of SEQ ID NO:1 is a sequence, optimized for in expression *E. coli,* as set forth in SEQ ID NO:24.

The resulting CyaA-derived polypeptide of the invention which may be obtained from a polynucleotide disclosed herein comprises or consists of two segments of the native CyaA, and in a particular embodiment may accordingly be the expression of two fragments, fused together or recombined, originating from the same *Bordetella* CyaA protein, preferably from *Bordetella pertussis.* By "*fragment*", it is meant a stretch or a concatenation of consecutive amino acid residues found in the sequence of the wild type *Bordetella* CyaA protein. The expression in a production cell of the fused fragments originating from native CyaA is obtained using a polynucleotide encoding their sequence in association with a polynucleotide comprising the coding sequence of the *cyaC* gene of *Bordetella* to enable post translational modification of the expressed amino acid sequence.

Accordingly, a "*CyaA-derived polypeptide*" encompasses the expressed amino acid sequence of the fused segments derived from CyaA and also such product which may have undergone post translational modifications. The expression also relates to variants of the CyaA-derived polypeptide of *B.pertussis*, in particular variants of the CyaA-derived polypeptide obtained from the amino acid sequence of SEQ ID No.2. Thus, advantageously, said "*CyaA-derived polypeptide*" used to carry out the invention is modified by post-translational acylation of at least one of its residues, in particular at least one of the two, preferably the two, lysine residues corresponding to the residues located in positions 860 and 983 of the full length sequence of B. *pertussis*, *B. hinzii* or *B parapertussis* CyaA or corresponding to the residues located in positions 859 and 982 of the full length sequence of B. *bronchiseptica* CyaA. By "*acylation*", it is meant herein palmitoylation, *i.e.,* addition of palmitate and/or palmitoleate group(s) on residue(s) of the CyaA-derived polypeptide used for the invention. Thus, said "*CyaA-derived polypeptide*" bears a palmitoyl group on some of these residues, preferably on one of the two, or the two, lysine residues corresponding to the residues 860 and 983 of the full length sequence of B. *pertussis, B. hinzii* or *B parapertussis* CyaA or corresponding to the residues located in positions 859 and 982 of the full length sequence of *B*. *bronchiseptica* CyaA. By "*corresponding to*", it is meant that the residue(s) which is (are) post-translationally modified in the CyaA-derived polypeptide of the invention is (are) the one(s) the position of which matches the lysines 860 and 983 in the sequence of CyaA of B. *pertussis, B. hinzii* or *B parapertussis* CyaA (SEQ ID NO:2, 4 and 6 respectively) or the lysines 859 and 982 in the sequence of *B*. *bronchiseptica* CyaA (SEQ ID NO:8). The identification of these lysine residues in the proteins of the invention can be carried out by the person skilled in the art, by aligning and comparing the sequence of the proteins of the invention with the sequence as defined in SEQ ID NO:2, 4, 6 or 8.

The process of palmitoylation is well known from the person skilled in the art and is mediated by the *cyaC* gene of a *Bordetella* species, preferably of the *Bordetella pertussis* CyaC coding sequence, the natural sequence of which is set forth in SEQ ID NO:21. A version of the CyaC coding sequence, optimized for production in *E. coli,* is set forth in SEQ ID NO:22. This (these) post translational modification(s) may be obtained by co-expression of the polynucleotide encoding CyaA protein, the polynucleotide encoding the CyaA-derived polypeptide of the invention and of the *cyaC* gene.

According to the definition provided above of the CyaA-derived polypeptide, the first segment or fragment (located in the N-terminal portion of the CyaA-derived polypeptide) begins with the first residue of SEQ ID NO:2 and ends with a residue located from position 183 to position 227 of SEQ ID NO:2.

This first segment or fragment has a size ranging from 183 to 227 residues, *i.e.,* is at least 183 residues in length and is at most 227 residues in length. In a particular embodiment, this segment or fragment is at least 183, at least 190, at least 200, at least 210 or at least 220 residues in length. In a particular embodiment, the size of this first segment or fragment is 183 residues or is 227 residues.

Thus, this segment or fragment begins with the first residue of SEQ ID NO:2 and ends with a residue selected from the group consisting of residues 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226 and 227 of SEQ ID NO: 2.

In other words, this first segment or fragment comprises or consists of a sequence which is selected from the group consisting of residues 1-183, 1-184, 1-185, 1-186, 1-187, 1-188, 1-189, 1-190, 1-191, 1-192, 1-193, 1-194, 1-195, 1-196, 1-197, 1-198, 1-199, 1-200, 1-201, 1-202, 1-203, 1-204, 1-205, 1-206, 1-207, 1-208, 1-209, 1-210, 1-211, 1-212, 1-213, 1- 214, 1-215, 1-216, 1-217, 1-218, 1-219, 1-220, 1-221, 1- 222, 1-223, 1-224, 1-225, 1-226 and 1-227 of SEQ ID NO: 2.

In a particular embodiment, this first segment or fragment comprises or consists of residues 1 to 227 of SEQ ID NO:2 or of residues 1 to 183 of SEQ ID NO:2.

In a particular embodiment, the polynucleotide encoding said first segment or fragment begins with the first nucleotide of SEQ ID NO:1 or of SEQ ID NO:24 and ends with a nucleotide located from position 549 to position 681 of SEQ ID NO:1 or of SEQ ID NO:24, provided that the length of said polynucleotide segment or fragment is a multiple of 3. Thus, the polynucleotide encoding this segment or fragment comprises or consists of a sequence which is selected from the group consisting of residues 1-549, 1-552, 1-555, 1-558, 1-561, 1-564, 1-567, 1-570, 1-573, 1-576, 1-579, 1-582, 1-585, 1-588, 1-591, 1-594, 1-597, 1-600, 1-603, 1-606, 1-609, 1-612, 1-615, 1-618, 1-621, 1-624, 1-627, 1-630, 1-633, 1-636, 1-639, 1- 642, 1-645, 1-648, 1-651, 1-654, 1-657, 1-660, 1-663, 1- 666, 1-669, 1-672, 1-675, 1-678 and 1-681 of SEQ ID NO: 1 or of SEQ ID NO:24.

The second segment or fragment (the C-terminal portion of the CyaA-derived polypeptide) begins with a residue located from position 321 to position 387 of SEQ ID NO:2 and ends with the last residue of SEQ ID NO:2.

This second segment or fragment has a size ranging from 1320 to 1386 residues, *i.e.,* is at least 1320 residues and is at most 1386 residues in length. In a particular embodiment, this segment or fragment is at least 1320, at least 1330, at least 1340, at least 1350, at least 1360, at least 1370 or at least 1380. In a particular embodiment, the size of this second segment or fragment is 1320 residues or is 1386 residues.

Thus, this second segment or fragment begins with a residue selected from the group consisting of residues 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386 and 387 of SEQ ID NO:2 and ends with the last residue (*i.e.,* residue 1706) of SEQ ID NO:2.

In other words, this second segment or fragment comprises or consists of a sequence which is selected from the group consisting of residues 321-1706, 322-1706, 323-1706, 324-1706, 325-1706, 326-1706, 327-1706, 328-1706, 329-1706, 330-1706, 331-1706, 332-1706, 333-1706, 334-1706, 335-1706, 336-1706, 337-1706, 338-1706, 339-1706, 340-1706, 341-1706, 342-1706, 343-1706, 344-1706, 345-1706, 346-1706, 347-1706, 348-1706, 349-1706, 350-1706, 351-1706, 352-1706, 353-1706, 354-1706, 355-1706, 356-1706, 357-1706, 358-1706, 359-1706, 360-1706, 361-1706, 362-1706, 363-1706, 364-1706, 365-1706, 366-1706, 367-1706, 368-1706, 369-1706, 370-1706, 371-1706, 372-1706, 373-1706, 374-1706, 375-1706, 376-1706, 377-1706, 378-1706, 379-1706, 380-1706, 381-1706, 382-1706, 383-1706, 384-1706, 385-1706, 386-1706 and 387-1706 of SEQ ID NO:2

In a particular embodiment, this second segment or fragment comprises or consists of residues 321-1706 of SEQ ID NO:2 or of residues 387-1076 of SEQ ID NO:2.

In a particular embodiment, the polynucleotide encoding said second segment or fragment begins with a nucleotide located from position 961 to position 1159 of SEQ ID NO:1 or of SEQ ID NO:24 and ends with the last nucleotide (*i.e.,* nucleotide 5118) of SEQ ID NO:1 or of SEQ ID NO:24, provided that the length of said nucleotide segment or fragment is a multiple of 3. Thus, the polynucleotide encoding this second segment or fragment comprises or consist of a sequence which is selected from the group consisting of residues 961-5118, 964-5118, 967-5118, 970-5118, 973-5118, 976-5118, 979-5118, 982-5118, 985-5118, 988-5118, 991-5118, 994-5118, 997-5118, 1000-5118, 1003-5118, 1006-5118, 1009-5118, 1012-5118, 1015-5118, 1018-5118, 1021-5118, 1024-5118, 1027-5118, 1030-5118, 1033-5118, 1036-5118, 1039-5118, 1042-5118, 1045-5118, 1048-5118, 1051-5118, 1054-5118, 1057-5118, 1060-5118, 1063-5118, 1066-5118, 1069-5118, 1072-5118, 1075-5118, 1078-5118, 1081-5118, 1084-5118, 1087-5118, 1090-5118, 1093-5118, 1096-5118, 1099-5118, 1102-5118, 1105-5118, 1108-5118, 1111-5118, 1114-5118, 1117-5118, 1120-5118, 1123-5118, 1126-5118, 1129-5118, 1132-5118, 1135-5118, 1138-5118, 1141-5118, 1144-5118, 1147-5118, 1150-5118, 1153-5118, 1156-5118 and 1159-5118 of SEQ ID NO:1 or of SEQ ID NO:69.

In a particular embodiment, the CyaA-derived polypeptide comprises or consists of a polypeptide of the sequence as set forth in SEQ ID NO:10; SEQ ID NO:10 consisting of a segment or fragment consisting of residues 1 to 227 of SEQ ID NO:2 fused to a segment or fragment consisting of residues 321 to 1706 of SEQ ID NO:2.

In another particular embodiment, the CyaA-derived polypeptide comprises or consists of a polypeptide of the sequence as set forth in SEQ ID NO:12; SEQ ID NO:12 consisting of a segment or fragment consisting of residues 1 to 183 of SEQ ID NO:2 fused to a segment or fragment consisting of residues 387 to 1706 of SEQ ID NO:2.

Other particular embodiments are also disclosed:
- the CyaA-derived polypeptide comprises or consists of a polypeptide of the sequence as set forth in SEQ ID NO:19, *i.e.,* a sequence consists of a segment or fragment consisting of residues 1 to 227 of SEQ ID NO:2 fused to a segment or fragment consisting of residues 387 to 1706 of SEQ ID NO:2, and
- the CyaA-derived polypeptide comprises or consists of a polypeptide of the sequence as set forth in SEQ ID NO:20, *i.e.,* a sequence consisting of a segment or fragment consisting of residues 1 to 183 of SEQ ID NO:2 fused to a segment or fragment consisting of residues 321 to 1706 of SEQ ID NO:2.

The expression *"fused to"* when reference is made to a protein or a polypeptide means that the peptide segments or fragments (e.g., several CyaA segments or fragments) are covalently linked together by a peptide bond. The order of these different peptide parts is described herein as from N-terminal to C-terminal, i.e., the last C-terminal residue of a first part is linked to the first N-residue of the other part by a peptide bond. The expression "*fused to"* when reference is made to a polynucleotide, means that two or more polynucleotide parts (e.g., several nucleotide CyaA segments or fragments) are covalently linked together by a phosphodiester bond. The order of these different nucleotide parts is described herein as from 5' to 3', i.e., the last 3' nucleotide of a first part is linked to the first 5' nucleotide of the other part by a phosphodiester bond. The polynucleotide consisting of the fusion of nucleotide sequences is in particular obtained as a recombinant polynucleotide, including by deletion of nucleotides in the native coding sequence of cyaA which are between the retained segments. The CyaA-derived polypeptide may be obtained as a result of expression in cells such as bacteria, in particular *E.coli* and the polynucleotide expressing the polypeptide may be obtained by genetic recombination or by synthesis of the sequence using the information of the polynucleotide encoding the CyaA-derived polypeptide.

The invention also concerns a polynucleotide encoding a variant CyaA-derived polypeptide, wherein said first segment or fragment is a polypeptide with at least 95% identity with a determined segment or fragment of the *Bordetella pertussis* CyaA protein as set forth in SEQ ID NO: 2, the sequence of said segment or fragment beginning with the first residue of SEQ ID NO:2 and ending with a residue located from position 183 to position 227 of SEQ ID NO:2, and/or wherein said second segment or fragment is a variant with at least 95% identity with a segment or fragment beginning with a residue located from position 321 to position 387 of SEQ ID NO:2 and ending with the last residue of SEQ ID NO:2.

By "a *variant with at least 95% identity*" when reference is made to a protein or a polypeptide, it is meant a protein sequence whose amino acid identity is at least 95%, at least 96%, at least 97%, at least 98% or at least 99% with the polypeptide from which it varies. The percentage of identity is calculated, comparing the full-length sequence of both said variant and said polypeptide of reference, in particular over the shorter of the two sequences. Thus, a variant has 95% of identity with a polypeptide, when 5% of its residues differ from the residues of this polypeptide, by one or more addition(s) and/or one or more deletion(s) and/or one or more substitution(s). In a particular embodiment, said variant differs from said polypeptide only by substitutions, preferably conservative substitutions and accordingly it keeps the same length as the sequence from which it varies. In another embodiment, said variant differs from said polypeptide by at least 1 single amino-acid deletion, preferably by 1, 2, 3, 4 or 5 single amino-acid deletion(s), and by substitutions, preferably conservative substitutions.

The invention also relates to polynucleotide variants having an identity of at least 75% with the polynucleotides encoding portions (or fragments) of SEQ ID NO:1. In a particular embodiment, the polynucleotide encoding said first fragment has an identity of 75% with a polynucleotide beginning with the first nucleotide of SEQ ID NO:1 and ends with a nucleotide located from position 549 to position 681 of SEQ ID NO:1 provided that the length of said nucleotide fragment is a multiple of 3. In another embodiment, independently or in combination with the above statement, the polynucleotide encoding said second fragment has an identity of 75% with a polynucleotide beginning with a nucleotide located from position 961 to position 1159 of SEQ ID NO:1 and ends with the last nucleotide (*i.e.,* nucleotide 5118) of SEQ ID NO:1, provided that the length of said nucleotide fragment is a multiple of 3. In a particular embodiment, the polynucleotides encoding said first and second segments or fragments originate from a polynucleotide, the full-length sequence of which has at least 75% identity with SEQ ID NO:1. An example of such variant is SEQ ID NO:24. In a particular embodiment, the polynucleotide variant results from degeneracy of the genetic code applied to the polynucleotide obtained from SEQ ID NO:1 as disclosed above or to the polynucleotide of SEQ ID NO:24. In a particular embodiment, the polynucleotide variant thus obtained has a degenerated base at the wobble position.

By "*a variant with at least 75% identity*" when reference is made to a polynucleotide, it is meant a nucleotide sequence whose nucleotide identity is at least 75%, at least 79%, at least 80%, at least 85%, at least 90%, at least 95%, at least 96%, at least 97%, at least 98% or at least 99% with the polynucleotide from which it varies. The percentage of identity is calculated, comparing the full-length sequence of both said variant and the polynucleotide from which it varies, in particular over the shorter of the two sequences. Thus, a variant has 75% of identity with a polynucleotide, when 25% of its nucleotides differ from the nucleotides of said polynucleotide, by one or more nucleotide addition(s) and/or one or more nucleotide deletion(s) and/or one or more nucleotide substitution(s). In a particular embodiment, said variant differs only by nucleotide substitutions, and accordingly it keeps the same length as the sequence from which it varies. In a particular embodiment, said variant differs only by nucleotide silent mutations, and accordingly it keeps encoding the same protein as the one encoded by the sequence from which it varies. In a particular embodiment, said variant differs only by nucleotide substitutions, a part of them being silent mutations, such that the sequence of the protein encoded by said polynucleotide variant has at least 95% of identity with a protein or polypeptide of the invention, or has 100% identity.

Nucleotide and protein identity percentages as indicated herein may be calculated by well-known programs based on the Needleman and Wunsch algorithm, such as MeAlign [18].

In a particular embodiment, the variant CyaA-derived polypeptide suitable for use in the invention keeps its capacity to bind to target cells and/or to translocate its adenylate cyclase (AC) domain into the cytosol of the target cells. In a particular embodiment, target cells are CD11b-expressing cells, *i.e.,* cells that express the CD11b/CD18 receptor on their surface (CD11b⁺). In particular, these cells are granulocytes/neutrophils, macrophages, NK cells, subsets of T CD8⁺, subsets of B cells, dendritic cells such as Langerhans cells, or myeloid dendritic cells.

The capacity of the variants of the invention to bind to target cells can be assayed especially according to the methods disclosed in EP03291486 or in WO02/22169 application. Furthermore, the capacity of the variant to translocate its N-terminal domain into the cytosol of target cells can be assayed by applying the method described in WO02/22169 application, or the method detailed in example A with the p105 peptide.

Preferred variants of CyaA-derived polypeptide obtained from the protein of SEQ ID NO.2 which is the full-length wild type sequence of the *Bordetella pertussis* CyaA protein are obtained from CyaA proteins of other *Bordetella sp.* provided herein by reference to their amino acid sequence as set forth in SEQ ID NO: 4 (CyaA protein of *Bordetella hinzii*), SEQ ID NO: 6 (CyaA protein of *Bordetella parapertussis*) and SEQ ID NO: 8 (CyaA protein of *Bordetella bronchiseptica*). The nucleotide sequence, encoding SEQ ID NOs: 4, 6 and 8, is as set forth in SEQ ID NOs:3, 5 and 7 respectively or is a variant of SEQ ID NOs:3, 5 and 7 by silent mutations. Accordingly, CyaA-derived polypeptides to carry out the present invention, which are variants of the polypeptide obtained by reference to the sequence of SEQ ID NO.2 are segments or fragments fused as disclosed herein which are obtained from the amino acid sequences of *Bordetella parapertussis, Bordetella bronchiseptica* or *Bordetella hinzii* and defined by alignment of these sequences with the segments defined in SEQ ID NO.2 for the purpose of the invention.

The CyaA-derived polypeptide defined in order to carry out the invention does not comprise or does not consist of SEQ ID NOs: 2, 4, 6 and 8. Moreover, a polynucleotide encoding a variant CyaA-derived polypeptide suitable to carry out the invention does not comprise or does not consist of SEQ ID NOs: 3, 5 or 7.

Particular CyaA-derived polypeptides which are suitable to carry out the invention and which result from the fusion of 2 segments or fragments of *Bordetella pertussis* CyaA according to the definition provided and their variants obtained from the fusion of the fragments equivalent to those of *B*. *pertussis* and taken from the amino acid sequence of CyaA of any of *Bordetella parapertussis, Bordetella bronchiseptica* or *Bordetella hinzii* are selected in the group of:
a polypeptide comprising or consisting of the sequence as set forth in SEQ ID NO:10 or a polypeptide variant obtained from *Bordetella parapertussis, Bordetella bronchiseptica* or *Bordetella hinzii* and characterized by an amino acid sequence matching the amino acid residue positions of SEQ ID No.10;
a polypeptide comprising or consisting of the sequence as set forth in SEQ ID NO:12 or a variant obtained from *Bordetella parapertussis, Bordetella bronchiseptica* or *Bordetella hinzii,* and characterized by an amino acid sequence matching the amino acid residue positions of SEQ ID No.12,
a polypeptide comprising or consisting of the sequence as set forth in SEQ ID NO:19 or a variant obtained from *Bordetella parapertussis, Bordetella bronchiseptica* or *Bordetella hinzii,* and characterized by an amino acid sequence matching the amino acid residue positions of SEQ ID No.19,
a polypeptide comprising or consisting of the sequence as set forth in SEQ ID NO:20 or a variant obtained from *Bordetella parapertussis, Bordetella bronchiseptica* or *Bordetella hinzii* and characterized by an amino acid sequence matching the amino acid residue positions of SEQ ID No.20.

### Polynucleotides and preparation of the CyaA-derived polypeptides

In a particular embodiment of the invention, a polynucleotide is used that encodes a variant CyaA-derived polypeptide, preferably as variant of a *B. pertussis* CyaA-derived polypeptide as defined herein, and such polynucleotide encodes a polypeptide comprising or consisting of:
(a) a segment or a fragment of the *Bordetella* CyaA protein as set forth in SEQ ID NO : 4, 6 or 8, the sequence of said segment of fragment beginning with the first residue of SEQ ID NO:4, 6 or 8 and ending with a residue located from position 183 to position 227 of SEQ ID NO:4, 6 or 8, fused to
(b) a segment or a fragment of the *Bordetella* CyaA protein as set forth respectively in SEQ ID NO : 4, 6 or 8, the sequence of said segment or fragment beginning with a residue located from position 321 to position 387 of SEQ ID NO: 4, 6 or 8 and ending with the last residue of SEQ ID NO: 4, 6 or 8.

The definitions given above for the particular CyaA-derived polypeptide comprising fragments of SEQ ID NO:2 apply identically to the variant CyaA-derived polypeptide comprising fragments of SEQ ID NO:4 and 6.

Regarding the variant CyaA-derived polypeptide comprising fragments of SEQ ID NO:8, all definitions apply identically, with the exception of the last residue of SEQ ID NO:8 is residue 1705 instead of residue 1706. Therefore, for the variant CyaA-derived polypeptide comprising segments or fragments of SEQ ID NO:8, all aspects referring to residue 1706 must be replaced by residue 1705. In particular, the second segment or fragment has a size ranging from 1319 to 1385 residues, and is preferably 1319 residues or 1385 residues in length. Regarding a polynucleotide encoding variant CyaA-derived polypeptide comprising segments or fragments of SEQ ID NO: 8, all definitions and embodiments referring to nucleotide 5118 must be replaced by nucleotide 5115.

Particular polynucleotides encoding variant CyaA-derived polypeptides suitable to carry out the invention are selected among the following polynucleotide consisting of:
1) a polynucleotide encoding the polypeptide as set forth in SEQ ID NO:13; SEQ ID NO:13 consists of a segment or a fragment consisting of residues 1 to 227 of SEQ ID NO:4 fused to a segment or a fragment consisting of residues 321 to 1706 of SEQ ID NO:4;
2) a polynucleotide encoding the polypeptide as set forth in SEQ ID NO:14; SEQ ID NO:14 consists of a segment or a fragment consisting of residues 1 to 183 of SEQ ID NO:4 fused to a segment or a fragment consisting of residues 387 to 1706 of SEQ ID NO:4;
3) a polynucleotide encoding the polypeptide as set forth in SEQ ID NO:15; SEQ ID NO:15 consists of a segment or a fragment consisting of residues 1 to 227 of SEQ ID NO:6 fused to a segment or a fragment consisting of residues 321 to 1706 of SEQ ID NO:6;
4) a polynucleotide encoding the polypeptide as set forth in SEQ ID NO:16; SEQ ID NO: 16 consists of a segment or a fragment consisting of residues 1 to 183 of SEQ ID NO:6 fused to a segment or a fragment consisting of residues 387 to 1706 of SEQ ID NO:6;
5) a polynucleotide encoding the polypeptide as set forth in SEQ ID NO:17; SEQ ID NO:17 consists of a segment or a fragment consisting of residues 1 to 227 of SEQ ID NO:8 fused to a segment or a fragment consisting of residues 321 to 1705 of SEQ ID NO:8; and
6) a polynucleotide encoding the polypeptide as set forth in SEQ ID NO:18; SEQ ID NO: 18 consists of a segment or a fragment consisting of residues 1 to 183 of SEQ ID NO:8 fused to a segment or a fragment consisting of residues 387 to 1705 of SEQ ID NO:8.

In a particular embodiment, the polynucleotide encoding the CyaA-derived polypeptide or the variant CyaA-derived polypeptide used in the invention may hence be defined as a deleted version of the full-length *Bordetella* CyaA coding nucleotide sequence, *i.e.,* a polynucleotide encoding a polypeptide consisting of a deletion fragment of SEQ ID NO :2, 4, 6 or 8 wherein the deletion encompasses a segment of the native sequence covering an amino acid segment the first amino acid residue of which is located from residue 184 to residue 228 of SEQ ID NO: 2, 4, 6 or 8 respectively, and the last amino acid residue of which is located from residue 320 to residue 386 of SEQ ID NO: 2, 4, 6 or 8 respectively. In a particular embodiment, said polynucleotide encodes a polypeptide consisting of a deleted version of SEQ ID NO :2, 4, 6 or 8, which is deleted for a polynucleotide encoding a polypeptide fragment whose first amino acid residue is selected from the group consisting of residues 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227 and 228 of SEQ ID NO: 2, 4, 6 or 8 respectively, and whose last amino acid residue is selected from the group consisting of residues 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385 or 386 of SEQ ID NO: 2, 4, 6 or 8 respectively.

A method to produce a polynucleotide encoding the CyaA-derived polypeptide in particular a variant CyaA-derived polypeptide as described herein is also part of the invention. This method comprises the steps of (a) deleting, from a polynucleotide encoding the *Bordetella* CyaA as set forth in SEQ ID NO : 2, 4, 6 or 8, a nucleotide fragment of consecutive nucleotide residues in said sequences, the first three nucleotides of which encode an amino acid residue located from residue 184 to residue 228 of SEQ ID NO : 2, 4, 6 or 8, and the last three nucleotides of which encode an amino acid residue located from residue 320 to residue 386 of SEQ ID NO : 2, 4, 6 or 8; and (b) recovering said polynucleotide.

Alternatively, the polynucleotide encoding the CyaA-derived polypeptide is chemically synthesized, using conventional methods, according to the sought CyaA-derived polypeptide sequence, and optionally taking into account the degeneracy of the genetic code and/or the optimization of expression.

The invention also uses the CyaA-derived polypeptides encoded by the polynucleotides thus defined. Particular CyaA-derived polypeptides consist of a sequence as set forth in SEQ ID NO: 10, 12, 13, 14, 15, 16, 17, 18, 19 and 20.

A polynucleotide encoding a CyaA-derived polypeptide, including a variant CyaA-derived polypeptide is used to produce in a cell, in particular in a bacterial cell, especially in *E.coli,* a polynucleotide encoding said CyaA-derived polypeptide, i.e., a B. pertussis CyaA-derived polypeptide or a variant CyaA-derived polypeptide thereof.

A method to produce a polynucleotide encoding a CyaA-derived polypeptide, comprises:
(a) deleting, from a polynucleotide encoding the *Bordetella* CyaA as set forth in SEQ ID NO : 2, 4, 6 or 8 or encoding a variant with at least 95% identity with SEQ ID NO: 2, a nucleotide segment or fragment, the first three nucleotides of which encode an amino acid residue located from residue 184 to residue 228 of SEQ ID NO : 2, 4, 6 or 8, whose the last 3 nucleotides of which encode an amino acid residue located from residue 320 to residue 386 of SEQ ID NO : 2, 4, 6 or 8; and
(b) recovering said polynucleotide encoding a CyaA-derived polypeptide.

A method to produce a CyaA-derived polypeptide comprises:
(a) deleting, from a polynucleotide encoding the *Bordetella* CyaA as set forth in SEQ ID NO : 2, 4, 6 or 8 or encoding a variant with at least 95% identity with SEQ ID NO : 2, a nucleotide fragment, the first three nucleotides of which encode an amino acid residue located from residue 184 to residue 228 of SEQ ID NO : 2, 4, 6 or 8, whose the last 3 nucleotides of which encode an amino acid residue located to from residue 320 to residue 386 of SEQ ID NO : 2, 4, 6 or 8;
(b) expressing, in a cell, especially in a bacterial cell and in particular in *E.coli,* the polynucleotide obtained in (a); and
(c) recovering said expressed CyaA-derived polypeptide.

The method to produce the CyaA-derived polypeptide may further comprise the step of combining in the polynucleotide construct thus obtained and a polynucleotide encoding the CyaC protein.

In a particular embodiment the CyaA-derived polypeptide is expressed in such cells using the plasmid pGTP-gtCyaAd93-CyaCopt which has the sequence of SEQ ID NO.25

Within the present invention, when reference is made to the "*first three nucleotides*" or the "*last three nucleotides*", it is understood that these three nucleotides refer to a codon which corresponds, according to the genetic code, to an amino acid residue identified by its position in SEQ ID NO: 2, 4, 6 or 8. Thus, the size of the polynucleotide nucleotide deletion is preferably a multiple of 3. Moreover, in addition to be a multiple of 3 in size, the polynucleotide nucleotide deletion is in frame, *i.e.,* the deletion removes the sought amino acid residues, without modifying the reading frame, nor modifying the residues surrounding (upstream and downstream) the deletion.

The preparation of the CyaA-derived polypeptide as an expression product of a polynucleotide encoding same is described in particular in WO 2014/016310 and may be achieved through the use of techniques well known to the person skilled in the art.

In a particular embodiment, the polynucleotide construct expressing the CyaA-derived polypeptide and CyaC protein comprises from 5' end to 3' end, the *cyaA* polynucleotide or gene, advantageously consisting of an optimized sequence for expression in a determined host or production cell, e.g. *E. Coli* and the *cyaC* polynucleotide or gene, advantageously consisting of an optimized sequence for expression in a determined host or production cell, e.g. *E. Coli.* This order of the insertion of the polynucleotides in the construct favours the expression of the CyaA-derived polypeptide and CyaC protein in respective quantities and conformation suitable for increasing efficiency of expression of post translational modified version of CyaA.

In a particular embodiment, except for the deletion of the segment or fragment [whose first amino acid residue is located from residue 184 to residue 228 of SEQ ID NO: 2, 4, 6 or 8 respectively, and whose last amino acid residue is located from residue 320 to residue 386 of SEQ ID NO: 2, 4, 6 or 8 respectively] carried out in the wild type *Bordetella* CyaA protein disclosed herein, the CyaA-derived polypeptide suitable to carry out the invention does not undergo any other variation (addition, deletion and/or substitution) as compared to SEQ ID NO:2, 4, 6 or 8.
As a consequence of its amino acid sequence, the CyaA derived protein is non-toxic, in particular non-cytotoxic, *i.e.,* its enzymatic activity adenylate cyclase has been inactivated as a result of the deletion of the segment or fragment whose first amino acid residue is located from residue 184 to residue 228 of SEQ ID NO: 2, 4, 6 or 8 respectively, and whose last amino acid residue is located from residue 320 to residue 386 of SEQ ID NO: 2, 4, 6 or 8 respectively (deletion of a segment or fragment is accordingly deletion of at least a fragment of 93 amino residues (designated "d93")). Therefore, in a particular embodiment, no insertion, deletion or substitution has been carried out. In particular, when the residues 188 and 189 of CyaA are still present in the proteins of the invention, no dipeptide (such as the dipeptide LQ or GS) is inserted between the residues 188 and 189.

Additional features and properties of the embodiments of the invention will be apparent in the Examples and Figures which follow.

### Legend of the figures

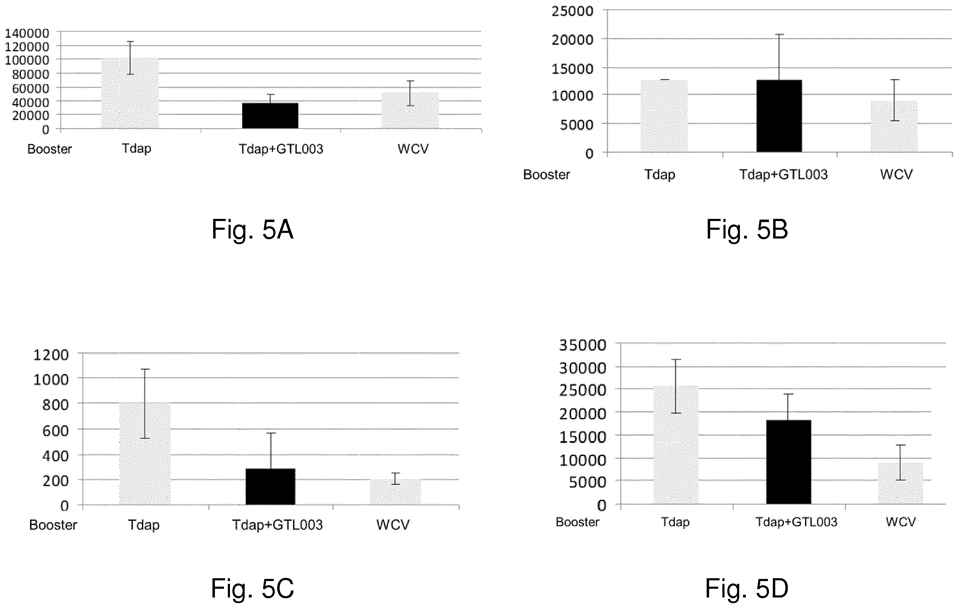

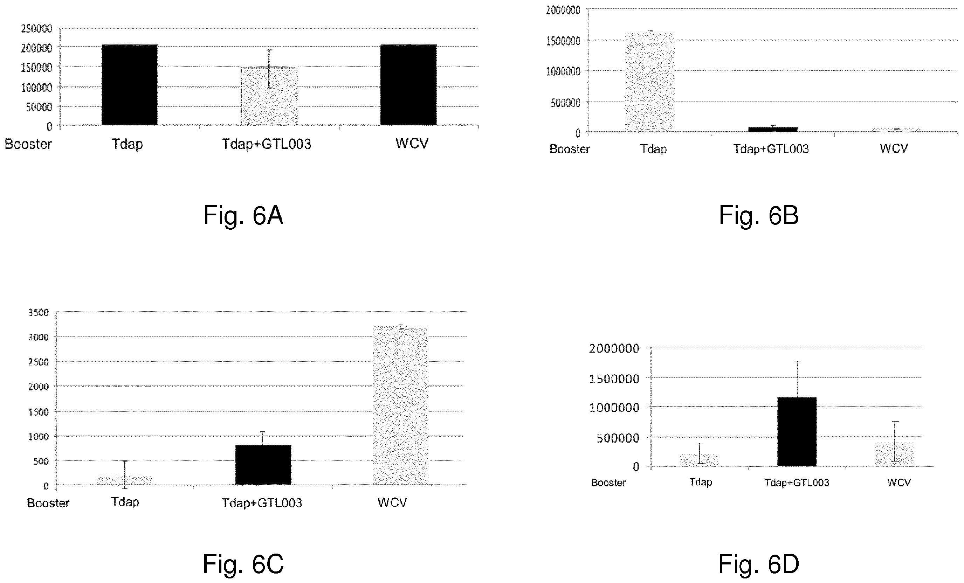

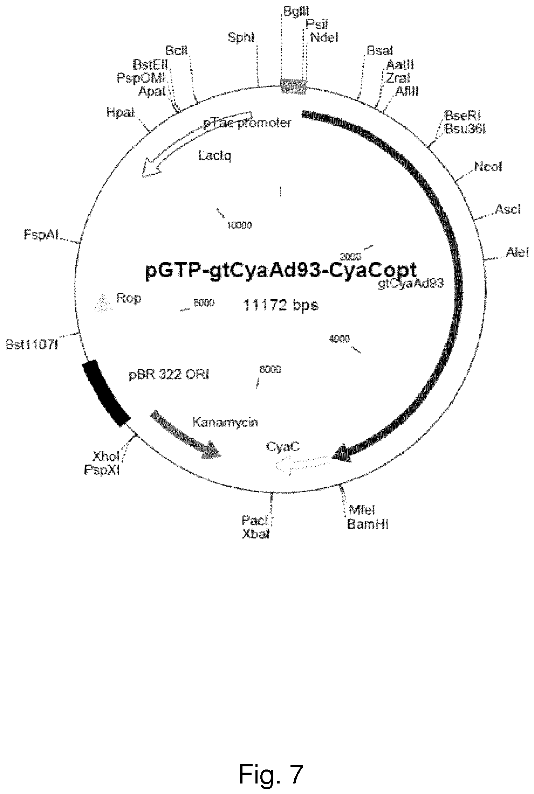

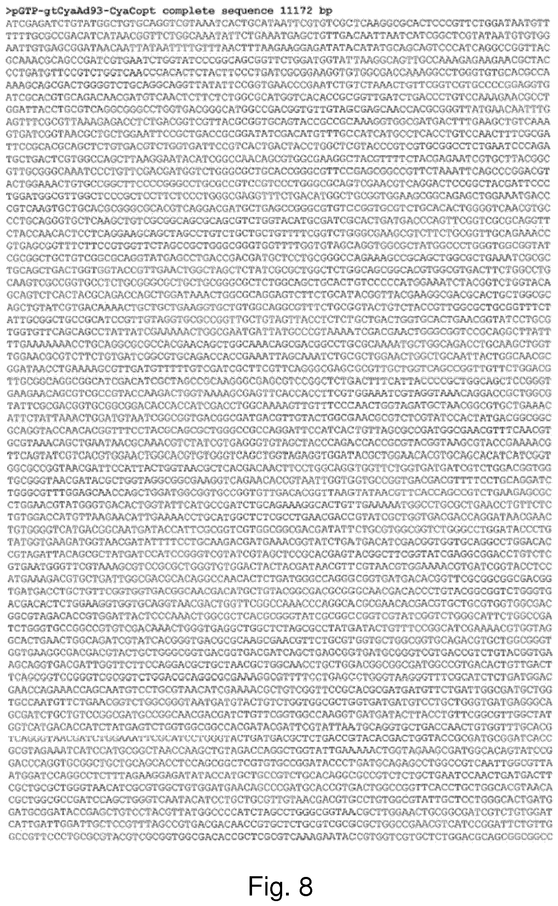

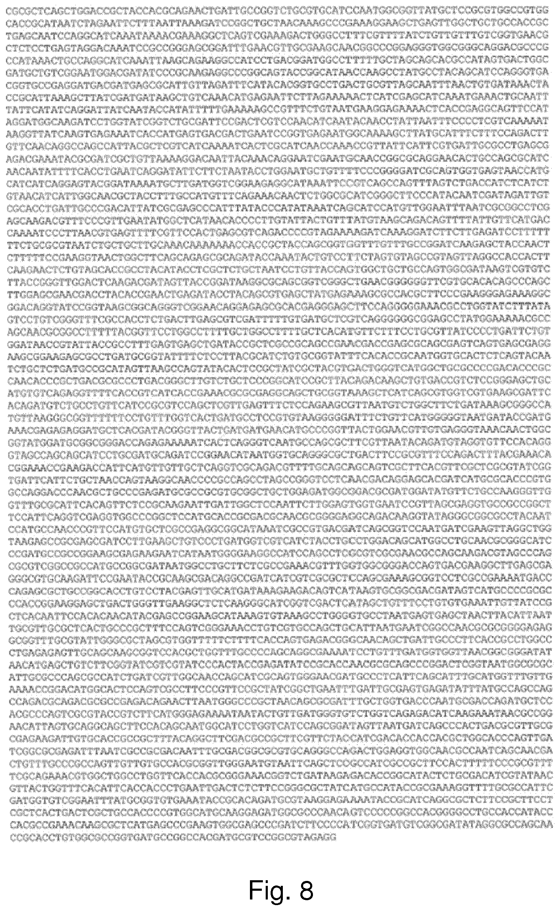

Figure 1:
   Cytokine responses after vaccination with Tdap+/- GTL003: Intramuscular route The results in histograms are representative of mean recall response of 4 animals. The splenocytes of 4 animals were pooled together and were cultured in duplicates. Each result is a mean response of duplicate wells. Levels of cytokines were analyzed using multiplex bead based ELISA and % variation (CV) between duplicates were below 20 %. Error bars representative of % CV of duplicate wells.
Figure 1A:
   Cytokine response of animals primed with Tdap and boosted either with Tdap or with Tdap + GTL003
   White bars: Tdap prime + Tdap boost
   Grey bars: Tdap prime + Tdap+GTL003 boost
Figure 1B:
   Cytokine response of animals primed with Tdap and boosted either with WCV or with Tdap + GTL003
   White bars: Tdap prime + tdap+GTL003 boost
   Grey bars: Tdap prime + WCV boost
Figure 2:
   Cytokine responses after vaccination with Tdap+/- GTL003: Intraperitoneal route
   The results in histograms are representative of mean recall response of 4 animals.
   The splenocytes of 4 animals were pooled together and were cultured in duplicates.
   Each result is a mean response of duplicate wells. Levels of cytokines were analyzed using multiplex bead based ELISA and % variation (CV) between duplicates were below 20 %. Error bars representative of % CV of duplicate wells.
Figure 2A:
   Cytokine response of animals primed with Tdap and boosted either with Tdap or with Tdap + GTL003
   White bars: Tdap prime + Tdap boost
   Grey bars: Tdap prime + tdap+GTL003 boost
Figure 2B:
   Cytokine response of animals primed with Tdap and boosted either with WCV or with Tdap + GTL003
   White bars: Tdap prime + tdap+GTL003 boost
   Grey bars: Tdap prime + WCV boost
Figure 3:
   Cytokine responses after immunization with Tdap or Tdap+GTL00: intraperitoneal INF-g immune response measured in animals vaccinated either with adjuvanted-Tdap alone or with adjuvanted-Tdap+GTL003
   *In vitro restimulations were performed with antigens contained in the Tdap or antigens contained in the Tdap* + *GTL003*
Figure 4:
   Cytokine responses after immunization with Tdap or Tdap+GTL00: intraperitoneal IL-17 immune response measured in animals vaccinated either with adjuvanted-Tdap alone or with adjuvanted-Tdap+GTL003
Figure 5:
   Isotyping Results (from intramuscular vaccination)
      - Results are from two pools of two animals each
      - Histograms represent mean GMT
      - Error bars represent 95 % CI of these titers
      - Pool to Pool variation observed as expected
      - In IM route, the effects were mixed Th1/Th2
Figure 5A:
   Pertussis toxin-IgG1 antibodies after a prime with Tdap and a boost with either Tdap, Tdap+GTL003 or WCV
Figure 5B:
   Pertussis toxin-IgG2a antibodies after a prime with Tdap and a boost with either Tdap, Tdap+GTL003 or WCV
Figure 5C:
   Pertussis toxin-IgG2b antibodies after a prime with Tdap and a boost with either Tdap, Tdap+GTL003 or WCV
Figure 5D: Pertussis toxin-IgG3 antibodies after a prime with Tdap and a boost with either Tdap, Tdap+GTL003 or WCV
Figure 6:
   Isotyping Results (from intramuscular vaccination)
      - Results are from two pools of two animals each
      - Histograms represent mean GMT
      - Error bars represent 95 % CI of these titers
      - Pool to Pool variation observed as expected
      - In IM route, the effects were mixed Th1/Th2
Figure 6A:
   FHA-IgG1 antibodies after a prime with Tdap and a boost with either Tdap, Tdap+GTL003 or WCV
Figure 6B:
   FHA-IgG2a antibodies after a prime with Tdap and a boost with either Tdap, Tdap+GTL003 or WCV
Figure 6C:
   FHA-IgG2b antibodies after a prime with Tdap and a boost with either Tdap, Tdap+GTL003 or WCV
Figure 6D:
   FHA-IgG3 antibodies after a prime with Tdap and a boost with either Tdap, Tdap+GTL003 or WCV
Figure 7:
   Plasmid maps of GTL003 (gtCyaad93)
Figure 8:
   Plasmid sequence (SEQ ID No.25)

### Examples

**Abbreviations**

| | |
|---|---|
| aa | aminoacid |
| ACT | GTL003 |
| aP | Acellular pertussis vaccine |
| APC | Antigen-presenting cells |
| CD | Cluster designation |
| CTL | Cytotoxic T lymphocytes |
| CyaA | Adenylate cyclase |
| DC | Dendritic cells |
| DTaP | Diphtheria and tetanus toxoids combined with acellular pertussis vaccine |
| DTwP | Diphteria and tetanus toxoids combined with whole cell pertussis inactivated bacteria vaccine |
| FHA | Filamentous hemagglutinin |
| FIM | Fimbriae |
| GTL003 | recombinant genetically detoxified CyaA described in WO 2014/016310 |
| id | Intradermal |
| IL-4 | Interleukin 4 |
| INF-γ | Interferon gamma |
| im | Intramuscular |
| ip | Intraperitoneal |
| KLH | Keyhole Limpet hemocyanin |
| LPS | Lipopolysaccharide |
| PRN | Pertactin |
| PT | Pertussis toxin |
| Tdap* | Tetanus and diphtheria toxoids combined with acellular pertussis reduced vaccine |
| TH1 | T-helper 1 cells |
| TH2 | T-helper 2 cells |
| TH17 | T-helper 17 cells |
| TLR | Toll like receptor |
| WCV | Whole cell pertussis vaccine |
| WHO | World health organization |
| wP | Whole cell pertussis vaccine |

| | |
|---|---|
| *Tdap distinguishes over DTap vaccine in that it contains a lower concentration of antigens: Tdap indeed reflects the concentration of antigens which is used for booster doses in conventional vaccines (such as Boostrix™ - GSK Biologicals S.A) | |

### 1. MATERIAL AND METHODS

### 1.1. MICE

13-23 grams female/male NIH mice were in bred at Serum Institute of India animal house. Mice were housed under pathogen-free conditions with water and food ad libitum. Procedures involving animals and their care were conformed to SIIL's guidelines that comply with national and international laws and policies and that are reviewed by the local ethical committee.

### 1.2. VACCINE

### Manufacturing and formulation of Tdap vaccine

The TdaP Vaccine is a blend of individually adsorbed antigens viz; Diphtheria Toxoid, Tetanus toxoid, Pertussis toxoid, Filamentous haemagglutinin and Pertactin. The tetanus component is purified toxoid manufactured by the chemical detoxification of toxin produced by Clostridium tetani. Tetanus toxoid is prepared from the toxin produced by the growth of this strain of Clostridium tetani in Semi-synthetic (meat free) medium using fermentation technology. The diphtheria toxoid is produced using Corynebacterium diphtheriae. Toxin is concentrated, partially purified and subsequently detoxified. And the toxoid is further purified to produce purified diphtheria toxoid.

The bulk Acellular pertussis is non-infectious and made up of sterile combination of three purified pertussis antigens i.e. Pertussis toxoid, Filamentous haemagglutinin and Pertactin. The fermentation is followed by chromatography based purifications followed by chemical detoxification of Toxoids. The Pertussis Toxin (PT) and Filamentous Haemagglutinin (FHA) are produced as extra cellular proteins which are secreted during fermentation process where as Pertactin (69 kDa outer membrane protein) is extracted from cells.

Tdap is a reduced antigen version of Acellular pertussis based DTaP vaccine. On December 17th 1991 the first Acellular pertussis vaccine viz; Acel-imune by lederle was licensed as a 4th and 5th Booster vaccine. These vaccines are classical vaccines.

### Construction and purification of GTL003

The DNA sequence of wild type CyaA (CyaAwt: GeneBank: CAE41066.1) was optimized and synthetized (GeneCust) for the expression in *E.Coli.* The optimized DNA sequence is named gtCyaA.

The gtCyaA was then inserted in the pGTPc608 plasmid that contains a pTAC inducible promoter (plasmid provided by GTP Technology, Labège, France).

The deletion of 93 aa in gtCyaA was generated between aa 227 and 321 by enzymatic restriction/ligation.

Purification protocol was already described in EP1 576 967 B1.

### 1.3. VACCINE FORMULATION AND ADMINISTRATION

Three vaccines were used to vaccinate mice against pertussis. All vaccines were adjuvanted with aluminum hydroxide in buffer. WCV was not adjuvanted.

**Table 1: Vaccine antigen and adjuvant doses injected per immunization**

| Vaccines | Antigen and adjuvant injected per immunization /per mouse | | | | | | |
|---|---|---|---|---|---|---|---|
| | DT | TT | PT | FHA | PRN | GTL003 | Alum |
| | (Lf) | (Lf) | (mcg) | (mcg) | (mcg) | (mcg) | (mg) |
| Tdap(1:10) | 0.25 | 0.5 | 0.8 | 0.8 | 0.25 | - | 0.39 |
| Tdap(1:10) + GTL003 | 0.25 | 0.5 | 0.8 | 0.8 | 0.25 | 25 | 0.39 |

WCV used in the present experiments corresponds to 0.5 IU / dose of *B*. *pertussis* (without alum) injected in mice.

In GTL003-containing vaccines, 25 µg of GTL003 were added to the Tdap vaccines preparation before injection. In prime boost protocol, mice were vaccinated twice via the same route, i.e. im, ip or sc, at day 0 and day 28.

**Table 2: summary of the vaccination schedule, In vitro stimulation was performed aP antigens +GTL003**

| **Groups** | **1st Dose at day 0** | **2nd Dose at day 28** | ***In vitro* Stimulus at day 42** |
|---|---|---|---|
| **Group 1** | Tdap (1:10) | Tdap (1:10) | aP antigens + GTL003 |
| **Group 2** | Tdap (1:10) | Tdap (1:10) + 25µg GTL003 | |
| **Group 3** | Tdap (1:10) | WCV | |

### 1.4. CYTOKINE RESPONSE PROFILING

Spleen cells from control and treatment groups were isolated using reported procedures. Briefly, Splenocytes were obtained by crushing the spleen using syringe plunger. The resulting Splenocytes were lysed using (0.15 M NH4Cl, 10 mM NaHCO3, 0.1 mM EDTA, pH 7.3). Splenocytes were cultured at 4 X 10⁶ cells/well in RPMI 1640 (2 mM L-Glutamine, 10% FCS) for 96 hours in presence of Pertussis antigens in presence and absence of GTL003. The cell supernatants were collected and cytokine levels were analyzed using commercial multiplex bead-based immunoassay kits according to the manufacturer's instructions (Bio-Rad Laboratories, Hercules, CA).

### 1.5. HUMORAL IMMUNE RESPONSE PROFILING

Antibody titres and isotyping was done using multiplex bead based immunoassay. Color-coded carboxylated microspheres representing distinct bead regions were obtained from Luminex Laboratories. Purified pertussis antigens (PT toxin, pertactin and FHA) were coupled to distinct activated beads essentially as described by van Gageldonk et al (25). Commercially available kit from Luminex technologies were used to couple the antigens to the beads. The kit is based on activation of beads by EDC and sulfo-NHS chemistry. Phycoerythrin (RPE)-conjugated goat anti-mouse total IgG, IgG1, IgG2a, IgG2b and IgG3 antibodies from Santa cruz were used for the assay. Purified pertussis antigens were obtained from Serum Institute of India Ltd. Humoral responses to GTL003 was analyzed using conventional ELISA. Briefly, optimized concentration of GTL003 was used for coating the plates. The sera samples were added and captured antibodies were detected using anti-mouse enzyme conjugated antibodies. The titre was determined as highest dilution of antibody showing the response higher than the cut-off OD.

### 1.6. RESPIRATORY CHALLENGE

Six mice each group were inoculated intraperitoneally with 0.5 ml from the test and reference vaccines (Mills et al. 1998, Xing et al. 1999). On day 21, B. pertussis suspension (2 X 10⁸ CFU/ml) was instilled in each mouse using intranasal challenge. Infected mice were sacrificed 7 days post-challenge. After ablation, the lung of each animal was homogenized and plated on BG and incubated at 37°C for five days for determination of viable counts (Colony Forming Units - CFU).

### 2. RESULTS

### 2.1. MICE IMMUNE RESPONSE AFTER PRIME WITH TDAP AND BOOST WITH TDAP+GTL003

To assess the impact of GTL003 in the booster vaccination on the immune response induced in primed mice, animals were primed with one injection of alum adjuvanted-Tdap at day 0 and received the boost injection containing alum adjuvanted-Tdap with or without GTL003 added to the formulation at day 28. In another group animals were primed with one injection of alum adjuvanted-Tdap at day 0 and received the boost injection containing Whole cell Vaccine without Alum at day 28. Three injection routes where tested: intra-muscular (IM), intra-peritoneal (IP) and subcutaneous (SC).

### 2.1.1. Cytokine immune response: Towards a Th1/Th17 immune response

Aluminium hydroxide adjuvanted-Tdap vaccinated people develop a more pronounced Th2 and lower Th1 immune response than induced by wP vaccination. This is thought to be the reason why immunity wanes more rapidly compared to immunity in people vaccinated with the WCV that is more prone to induce a TH1 and TH17 types of immune response (10, 15). (Bancroft)

### IM immunization

Animals primed by IM injection of Tdap/Alum and boosted with Tdap/Alum + GTL003 developed a higher antigen-specific IFN-g immune response as well as a higher antigen-specific IL-17 immune response compared to animals primed with Tdap and boosted with Tdap alone. Ratios were calculated between the level of IL-4 or IL5 *vs* IFN-g secreted by antigen-stimulated spleen cells to assess the impact of the booster of this TH1/TH2 ratio.

**Table 3: Cytokine response measured after mice immunization either with Tdap (prime) + Tdap (boost) or with Tdap (prime) / Tdap+GTL003 (Boost) (pg/ml). Ratio of IFN-g/IL-4 and IFNg/IL5 secreted cytokines is also calculated. Splenocytes restimulations were performed with Ap antigens + GTL003**

| **Group** | | **Stimulus** | **Route** | **IFN Gamma** | **IL4** | **IL5** | **IL-17** | **IFNg/IL4** | **IFNg/IL5** |
|---|---|---|---|---|---|---|---|---|---|
| **Prime** | **Boost** | | | | | | | | |
| **TdaP (1:10)** | TdaP (1:10) | Ap+GTL003 | IM | 388 | 210 | 610 | 389 | 1,85 | 0.64 |
| **TdaP (1:10)** | TdaP(1:10)+ GTL003 | Ap+GTL003 | IM | 1800 | 753 | 278 | 969 | 2,39 | 6.,47 |

As shown in *Table 3,* surprisingly, the addition of GTL003 in the booster skewed the immune response towards a Th1 immune response, i.e. increased IFNg/IL-4 and IFNg/IL-5 ratio compared to the TdaP boost without GTL003. Of note, the IL-17 response was also increased.

When we then compared the effect of GTL003 on the Th1 immune response when added to a Tdap+ GTL003 boost *vs* the WCV boost, the IFNg immune response was higher in animals that have been boosted with Tdap+GTL003 (*Table 4*) whereas the II-17 is equivalent.

**Table 4: Cytokine response measured after mice immunization either with Tdap (prime) + Tdap (boost) or with Tdap (prime) + WCV (Boost) (pg/ml). Ratio of IFN-g/IL-4 and IFNg/IL5 secreted cytokines is also calculated. Splenocytes restimulations were performed with Ap antigens + GTL003.**

| **Group** | | **Stimulus** | **Route** | **IFNg** | **IL4** | **IL5** | **IL-17** | **IFNg/IL4** | **IFNg/IL5** |
|---|---|---|---|---|---|---|---|---|---|
| **Prime** | **Boost** | | | | | | | | |
| **TdaP (1:10)** | TdaP+ GTL003(1:10) | Ap+GTL003 | IM | 1800 | 753 | 278 | 969 | 2,39 | 6,47 |
| **TdaP (1:10)** | WCV | Ap+GTL003 | IM | 811 | 435 | 729 | 914 | 1,86 | 1,11 |

When looking at the IFNg/IL-4 ratio and IFNg/IL-5 ratio, they were more-Th1 oriented response in the Tdap+GTL003 boost compared to the WCV boost.

### IP immunization:

The same immunizations were performed *via* the intraperitoneal route. Surprisingly, the results were not the same as via the IM route. The Tdap prime/Tdap boost induced a better TH1 profile compared to the Tdap prime/Tdap+GTL003 boost. No skew of the response towards a TH1 is observed when adding GTL003 in the Tdap boost (*Table 5*) or WCV in the boost (table 6)

**Table 5: Cytokine response measured after mice immunization either with Tdap (prime) + Tdap (boost) or with Tdap (prime) / Tdap+GTL003 (Boost) (pg/ml). Ratio of IFN-g/IL-4 and IFNg/IL5 secreted cytokines was also calculated. Splenocytes restimulations were performed with Ap antigens + GTL003.**

| **Group** | | **Stimulus** | **Route** | **IFNg** | **IL4** | **IL5** | **IL-17** | **IFNg/IL4** | **IFNg/IL5** |
|---|---|---|---|---|---|---|---|---|---|
| **Prime** | **Boost** | | | | | | | | |
| **TdaP (1:10)** | TdaP (1:10) | Ap+GTL003 | IP | 838 | 1207 | 1816 | 1810 | 0,69 | 0,46 |
| **TdaP (1:10)** | TdaP+ GTL003 (1:10) | Ap+GTL003 | IP | 341 | 2207 | 1492 | 209 | 0,15 | 0,23 |

Finally, when comparing the WCV boost with Tdap+GTL003 boost via the IP route, WCV boost allowed a slightly better IFNg and IL-17 immune response (*Table 6*). The overall response was nevertheless Th2 oriented.

**Table 6: Cytokine response measured after mice immunization either with Tdap (prime) / Tdap GTL003 (boost) or with Tdap (prime) + WCV (Boost) (pg/ml). Ratio of IFN-g/IL-4 and IFNg/IL5 secreted cytokines was also calculated. Splenocytes restimulations were performed with Ap antigens + GTL003.**

| **Group** | | **Stimulus** | **Route** | **IFNg** | **IL4** | **IL5** | **IL-17** | **IFNg/IL4** | **IFNg/IL5** |
|---|---|---|---|---|---|---|---|---|---|
| **Prime** | **Boost** | | | | | | | | |
| **TdaP (1:10)** | TdaP+ GTL003 (1:10) | Ap+GTL003 | IP | 341 | 2207 | 1492 | 209 | 0,15 | 0,23 |
| **TdaP (1:10)** | WCV | Ap+GTL003 | IP | 751 | 827 | 2719 | 1637 | 0,91 | 0,28 |

### SC immunization:

No significant difference was observed between the Tdap prime/ Tdap boost vs Tdap prime /tdap+GTL003 boost in terms of cytokine responses.

### 2.1.2. Humoral immune response

The antibody response to FHA, and PT was measured in the sera of vaccinated animals with Tdap and boosted either with Tdap+GTL003 or WCV.

### FHA-specific antibodies:

Animals boosted with Tdap+GTL003 had a slight decrease in IgG1 antibodiy response with almost no IgG2a, an increase in IgG2b and IgG3 levels allowing to say that the Ab response is a mixed Th1/Th2 profile but with a trend towards a Th1 IgG isotype response in the presence of GTL003.

Animals boosted with WCV developed a similar pattern of response except that IgG1 Ab level did not change while IgG2b ab level increased dramatically.

### PT-specific antibodies:

A decrease of Th2 IgG1 isotype response post- Tdap + GTL003 boost was measured. IgG2a Ab were the same and IgG2b and IgG3 Ab decreased. The same pattern of responses was obtained in animals boosted with WCV.

### PRN-specific antibodies:

The levels of PRN-specific IgG were below the limit of detection of our assays at the dilutions used for all vaccine conditions

### GTL003-specific antibody:

The anti-GTL003 IgG titers of are significantly higher after boosting with Tdap + GTL003 than with WCV. This is suggesting that the GTL003-containing vaccine boost will provide a better anti-CyaA mediated protection against whooping cough than WCV.

**Table 7: Serum Anti-Cya antibody levels in animals immunized with GTL003 or WCV . Animals were primed with Tdap**

| Route | 1st dose | 2^{nd} dose | GMT |
|---|---|---|---|
| IM | Tdap | Whole cell vaccine | 800 |
| IM | Tdap (1:10) | Tdap (1:10)+GTL003 | 12800 |

### 2.2. PROTECTION OF MICE VACCINATED WITH TDAP + GTL003 COMPARED TO WP OR TDAP ALONE

BalbC mice were vaccinated at day 0, intraperitoneally, with either wP, Tdap alone or Tdap with increasing doses of GTL003 (10 µg, 25µg, 50µg and 100µg). Three dilutions were tested as shown in Table 8. Animals were challenged 21 days after vaccination with *Bordetella pertussis* strain. Seven days after challenge, lungs were analyzed for the presence of pertussis colonization.

**Table 8: Intranasal Challenge assay post vaccination**

| Lung CFU distribution | | | |
|---|---|---|---|
| Vaccine dilution | 2 | 10 | 50 |
| Whole Cell vaccine | 508 | 4383 | 86000 |
| Tdap (Control group) | 188 | 16931 | 62167 |
| Tdap+GTL003 (10µg) | **37** | **52** | **1935** |

Tdap + GTL003 vaccine induces overall a better protection against *B*. *pertussis* than Tdap alone and equivalent or better than WCV. For vaccine dilution 10, protection was observed with Tdap+ 10µg GTL003 but also 25 (1028 CFU) and 50 µg (2625 CFU) GTL003 compared to Tdap alone and WCV.

Analysis of the immune response after a single intra-peritoneal injection of Tdap+GTL003 was performed to understand the protection gain in the presence of GTL003.

### 2.3. Mice immune response after single dose vaccination with Tdap + GTL003

### 2.3.1 Humoral immune response

Four groups of mice were vaccinated either with the Aluminium hydroxide adjuvanted-Tdap alone or adjuvanted-Tdap mixed with increasing doses of GTL003 as presented in the table hereafter.

**Table 9: Serum anti-aP antibody levels in vaccinated mice**

| **Vaccine composition** | | **Total IgG titers** | | |
|---|---|---|---|---|
| ***Vaccinated Groups*** | ***GTL003 µg*** | ***Anti-PT Ab*** | ***Anti-FHA Ab*** | ***Anti-PRN Ab*** |
| Tdap (ref) | 0 | 49,2 | 117,2 | 6,7 |
| Tdap + GTL003 | 4 | **79,7** | **127** | **10,54** |
| Tdap + GTL003 | 40 | **96,8** | **142,9** | **14,38** |
| Tdap + GTL003 | 120 | 73 | **194,2** | 5,03 |

A slight increase of antibodies against PT, FHA and PRN was detected only in groups of mice immunized with pertussis vaccine containing GTL003.

When comparing the production of antibodies against CyaA, the response was higher with adjuvanted-Tdap + GTL003 vaccinated animals compared with those vaccinated with the WCV. Of note, WCV contains wild type CyaA.

**Table 10: Serum anti-CyaA antibody levels in vaccinated mice.**

| **Route** | **Prime** | **Boost** | **Anti-cyaA IgG Titer** |
|---|---|---|---|
| IP | Tdap (1 :10) | Whole cell vaccine | 800 |
| IP | Tdap (1:10) | Tdap (1:10)+ GTL003 | **12800** |

### 2.3.2 Cytokine production by spleen and macrophages

The secreted cytokines by spleen cells restimulated *in vitro* with Ap antigens from mice vaccinated with either adjuvanted-Tdap + GTL003 or Tdap alone or WCV were analyzed in unchallenged mice, i.e. mice that have not been challenged *in vivo* by live *B*. *pertussis* post vaccination.

**Table 11: Cytokine immune response measured in spleens and macrophages of vaccinated, but unchallenged mice, after in vitro stimulation with vaccine antigens.**

| **Readout** | **Groups according to GTL003 dose (10, 25, 50, 100µg) injected IP with Tdap +/- GTL003 or WCV (Tdap + GTL003 dose in µg)** | | | | |
|---|---|---|---|---|---|
| | Tdap | **Tdap+25** | **Tdap+50** | Tdap+10 0 | WCV |
| **Th1 cytokines** | | | | | |
| **IL**-**12 (p70)** | Reference | **No change** | **No change** | No change | - |
| **IFN-g** | Reference | + | - | - | - |
| **Th2 cytokines** | | | | | |
| **IL-4** | Reference | - | - | - | NA |
| **IL-5** | Reference | **No change** | **No change** | - | NA |
| **IL-13** | Reference | **No change** | **+** | No change | - |
| **IL-9** | Reference | **No change** | **-** | - | No change |
| **Th17 cytokines** | | | | | |
| **IL**-**17A** | Reference | **++** | **+** | ++ | NA |
| **Macrophage activity** | | | | | |
| **Nitric Oxide** | Reference | **++** | **+++** | + | NA |

| | | | | | |
|---|---|---|---|---|---|
| +, ++, +++: 1-2x, 2-4x and >4x increase in cytokine level compared to cytokine induced by Tdap alone (reference), respectively; -: decrease | | | | | |

The addition of GTL003 at the concentration of either 25 or 50µg to the Tdap vaccine induces an increase of the TH1 and TH17 immune responses. Unexpectedly, GTL003 induces the same antigen-specific effect on nitric oxide (NO) in stimulated spleen cells while the injection has been performed intra-peritonealy compared to mice vaccinated with Tdap alone. In previous experiments, NO has been shown to increase after peritoneal macrophages stimulation after intra-peritoneal injection that could be expected because of the recruitment of macrophages at injection site.

The same conclusions on the effect of the addition of GTL003 to Tdap vaccine are drawn in vaccinated mice followed by an intranasal challenge with live *B*. *pertussis.*

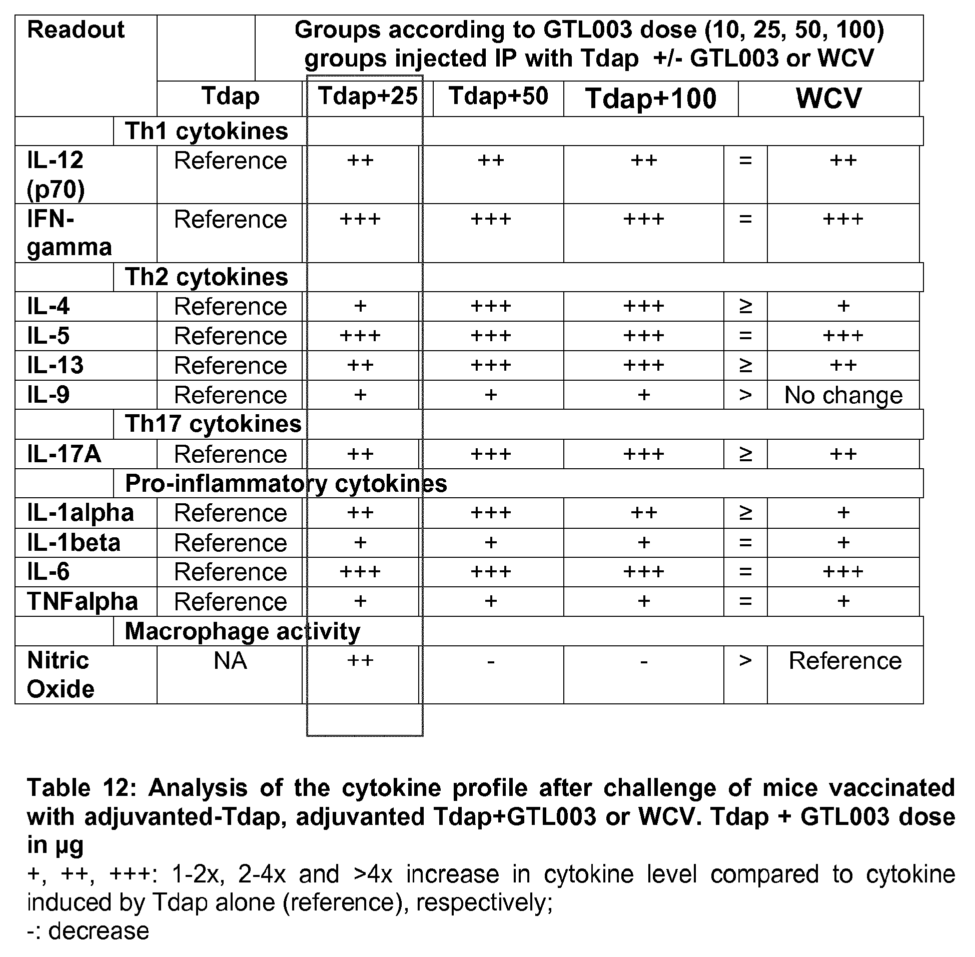

A dramatic overall increase in the immune response in the groups vaccinated with GTL003-containing adjuvanted-Tdap + GTL003 compared to adjuvanted Tdap without GTL003 was observed. This response was equivalent or superior to WCV. A strong increase of IFN-g, IL17 and Nitric Oxide were observed.

The analysis of the IFN-g after stimulation either with Tdap antigens or Tdap+GTL003 showed that the response against all antigens was increased in the groups immunized with GTL003-containing vaccine.

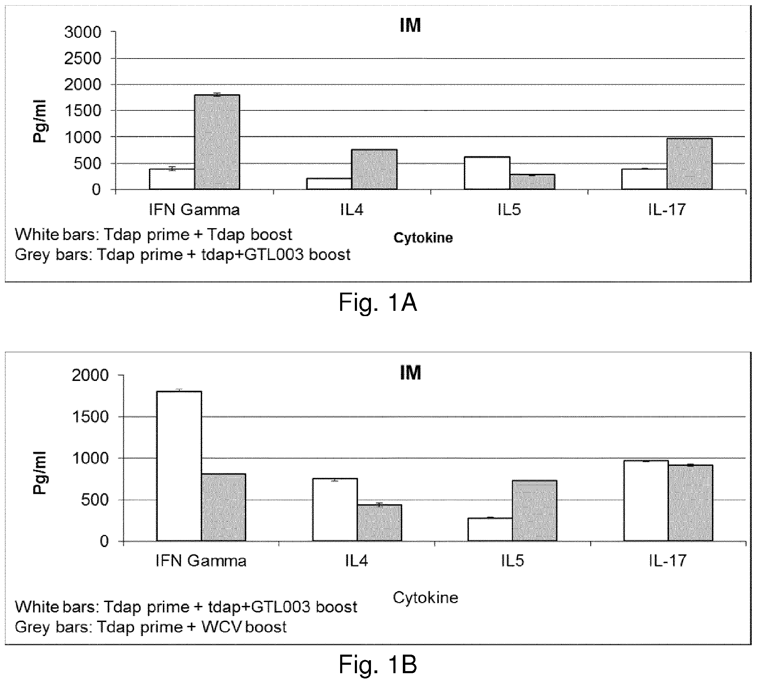

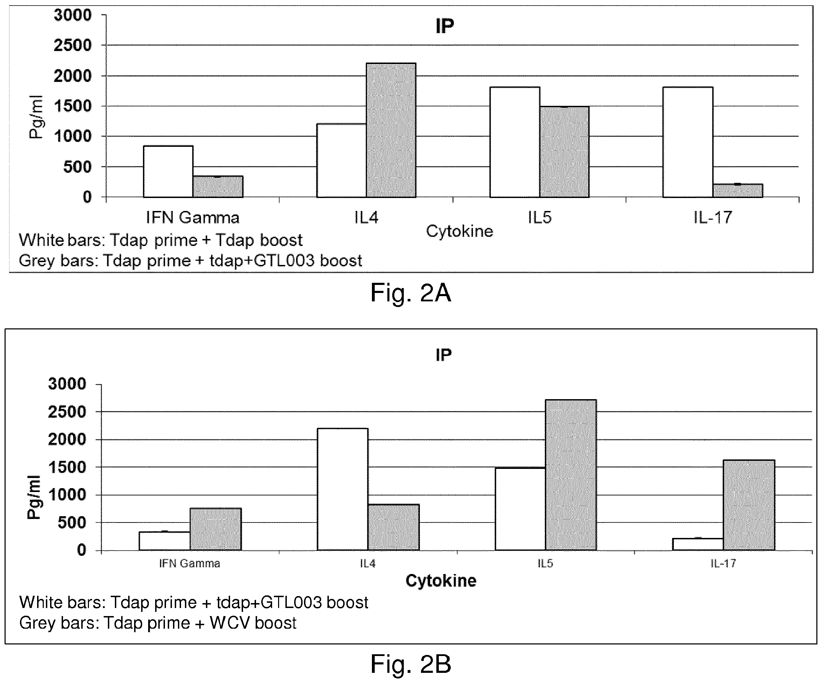

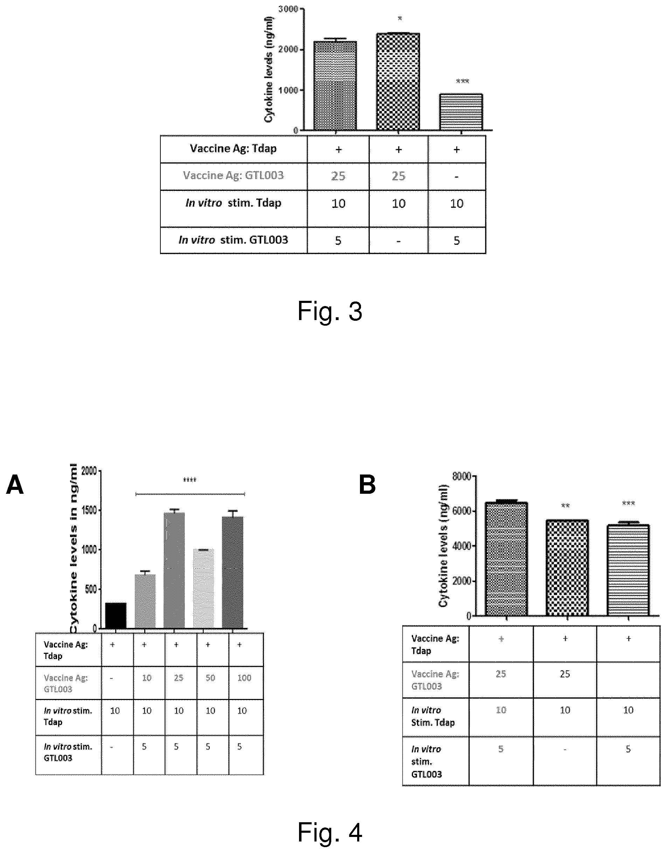

For IL-17, the level of cytokine measured was higher in the presence of GTL003 and increased between 10 and 25 µg of GTL003 (Fig 3A). Unexpectedly, the increased response in those animals vaccinated with Tdap+GTL003 was GTL003-specific (Fig 4 B).

### CONCLUSION:

These results show for the first time the ability of GTL003 to enhance the Th1 immune response against pertussis antigens when included in a Tdap vaccine containing aluminium, a Th2 adjuvant.
- The effect is observed after intramuscular injection when GTL003 is in the Tdap boost which is unexpected following what the literature reports;
- The effect is observed after a single intraperitoneal injection (standard route of vaccination in a challenge assay) allowing a better protection of animals, which is supported by the cytokine and humoral responses that are superior to the WCV; this is also unexpected in the presence of aluminium

### References

1. Cherry, J. D. 1996. Historical review of pertussis and the classical vaccine. J Infect Dis 174 Suppl 3:S259-263.
2. Mattoo, S., and J. D. Cherry. 2005. Molecular pathogenesis, epidemiology, and clinical manifestations of respiratory infections due to Bordetella pertussis and other Bordetella subspecies. Clin Microbiol Rev 18:326-382.
3. Aoyama, T., Y. Murase, T. Kato, and T. Iwata. 1985. Efficacy of an acellular pertussis vaccine in Japan. J Pediatr 107:180-183.
4. 1992. American Academy of Pediatrics Committee on Infectious Disease: Acellular pertussis vaccines: recommendations for use as the fourth and fifth doses. Pediatrics 90:121-123.
5. Sealey, K. L., T. Belcher, and A. Preston. 2016. Bordetella pertussis epidemiology and evolution in the light of pertussis resurgence. Infect Genet Evol 40:136-143.
6. Edwards, K. M., and G. A. Berbers. 2014. Immune responses to pertussis vaccines and disease. J Infect Dis 209 Suppl 1:S10-15.
7. Allen, A. 2013. Public health. The pertussis paradox. Science 341:454-455.
8. Cherry, J. D. 2012. Epidemic pertussis in 2012--the resurgence of a vaccine-preventable disease. N Engl J Med 367:785-787.
9. Black, R. E., S. Cousens, H. L. Johnson, J. E. Lawn, I. Rudan, D. G. Bassani, P. Jha, H. Campbell, C. F. Walker, R. Cibulskis, T. Eisele, L. Liu, and C. Mathers. 2010. Global, regional, and national causes of child mortality in 2008: a systematic analysis. Lancet 375:1969-1987.
10. Warfel, J. M., and K. M. Edwards. 2015. Pertussis vaccines and the challenge of inducing durable immunity. Curr Opin Immunol 35:48-54.
11. Witt, M. A., L. Arias, P. H. Katz, E. T. Truong, and D. J. Witt. 2013. Reduced risk of pertussis among persons ever vaccinated with whole cell pertussis vaccine compared to recipients of acellular pertussis vaccines in a large US cohort. Clin Infect Dis 56:1248-1254.
12. Klein, N. P., J. Bartlett, A. Rowhani-Rahbar, B. Fireman, and R. Baxter. 2012. Waning protection after fifth dose of acellular pertussis vaccine in children. N Engl J Med 367:1012-1019.
13. Koepke, R., J. C. Eickhoff, R. A. Ayele, A. B. Petit, S. L. Schauer, D. J. Hopfensperger, J. H. Conway, and J. P. Davis. 2014. Estimating the effectiveness of tetanus-diphtheria-acellular pertussis vaccine (Tdap) for preventing pertussis: evidence of rapidly waning immunity and difference in effectiveness by Tdap brand. J Infect Dis 210:942-953.
14. Warfel, J. M., L. I. Zimmerman, and T. J. Merkel. 2014. Acellular pertussis vaccines protect against disease but fail to prevent infection and transmission in a nonhuman primate model. Proc Natl Acad Sci U S A 111:787-792.
15. Higgins, S. C., A. G. Jarnicki, E. C. Lavelle, and K. H. Mills. 2006. TLR4 mediates vaccine-induced protective cellular immunity to Bordetella pertussis: role of IL-17-producing T cells. J Immunol 177:7980-7989.
16. Guermonprez, P., N. Khelef, E. Blouin, P. Rieu, P. Ricciardi-Castagnoli, N. Guiso, D. Ladant, and C. Leclerc. 2001. The adenylate cyclase toxin of Bordetella pertussis binds to target cells via the alpha(M)beta(2) integrin (CD11 b/CD18). J Exp Med 193:1035-1044.
17. Hewlett, E. L., L. Gray, M. Allietta, I. Ehrmann, V. M. Gordon, and M. C. Gray. 1991. Adenylate cyclase toxin from Bordetella pertussis. Conformational change associated with toxin activity. J Biol Chem 266:17503-17508.
18. Vojtova, J., J. Kamanova, and P. Sebo. 2006. Bordetella adenylate cyclase toxin: a swift saboteur of host defense. Curr Opin Microbiol 9:69-75.
19. Goodwin, M. S., and A. A. Weiss. 1990. Adenylate cyclase toxin is critical for colonization and pertussis toxin is critical for lethal infection by Bordetella pertussis in infant mice. Infect Immun 58:3445-3447.
20. Khelef, N., H. Sakamoto, and N. Guiso. 1992. Both adenylate cyclase and hemolytic activities are required by Bordetella pertussis to initiate infection. Microb Pathog 12:227-235.
21. Macdonald-Fyall, J., D. Xing, M. Corbel, S. Baillie, R. Parton, and J. Coote. 2004. Adjuvanticity of native and detoxified adenylate cyclase toxin of Bordetella pertussis towards co-administered antigens. Vaccine 22:4270-4281.
22. Cheung, G. Y., D. Xing, S. Prior, M. J. Corbel, R. Parton, and J. G. Coote. 2006. Effect of different forms of adenylate cyclase toxin of Bordetella pertussis on protection afforded by an acellular pertussis vaccine in a murine model. Infect Immun 74:6797-6805.
23. Dunne, A., P. J. Ross, E. Pospisilova, J. Masin, A. Meaney, C. E. Sutton, Y. Iwakura, J. Tschopp, P. Sebo, and K. H. Mills. 2010. Inflammasome activation by adenylate cyclase toxin directs Th17 responses and protection against Bordetella pertussis. J Immunol 185:1711-1719.
24. Dadaglio, G., C. Fayolle, X. Zhang, B. Ryffel, M. Oberkampf, T. Felix, S. Hervas-Stubbs, R. Osicka, P. Sebo, D. Ladant, and C. Leclerc. 2014. Antigen targeting to CD11 b+ dendritic cells in association with TLR4/TRIF signaling promotes strong CD8+ T cell responses. J Immunol 193:1787-1798.
25. van Gageldonk P. G., van Schaijk F. G., van der Klis F. R., Berbers G. A. 2008. Development and validation of a multiplex immunoassay for the simultaneous determination of serum antibodies to Bordetella pertussis, diphtheria and tetanus. J. Immunol. Methods 335:79-89
26. Bancroft et al, Cellular Immunology, 2016
27. Ryan et al, Clin Exp Immunol 2000

## Claims

1. An immunogenic composition which is chosen in the group of:
a) an acellular immunogenic composition suitable for the protection against a condition causally related to the infection by a *Bordetella* strain, in particular *Bordetella pertussis* in a human host, or
b) a combination immunogenic composition comprising said acellular immunogenic composition suitable for the protection against a condition causally related to the infection by a *Bordetella* strain, in particular *Bordetella pertussis* in a human host
wherein said acellular immunogenic composition comprises:
- a non-toxic polypeptide, in particular a non-cytotoxic polypeptide, derived from the adenylate cyclase protein (CyaA-derived polypeptide) of a *Bordetella* strain and,
- optionally an adjuvant of the TH1 immune response or an adjuvant of the TH2 immune response or a combination of both.

2. An immunogenic composition according to claim 1, wherein the non-cytotoxic polypeptide, derived from the adenylate cyclase protein (CyaA-derived polypeptide) of a *Bordetella* strain is **(i)** a polypeptide the amino acid sequence of which is obtained from CyaA of *Bordetella pertussis* and contains a deletion of a continuous segment of at least 93 amino acid residues from position 227 to position 321 in the amino acid sequence of the native CyaA of *Bordetella pertussis* or **(ii)** a polypeptide which is a variant of the polypeptide in (i) the amino acid sequence of said variant polypeptide being obtained from CyaA of *Bordetella parapertussis, Bordetella bronchiseptica* or *Bordetella hinzii,* and containing the deletion of a continuous segment of at least 93 amino acid residues defined by the positions in the native CyaA sequence of *Bordetella parapertussis, Bordetella bronchiseptica* or *Bordetella hinzii* matching positions 227 and 321 in CyaA of said continuous segment of at least 93 amino acid residues of *Bordetella pertussis.*

3. An acellular immunogenic composition according to claim 1 or 2 suitable for the protection against a condition causally related to the infection by a *Bordetella* strain, in particular *Bordetella pertussis,* in a human which comprises:
- at least two antigens from a *Bordetella* strain wherein one antigen is **(i)** a non-toxic polypeptide, in particular a non-cytotoxic polypeptide, derived from the adenylcyclase protein (CyaA-derived polypeptide) of a *Bordetella* strain, said polypeptide being in particular **(i.1)** either a polypeptide the amino acid sequence of which is obtained from CyaA of *Bordetella pertussis* and contains a deletion of a continuous segment of at least 93 amino acid residues from position 227 to position 321 in the amino acid sequence of the native CyaA of *Bordetella pertussis,* **(i.2)** or a polypeptide which is a variant of the polypeptide in (i) the amino acid sequence of said variant polypeptide being obtained from CyaA of *Bordetella parapertussis, Bordetella bronchiseptica* or *Bordetella hinzii,* and containing the deletion of a continuous segment of at least 93 amino acid residues defined by the positions in the native CyaA sequence of *Bordetella parapertussis, Bordetella bronchiseptica* or *Bordetella hinzii* matching positions 227 and 321 in CyaA of said continuous segment of at least 93 amino acid residues of *Bordetella pertussis,* and wherein further antigen(s) is (are) selected in the group of **(ii)** *Pertussis* toxoid (PT), **(iii)** Filamentous Haemagglutinin (FHA), **(iv)** Pertactin (PRN) and **(v)** Fimbriae (Fim),
wherein a protective immune response against the *Bordetella* strain is raised against said antigens and,
- optionally an adjuvant of the TH1 immune response or an adjuvant of the TH2 immune response or a combination of both.

4. A Combination immunogenic composition according to claim 1 or to claim 2, comprising further antigens as active ingredients for the elicitation of an immune protection against determined pathogens which are **(vi)** at least one antigen of *Clostridium tetani* consisting of the Tetanus toxin in detoxified form, **(vii)** an antigen of *Corynebacterium* complex consisting of the diphtheria toxin in detoxified from, and optionally **(viii)** further antigens of different pathogen(s).

5. A combination immunogenic composition according to any one of claims 1 to 4 which comprises further antigens as active ingredients for the elicitation of an immune protection against determined pathogens which are selected in the group of Hepatitis B surface antigen (HBs), inactivated poliovirus (IPV) of one or several virus strains, *Haemophilus influenza* type b polysaccharide.

6. An acellular immunogenic composition according to any one of claims 1 to 3 or a combination immunogenic composition according to any one of claims 1, 4 or 5, wherein the antigens selected in the group of Bordetella toxin in detoxified form (PT), Filamentous Haemagglutinin (FHA), Pertactin (PRN) and Fimbriae (Fim) are independently of each other from *Bordetella pertussis, Bordetella parapertussis, Bordetella bronchiseptica* or *Bordetella hinzii,* preferably from B. *pertussis,* and preferably are all from the same *Bordetella* strain.

7. Combination immunogenic composition according to any one of claims 1, 2 or 4 to 5 wherein one dose of 0.5ml of the immunogenic composition contains:
*Bordetella* toxin in detoxified form, in particular *Pertussis* toxoid: 1 to 50 micrograms;
Filamentous Haemagglutinin: 1 to 50 µg;
Pertactin: 1 to 20µg;
optionally Fimbriae: 2 to 25 µg;
Tetanus toxoid: at least 0.25 and less than 25 Lf;
Diphtheria toxoid: at least 0.25 and less than 50 Lf;
and optionally,
Hepatitis B surface antigen: 5µg to 25µg
inactivated poliovirus: from 5 to 45, in particular from 8 to 40 D-antigen unit per strain;
*Haemophilus influenza* type b polysaccharide: 1 to 20µg.

8. Combination immunogenic composition according to any one of claims 1, 2 or 4 to 6, wherein one dose of 0.5ml of the immunogenic composition comprises:
*Bordetella* toxin in detoxified form in particular *Pertusssis* toxoid: 8µg;
Filamentous Haemagglutinin:8µg;
Pertactin: 2.5µg;
Tetanus toxoid: 5Lf;
Diphteria toxoid : 2.5Lf.

9. An immunogenic composition, in particular a combination immunogenic composition, according to any one of claims 1 to 8 wherein one dose of the immunogenic composition comprises 2.5 to 600 micrograms, 2.5 to 500µg, 2.5 to 400µg, 2.5 to 300µg, 2.5 to 200µg or 2.5 to 100µg and preferably comprises 25µg of the CyaA-derived polypeptide of *Bordetella pertussis.*

10. An immunogenic composition, in particular a combination immunogenic composition, according to any one of claims 1 to 9 wherein the adjuvant of the TH2 response is an aluminum salt, in particular is aluminum hydroxide, aluminum hydroxyphosphate sulfate or aluminum phosphate.

11. An immunogenic composition, in particular a combination immunogenic composition according to any one of claims 1 to 10 wherein the adjuvant of the TH1 response is selected:
i. in the group of MPL-containing adjuvant, in particular AS15, AS01B, AS01D, or AS01E; CpG-containing adjuvant, in particular AS15, QS21-containing adjuvant, in particular AS01 B, D, and E or AS15, immune stimulating complexes (ISCOMs), IC31-containing adjuvant, chitosan-containing adjuvant, liposomal formulation-based adjuvant, in particular AS01 B D, E or AS15 or ISCOMs, lipid-based emulsions such as MF59, or
ii. In the group of adjuvants which are or contain a ligand of a toll-like receptor selected among:
- a ligand of toll-like receptor 4 (TLR-4), in particular monophosphoryl lipid A (MPL) or Glucopyranosyl Lipid A (GLA) or,
- a ligand of toll-like receptor 9 (TLR-9), in particular a synthetic oligodeoxynucleotides (ODNs) containing unmethylated CpG motifs and more particularly HBsAg-1018 or,
- a ligand of toll-like receptor 3 (TLR-3), in particular a synthetic analog of double-stranded RNA (dsRNA) and more particularly a composition containing Poly(I:C) such as polyinosinic-polycytidylic acid plus poly-L-lysine double-stranded RNA in the presence of carboxymethylcellulose (Poly (ICLC).or,
- a ligand of toll-like receptor 2 (TLR-2), in particular synthetic lipopeptides or a recombinant lipoprotein and,
- a ligand of toll-like receptor 5 (TLR-5), in particular a recombinant bacterial flagellin.

12. An immunogenic composition, in particular a combination immunogenic composition, according to any one of claims 1 to 11 wherein the CyaA-derived polypeptide of *Bordetella* consists of:
1) a segment or a fragment of *Bordetella pertussis* CyaA protein as set forth in SEQ ID No. 2, the sequence of said segment or fragment beginning with the first residue of SEQ ID No.2 and ending with a residue located from position 183 to position 227 of SEQ ID No.2 or a polypeptide variant obtained from *Bordetella parapertussis, Bordetella bronchiseptica* or *Bordetella hinzii* and having at least 95% identity in amino acid residues with the segment or fragment consisting of residues 1 to 183 or 1 to 227 in SEQ ID No.2, fused to
2) a segment or a fragment of *Bordetella* pertussis CyaA protein as set forth in SEQ ID No. 2, the sequence of said segment or fragment beginning with a residue located from position 321 to position 387 of SEQ ID No.2 and ending with the last residue of SEQ ID No.2 or a polypeptide variant obtained from *Bordetella parapertussis, Bordetella bronchiseptica* or *Bordetella hinzii,* and having at least 95% identity in amino acid residues with the segment or fragment consisting of residues 321 to final residue or 387 to final residue in SEQ ID No.2.

13. An immunogenic composition, in particular a combination immunogenic composition according to any one of claims 1 to 12 wherein the CyaA-derived polypeptide of *Bordetella* consists of:
1) a polypeptide comprising or consisting of the sequence as set forth in SEQ ID NO:10 or a polypeptide variant obtained from *Bordetella parapertussis, Bordetella bronchiseptica* or *Bordetella hinzii* and **characterized by** an amino acid sequence matching the amino acid residue positions of SEQ ID No.10;
2) a polypeptide comprising or consisting of the sequence as set forth in SEQ ID NO:12 or a variant obtained from *Bordetella parapertussis, Bordetella bronchiseptica* or *Bordetella hinzii,* and **characterized by** an amino acid sequence matching the amino acid residue positions of SEQ ID No.12,
3) a polypeptide comprising or consisting of the sequence as set forth in SEQ ID NO:19 or a variant obtained from *Bordetella parapertussis, Bordetella bronchiseptica* or *Bordetella hinzii,* and **characterized by** an amino acid sequence matching the amino acid residue positions of SEQ ID No.19,
4) a polypeptide comprising or consisting of the sequence as set forth in SEQ ID NO:20 or a variant obtained from *Bordetella parapertussis, Bordetella bronchiseptica* or *Bordetella hinzii* and **characterized by** an amino acid sequence matching the amino acid residue positions of SEQ ID No.20.

14. An immunogenic composition, in particular a combination immunogenic composition according to any one of claims 1 to 13, wherein the CyaA-derived polypeptide is the expression product in *E.coli* cells of the nucleic acid molecule gtCyaad93-CyaC-opt borne by plasmid pGTP- gtCyaad93-CyaC-opt of SEQ ID No. 25.

15. A medicinal composition for administration to a human host which comprises an immunogenic composition according to any one of claims 1 to 14 and which is provided as an administration form selected among a powder, a powder and solution for reconstitution, a liquid, in particular a suspension or a solution, a lyophilized component and a combination of such administration forms.

16. A medicinal composition according to claim 15 wherein one dose for administration to a human host contains 0.25 ml to 1 ml of liquid, in particular of reconstituted product.

17. A medicinal composition according to claim 15 or 16, for administration to a human host which is provided as a pharmaceutical form formulated for administration by injection, in particular for intramuscular injection.

18. A method for preparing a vaccine comprising as active ingredients for protection against determined pathogens or condition causally related to such pathogens, antigens of said pathogens and furthermore a polypeptide derived from the adenylate cyclase protein (CyaA-derived polypeptide) of a *Bordetella* strain as defined in any of claims 1 or 13, wherein the method comprises the step(s) of:
a) providing antigens selected in the group of **(i)** at least one of the antigens from *Bordetella* selected in the group of *Pertussis toxin in detoxified form* (PT), Filamentous Haemagglutinin (FHA), Pertactin (PRN) and Fimbriae (Fim) and preferably the first three antigens or all of these antigens, **(ii)** at least one antigen of *Clostridium tetani* consisting of the Tetanus toxin in detoxified form, **(iii)** an antigen of *Corynebacterium* complex consisting of the diphtheria toxin in detoxified form, and **(iv)** the CyaA-derived polypeptide as defined in any one of claims 1 or 13 to 15 and optionally **(v)** further antigens of different pathogen(s) as active ingredients for the elicitation of an immune response against said determined pathogens formulated as one or several component(s) and,
b) admixing said component(s) with one or more compounds that enhance the immune response when the vaccine is administered to a host such as adjuvant(s) of the TH2- oriented immune response, adjuvant of the TH1-oriented immune response, adjuvant of the TH17- oriented immune response or a combination of such adjuvants,
wherein steps a) and b) are optionally carried out as a single step.

19. A method according to claim 18 wherein the active ingredients for protection are as defined in any one of claims 2 to 14.

20. A combination immunogenic composition according to any one of claims 1 to 14 or a medicinal composition according to any one of claims 15 to 17 for use in active immunization of a human host against diphtheria, tetanus and condition causally related to *pertussis* infection, and optionally against hepatitis B, poliomyelitis and/or disease caused by *Haemophilus influenza* type b.

21. A combination immunogenic composition according to any one of claims 1 to 13 or a medicinal composition according to any one of claims 14 to 16 for use in active immunization of a human host against the persistence of *Pertussis* bacteria after infection of a human host, or against the transmission and/or against the colonization of airways of the human host infected by a *Bordetella* strain, in particular B.*pertussis.*

22. A combination immunogenic composition according to any one of claims 1 to 13 or a medicinal composition according to any one of claims 15 to 17 for use according to claim 19 or 20 as a dose for administration after a first dose, in particular (i) as a second or as a further dose of a multiple-dose setting or (ii) as a booster dose, in individuals who previously received first vaccination dose or primary vaccination with a monovalent or a combination vaccine against at least one of the diseases selected in the group of tetanus, diphtheria and a condition causally related to infection by a *Bordetella* strain, in particular *Bordetella pertussis,* and optionally hepatitis B, poliomyelitis and disease caused by *Haemophilus influenza* type b.

23. A combination immunogenic composition according to any one of claims 1 to 14 or a medicinal composition according to any one of claims 16 to 18 for use according to any one of claims 20 to 22 as a dose for administration as a second or as a further dose, in particular as a booster dose and wherein said second, further, or in particular booster dose, is different from the first or from the previously administered dose(s) in that said first or previous doses is(are) devoid of non-toxic CyaA derived polypeptide as defined in claims 1, 2 or 12 to 14.

24. A combination immunogenic composition or a medicinal composition for use according to any one of claims 20 to 23, wherein the host is a child at birth or later, in particular until 18 months of age

25. A combination immunogenic composition for use according to claim 22 or 23, as a booster dose in a prime/boost vaccination setting wherein the host is a Child over 4 years of age, an adolescent over 11 or an adult, in particular an elderly person, a pregnant woman or relatives close to a pregnant woman, said host having previously received primary vaccination with a different acellular vaccine against a condition causally related to infection by *Bordetella* strain, in particular *Bordetella pertussis* either administered as a monovalent *pertussis* vaccine or a combination vaccine against whopping cough.


SEQUENCE LISTING

<110>  GENTICEL
       Serum Institute of India Private Limited

<120>  Immunogenic composition comprising CyaA-derived polypeptide
       promoting  a TH1/TH17-oriented immune response

<130>  B12224-AD/SDU

<140>  EP XXXXXXXX.X
<141>  2016-12-27

<160>  25

<170>  PatentIn version 3.5

<210>  1
<211>  5121
<212>  DNA
<213>  Bordetella pertussis (wild type CyaA)


<220>
<221>  CDS
<222>  (1)..(5121)

<400>  1
atg cag caa tcg cat cag gct ggt tac gca aac gcc gcc gac cgg gag        48
Met Gln Gln Ser His Gln Ala Gly Tyr Ala Asn Ala Ala Asp Arg Glu
1               5                   10                  15

tct ggc atc ccc gca gcc gta ctc gat ggc atc aag gcc gtg gcg aag        96
Ser Gly Ile Pro Ala Ala Val Leu Asp Gly Ile Lys Ala Val Ala Lys
            20                  25                  30

gaa aaa aac gcc aca ttg atg ttc cgc ctg gtc aac ccc cat tcc acc       144
Glu Lys Asn Ala Thr Leu Met Phe Arg Leu Val Asn Pro His Ser Thr
        35                  40                  45

agc ctg att gcc gaa ggg gtg gcc acc aaa gga ttg ggc gtg cac gcc       192
Ser Leu Ile Ala Glu Gly Val Ala Thr Lys Gly Leu Gly Val His Ala
    50                  55                  60

aag tcg tcc gat tgg ggg ttg cag gcg ggc tac att ccc gtc aac ccg       240
Lys Ser Ser Asp Trp Gly Leu Gln Ala Gly Tyr Ile Pro Val Asn Pro
65                  70                  75                  80

aat ctt tcc aaa ctg ttc ggc cgt gcg ccc gag gtg atc gcg cgg gcc       288
Asn Leu Ser Lys Leu Phe Gly Arg Ala Pro Glu Val Ile Ala Arg Ala
                85                  90                  95

gac aac gac gtc aac agc agc ctg gcg cat ggc cat acc gcg gtc gac       336
Asp Asn Asp Val Asn Ser Ser Leu Ala His Gly His Thr Ala Val Asp
            100                 105                 110

ctg acg ctg tcg aaa gag cgg ctt gac tat ctg cgg caa gcg ggc ctg       384
Leu Thr Leu Ser Lys Glu Arg Leu Asp Tyr Leu Arg Gln Ala Gly Leu
        115                 120                 125

gtc acc ggc atg gcc gat ggc gtg gtc gcg agc aac cac gca ggc tac       432
Val Thr Gly Met Ala Asp Gly Val Val Ala Ser Asn His Ala Gly Tyr
    130                 135                 140

gag cag ttc gag ttt cgc gtg aag gaa acc tcg gac ggg cgc tat gcc 480
Glu Gln Phe Glu Phe Arg Val Lys Glu Thr Ser Asp Gly Arg Tyr Ala
145                 150                 155                 160

gtg cag tat cgc cgc aag ggc ggc gac gat ttc gag gcg gtc aag gtg 528
Val Gln Tyr Arg Arg Lys Gly Gly Asp Asp Phe Glu Ala Val Lys Val
                165                 170                 175

atc ggc aat gcc gcc ggt att cca ctg acg gcg gat atc gac atg ttc 576
Ile Gly Asn Ala Ala Gly Ile Pro Leu Thr Ala Asp Ile Asp Met Phe
            180                 185                 190

gcc att atg ccg cat ctg tcc aac ttc cgc gac tcg gcg cgc agt tcg 624
Ala Ile Met Pro His Leu Ser Asn Phe Arg Asp Ser Ala Arg Ser Ser
        195                 200                 205

gtg acc agc ggc gat tcg gtg acc gat tac ctg gcg cgc acg cgg cgg 672
Val Thr Ser Gly Asp Ser Val Thr Asp Tyr Leu Ala Arg Thr Arg Arg
    210                 215                 220

gcc gcc agc gag gcc acg ggc ggc ctg gat cgc gaa cgc atc gac ttg 720
Ala Ala Ser Glu Ala Thr Gly Gly Leu Asp Arg Glu Arg Ile Asp Leu
225                 230                 235                 240

ttg tgg aaa atc gct cgc gcc ggc gcc cgt tcc gca gtg ggc acc gag 768
Leu Trp Lys Ile Ala Arg Ala Gly Ala Arg Ser Ala Val Gly Thr Glu
                245                 250                 255

gcg cgt cgc cag ttc cgc tac gac ggc gac atg aat atc ggc gtg atc 816
Ala Arg Arg Gln Phe Arg Tyr Asp Gly Asp Met Asn Ile Gly Val Ile
            260                 265                 270

acc gat ttc gag ctg gaa gtg cgc aat gcg ctg aac agg cgg gcg cac 864
Thr Asp Phe Glu Leu Glu Val Arg Asn Ala Leu Asn Arg Arg Ala His
        275                 280                 285

gcc gtc ggc gcg cag gac gtg gtc cag cat ggc act gag cag aac aat 912
Ala Val Gly Ala Gln Asp Val Val Gln His Gly Thr Glu Gln Asn Asn
    290                 295                 300

cct ttc ccg gag gca gat gag aag att ttc gtc gta tcg gcc acc ggt 960
Pro Phe Pro Glu Ala Asp Glu Lys Ile Phe Val Val Ser Ala Thr Gly
305                 310                 315                 320

gaa agc cag atg ctc acg cgc ggg caa ctg aag gaa tac att ggc cag 1008
Glu Ser Gln Met Leu Thr Arg Gly Gln Leu Lys Glu Tyr Ile Gly Gln
                325                 330                 335

cag cgc ggc gag ggc tat gtc ttc tac gag aac cgt gca tac ggc gtg 1056
Gln Arg Gly Glu Gly Tyr Val Phe Tyr Glu Asn Arg Ala Tyr Gly Val
            340                 345                 350

gcg ggg aaa agc ctg ttc gac gat ggg ctg gga gcc gcg ccc ggc gtg 1104
Ala Gly Lys Ser Leu Phe Asp Asp Gly Leu Gly Ala Ala Pro Gly Val
        355                 360                 365

ccg agc gga cgt tcg aag ttc tcg ccg gat gta ctg gaa acg gtg ccg 1152
Pro Ser Gly Arg Ser Lys Phe Ser Pro Asp Val Leu Glu Thr Val Pro
    370                 375                 380

gcg tca ccc gga ttg cgg cgg ccg tcg ctg ggc gca gtg gaa cgc cag 1200
Ala Ser Pro Gly Leu Arg Arg Pro Ser Leu Gly Ala Val Glu Arg Gln

385                 390                 395                 400

gat tcc ggc tat gac agc ctt gat ggg gtg gga tcg cga tcg ttc tcg     1248
Asp Ser Gly Tyr Asp Ser Leu Asp Gly Val Gly Ser Arg Ser Phe Ser
                405                 410                 415

ttg ggc gag gtg tcc gac atg gcc gcc gtg gaa gcg gcg gaa ctg gaa     1296
Leu Gly Glu Val Ser Asp Met Ala Ala Val Glu Ala Ala Glu Leu Glu
            420                 425                 430

atg acc cgg caa gtc ttg cac gcc ggg gcg cgg cag gac gat gcc gag     1344
Met Thr Arg Gln Val Leu His Ala Gly Ala Arg Gln Asp Asp Ala Glu
        435                 440                 445

ccg ggc gtg agc ggt gcg tcg gcg cac tgg ggg cag cgg gcg ctg cag     1392
Pro Gly Val Ser Gly Ala Ser Ala His Trp Gly Gln Arg Ala Leu Gln
    450                 455                 460

ggc gcc cag gcg gtg gcg gcg gcg cag cgg ctg gtt cat gcc att gcc     1440
Gly Ala Gln Ala Val Ala Ala Ala Gln Arg Leu Val His Ala Ile Ala
465                 470                 475                 480

ctg atg acg caa ttc ggc cgg gcc ggt tcc acc aac acg ccg cag gaa     1488
Leu Met Thr Gln Phe Gly Arg Ala Gly Ser Thr Asn Thr Pro Gln Glu
                485                 490                 495

gcg gcc tcg ttg tcg gcg gcc gtg ttc ggc ttg ggc gag gcc agc agc     1536
Ala Ala Ser Leu Ser Ala Ala Val Phe Gly Leu Gly Glu Ala Ser Ser
            500                 505                 510

gcc gtg gcc gaa acc gtg agc ggt ttt ttc cgc ggg tct tcg cgc tgg     1584
Ala Val Ala Glu Thr Val Ser Gly Phe Phe Arg Gly Ser Ser Arg Trp
        515                 520                 525

gcc ggc ggt ttc ggc gtg gct ggc ggc gcg atg gcg ctg gga ggc ggc     1632
Ala Gly Gly Phe Gly Val Ala Gly Gly Ala Met Ala Leu Gly Gly Gly
    530                 535                 540

atc gcc gcg gcc gtt ggc gcc ggg atg tcg ttg acc gat gac gcg ccg     1680
Ile Ala Ala Ala Val Gly Ala Gly Met Ser Leu Thr Asp Asp Ala Pro
545                 550                 555                 560

gcc gga cag aag gcc gcc gcc ggc gcc gag atc gcg ctg cag ttg aca     1728
Ala Gly Gln Lys Ala Ala Ala Gly Ala Glu Ile Ala Leu Gln Leu Thr
                565                 570                 575

ggt gga acg gtc gag ctg gct tct tcc atc gcg ttg gcg ctg gcc gcg     1776
Gly Gly Thr Val Glu Leu Ala Ser Ser Ile Ala Leu Ala Leu Ala Ala
            580                 585                 590

gcg cgc ggc gtg acc agc ggc ttg cag gtg gcc ggg gcg tcg gcc ggg     1824
Ala Arg Gly Val Thr Ser Gly Leu Gln Val Ala Gly Ala Ser Ala Gly
        595                 600                 605

gcg gct gcc ggc gca ttg gcc gcg gcg ctc agt ccc atg gag atc tac     1872
Ala Ala Ala Gly Ala Leu Ala Ala Ala Leu Ser Pro Met Glu Ile Tyr
    610                 615                 620

ggc ctg gtg cag caa tcg cac tat gcg gat cag ctg gac aag ctg gcg     1920
Gly Leu Val Gln Gln Ser His Tyr Ala Asp Gln Leu Asp Lys Leu Ala
625                 630                 635                 640

cag gaa tcg agc gca tac ggt tac gag ggc gac gcc ttg ctg gcc cag     1968

Gln Glu Ser Ser Ala Tyr Gly Tyr Glu Gly Asp Ala Leu Leu Ala Gln
                645                 650                 655

ctg tat cgc gac aag acg gcc gcc gag ggc gcc gtc gcc ggc gtc tcc      2016
Leu Tyr Arg Asp Lys Thr Ala Ala Glu Gly Ala Val Ala Gly Val Ser
            660                 665                 670

gcc gtc ctg agc acg gtg ggg gcg gcg gtg tcg atc gcc gcg gcg gcc      2064
Ala Val Leu Ser Thr Val Gly Ala Ala Val Ser Ile Ala Ala Ala Ala
        675                 680                 685

agc gtg gta ggg gcc ccg gtg gcg gtg gtc act tcc ttg ctg acc ggg      2112
Ser Val Val Gly Ala Pro Val Ala Val Val Thr Ser Leu Leu Thr Gly
    690                 695                 700

gct ctc aac ggc atc ctg cgc ggc gtg cag cag ccc atc atc gaa aag      2160
Ala Leu Asn Gly Ile Leu Arg Gly Val Gln Gln Pro Ile Ile Glu Lys
705                 710                 715                 720

ctg gcc aac gat tac gct cgc aag atc gac gag ctg ggc ggg ccg caa      2208
Leu Ala Asn Asp Tyr Ala Arg Lys Ile Asp Glu Leu Gly Gly Pro Gln
                725                 730                 735

gcg tac ttc gag aaa aac ctg cag gcg cgt cac gaa caa ctg gcc aat      2256
Ala Tyr Phe Glu Lys Asn Leu Gln Ala Arg His Glu Gln Leu Ala Asn
            740                 745                 750

tcg gac ggc cta cgg aaa atg ctg gcc gac ctg cag gcc ggt tgg aac      2304
Ser Asp Gly Leu Arg Lys Met Leu Ala Asp Leu Gln Ala Gly Trp Asn
        755                 760                 765

gcc agc agc gtg atc ggg gtg cag acg aca gag atc tcc aag tcg gcg      2352
Ala Ser Ser Val Ile Gly Val Gln Thr Thr Glu Ile Ser Lys Ser Ala
    770                 775                 780

ctc gaa ctg gcc gcc att acc ggc aac gcg gac aac ctg aaa tcc gtc      2400
Leu Glu Leu Ala Ala Ile Thr Gly Asn Ala Asp Asn Leu Lys Ser Val
785                 790                 795                 800

gac gtg ttc gtg gac cgc ttc gtc cag ggc gag cgg gtg gcc ggc cag      2448
Asp Val Phe Val Asp Arg Phe Val Gln Gly Glu Arg Val Ala Gly Gln
                805                 810                 815

ccg gtg gtc ctc gac gtc gcc gcc ggc ggc atc gat atc gcc agc cgc      2496
Pro Val Val Leu Asp Val Ala Ala Gly Gly Ile Asp Ile Ala Ser Arg
            820                 825                 830

aag ggc gag cgg ccg gcg ctg acg ttc atc acg ccg ctg gcc gcg cca      2544
Lys Gly Glu Arg Pro Ala Leu Thr Phe Ile Thr Pro Leu Ala Ala Pro
        835                 840                 845

gga gaa gag cag cgc cgg cgc acg aaa acg ggc aag agc gaa ttc acc      2592
Gly Glu Glu Gln Arg Arg Arg Thr Lys Thr Gly Lys Ser Glu Phe Thr
    850                 855                 860

aca ttc gtc gag atc gtg ggc aag cag gac cgc tgg cgc atc cgg gac      2640
Thr Phe Val Glu Ile Val Gly Lys Gln Asp Arg Trp Arg Ile Arg Asp
865                 870                 875                 880

ggc gcg gcc gac acc acc atc gat ctg gcc aag gtg gtg tcg caa ctg      2688
Gly Ala Ala Asp Thr Thr Ile Asp Leu Ala Lys Val Val Ser Gln Leu
                885                 890                 895

gtc gac gcc aat ggc gtg ctc aag cac agc atc aaa ctg gat gtg atc 2736
Val Asp Ala Asn Gly Val Leu Lys His Ser Ile Lys Leu Asp Val Ile
            900                 905                 910

ggc gga gat ggc gat gac gtc gtg ctt gcc aat gct tcg cgc atc cat 2784
Gly Gly Asp Gly Asp Asp Val Val Leu Ala Asn Ala Ser Arg Ile His
        915                 920                 925

tat gac ggc ggc gcg ggc acc aac acg gtc agc tat gcc gcc ctg ggt 2832
Tyr Asp Gly Gly Ala Gly Thr Asn Thr Val Ser Tyr Ala Ala Leu Gly
    930                 935                 940

cga cag gat tcc att acc gtg tcc gcc gac ggg gaa cgt ttc aac gtg 2880
Arg Gln Asp Ser Ile Thr Val Ser Ala Asp Gly Glu Arg Phe Asn Val
945                 950                 955                 960

cgc aag cag ttg aac aac gcc aac gtg tat cgc gaa ggc gtg gct acc 2928
Arg Lys Gln Leu Asn Asn Ala Asn Val Tyr Arg Glu Gly Val Ala Thr
                965                 970                 975

cag aca acc gcc tac ggc aag cgc acg gag aat gtc caa tac cgc cat 2976
Gln Thr Thr Ala Tyr Gly Lys Arg Thr Glu Asn Val Gln Tyr Arg His
            980                 985                 990

gtc gag ctg gcc cgt gtc ggg caa ctg gtg gag gtc gac acg ctc gag 3024
Val Glu Leu Ala Arg Val Gly Gln Leu Val Glu Val Asp Thr Leu Glu
        995                 1000                1005

cat gtg cag cac atc atc ggc ggg gcc ggc aac gat tcg atc acc 3069
His Val Gln His Ile Ile Gly Gly Ala Gly Asn Asp Ser Ile Thr
    1010                1015                1020

ggc aat gcg cac gac aac ttc cta gcc ggc ggg tcg ggc gac gac 3114
Gly Asn Ala His Asp Asn Phe Leu Ala Gly Gly Ser Gly Asp Asp
    1025                1030                1035

agg ctg gat ggc ggc gcc ggc aac gac acc ctg gtt ggc ggc gag 3159
Arg Leu Asp Gly Gly Ala Gly Asn Asp Thr Leu Val Gly Gly Glu
    1040                1045                1050

ggc caa aac acg gtc atc ggc ggc gcc ggc gac gac gta ttc ctg 3204
Gly Gln Asn Thr Val Ile Gly Gly Ala Gly Asp Asp Val Phe Leu
    1055                1060                1065

cag gac ctg ggg gta tgg agc aac cag ctc gat ggc ggc gcg ggc 3249
Gln Asp Leu Gly Val Trp Ser Asn Gln Leu Asp Gly Gly Ala Gly
    1070                1075                1080

gtc gat acc gtg aag tac aac gtg cac cag cct tcc gag gag cgc 3294
Val Asp Thr Val Lys Tyr Asn Val His Gln Pro Ser Glu Glu Arg
    1085                1090                1095

ctc gaa cgc atg ggc gac acg ggc atc cat gcc gat ctt caa aag 3339
Leu Glu Arg Met Gly Asp Thr Gly Ile His Ala Asp Leu Gln Lys
    1100                1105                1110

ggc acg gtc gag aag tgg ccg gcc ctg aac ctg ttc agc gtc gac 3384
Gly Thr Val Glu Lys Trp Pro Ala Leu Asn Leu Phe Ser Val Asp
    1115                1120                1125

cat gtc aag aat atc gag aat ctg cac ggc tcc cgc ctg aac gac 3429
His Val Lys Asn Ile Glu Asn Leu His Gly Ser Arg Leu Asn Asp
    1130                1135                1140

cgc atc gcc ggc gac gac cag gac aac gag ctc tgg ggc cac gat 3474
Arg Ile Ala Gly Asp Asp Gln Asp Asn Glu Leu Trp Gly His Asp
    1145                1150                1155

ggc aac gac acg ata cgc ggc cgg ggc ggc gac gac atc ctg cgc 3519
Gly Asn Asp Thr Ile Arg Gly Arg Gly Gly Asp Asp Ile Leu Arg
    1160                1165                1170

ggc ggc ctg ggc ctg gac acg ctg tat ggc gag gac ggc aac gac 3564
Gly Gly Leu Gly Leu Asp Thr Leu Tyr Gly Glu Asp Gly Asn Asp
    1175                1180                1185

atc ttc ctg cag gac gac gag acc gtc agc gat gac atc gac ggc 3609
Ile Phe Leu Gln Asp Asp Glu Thr Val Ser Asp Asp Ile Asp Gly
    1190                1195                1200

ggc gcg ggg ctg gac acc gtc gac tac tcc gcc atg atc cat cca 3654
Gly Ala Gly Leu Asp Thr Val Asp Tyr Ser Ala Met Ile His Pro
    1205                1210                1215

ggc agg atc gtt gcg ccg cat gaa tac ggc ttc ggg atc gag gcg 3699
Gly Arg Ile Val Ala Pro His Glu Tyr Gly Phe Gly Ile Glu Ala
    1220                1225                1230

gac ctg tcc agg gaa tgg gtg cgc aag gcg tcc gcg ctg ggc gtg 3744
Asp Leu Ser Arg Glu Trp Val Arg Lys Ala Ser Ala Leu Gly Val
    1235                1240                1245

gac tat tac gat aat gtc cgc aat gtc gaa aac gtc atc ggt acg 3789
Asp Tyr Tyr Asp Asn Val Arg Asn Val Glu Asn Val Ile Gly Thr
    1250                1255                1260

agc atg aag gat gtg ctc atc ggc gac gcg caa gcc aat acc ctg 3834
Ser Met Lys Asp Val Leu Ile Gly Asp Ala Gln Ala Asn Thr Leu
    1265                1270                1275

atg ggc cag ggc ggc gac gat acc gtg cgc ggc ggc gac ggc gat 3879
Met Gly Gln Gly Gly Asp Asp Thr Val Arg Gly Gly Asp Gly Asp
    1280                1285                1290

gat ctg ctg ttc ggc ggc gac ggc aac gac atg ctg tat ggc gac 3924
Asp Leu Leu Phe Gly Gly Asp Gly Asn Asp Met Leu Tyr Gly Asp
    1295                1300                1305

gcc ggc aac gac acc ctc tac ggg ggg ctg ggc gac gat acc ctt 3969
Ala Gly Asn Asp Thr Leu Tyr Gly Gly Leu Gly Asp Asp Thr Leu
    1310                1315                1320

gaa ggc ggc gcg ggc aac gat tgg ttc ggc cag acg cag gcg cgc 4014
Glu Gly Gly Ala Gly Asn Asp Trp Phe Gly Gln Thr Gln Ala Arg
    1325                1330                1335

gag cat gac gtg ctg cgc ggc gga gat ggg gtg gat acc gtc gat 4059
Glu His Asp Val Leu Arg Gly Gly Asp Gly Val Asp Thr Val Asp
    1340                1345                1350

tac agc cag acc ggc gcg cat gcc ggc att gcc gcg ggt cgc atc 4104
Tyr Ser Gln Thr Gly Ala His Ala Gly Ile Ala Ala Gly Arg Ile
    1355                1360                1365

ggg ctg ggc atc ctg gct gac ctg ggc gcc ggc cgc gtc gac aag 4149
Gly Leu Gly Ile Leu Ala Asp Leu Gly Ala Gly Arg Val Asp Lys

1370                    1375                    1380

ctg ggc gag gcc ggc agc agc gcc tac gat acg gtt tcc ggt atc          4194
Leu Gly Glu Ala Gly Ser Ser Ala Tyr Asp Thr Val Ser Gly Ile
    1385                    1390                    1395

gag aac gtg gtg ggc acg gaa ctg gcc gac cgc atc acg ggc gat          4239
Glu Asn Val Val Gly Thr Glu Leu Ala Asp Arg Ile Thr Gly Asp
    1400                    1405                    1410

gcg cag gcc aac gtg ctg cgc ggc gcg ggt ggc gcc gac gtg ctt          4284
Ala Gln Ala Asn Val Leu Arg Gly Ala Gly Gly Ala Asp Val Leu
    1415                    1420                    1425

gcg ggc ggc gag ggc gac gat gtg ctg ctg ggc ggc gac ggc gac          4329
Ala Gly Gly Glu Gly Asp Asp Val Leu Leu Gly Gly Asp Gly Asp
    1430                    1435                    1440

gac cag ctg tcg ggc gac gcc gga cgc gat cgc ttg tac ggc gaa          4374
Asp Gln Leu Ser Gly Asp Ala Gly Arg Asp Arg Leu Tyr Gly Glu
    1445                    1450                    1455

gcc ggt gac gac tgg ttc ttc cag gat gcc gcc aat gcc ggc aat          4419
Ala Gly Asp Asp Trp Phe Phe Gln Asp Ala Ala Asn Ala Gly Asn
    1460                    1465                    1470

ctg ctc gac ggc ggc gac ggc cgc gat acc gtg gat ttc agc ggc          4464
Leu Leu Asp Gly Gly Asp Gly Arg Asp Thr Val Asp Phe Ser Gly
    1475                    1480                    1485

ccg ggc cgg ggc ctc gac gcc ggc gca aag ggc gta ttc ctg agc          4509
Pro Gly Arg Gly Leu Asp Ala Gly Ala Lys Gly Val Phe Leu Ser
    1490                    1495                    1500

ttg ggc aag ggg ttc gcc agc ctg atg gac gaa ccc gaa acc agc          4554
Leu Gly Lys Gly Phe Ala Ser Leu Met Asp Glu Pro Glu Thr Ser
    1505                    1510                    1515

aac gtg ttg cgc aat atc gag aac gcc gtg ggc agc gcg cgt gat          4599
Asn Val Leu Arg Asn Ile Glu Asn Ala Val Gly Ser Ala Arg Asp
    1520                    1525                    1530

gac gtg ctg atc ggc gac gca ggc gcc aac gtc ctc aat ggc ctg          4644
Asp Val Leu Ile Gly Asp Ala Gly Ala Asn Val Leu Asn Gly Leu
    1535                    1540                    1545

gcg ggc aac gac gtg ctg tcc ggc ggc gct ggc gac gat gtg ctg          4689
Ala Gly Asn Asp Val Leu Ser Gly Gly Ala Gly Asp Asp Val Leu
    1550                    1555                    1560

ctg ggc gac gag ggc tcg gac ctg ctc agc ggc gat gcg ggc aac          4734
Leu Gly Asp Glu Gly Ser Asp Leu Leu Ser Gly Asp Ala Gly Asn
    1565                    1570                    1575

gac gat ctg ttc ggc ggg cag ggc gat gat act tat ctg ttc ggg          4779
Asp Asp Leu Phe Gly Gly Gln Gly Asp Asp Thr Tyr Leu Phe Gly
    1580                    1585                    1590

gtc ggg tac ggg cac gac acg atc tac gaa tcg ggc ggc ggc cat          4824
Val Gly Tyr Gly His Asp Thr Ile Tyr Glu Ser Gly Gly Gly His
    1595                    1600                    1605

gac acc atc cgc atc aac gcg ggg gcg gac cag ctg tgg ttc gcg          4869

Asp Thr Ile Arg Ile Asn Ala Gly Ala Asp Gln Leu Trp Phe Ala
    1610                1615                1620

cgc cag ggc aac gac ctg gag atc cgc att ctc ggc acc gac gat        4914
Arg Gln Gly Asn Asp Leu Glu Ile Arg Ile Leu Gly Thr Asp Asp
    1625                1630                1635

gca ctt acc gtg cac gac tgg tat cgc gac gcc gat cac cgg gtg        4959
Ala Leu Thr Val His Asp Trp Tyr Arg Asp Ala Asp His Arg Val
    1640                1645                1650

gaa atc atc cat gcc gcc aac cag gcg gta gac cag gca ggc atc        5004
Glu Ile Ile His Ala Ala Asn Gln Ala Val Asp Gln Ala Gly Ile
    1655                1660                1665

gaa aag ctg gtc gag gca atg gcg cag tat ccg gac ccc ggc gcg        5049
Glu Lys Leu Val Glu Ala Met Ala Gln Tyr Pro Asp Pro Gly Ala
    1670                1675                1680

gcg gcg gct gcc ccg ccg gcg gcg cgc gtg ccg gac acg ctg atg        5094
Ala Ala Ala Ala Pro Pro Ala Ala Arg Val Pro Asp Thr Leu Met
    1685                1690                1695

cag tcc ctg gct gtc aac tgg cgc tga                                5121
Gln Ser Leu Ala Val Asn Trp Arg
    1700                1705


<210>  2
<211>  1706
<212>  PRT
<213>  Bordetella pertussis (wild type CyaA)

<400>  2

Met Gln Gln Ser His Gln Ala Gly Tyr Ala Asn Ala Ala Asp Arg Glu
1               5                   10                  15


Ser Gly Ile Pro Ala Ala Val Leu Asp Gly Ile Lys Ala Val Ala Lys
            20                  25                  30


Glu Lys Asn Ala Thr Leu Met Phe Arg Leu Val Asn Pro His Ser Thr
        35                  40                  45


Ser Leu Ile Ala Glu Gly Val Ala Thr Lys Gly Leu Gly Val His Ala
    50                  55                  60


Lys Ser Ser Asp Trp Gly Leu Gln Ala Gly Tyr Ile Pro Val Asn Pro
65                  70                  75                  80


Asn Leu Ser Lys Leu Phe Gly Arg Ala Pro Glu Val Ile Ala Arg Ala
                85                  90                  95


Asp Asn Asp Val Asn Ser Ser Leu Ala His Gly His Thr Ala Val Asp
            100                 105                 110

Leu Thr Leu Ser Lys Glu Arg Leu Asp Tyr Leu Arg Gln Ala Gly Leu
        115                 120                 125

Val Thr Gly Met Ala Asp Gly Val Val Ala Ser Asn His Ala Gly Tyr
    130                 135                 140

Glu Gln Phe Glu Phe Arg Val Lys Glu Thr Ser Asp Gly Arg Tyr Ala
145                 150                 155                 160

Val Gln Tyr Arg Arg Lys Gly Gly Asp Asp Phe Glu Ala Val Lys Val
                165                 170                 175

Ile Gly Asn Ala Ala Gly Ile Pro Leu Thr Ala Asp Ile Asp Met Phe
            180                 185                 190

Ala Ile Met Pro His Leu Ser Asn Phe Arg Asp Ser Ala Arg Ser Ser
        195                 200                 205

Val Thr Ser Gly Asp Ser Val Thr Asp Tyr Leu Ala Arg Thr Arg Arg
    210                 215                 220

Ala Ala Ser Glu Ala Thr Gly Gly Leu Asp Arg Glu Arg Ile Asp Leu
225                 230                 235                 240

Leu Trp Lys Ile Ala Arg Ala Gly Ala Arg Ser Ala Val Gly Thr Glu
                245                 250                 255

Ala Arg Arg Gln Phe Arg Tyr Asp Gly Asp Met Asn Ile Gly Val Ile
            260                 265                 270

Thr Asp Phe Glu Leu Glu Val Arg Asn Ala Leu Asn Arg Arg Ala His
        275                 280                 285

Ala Val Gly Ala Gln Asp Val Val Gln His Gly Thr Glu Gln Asn Asn
    290                 295                 300

Pro Phe Pro Glu Ala Asp Glu Lys Ile Phe Val Val Ser Ala Thr Gly
305                 310                 315                 320

Glu Ser Gln Met Leu Thr Arg Gly Gln Leu Lys Glu Tyr Ile Gly Gln
                325                 330                 335

Gln Arg Gly Glu Gly Tyr Val Phe Tyr Glu Asn Arg Ala Tyr Gly Val
            340                 345                 350

Ala Gly Lys Ser Leu Phe Asp Asp Gly Leu Gly Ala Ala Pro Gly Val
        355                 360                 365

Pro Ser Gly Arg Ser Lys Phe Ser Pro Asp Val Leu Glu Thr Val Pro
    370                 375                 380

Ala Ser Pro Gly Leu Arg Arg Pro Ser Leu Gly Ala Val Glu Arg Gln
385                 390                 395                 400

Asp Ser Gly Tyr Asp Ser Leu Asp Gly Val Gly Ser Arg Ser Phe Ser
                405                 410                 415

Leu Gly Glu Val Ser Asp Met Ala Ala Val Glu Ala Ala Glu Leu Glu
            420                 425                 430

Met Thr Arg Gln Val Leu His Ala Gly Ala Arg Gln Asp Asp Ala Glu
        435                 440                 445

Pro Gly Val Ser Gly Ala Ser Ala His Trp Gly Gln Arg Ala Leu Gln
    450                 455                 460

Gly Ala Gln Ala Val Ala Ala Ala Gln Arg Leu Val His Ala Ile Ala
465                 470                 475                 480

Leu Met Thr Gln Phe Gly Arg Ala Gly Ser Thr Asn Thr Pro Gln Glu
                485                 490                 495

Ala Ala Ser Leu Ser Ala Ala Val Phe Gly Leu Gly Glu Ala Ser Ser
            500                 505                 510

Ala Val Ala Glu Thr Val Ser Gly Phe Phe Arg Gly Ser Ser Arg Trp
        515                 520                 525

Ala Gly Gly Phe Gly Val Ala Gly Gly Ala Met Ala Leu Gly Gly Gly
    530                 535                 540

Ile Ala Ala Ala Val Gly Ala Gly Met Ser Leu Thr Asp Asp Ala Pro
545                 550                 555                 560

Ala Gly Gln Lys Ala Ala Ala Gly Ala Glu Ile Ala Leu Gln Leu Thr
                565                 570                 575

Gly Gly Thr Val Glu Leu Ala Ser Ser Ile Ala Leu Ala Leu Ala Ala
            580                 585                 590

Ala Arg Gly Val Thr Ser Gly Leu Gln Val Ala Gly Ala Ser Ala Gly
        595                 600                 605

Ala Ala Ala Gly Ala Leu Ala Ala Ala Leu Ser Pro Met Glu Ile Tyr
    610                 615                 620

Gly Leu Val Gln Gln Ser His Tyr Ala Asp Gln Leu Asp Lys Leu Ala
625                 630                 635                 640

Gln Glu Ser Ser Ala Tyr Gly Tyr Glu Gly Asp Ala Leu Leu Ala Gln
                645                 650                 655

Leu Tyr Arg Asp Lys Thr Ala Ala Glu Gly Ala Val Ala Gly Val Ser
            660                 665                 670

Ala Val Leu Ser Thr Val Gly Ala Ala Val Ser Ile Ala Ala Ala Ala
        675                 680                 685

Ser Val Val Gly Ala Pro Val Ala Val Val Thr Ser Leu Leu Thr Gly
    690                 695                 700

Ala Leu Asn Gly Ile Leu Arg Gly Val Gln Gln Pro Ile Ile Glu Lys
705                 710                 715                 720

Leu Ala Asn Asp Tyr Ala Arg Lys Ile Asp Glu Leu Gly Gly Pro Gln
                725                 730                 735

Ala Tyr Phe Glu Lys Asn Leu Gln Ala Arg His Glu Gln Leu Ala Asn
            740                 745                 750

Ser Asp Gly Leu Arg Lys Met Leu Ala Asp Leu Gln Ala Gly Trp Asn
        755                 760                 765

Ala Ser Ser Val Ile Gly Val Gln Thr Thr Glu Ile Ser Lys Ser Ala
    770                 775                 780

Leu Glu Leu Ala Ala Ile Thr Gly Asn Ala Asp Asn Leu Lys Ser Val
785                 790                 795                 800

Asp Val Phe Val Asp Arg Phe Val Gln Gly Glu Arg Val Ala Gly Gln
                805                 810                 815

Pro Val Val Leu Asp Val Ala Ala Gly Gly Ile Asp Ile Ala Ser Arg
            820                 825                 830

Lys Gly Glu Arg Pro Ala Leu Thr Phe Ile Thr Pro Leu Ala Ala Pro
        835                 840                 845

Gly Glu Glu Gln Arg Arg Arg Thr Lys Thr Gly Lys Ser Glu Phe Thr
    850                 855                 860

Thr Phe Val Glu Ile Val Gly Lys Gln Asp Arg Trp Arg Ile Arg Asp

865                 870                 875                 880

Gly Ala Ala Asp Thr Thr Ile Asp Leu Ala Lys Val Val Ser Gln Leu
                885                 890                 895

Val Asp Ala Asn Gly Val Leu Lys His Ser Ile Lys Leu Asp Val Ile
            900                 905                 910

Gly Gly Asp Gly Asp Asp Val Val Leu Ala Asn Ala Ser Arg Ile His
        915                 920                 925

Tyr Asp Gly Gly Ala Gly Thr Asn Thr Val Ser Tyr Ala Ala Leu Gly
    930                 935                 940

Arg Gln Asp Ser Ile Thr Val Ser Ala Asp Gly Glu Arg Phe Asn Val
945                 950                 955                 960

Arg Lys Gln Leu Asn Asn Ala Asn Val Tyr Arg Glu Gly Val Ala Thr
                965                 970                 975

Gln Thr Thr Ala Tyr Gly Lys Arg Thr Glu Asn Val Gln Tyr Arg His
            980                 985                 990

Val Glu Leu Ala Arg Val Gly Gln  Leu Val Glu Val Asp  Thr Leu Glu
        995                 1000                 1005

His Val  Gln His Ile Ile Gly  Gly Ala Gly Asn Asp  Ser Ile Thr
    1010                 1015                 1020

Gly Asn  Ala His Asp Asn Phe  Leu Ala Gly Gly Ser  Gly Asp Asp
    1025                 1030                 1035

Arg Leu  Asp Gly Gly Ala Gly  Asn Asp Thr Leu Val  Gly Gly Glu
    1040                 1045                 1050

Gly Gln  Asn Thr Val Ile Gly  Gly Ala Gly Asp Asp  Val Phe Leu
    1055                 1060                 1065

Gln Asp  Leu Gly Val Trp Ser  Asn Gln Leu Asp Gly  Gly Ala Gly
    1070                 1075                 1080

Val Asp  Thr Val Lys Tyr Asn  Val His Gln Pro Ser  Glu Glu Arg
    1085                 1090                 1095

Leu Glu  Arg Met Gly Asp Thr  Gly Ile His Ala Asp  Leu Gln Lys
    1100                 1105                 1110

Gly Thr Val Glu Lys Trp Pro Ala Leu Asn Leu Phe Ser Val Asp
    1115                1120                1125

His Val Lys Asn Ile Glu Asn Leu His Gly Ser Arg Leu Asn Asp
    1130                1135                1140

Arg Ile Ala Gly Asp Asp Gln Asp Asn Glu Leu Trp Gly His Asp
    1145                1150                1155

Gly Asn Asp Thr Ile Arg Gly Arg Gly Gly Asp Asp Ile Leu Arg
    1160                1165                1170

Gly Gly Leu Gly Leu Asp Thr Leu Tyr Gly Glu Asp Gly Asn Asp
    1175                1180                1185

Ile Phe Leu Gln Asp Asp Glu Thr Val Ser Asp Asp Ile Asp Gly
    1190                1195                1200

Gly Ala Gly Leu Asp Thr Val Asp Tyr Ser Ala Met Ile His Pro
    1205                1210                1215

Gly Arg Ile Val Ala Pro His Glu Tyr Gly Phe Gly Ile Glu Ala
    1220                1225                1230

Asp Leu Ser Arg Glu Trp Val Arg Lys Ala Ser Ala Leu Gly Val
    1235                1240                1245

Asp Tyr Tyr Asp Asn Val Arg Asn Val Glu Asn Val Ile Gly Thr
    1250                1255                1260

Ser Met Lys Asp Val Leu Ile Gly Asp Ala Gln Ala Asn Thr Leu
    1265                1270                1275

Met Gly Gln Gly Gly Asp Asp Thr Val Arg Gly Gly Asp Gly Asp
    1280                1285                1290

Asp Leu Leu Phe Gly Gly Asp Gly Asn Asp Met Leu Tyr Gly Asp
    1295                1300                1305

Ala Gly Asn Asp Thr Leu Tyr Gly Gly Leu Gly Asp Asp Thr Leu
    1310                1315                1320

Glu Gly Gly Ala Gly Asn Asp Trp Phe Gly Gln Thr Gln Ala Arg
    1325                1330                1335

Glu His Asp Val Leu Arg Gly Gly Asp Gly Val Asp Thr Val Asp
    1340                1345                1350

Tyr Ser Gln Thr Gly Ala His Ala Gly Ile Ala Ala Gly Arg Ile
    1355                1360                1365

Gly Leu Gly Ile Leu Ala Asp Leu Gly Ala Gly Arg Val Asp Lys
    1370                1375                1380

Leu Gly Glu Ala Gly Ser Ser Ala Tyr Asp Thr Val Ser Gly Ile
    1385                1390                1395

Glu Asn Val Val Gly Thr Glu Leu Ala Asp Arg Ile Thr Gly Asp
    1400                1405                1410

Ala Gln Ala Asn Val Leu Arg Gly Ala Gly Gly Ala Asp Val Leu
    1415                1420                1425

Ala Gly Gly Glu Gly Asp Asp Val Leu Leu Gly Gly Asp Gly Asp
    1430                1435                1440

Asp Gln Leu Ser Gly Asp Ala Gly Arg Asp Arg Leu Tyr Gly Glu
    1445                1450                1455

Ala Gly Asp Asp Trp Phe Phe Gln Asp Ala Ala Asn Ala Gly Asn
    1460                1465                1470

Leu Leu Asp Gly Gly Asp Gly Arg Asp Thr Val Asp Phe Ser Gly
    1475                1480                1485

Pro Gly Arg Gly Leu Asp Ala Gly Ala Lys Gly Val Phe Leu Ser
    1490                1495                1500

Leu Gly Lys Gly Phe Ala Ser Leu Met Asp Glu Pro Glu Thr Ser
    1505                1510                1515

Asn Val Leu Arg Asn Ile Glu Asn Ala Val Gly Ser Ala Arg Asp
    1520                1525                1530

Asp Val Leu Ile Gly Asp Ala Gly Ala Asn Val Leu Asn Gly Leu
    1535                1540                1545

Ala Gly Asn Asp Val Leu Ser Gly Gly Ala Gly Asp Asp Val Leu
    1550                1555                1560

Leu Gly Asp Glu Gly Ser Asp Leu Leu Ser Gly Asp Ala Gly Asn
    1565                1570                1575

Asp Asp Leu Phe Gly Gly Gln Gly Asp Asp Thr Tyr Leu Phe Gly
    1580                1585                1590

Val Gly Tyr Gly His Asp Thr Ile Tyr Glu Ser Gly Gly Gly His
    1595                1600                1605

Asp Thr Ile Arg Ile Asn Ala Gly Ala Asp Gln Leu Trp Phe Ala
    1610                1615                1620

Arg Gln Gly Asn Asp Leu Glu Ile Arg Ile Leu Gly Thr Asp Asp
    1625                1630                1635

Ala Leu Thr Val His Asp Trp Tyr Arg Asp Ala Asp His Arg Val
    1640                1645                1650

Glu Ile Ile His Ala Ala Asn Gln Ala Val Asp Gln Ala Gly Ile
    1655                1660                1665

Glu Lys Leu Val Glu Ala Met Ala Gln Tyr Pro Asp Pro Gly Ala
    1670                1675                1680

Ala Ala Ala Ala Pro Pro Ala Ala Arg Val Pro Asp Thr Leu Met
    1685                1690                1695

Gln Ser Leu Ala Val Asn Trp Arg
    1700                1705

<210> 3
<211> 5121
<212> DNA
<213> Bordetella hinzii (wild type CyaA)

<220>
<221> CDS
<222> (1)..(5121)

<400> 3
atg cag caa tcg cat cag gct ggt tac gca aac gcc gcc gac cgg gag 48
Met Gln Gln Ser His Gln Ala Gly Tyr Ala Asn Ala Ala Asp Arg Glu
1               5                   10                  15

tct ggc atc ccc gca gcc gta ctc gat ggc atc aag gcc gtg gcg aag 96
Ser Gly Ile Pro Ala Ala Val Leu Asp Gly Ile Lys Ala Val Ala Lys
            20                  25                  30

gaa aaa aac gcc aca ttg atg ttc cgc ctg gtc aac ccc cat tcc acc 144
Glu Lys Asn Ala Thr Leu Met Phe Arg Leu Val Asn Pro His Ser Thr
        35                  40                  45

agc ctg att gcc gaa ggg gtg gcc acc aaa gga ttg ggc gtg cac gcc 192
Ser Leu Ile Ala Glu Gly Val Ala Thr Lys Gly Leu Gly Val His Ala
    50                  55                  60

aag tcg tcc gat tgg ggg ttg cag gcg ggc tac att ccc gtc aac ccg 240
Lys Ser Ser Asp Trp Gly Leu Gln Ala Gly Tyr Ile Pro Val Asn Pro

65                  70                  75                  80

aat ctt tcc aaa ctg ttc ggc cgt gcg ccc gag gtg atc gcg cgg gcc      288
Asn Leu Ser Lys Leu Phe Gly Arg Ala Pro Glu Val Ile Ala Arg Ala
                85                  90                  95

gac aac gac gtc aac agc agc ctg gcg cat ggc cat acc gcg gtc gac      336
Asp Asn Asp Val Asn Ser Ser Leu Ala His Gly His Thr Ala Val Asp
            100                 105                 110

ctg acg ctg tcg aaa gag cgg ctt gac tat ctg cgg caa gcg ggc ctg      384
Leu Thr Leu Ser Lys Glu Arg Leu Asp Tyr Leu Arg Gln Ala Gly Leu
        115                 120                 125

gtc acc ggc atg gcc gat ggc gtg gtc gcg agc aac cac gca ggc tac      432
Val Thr Gly Met Ala Asp Gly Val Val Ala Ser Asn His Ala Gly Tyr
    130                 135                 140

gag cag ttc gag ttt cgc gtg aag gaa acc tcg gac ggg cgc tat gcc      480
Glu Gln Phe Glu Phe Arg Val Lys Glu Thr Ser Asp Gly Arg Tyr Ala
145                 150                 155                 160

gtg cag tat cgc cgc aag ggc ggc gac gat ttc gag gcg gtc aag gtg      528
Val Gln Tyr Arg Arg Lys Gly Gly Asp Asp Phe Glu Ala Val Lys Val
                165                 170                 175

atc ggc aat gcc gcc ggt att cca ctg acg gcg gat atc gac atg ttc      576
Ile Gly Asn Ala Ala Gly Ile Pro Leu Thr Ala Asp Ile Asp Met Phe
            180                 185                 190

gcc att atg ccg cat ctg tcc aac ttc cgc gac tcg gcg cgc agt tcg      624
Ala Ile Met Pro His Leu Ser Asn Phe Arg Asp Ser Ala Arg Ser Ser
        195                 200                 205

gtg acc agc ggc gat tcg gtg acc gat tac ctg gcg cgc acg cgg cgg      672
Val Thr Ser Gly Asp Ser Val Thr Asp Tyr Leu Ala Arg Thr Arg Arg
    210                 215                 220

gcc gcc agc gag gcc acg ggc ggc ctg gat cgc gaa cgc atc gac ttg      720
Ala Ala Ser Glu Ala Thr Gly Gly Leu Asp Arg Glu Arg Ile Asp Leu
225                 230                 235                 240

ttg tgg aaa atc gct cgc gcc ggc gcc cgt tcc gca gtg ggc acc gag      768
Leu Trp Lys Ile Ala Arg Ala Gly Ala Arg Ser Ala Val Gly Thr Glu
                245                 250                 255

gcg cgt cgc cag ttc cgc tac gac ggc gac atg aat atc ggc gtg atc      816
Ala Arg Arg Gln Phe Arg Tyr Asp Gly Asp Met Asn Ile Gly Val Ile
            260                 265                 270

acc gat ttc gag ctg gaa gtg cgc aat gcg ctg aac agg cgg gcg cac      864
Thr Asp Phe Glu Leu Glu Val Arg Asn Ala Leu Asn Arg Arg Ala His
        275                 280                 285

gcc gtc ggc gcg cag gac gtg gtc cag cat ggc act gag cag aac aat      912
Ala Val Gly Ala Gln Asp Val Val Gln His Gly Thr Glu Gln Asn Asn
    290                 295                 300

cct ttc ccg gag gca gat gag aag att ttc gtc gta tcg gcc acc ggt      960
Pro Phe Pro Glu Ala Asp Glu Lys Ile Phe Val Val Ser Ala Thr Gly
305                 310                 315                 320

gaa agc cag atg ctc acg cgc ggg caa ctg aag gaa tac att ggc cag     1008

Glu Ser Gln Met Leu Thr Arg Gly Gln Leu Lys Glu Tyr Ile Gly Gln
                325                 330                 335

cag cgc ggc gag ggc tat gtc ttc tac gag aac cgt gca tac ggc gtg      1056
Gln Arg Gly Glu Gly Tyr Val Phe Tyr Glu Asn Arg Ala Tyr Gly Val
            340                 345                 350

gcg ggg aaa agc ctg ttc gac gat ggg ctg gga gcc gcg ccc ggc gtg      1104
Ala Gly Lys Ser Leu Phe Asp Asp Gly Leu Gly Ala Ala Pro Gly Val
        355                 360                 365

ccg agc gga cgt tcg aag ttc tcg ccg gat gta ctg gaa acg gtg ccg      1152
Pro Ser Gly Arg Ser Lys Phe Ser Pro Asp Val Leu Glu Thr Val Pro
    370                 375                 380

gcg tca ccc gga ttg cgg cgg ccg tcg ctg ggc gca gtg gaa cgc cag      1200
Ala Ser Pro Gly Leu Arg Arg Pro Ser Leu Gly Ala Val Glu Arg Gln
385                 390                 395                 400

gat tcc ggc tat gac agc ctt gat ggg gtg gga tcg cga tcg ttc tcg      1248
Asp Ser Gly Tyr Asp Ser Leu Asp Gly Val Gly Ser Arg Ser Phe Ser
                405                 410                 415

ttg ggc gag gtg tcc gac atg gcc gcc gtg gaa gcg gcg gaa ctg gaa      1296
Leu Gly Glu Val Ser Asp Met Ala Ala Val Glu Ala Ala Glu Leu Glu
            420                 425                 430

atg acc cgg caa gtc ttg cac gcc ggg gcg cgg cag gac gat gcc gag      1344
Met Thr Arg Gln Val Leu His Ala Gly Ala Arg Gln Asp Asp Ala Glu
        435                 440                 445

ccg ggc gtg agc ggt gcg tcg gcg cac tgg ggg cag cgg gcg ctg cag      1392
Pro Gly Val Ser Gly Ala Ser Ala His Trp Gly Gln Arg Ala Leu Gln
    450                 455                 460

ggc gcc cag gcg gtg gcg gcg gcg cag cgg ctg gtt cat gcc att gcc      1440
Gly Ala Gln Ala Val Ala Ala Ala Gln Arg Leu Val His Ala Ile Ala
465                 470                 475                 480

ctg atg acg caa ttc ggc cgg gcc ggt tcc acc aac acg ccg cag gaa      1488
Leu Met Thr Gln Phe Gly Arg Ala Gly Ser Thr Asn Thr Pro Gln Glu
                485                 490                 495

gcg gcc tcg ttg tcg gcg gcc gtg ttc ggc ttg ggc gag gcc agc agc      1536
Ala Ala Ser Leu Ser Ala Ala Val Phe Gly Leu Gly Glu Ala Ser Ser
            500                 505                 510

gcc gtg gcc gaa acc gtg agc ggt ttt ttc cgc ggg tct tcg cgc tgg      1584
Ala Val Ala Glu Thr Val Ser Gly Phe Phe Arg Gly Ser Ser Arg Trp
        515                 520                 525

gcc ggc ggt ttc ggc gtg gct ggc ggc gcg atg gcg ctg gga ggc ggc      1632
Ala Gly Gly Phe Gly Val Ala Gly Gly Ala Met Ala Leu Gly Gly Gly
    530                 535                 540

atc gcc gcg gcc gtt ggc gcc ggg atg tcg ttg acc gat gac gcg ccg      1680
Ile Ala Ala Ala Val Gly Ala Gly Met Ser Leu Thr Asp Asp Ala Pro
545                 550                 555                 560

gcc gga cag aag gcc gcc gcc ggc gcc gag atc gcg ctg cag ttg aca      1728
Ala Gly Gln Lys Ala Ala Ala Gly Ala Glu Ile Ala Leu Gln Leu Thr
                565                 570                 575

ggt gga acg gtc gag ctg gct tct tcc atc gcg ttg gcg ctg gcc gcg 1776
Gly Gly Thr Val Glu Leu Ala Ser Ser Ile Ala Leu Ala Leu Ala Ala
            580                 585                 590

gcg cgc ggc gtg acc agc ggc ttg cag gtg gcc ggg gcg tcg gcc ggg 1824
Ala Arg Gly Val Thr Ser Gly Leu Gln Val Ala Gly Ala Ser Ala Gly
        595                 600                 605

gcg gct gcc ggc gca ttg gcc gcg gcg ctc agt ccc atg gag atc tac 1872
Ala Ala Ala Gly Ala Leu Ala Ala Ala Leu Ser Pro Met Glu Ile Tyr
    610                 615                 620

ggc ctg gtg cag caa tcg cac tat gcg gat cag ctg gac aag ctg gcg 1920
Gly Leu Val Gln Gln Ser His Tyr Ala Asp Gln Leu Asp Lys Leu Ala
625                 630                 635                 640

cag gaa tcg agc gca tac ggt tac gag ggc gac gcc ttg ctg gcc cag 1968
Gln Glu Ser Ser Ala Tyr Gly Tyr Glu Gly Asp Ala Leu Leu Ala Gln
                645                 650                 655

ctg tat cgc gac aag acg gcc gcc gag ggc gcc gtc gcc ggc gtc tcc 2016
Leu Tyr Arg Asp Lys Thr Ala Ala Glu Gly Ala Val Ala Gly Val Ser
            660                 665                 670

gcc gtc ctg agc acg gtg ggg gcg gcg gtg tcg atc gcc gcg gcg gcc 2064
Ala Val Leu Ser Thr Val Gly Ala Ala Val Ser Ile Ala Ala Ala Ala
        675                 680                 685

agc gtg gta ggg gcc ccg gtg gcg gtg gtc act tcc ttg ctg acc ggg 2112
Ser Val Val Gly Ala Pro Val Ala Val Val Thr Ser Leu Leu Thr Gly
    690                 695                 700

gct ctc aac ggc atc ctg cgc ggc gtg cag cag ccc atc atc gaa aag 2160
Ala Leu Asn Gly Ile Leu Arg Gly Val Gln Gln Pro Ile Ile Glu Lys
705                 710                 715                 720

ctg gcc aac gat tac gct cgc aag atc gac gag ctg ggc ggg ccg caa 2208
Leu Ala Asn Asp Tyr Ala Arg Lys Ile Asp Glu Leu Gly Gly Pro Gln
                725                 730                 735

gcg tac ttc gag aaa aac ctg cag gcg cgt cac gaa caa ctg gcc aat 2256
Ala Tyr Phe Glu Lys Asn Leu Gln Ala Arg His Glu Gln Leu Ala Asn
            740                 745                 750

tcg gac ggc cta cgg aaa atg ctg gcc gac ctg cag gcc ggt tgg aac 2304
Ser Asp Gly Leu Arg Lys Met Leu Ala Asp Leu Gln Ala Gly Trp Asn
        755                 760                 765

gcc agc agc gtg atc ggg gtg cag acg aca gag atc tcc aag tcg gcg 2352
Ala Ser Ser Val Ile Gly Val Gln Thr Thr Glu Ile Ser Lys Ser Ala
    770                 775                 780

ctc gaa ctg gcc gcc att acc ggc aac gcg gac aac ctg aaa tcc gtc 2400
Leu Glu Leu Ala Ala Ile Thr Gly Asn Ala Asp Asn Leu Lys Ser Val
785                 790                 795                 800

gac gtg ttc gtg gac cgc ttc gtc cag ggc gag cgg gtg gcc ggc cag 2448
Asp Val Phe Val Asp Arg Phe Val Gln Gly Glu Arg Val Ala Gly Gln
                805                 810                 815

ccg gtg gtc ctc gac gtc gcc gcc ggc ggc atc gat atc gcc agc cgc 2496
Pro Val Val Leu Asp Val Ala Ala Gly Gly Ile Asp Ile Ala Ser Arg
            820                 825                 830

aag ggc gag cgg ccg gcg ctg acg ttc atc acg ccg ctg gcc gcg cca 2544
Lys Gly Glu Arg Pro Ala Leu Thr Phe Ile Thr Pro Leu Ala Ala Pro
        835                 840                 845

gga gaa gag cag cgc cgg cgc acg aaa acg ggc aag agc gaa ttc acc 2592
Gly Glu Glu Gln Arg Arg Arg Thr Lys Thr Gly Lys Ser Glu Phe Thr
    850                 855                 860

aca ttc gtc gag atc gtg ggc aag cag gac cgc tgg cgc atc cgg gac 2640
Thr Phe Val Glu Ile Val Gly Lys Gln Asp Arg Trp Arg Ile Arg Asp
865                 870                 875                 880

ggc gcg gcc gac acc acc atc gat ctg gcc aag gtg gtg tcg caa ctg 2688
Gly Ala Ala Asp Thr Thr Ile Asp Leu Ala Lys Val Val Ser Gln Leu
                885                 890                 895

gtc gac gcc aat ggc gtg ctc aag cac agc atc aaa ctg gat gtg atc 2736
Val Asp Ala Asn Gly Val Leu Lys His Ser Ile Lys Leu Asp Val Ile
            900                 905                 910

ggc gga gat ggc gat gac gtc gtg ctt gcc aat gct tcg cgc atc cat 2784
Gly Gly Asp Gly Asp Asp Val Val Leu Ala Asn Ala Ser Arg Ile His
        915                 920                 925

tat gac ggc ggc gcg ggc acc aac acg gtc agc tat gcc gcc ctg ggt 2832
Tyr Asp Gly Gly Ala Gly Thr Asn Thr Val Ser Tyr Ala Ala Leu Gly
    930                 935                 940

cga cag gat tcc att acc gtg tcc gcc gac ggg gaa cgt ttc aac gtg 2880
Arg Gln Asp Ser Ile Thr Val Ser Ala Asp Gly Glu Arg Phe Asn Val
945                 950                 955                 960

cgc aag cag ttg aac aac gcc aac gtg tat cgc gaa ggc gtg gct acc 2928
Arg Lys Gln Leu Asn Asn Ala Asn Val Tyr Arg Glu Gly Val Ala Thr
                965                 970                 975

cag aca acc gcc tac ggc aag cgc acg gag aat gtc caa tac cgc cat 2976
Gln Thr Thr Ala Tyr Gly Lys Arg Thr Glu Asn Val Gln Tyr Arg His
            980                 985                 990

gtc gag ctg gcc cgt gtc ggg caa ctg gtg gag gtc gac acg ctc gag 3024
Val Glu Leu Ala Arg Val Gly Gln Leu Val Glu Val Asp Thr Leu Glu
        995                 1000                1005

cat gtg cag cac atc atc ggc ggg gcc ggc aac gat tcg atc acc 3069
His Val Gln His Ile Ile Gly Gly Ala Gly Asn Asp Ser Ile Thr
    1010                1015                1020

ggc aat gcg cac gac aac ttc cta gcc ggc ggg tcg ggc gac gac 3114
Gly Asn Ala His Asp Asn Phe Leu Ala Gly Gly Ser Gly Asp Asp
    1025                1030                1035

agg ctg gat ggc ggc gcc ggc aac gac acc ctg gtt ggc ggc gag 3159
Arg Leu Asp Gly Gly Ala Gly Asn Asp Thr Leu Val Gly Gly Glu
    1040                1045                1050

ggc caa aac acg gtc atc ggc ggc gcc ggc gac gac gta ttc ctg 3204
Gly Gln Asn Thr Val Ile Gly Gly Ala Gly Asp Asp Val Phe Leu
    1055                1060                1065

cag gac ctg ggg gta tgg agc aac cag ctc gat ggc ggc gcg ggc 3249
Gln Asp Leu Gly Val Trp Ser Asn Gln Leu Asp Gly Gly Ala Gly

1070                    1075                    1080

gtc gat acc gtg aag tac aac gtg cac cag cct tcc gag gag cgc          3294
Val Asp Thr Val Lys Tyr Asn Val His Gln Pro Ser Glu Glu Arg
    1085                    1090                    1095

ctc gaa cgc atg ggc gac acg ggc atc cat gcc gat ctt caa aag          3339
Leu Glu Arg Met Gly Asp Thr Gly Ile His Ala Asp Leu Gln Lys
    1100                    1105                    1110

ggc acg gtc gag aag tgg ccg gcc ctg aac ctg ttc agc gtc gac          3384
Gly Thr Val Glu Lys Trp Pro Ala Leu Asn Leu Phe Ser Val Asp
    1115                    1120                    1125

cat gtc aag aat atc gag aat ctg cac ggc tcc cgc ctg aac gac          3429
His Val Lys Asn Ile Glu Asn Leu His Gly Ser Arg Leu Asn Asp
    1130                    1135                    1140

cgc atc gcc ggc gac gac cag gac aac gag ctc tgg ggc cac gat          3474
Arg Ile Ala Gly Asp Asp Gln Asp Asn Glu Leu Trp Gly His Asp
    1145                    1150                    1155

ggc aac gac acg ata cgc ggc cgg ggc ggc gac gac atc ctg cgc          3519
Gly Asn Asp Thr Ile Arg Gly Arg Gly Gly Asp Asp Ile Leu Arg
    1160                    1165                    1170

ggc ggc ctg ggc ctg gac acg ctg tat ggc gag gac ggc aac gac          3564
Gly Gly Leu Gly Leu Asp Thr Leu Tyr Gly Glu Asp Gly Asn Asp
    1175                    1180                    1185

atc ttc ctg cag gac gac gag acc gtc agc gat gac atc gac ggc          3609
Ile Phe Leu Gln Asp Asp Glu Thr Val Ser Asp Asp Ile Asp Gly
    1190                    1195                    1200

ggc gcg ggg ctg gac acc gtc gac tac tcc gcc atg atc cat cca          3654
Gly Ala Gly Leu Asp Thr Val Asp Tyr Ser Ala Met Ile His Pro
    1205                    1210                    1215

ggc agg atc gtt gcg ccg cat gaa tac ggc ttc ggg atc gag gcg          3699
Gly Arg Ile Val Ala Pro His Glu Tyr Gly Phe Gly Ile Glu Ala
    1220                    1225                    1230

gac ctg tcc agg gaa tgg gtg cgc aag gcg tcc gcg ctg ggc gtg          3744
Asp Leu Ser Arg Glu Trp Val Arg Lys Ala Ser Ala Leu Gly Val
    1235                    1240                    1245

gac tat tac gat aat gtc cgc aat gtc gaa aac gtc atc ggt acg          3789
Asp Tyr Tyr Asp Asn Val Arg Asn Val Glu Asn Val Ile Gly Thr
    1250                    1255                    1260

agc atg aag gat gtg ctc atc ggc gac gcg caa gcc aat acc ctg          3834
Ser Met Lys Asp Val Leu Ile Gly Asp Ala Gln Ala Asn Thr Leu
    1265                    1270                    1275

atg ggc cag ggc ggc gac gat acc gtg cgc ggc ggc gac ggc gat          3879
Met Gly Gln Gly Gly Asp Asp Thr Val Arg Gly Gly Asp Gly Asp
    1280                    1285                    1290

gat ctg ctg ttc ggc ggc gac ggc aac gac atg ctg tat ggc gac          3924
Asp Leu Leu Phe Gly Gly Asp Gly Asn Asp Met Leu Tyr Gly Asp
    1295                    1300                    1305

gcc ggc aac gac acc ctc tac ggg ggg ctg ggc gac gat acc ctt          3969

Ala Gly Asn Asp Thr Leu Tyr Gly Gly Leu Gly Asp Asp Thr Leu
    1310                1315                1320

gaa ggc ggc gcg ggc aac gat tgg ttc ggc cag acg cag gcg cgc 4014
Glu Gly Gly Ala Gly Asn Asp Trp Phe Gly Gln Thr Gln Ala Arg
    1325                1330                1335

gag cat gac gtg ctg cgc ggc gga gat ggg gtg gat acc gtc gat 4059
Glu His Asp Val Leu Arg Gly Gly Asp Gly Val Asp Thr Val Asp
    1340                1345                1350

tac agc cag acc ggc gcg cat gcc ggc att gcc gcg ggt cgc atc 4104
Tyr Ser Gln Thr Gly Ala His Ala Gly Ile Ala Ala Gly Arg Ile
    1355                1360                1365

ggg ctg ggc atc ctg gct gac ctg ggc gcc ggc cgc gtc gac aag 4149
Gly Leu Gly Ile Leu Ala Asp Leu Gly Ala Gly Arg Val Asp Lys
    1370                1375                1380

ctg ggc gag gcc ggc agc agc gcc tac gat acg gtt tcc ggt atc 4194
Leu Gly Glu Ala Gly Ser Ser Ala Tyr Asp Thr Val Ser Gly Ile
    1385                1390                1395

gag aac gtg gtg ggc acg gaa ctg gcc gac cgc atc acg ggc gat 4239
Glu Asn Val Val Gly Thr Glu Leu Ala Asp Arg Ile Thr Gly Asp
    1400                1405                1410

gcg cag gcc aac gtg ctg cgc ggc gcg ggt ggc gcc gac gtg ctt 4284
Ala Gln Ala Asn Val Leu Arg Gly Ala Gly Gly Ala Asp Val Leu
    1415                1420                1425

gcg ggc ggc gag ggc gac gat gtg ctg ctg ggc ggc gac ggc gac 4329
Ala Gly Gly Glu Gly Asp Asp Val Leu Leu Gly Gly Asp Gly Asp
    1430                1435                1440

gac cag ctg tcg ggc gac gcc gga cgc gat cgc ttg tac ggc gaa 4374
Asp Gln Leu Ser Gly Asp Ala Gly Arg Asp Arg Leu Tyr Gly Glu
    1445                1450                1455

gcc ggt gac gac tgg ttc ttc cag gat gcc gcc aat gcc ggc aat 4419
Ala Gly Asp Asp Trp Phe Phe Gln Asp Ala Ala Asn Ala Gly Asn
    1460                1465                1470

ctg ctc gac ggc ggc gac ggc cgc gat acc gtg gat ttc agc ggc 4464
Leu Leu Asp Gly Gly Asp Gly Arg Asp Thr Val Asp Phe Ser Gly
    1475                1480                1485

ccg ggc cgg ggc ctc gac gcc ggc gca aag ggc gta ttc ctg agc 4509
Pro Gly Arg Gly Leu Asp Ala Gly Ala Lys Gly Val Phe Leu Ser
    1490                1495                1500

ttg ggc aag ggg ttc gcc agc ctg atg gac gaa ccc gaa acc agc 4554
Leu Gly Lys Gly Phe Ala Ser Leu Met Asp Glu Pro Glu Thr Ser
    1505                1510                1515

aac gtg ttg cgc aat atc gag aac gcc gtg ggc agc gcg cgt gat 4599
Asn Val Leu Arg Asn Ile Glu Asn Ala Val Gly Ser Ala Arg Asp
    1520                1525                1530

gac gtg ctg atc ggc gac gca ggc gcc aac gtc ctc aat ggc ctg 4644
Asp Val Leu Ile Gly Asp Ala Gly Ala Asn Val Leu Asn Gly Leu
    1535                1540                1545

gcg ggc aac gac gtg ctg tcc ggc ggc gct ggc gac gat gtg ctg 4689
Ala Gly Asn Asp Val Leu Ser Gly Gly Ala Gly Asp Asp Val Leu
    1550                1555                1560

ctg ggc gac gag ggc tcg gac ctg ctc agc ggc gat gcg ggc aac 4734
Leu Gly Asp Glu Gly Ser Asp Leu Leu Ser Gly Asp Ala Gly Asn
    1565                1570                1575

gac gat ctg ttc ggc ggg cag ggc gat gat act tat ctg ttc ggg 4779
Asp Asp Leu Phe Gly Gly Gln Gly Asp Asp Thr Tyr Leu Phe Gly
    1580                1585                1590

gtc ggg tac ggg cac gac acg atc tac gaa tcg ggc ggc ggc cat 4824
Val Gly Tyr Gly His Asp Thr Ile Tyr Glu Ser Gly Gly Gly His
    1595                1600                1605

gac acc atc cgc atc aac gcg ggg gcg gac cag ctg tgg ttc gcg 4869
Asp Thr Ile Arg Ile Asn Ala Gly Ala Asp Gln Leu Trp Phe Ala
    1610                1615                1620

cgc cag ggc aac gac ctg gag atc cgc att ctc ggc acc gac gat 4914
Arg Gln Gly Asn Asp Leu Glu Ile Arg Ile Leu Gly Thr Asp Asp
    1625                1630                1635

gca ctt acc gtg cac gac tgg tat cgc gac gcc gat cac cgg gtg 4959
Ala Leu Thr Val His Asp Trp Tyr Arg Asp Ala Asp His Arg Val
    1640                1645                1650

gaa atc atc cat gcc gcc aac cag gcg gta gac cag gca ggc atc 5004
Glu Ile Ile His Ala Ala Asn Gln Ala Val Asp Gln Ala Gly Ile
    1655                1660                1665

gaa aag ctg gtc gag gca atg gcg cag tat ccg gac ccc ggc gcg 5049
Glu Lys Leu Val Glu Ala Met Ala Gln Tyr Pro Asp Pro Gly Ala
    1670                1675                1680

gcg gcg gct gcc ccg ccg gcg gcg cgc gtg ccg gac acg ctg atg 5094
Ala Ala Ala Ala Pro Pro Ala Ala Arg Val Pro Asp Thr Leu Met
    1685                1690                1695

cag tcc ctg gct gtc aac tgg cgc tga 5121
Gln Ser Leu Ala Val Asn Trp Arg
    1700                1705

<210> 4
<211> 1706
<212> PRT
<213> Bordetella hinzii (wild type CyaA)

<400> 4

Met Gln Gln Ser His Gln Ala Gly Tyr Ala Asn Ala Ala Asp Arg Glu
1               5                   10                  15

Ser Gly Ile Pro Ala Ala Val Leu Asp Gly Ile Lys Ala Val Ala Lys
            20                  25                  30

Glu Lys Asn Ala Thr Leu Met Phe Arg Leu Val Asn Pro His Ser Thr
        35                  40                  45

Ser Leu Ile Ala Glu Gly Val Ala Thr Lys Gly Leu Gly Val His Ala
    50                  55                  60

Lys Ser Ser Asp Trp Gly Leu Gln Ala Gly Tyr Ile Pro Val Asn Pro
65                  70                  75                  80

Asn Leu Ser Lys Leu Phe Gly Arg Ala Pro Glu Val Ile Ala Arg Ala
                85                  90                  95

Asp Asn Asp Val Asn Ser Ser Leu Ala His Gly His Thr Ala Val Asp
            100                 105                 110

Leu Thr Leu Ser Lys Glu Arg Leu Asp Tyr Leu Arg Gln Ala Gly Leu
        115                 120                 125

Val Thr Gly Met Ala Asp Gly Val Val Ala Ser Asn His Ala Gly Tyr
    130                 135                 140

Glu Gln Phe Glu Phe Arg Val Lys Glu Thr Ser Asp Gly Arg Tyr Ala
145                 150                 155                 160

Val Gln Tyr Arg Arg Lys Gly Gly Asp Asp Phe Glu Ala Val Lys Val
                165                 170                 175

Ile Gly Asn Ala Ala Gly Ile Pro Leu Thr Ala Asp Ile Asp Met Phe
            180                 185                 190

Ala Ile Met Pro His Leu Ser Asn Phe Arg Asp Ser Ala Arg Ser Ser
        195                 200                 205

Val Thr Ser Gly Asp Ser Val Thr Asp Tyr Leu Ala Arg Thr Arg Arg
    210                 215                 220

Ala Ala Ser Glu Ala Thr Gly Gly Leu Asp Arg Glu Arg Ile Asp Leu
225                 230                 235                 240

Leu Trp Lys Ile Ala Arg Ala Gly Ala Arg Ser Ala Val Gly Thr Glu
                245                 250                 255

Ala Arg Arg Gln Phe Arg Tyr Asp Gly Asp Met Asn Ile Gly Val Ile
            260                 265                 270

Thr Asp Phe Glu Leu Glu Val Arg Asn Ala Leu Asn Arg Arg Ala His
        275                 280                 285

Ala Val Gly Ala Gln Asp Val Val Gln His Gly Thr Glu Gln Asn Asn
    290                 295                 300

Pro Phe Pro Glu Ala Asp Glu Lys Ile Phe Val Val Ser Ala Thr Gly
305                 310                 315                 320

Glu Ser Gln Met Leu Thr Arg Gly Gln Leu Lys Glu Tyr Ile Gly Gln
                325                 330                 335

Gln Arg Gly Glu Gly Tyr Val Phe Tyr Glu Asn Arg Ala Tyr Gly Val
            340                 345                 350

Ala Gly Lys Ser Leu Phe Asp Asp Gly Leu Gly Ala Ala Pro Gly Val
        355                 360                 365

Pro Ser Gly Arg Ser Lys Phe Ser Pro Asp Val Leu Glu Thr Val Pro
    370                 375                 380

Ala Ser Pro Gly Leu Arg Arg Pro Ser Leu Gly Ala Val Glu Arg Gln
385                 390                 395                 400

Asp Ser Gly Tyr Asp Ser Leu Asp Gly Val Gly Ser Arg Ser Phe Ser
                405                 410                 415

Leu Gly Glu Val Ser Asp Met Ala Ala Val Glu Ala Ala Glu Leu Glu
            420                 425                 430

Met Thr Arg Gln Val Leu His Ala Gly Ala Arg Gln Asp Asp Ala Glu
        435                 440                 445

Pro Gly Val Ser Gly Ala Ser Ala His Trp Gly Gln Arg Ala Leu Gln
    450                 455                 460

Gly Ala Gln Ala Val Ala Ala Ala Gln Arg Leu Val His Ala Ile Ala
465                 470                 475                 480

Leu Met Thr Gln Phe Gly Arg Ala Gly Ser Thr Asn Thr Pro Gln Glu
                485                 490                 495

Ala Ala Ser Leu Ser Ala Ala Val Phe Gly Leu Gly Glu Ala Ser Ser
            500                 505                 510

Ala Val Ala Glu Thr Val Ser Gly Phe Phe Arg Gly Ser Ser Arg Trp
        515                 520                 525

Ala Gly Gly Phe Gly Val Ala Gly Gly Ala Met Ala Leu Gly Gly Gly
    530                 535                 540

Ile Ala Ala Ala Val Gly Ala Gly Met Ser Leu Thr Asp Asp Ala Pro

545                 550                 555                 560

Ala Gly Gln Lys Ala Ala Ala Gly Ala Glu Ile Ala Leu Gln Leu Thr
                565                 570                 575

Gly Gly Thr Val Glu Leu Ala Ser Ser Ile Ala Leu Ala Leu Ala Ala
            580                 585                 590

Ala Arg Gly Val Thr Ser Gly Leu Gln Val Ala Gly Ala Ser Ala Gly
        595                 600                 605

Ala Ala Ala Gly Ala Leu Ala Ala Ala Leu Ser Pro Met Glu Ile Tyr
    610                 615                 620

Gly Leu Val Gln Gln Ser His Tyr Ala Asp Gln Leu Asp Lys Leu Ala
625                 630                 635                 640

Gln Glu Ser Ser Ala Tyr Gly Tyr Glu Gly Asp Ala Leu Leu Ala Gln
                645                 650                 655

Leu Tyr Arg Asp Lys Thr Ala Ala Glu Gly Ala Val Ala Gly Val Ser
            660                 665                 670

Ala Val Leu Ser Thr Val Gly Ala Ala Val Ser Ile Ala Ala Ala Ala
        675                 680                 685

Ser Val Val Gly Ala Pro Val Ala Val Val Thr Ser Leu Leu Thr Gly
    690                 695                 700

Ala Leu Asn Gly Ile Leu Arg Gly Val Gln Gln Pro Ile Ile Glu Lys
705                 710                 715                 720

Leu Ala Asn Asp Tyr Ala Arg Lys Ile Asp Glu Leu Gly Gly Pro Gln
                725                 730                 735

Ala Tyr Phe Glu Lys Asn Leu Gln Ala Arg His Glu Gln Leu Ala Asn
            740                 745                 750

Ser Asp Gly Leu Arg Lys Met Leu Ala Asp Leu Gln Ala Gly Trp Asn
        755                 760                 765

Ala Ser Ser Val Ile Gly Val Gln Thr Thr Glu Ile Ser Lys Ser Ala
    770                 775                 780

Leu Glu Leu Ala Ala Ile Thr Gly Asn Ala Asp Asn Leu Lys Ser Val
785                 790                 795                 800

Asp Val Phe Val Asp Arg Phe Val Gln Gly Glu Arg Val Ala Gly Gln
                805                 810                 815

Pro Val Val Leu Asp Val Ala Ala Gly Gly Ile Asp Ile Ala Ser Arg
            820                 825                 830

Lys Gly Glu Arg Pro Ala Leu Thr Phe Ile Thr Pro Leu Ala Ala Pro
        835                 840                 845

Gly Glu Glu Gln Arg Arg Arg Thr Lys Thr Gly Lys Ser Glu Phe Thr
    850                 855                 860

Thr Phe Val Glu Ile Val Gly Lys Gln Asp Arg Trp Arg Ile Arg Asp
865                 870                 875                 880

Gly Ala Ala Asp Thr Thr Ile Asp Leu Ala Lys Val Val Ser Gln Leu
                885                 890                 895

Val Asp Ala Asn Gly Val Leu Lys His Ser Ile Lys Leu Asp Val Ile
            900                 905                 910

Gly Gly Asp Gly Asp Asp Val Val Leu Ala Asn Ala Ser Arg Ile His
        915                 920                 925

Tyr Asp Gly Gly Ala Gly Thr Asn Thr Val Ser Tyr Ala Ala Leu Gly
    930                 935                 940

Arg Gln Asp Ser Ile Thr Val Ser Ala Asp Gly Glu Arg Phe Asn Val
945                 950                 955                 960

Arg Lys Gln Leu Asn Asn Ala Asn Val Tyr Arg Glu Gly Val Ala Thr
                965                 970                 975

Gln Thr Thr Ala Tyr Gly Lys Arg Thr Glu Asn Val Gln Tyr Arg His
            980                 985                 990

Val Glu Leu Ala Arg Val Gly Gln Leu Val Glu Val Asp Thr Leu Glu
        995                 1000                1005

His Val Gln His Ile Ile Gly Gly Ala Gly Asn Asp Ser Ile Thr
    1010                1015                1020

Gly Asn Ala His Asp Asn Phe Leu Ala Gly Gly Ser Gly Asp Asp
    1025                1030                1035

Arg Leu Asp Gly Gly Ala Gly Asn Asp Thr Leu Val Gly Gly Glu
    1040                1045                1050

Gly Gln Asn Thr Val Ile Gly Gly Ala Gly Asp Asp Val Phe Leu
    1055                1060                1065

Gln Asp Leu Gly Val Trp Ser Asn Gln Leu Asp Gly Gly Ala Gly
    1070                1075                1080

Val Asp Thr Val Lys Tyr Asn Val His Gln Pro Ser Glu Glu Arg
    1085                1090                1095

Leu Glu Arg Met Gly Asp Thr Gly Ile His Ala Asp Leu Gln Lys
    1100                1105                1110

Gly Thr Val Glu Lys Trp Pro Ala Leu Asn Leu Phe Ser Val Asp
    1115                1120                1125

His Val Lys Asn Ile Glu Asn Leu His Gly Ser Arg Leu Asn Asp
    1130                1135                1140

Arg Ile Ala Gly Asp Asp Gln Asp Asn Glu Leu Trp Gly His Asp
    1145                1150                1155

Gly Asn Asp Thr Ile Arg Gly Arg Gly Gly Asp Asp Ile Leu Arg
    1160                1165                1170

Gly Gly Leu Gly Leu Asp Thr Leu Tyr Gly Glu Asp Gly Asn Asp
    1175                1180                1185

Ile Phe Leu Gln Asp Asp Glu Thr Val Ser Asp Asp Ile Asp Gly
    1190                1195                1200

Gly Ala Gly Leu Asp Thr Val Asp Tyr Ser Ala Met Ile His Pro
    1205                1210                1215

Gly Arg Ile Val Ala Pro His Glu Tyr Gly Phe Gly Ile Glu Ala
    1220                1225                1230

Asp Leu Ser Arg Glu Trp Val Arg Lys Ala Ser Ala Leu Gly Val
    1235                1240                1245

Asp Tyr Tyr Asp Asn Val Arg Asn Val Glu Asn Val Ile Gly Thr
    1250                1255                1260

Ser Met Lys Asp Val Leu Ile Gly Asp Ala Gln Ala Asn Thr Leu
    1265                1270                1275

Met Gly Gln Gly Gly Asp Asp Thr Val Arg Gly Gly Asp Gly Asp
    1280                1285                1290

Asp Leu Leu Phe Gly Gly Asp Gly Asn Asp Met Leu Tyr Gly Asp
    1295                1300                1305

Ala Gly Asn Asp Thr Leu Tyr Gly Gly Leu Gly Asp Asp Thr Leu
    1310                1315                1320

Glu Gly Gly Ala Gly Asn Asp Trp Phe Gly Gln Thr Gln Ala Arg
    1325                1330                1335

Glu His Asp Val Leu Arg Gly Gly Asp Gly Val Asp Thr Val Asp
    1340                1345                1350

Tyr Ser Gln Thr Gly Ala His Ala Gly Ile Ala Ala Gly Arg Ile
    1355                1360                1365

Gly Leu Gly Ile Leu Ala Asp Leu Gly Ala Gly Arg Val Asp Lys
    1370                1375                1380

Leu Gly Glu Ala Gly Ser Ser Ala Tyr Asp Thr Val Ser Gly Ile
    1385                1390                1395

Glu Asn Val Val Gly Thr Glu Leu Ala Asp Arg Ile Thr Gly Asp
    1400                1405                1410

Ala Gln Ala Asn Val Leu Arg Gly Ala Gly Gly Ala Asp Val Leu
    1415                1420                1425

Ala Gly Gly Glu Gly Asp Asp Val Leu Leu Gly Gly Asp Gly Asp
    1430                1435                1440

Asp Gln Leu Ser Gly Asp Ala Gly Arg Asp Arg Leu Tyr Gly Glu
    1445                1450                1455

Ala Gly Asp Asp Trp Phe Phe Gln Asp Ala Ala Asn Ala Gly Asn
    1460                1465                1470

Leu Leu Asp Gly Gly Asp Gly Arg Asp Thr Val Asp Phe Ser Gly
    1475                1480                1485

Pro Gly Arg Gly Leu Asp Ala Gly Ala Lys Gly Val Phe Leu Ser
    1490                1495                1500

Leu Gly Lys Gly Phe Ala Ser Leu Met Asp Glu Pro Glu Thr Ser
    1505                1510                1515

Asn Val Leu Arg Asn Ile Glu Asn Ala Val Gly Ser Ala Arg Asp

1520                    1525                    1530

Asp Val  Leu Ile Gly Asp Ala  Gly Ala Asn Val Leu  Asn Gly Leu
    1535                    1540                    1545

Ala Gly  Asn Asp Val Leu Ser  Gly Gly Ala Gly Asp  Asp Val Leu
    1550                    1555                    1560

Leu Gly  Asp Glu Gly Ser Asp  Leu Leu Ser Gly Asp  Ala Gly Asn
    1565                    1570                    1575

Asp Asp  Leu Phe Gly Gly Gln  Gly Asp Asp Thr Tyr  Leu Phe Gly
    1580                    1585                    1590

Val Gly  Tyr Gly His Asp Thr  Ile Tyr Glu Ser Gly  Gly Gly His
    1595                    1600                    1605

Asp Thr  Ile Arg Ile Asn Ala  Gly Ala Asp Gln Leu  Trp Phe Ala
    1610                    1615                    1620

Arg Gln  Gly Asn Asp Leu Glu  Ile Arg Ile Leu Gly  Thr Asp Asp
    1625                    1630                    1635

Ala Leu  Thr Val His Asp Trp  Tyr Arg Asp Ala Asp  His Arg Val
    1640                    1645                    1650

Glu Ile  Ile His Ala Ala Asn  Gln Ala Val Asp Gln  Ala Gly Ile
    1655                    1660                    1665

Glu Lys  Leu Val Glu Ala Met  Ala Gln Tyr Pro Asp  Pro Gly Ala
    1670                    1675                    1680

Ala Ala  Ala Ala Pro Pro Ala  Ala Arg Val Pro Asp  Thr Leu Met
    1685                    1690                    1695

Gln Ser  Leu Ala Val Asn Trp  Arg
    1700                    1705

<210>  5
<211>  5121
<212>  DNA
<213>  Bordetella parapertussis (wild type CyaA)

<220>
<221>  CDS
<222>  (1)..(5121)

<400>  5
atg cag caa tcg cat cag gct ggt tac gca aac gcc gcc gac cgg gag          48

Met Gln Gln Ser His Gln Ala Gly Tyr Ala Asn Ala Ala Asp Arg Glu
1               5                   10                  15

tct ggc atc ccc gca gcc gta ctc gat ggc atc aag gcc gtg gcg aag      96
Ser Gly Ile Pro Ala Ala Val Leu Asp Gly Ile Lys Ala Val Ala Lys
            20                  25                  30

gaa aaa aac gcc aca ttg atg ttc cgc ctg gtc aac ccc cat tcc acc      144
Glu Lys Asn Ala Thr Leu Met Phe Arg Leu Val Asn Pro His Ser Thr
        35                  40                  45

agc ctg att gcc gaa ggg gtg gcc acc aaa gga ttg ggc gtg cac gcc      192
Ser Leu Ile Ala Glu Gly Val Ala Thr Lys Gly Leu Gly Val His Ala
    50                  55                  60

aag tcg tcc gat tgg ggg ttg cag gcg ggc tac att ccc gtc aac ccg      240
Lys Ser Ser Asp Trp Gly Leu Gln Ala Gly Tyr Ile Pro Val Asn Pro
65                  70                  75                  80

aat ctt tcc aaa ctg ttc ggc cgt gcg ccc gag gtg atc gcg cgg gcc      288
Asn Leu Ser Lys Leu Phe Gly Arg Ala Pro Glu Val Ile Ala Arg Ala
                85                  90                  95

gac aac gac gtc aac agc agc ctg gcg cat ggc cat acc gcg gtc gac      336
Asp Asn Asp Val Asn Ser Ser Leu Ala His Gly His Thr Ala Val Asp
            100                 105                 110

ctg acg ctg tcg aaa gag cgg ctt gac tat ctg cgg caa gcg ggc ctg      384
Leu Thr Leu Ser Lys Glu Arg Leu Asp Tyr Leu Arg Gln Ala Gly Leu
        115                 120                 125

gtc acc ggc atg gcc gat ggc gtg gtc gcg agc aac cac gca ggc tac      432
Val Thr Gly Met Ala Asp Gly Val Val Ala Ser Asn His Ala Gly Tyr
    130                 135                 140

gag cag ttc gag ttt cgc gtg aag gaa acc tcg gac ggg cgc tat gcc      480
Glu Gln Phe Glu Phe Arg Val Lys Glu Thr Ser Asp Gly Arg Tyr Ala
145                 150                 155                 160

gtg cag tat cgc cgc aag ggc ggc gac gat ttc gag gcg gtc aag gtg      528
Val Gln Tyr Arg Arg Lys Gly Gly Asp Asp Phe Glu Ala Val Lys Val
                165                 170                 175

atc ggc aat gcc gcc ggt att cca ctg acg gcg gat atc gac atg ttc      576
Ile Gly Asn Ala Ala Gly Ile Pro Leu Thr Ala Asp Ile Asp Met Phe
            180                 185                 190

gcc atc atg ccg cat ctg tcc aac ttc cgc gac tcg gcg cgc agt tcg      624
Ala Ile Met Pro His Leu Ser Asn Phe Arg Asp Ser Ala Arg Ser Ser
        195                 200                 205

gtg acc agc ggc gat tcg gtg acc gat tac ctg gcg cgc acg cgg cgg      672
Val Thr Ser Gly Asp Ser Val Thr Asp Tyr Leu Ala Arg Thr Arg Arg
    210                 215                 220

gcc gcc agc gag gcc acg ggc ggc ctg gat cgc gaa cgc atc gac ttg      720
Ala Ala Ser Glu Ala Thr Gly Gly Leu Asp Arg Glu Arg Ile Asp Leu
225                 230                 235                 240

ttg tgg aaa atc gct cgc gcc ggc gcc cgt tcc gca gtg ggc acc gag      768
Leu Trp Lys Ile Ala Arg Ala Gly Ala Arg Ser Ala Val Gly Thr Glu
                245                 250                 255

gcg cgt cgc cag ttc cgc tac gac ggc gac atg aat atc ggc gtg atc 816
Ala Arg Arg Gln Phe Arg Tyr Asp Gly Asp Met Asn Ile Gly Val Ile
            260                 265                 270

acc gat ttc gag ctg gaa gtg cgc aat gcg ctg aac agg cgg gcg cac 864
Thr Asp Phe Glu Leu Glu Val Arg Asn Ala Leu Asn Arg Arg Ala His
        275                 280                 285

gcc gtc ggc gcg cag gac gtg gtc cag cat ggc act gag cag aac aat 912
Ala Val Gly Ala Gln Asp Val Val Gln His Gly Thr Glu Gln Asn Asn
    290                 295                 300

cct ttc ccg gag gca gat gag aag att ttc gtc gta tcg gcc acc ggt 960
Pro Phe Pro Glu Ala Asp Glu Lys Ile Phe Val Val Ser Ala Thr Gly
305                 310                 315                 320

gaa agc cag atg ctc acg cgc ggg caa ctg aag gaa tac att ggc cag 1008
Glu Ser Gln Met Leu Thr Arg Gly Gln Leu Lys Glu Tyr Ile Gly Gln
                325                 330                 335

cag cgc ggc gag ggc tat gtc ttc tac gag aac cgt gca tac ggc gtg 1056
Gln Arg Gly Glu Gly Tyr Val Phe Tyr Glu Asn Arg Ala Tyr Gly Val
            340                 345                 350

gcg ggg aaa agc ctg ttc gac gat ggg ctg gga gcc gcg ccc ggc gtg 1104
Ala Gly Lys Ser Leu Phe Asp Asp Gly Leu Gly Ala Ala Pro Gly Val
        355                 360                 365

ccg ggc gga cgt tcg aag tcc tcg ccg gat gta ctg gaa acg gtg ccg 1152
Pro Gly Gly Arg Ser Lys Ser Ser Pro Asp Val Leu Glu Thr Val Pro
    370                 375                 380

gcg tca ccc gga ttg cgg cgg ccg tcg ctg ggc gca gtg gaa cgc cag 1200
Ala Ser Pro Gly Leu Arg Arg Pro Ser Leu Gly Ala Val Glu Arg Gln
385                 390                 395                 400

gat tcc ggc tat gac agc ctt gat ggg gtg gga tcg cga tcg ttc tcg 1248
Asp Ser Gly Tyr Asp Ser Leu Asp Gly Val Gly Ser Arg Ser Phe Ser
                405                 410                 415

ttg ggc gag gtg tcc gac atg gcc gcc gtg gaa gcg gcg gaa ctg gaa 1296
Leu Gly Glu Val Ser Asp Met Ala Ala Val Glu Ala Ala Glu Leu Glu
            420                 425                 430

atg acc cgg caa gtc ttg cac gcc ggg gcg cgg cag gac gat gcc gag 1344
Met Thr Arg Gln Val Leu His Ala Gly Ala Arg Gln Asp Asp Ala Glu
        435                 440                 445

ccg ggc gtg agc ggt gcg tcg gcg cac tgg ggg cag cgg gcg ctg cag 1392
Pro Gly Val Ser Gly Ala Ser Ala His Trp Gly Gln Arg Ala Leu Gln
    450                 455                 460

ggc gcc cag gcg gtg gcg gcg gcg cag cgg ctg gtt cat gcc att gcc 1440
Gly Ala Gln Ala Val Ala Ala Ala Gln Arg Leu Val His Ala Ile Ala
465                 470                 475                 480

ctg atg acg caa ttc ggc cgg gcc ggt tcc acc aac acg ccg cag gaa 1488
Leu Met Thr Gln Phe Gly Arg Ala Gly Ser Thr Asn Thr Pro Gln Glu
                485                 490                 495

gcg gcc tcg ttg tcg gcg gcc gtg ttc ggc ttg ggc gag gcc agc agc 1536
Ala Ala Ser Leu Ser Ala Ala Val Phe Gly Leu Gly Glu Ala Ser Ser
            500                 505                 510

gcc gtg gcc gaa acc gtg agc ggt ttt ttc cgc ggg tct tcg cgc tgg 1584
Ala Val Ala Glu Thr Val Ser Gly Phe Phe Arg Gly Ser Ser Arg Trp
        515                 520                 525

gcc ggc ggt ttc ggc gtg gct ggc ggc gcg atg gcg ctg gga ggc ggc 1632
Ala Gly Gly Phe Gly Val Ala Gly Gly Ala Met Ala Leu Gly Gly Gly
    530                 535                 540

atc gcc gcg gcc gtt ggc gcc ggg atg tcg ttg acc gat gac gcg ccg 1680
Ile Ala Ala Ala Val Gly Ala Gly Met Ser Leu Thr Asp Asp Ala Pro
545                 550                 555                 560

gcc gga cag aag gcc gcc gtc ggc gcc gag atc gcg ctg cag ttg aca 1728
Ala Gly Gln Lys Ala Ala Val Gly Ala Glu Ile Ala Leu Gln Leu Thr
                565                 570                 575

ggt gga acg gtc gag ctg gct tct tcc atc gcg ttg gcg ctg gcc gcg 1776
Gly Gly Thr Val Glu Leu Ala Ser Ser Ile Ala Leu Ala Leu Ala Ala
            580                 585                 590

gcg cgc ggc gtg acc agc ggc ttg cag gtg gcg ggg gcg tcg gcc ggg 1824
Ala Arg Gly Val Thr Ser Gly Leu Gln Val Ala Gly Ala Ser Ala Gly
        595                 600                 605

gcg gct gcc ggc gca ttg gcc gcg gcg ctc agt ccc atg gag atc tac 1872
Ala Ala Ala Gly Ala Leu Ala Ala Ala Leu Ser Pro Met Glu Ile Tyr
    610                 615                 620

ggc ctg gtg cag caa tcg cac tat gcg gat cag ctg gac aag ctg gcg 1920
Gly Leu Val Gln Gln Ser His Tyr Ala Asp Gln Leu Asp Lys Leu Ala
625                 630                 635                 640

cag gaa tcg agc gca tac ggt tac gag ggc gac gcc ttg ctg gcc cag 1968
Gln Glu Ser Ser Ala Tyr Gly Tyr Glu Gly Asp Ala Leu Leu Ala Gln
                645                 650                 655

ctg tat cgc gac aag acg gcc gcc gag ggc gcc gtc gcc ggc gtc tcc 2016
Leu Tyr Arg Asp Lys Thr Ala Ala Glu Gly Ala Val Ala Gly Val Ser
            660                 665                 670

gcc gtc ctg agc acg gtg ggg gcg gcg gtg tcg atc gcc gcg gcg gcc 2064
Ala Val Leu Ser Thr Val Gly Ala Ala Val Ser Ile Ala Ala Ala Ala
        675                 680                 685

agc gtg gta ggc gcc ccg gtg gcg gtg gtc act tcc ttg ttg acc ggg 2112
Ser Val Val Gly Ala Pro Val Ala Val Val Thr Ser Leu Leu Thr Gly
    690                 695                 700

gct ctc aac ggc atc ctg cgc ggc gtg cag cag ccc atc atc gaa aag 2160
Ala Leu Asn Gly Ile Leu Arg Gly Val Gln Gln Pro Ile Ile Glu Lys
705                 710                 715                 720

ctg gcc aat gat tac gct cgc aag atc gac gag ctg ggc ggg ccg caa 2208
Leu Ala Asn Asp Tyr Ala Arg Lys Ile Asp Glu Leu Gly Gly Pro Gln
                725                 730                 735

gcg tac ttc gag aaa aac ctg cag gcg cgt cac gaa caa ctg gcc aat 2256
Ala Tyr Phe Glu Lys Asn Leu Gln Ala Arg His Glu Gln Leu Ala Asn
            740                 745                 750

tcg gac ggc cta cgg aaa atg ctg gct gac ctg cag gcc ggg tgg aac 2304
Ser Asp Gly Leu Arg Lys Met Leu Ala Asp Leu Gln Ala Gly Trp Asn

755                 760                 765

gcc agc agc gtg atc ggg gtg cag acg aca gag att tcc aag tcg gcg        2352
Ala Ser Ser Val Ile Gly Val Gln Thr Thr Glu Ile Ser Lys Ser Ala
    770                 775                 780

ctc gaa ctg gcc gcc att acc ggc aac gcg gac aac ctg aaa tcc gcc        2400
Leu Glu Leu Ala Ala Ile Thr Gly Asn Ala Asp Asn Leu Lys Ser Ala
785                 790                 795                 800

gac gtg ttc gtg gac cgc ttc atc cag ggc gag cgg gtg gcc ggc cag        2448
Asp Val Phe Val Asp Arg Phe Ile Gln Gly Glu Arg Val Ala Gly Gln
                805                 810                 815

ccg gtg gta ctc gac gtc gcc gcc ggc ggc atc gat atc gcc agc cgc        2496
Pro Val Val Leu Asp Val Ala Ala Gly Gly Ile Asp Ile Ala Ser Arg
            820                 825                 830

aag ggc gag cgg ccg gcg ctg acg ttc atc acg ccg ctg gcc gcg cca        2544
Lys Gly Glu Arg Pro Ala Leu Thr Phe Ile Thr Pro Leu Ala Ala Pro
        835                 840                 845

gga gaa gag cag cgc cgg cgc acg aag acg ggc aag agc gaa ttc acc        2592
Gly Glu Glu Gln Arg Arg Arg Thr Lys Thr Gly Lys Ser Glu Phe Thr
    850                 855                 860

aca ttc gtc gag atc gtg ggc aag cag gac cgc tgg cgc atc cgg gac        2640
Thr Phe Val Glu Ile Val Gly Lys Gln Asp Arg Trp Arg Ile Arg Asp
865                 870                 875                 880

ggc gcg gcc gac acc acc atc gat ctg gcc aag gtg gtg tcg caa ctg        2688
Gly Ala Ala Asp Thr Thr Ile Asp Leu Ala Lys Val Val Ser Gln Leu
                885                 890                 895

gtc gac gcc aat ggc gtg ctc aag cac agc atc aaa ctg gag gtg atc        2736
Val Asp Ala Asn Gly Val Leu Lys His Ser Ile Lys Leu Glu Val Ile
            900                 905                 910

ggc gga gat ggc gat gat gtc gtg ctt gcc aat gct tcg cgc atc cat        2784
Gly Gly Asp Gly Asp Asp Val Val Leu Ala Asn Ala Ser Arg Ile His
        915                 920                 925

tac gac ggc ggc gcg gga acc aac acg gtc agc tat gcc gcc ctg ggc        2832
Tyr Asp Gly Gly Ala Gly Thr Asn Thr Val Ser Tyr Ala Ala Leu Gly
    930                 935                 940

cga cag gat tcc att acc gtg tcc gcc gac ggg gaa cgt ttc aac gtg        2880
Arg Gln Asp Ser Ile Thr Val Ser Ala Asp Gly Glu Arg Phe Asn Val
945                 950                 955                 960

cgc aag cag ttg aac aac gcc aac gtg tat cgc gaa ggc gtg gct acc        2928
Arg Lys Gln Leu Asn Asn Ala Asn Val Tyr Arg Glu Gly Val Ala Thr
                965                 970                 975

cag aaa acc gcc tac ggc aag cgc acg gag aat gtc caa tac cgc cat        2976
Gln Lys Thr Ala Tyr Gly Lys Arg Thr Glu Asn Val Gln Tyr Arg His
            980                 985                 990

gtc gag ctg gcc cgt gtc ggg caa  ctg gtg gag gtc gac  acg ctc gag        3024
Val Glu Leu Ala Arg Val Gly Gln  Leu Val Glu Val Asp  Thr Leu Glu
        995                 1000                 1005

cat gtg  cag cac atc atc ggc  ggg gcc ggc aac gat  tcg atc acc        3069

His Val Gln His Ile Ile Gly Gly Ala Gly Asn Asp Ser Ile Thr
    1010                1015                1020

ggc aat gcg cac gac aac ttc ctg gcc ggc ggg gcg ggc gac gac      3114
Gly Asn Ala His Asp Asn Phe Leu Ala Gly Gly Ala Gly Asp Asp
    1025                1030                1035

agg ctg gat ggc ggc gcc ggc aac gac aca ctg gtc ggc ggc gag      3159
Arg Leu Asp Gly Gly Ala Gly Asn Asp Thr Leu Val Gly Gly Glu
    1040                1045                1050

ggc cac aac acg gtc gtc ggc ggc gct ggc gac gac gta ttc ctg      3204
Gly His Asn Thr Val Val Gly Gly Ala Gly Asp Asp Val Phe Leu
    1055                1060                1065

cag gac ctg ggg gta tgg agc aac cag ctc gat ggc ggc gcg ggc      3249
Gln Asp Leu Gly Val Trp Ser Asn Gln Leu Asp Gly Gly Ala Gly
    1070                1075                1080

gtc gat acc gtg aag tac aac gtg cac cag cct tcc gag gaa cgc      3294
Val Asp Thr Val Lys Tyr Asn Val His Gln Pro Ser Glu Glu Arg
    1085                1090                1095

ctc gaa cgc atg ggc gac acg ggc atc cat gcc gat ctt caa aag      3339
Leu Glu Arg Met Gly Asp Thr Gly Ile His Ala Asp Leu Gln Lys
    1100                1105                1110

ggc acg gtc gag aag tgg ccg gcc ctg aac ctg ttc agc gtc gac      3384
Gly Thr Val Glu Lys Trp Pro Ala Leu Asn Leu Phe Ser Val Asp
    1115                1120                1125

cat gtc aag aat atc gag aat ctg cac ggc tcc agc ctg aac gac      3429
His Val Lys Asn Ile Glu Asn Leu His Gly Ser Ser Leu Asn Asp
    1130                1135                1140

agc atc gcc ggc gac gac cgg gac aac gag ctc tgg ggc gac gat      3474
Ser Ile Ala Gly Asp Asp Arg Asp Asn Glu Leu Trp Gly Asp Asp
    1145                1150                1155

ggc aac gac acg ata cac ggc cgg ggc ggc gac gat atc ctg cgc      3519
Gly Asn Asp Thr Ile His Gly Arg Gly Gly Asp Asp Ile Leu Arg
    1160                1165                1170

ggc ggc ctg ggc ctg gac acg ctg tat ggc gag gac ggc aac gac      3564
Gly Gly Leu Gly Leu Asp Thr Leu Tyr Gly Glu Asp Gly Asn Asp
    1175                1180                1185

atc ttc ctg cag gac gac gag acc gtc agc gat gac atc gac ggc      3609
Ile Phe Leu Gln Asp Asp Glu Thr Val Ser Asp Asp Ile Asp Gly
    1190                1195                1200

ggc gcg ggg ctg gac acc gtc gac tat tcc gcc atg atc cat gca      3654
Gly Ala Gly Leu Asp Thr Val Asp Tyr Ser Ala Met Ile His Ala
    1205                1210                1215

ggc aag atc gtt gcg ccg cat gaa tac ggc ttc ggg atc gag gcg      3699
Gly Lys Ile Val Ala Pro His Glu Tyr Gly Phe Gly Ile Glu Ala
    1220                1225                1230

gac ctg tcc gaa ggg tgg gtg cgc aag gcg gcc cgg cgc ggc atg      3744
Asp Leu Ser Glu Gly Trp Val Arg Lys Ala Ala Arg Arg Gly Met
    1235                1240                1245

ggc tac tac gac agt gtc cgc agt gtc gaa aac gtc atc ggc acg 3789
Gly Tyr Tyr Asp Ser Val Arg Ser Val Glu Asn Val Ile Gly Thr
    1250                1255                1260

agc atg aag gat gtg ctc atc ggc gac gcg caa gcc aat acc ctg 3834
Ser Met Lys Asp Val Leu Ile Gly Asp Ala Gln Ala Asn Thr Leu
    1265                1270                1275

atg ggc cag ggc ggc gac gat acc gtg cgc ggc ggc gac ggc gat 3879
Met Gly Gln Gly Gly Asp Asp Thr Val Arg Gly Gly Asp Gly Asp
    1280                1285                1290

gat ctg ctg ttc ggc ggc gac ggc aac gac atg ctg tat gga gac 3924
Asp Leu Leu Phe Gly Gly Asp Gly Asn Asp Met Leu Tyr Gly Asp
    1295                1300                1305

gcc ggc aac gac acc ctc tac gga ggg ctg ggc gac gat acc ctt 3969
Ala Gly Asn Asp Thr Leu Tyr Gly Gly Leu Gly Asp Asp Thr Leu
    1310                1315                1320

gaa ggc ggc gcg ggc aac gat tgg ttc ggc cag acg ccg gcg cgc 4014
Glu Gly Gly Ala Gly Asn Asp Trp Phe Gly Gln Thr Pro Ala Arg
    1325                1330                1335

gag cat gac gtg ctg cgc ggc ggg gct ggg gtg gat acc gtg gat 4059
Glu His Asp Val Leu Arg Gly Gly Ala Gly Val Asp Thr Val Asp
    1340                1345                1350

tac agc cag gcg ggc gcg cat gcc ggc gtt gcc acg ggt cgc atc 4104
Tyr Ser Gln Ala Gly Ala His Ala Gly Val Ala Thr Gly Arg Ile
    1355                1360                1365

ggg ctg ggt att ctg gcg gac ctg ggc gcc ggc cgc gtc gac aag 4149
Gly Leu Gly Ile Leu Ala Asp Leu Gly Ala Gly Arg Val Asp Lys
    1370                1375                1380

ctg ggc gag gcc ggc agc agc gcc tac gat acg gtt tcc ggc atc 4194
Leu Gly Glu Ala Gly Ser Ser Ala Tyr Asp Thr Val Ser Gly Ile
    1385                1390                1395

gaa aat gtg gtg ggc acg gaa ctg gcc gac cgc atc acg ggc gat 4239
Glu Asn Val Val Gly Thr Glu Leu Ala Asp Arg Ile Thr Gly Asp
    1400                1405                1410

gcg cag gcc aac gta ctg cgc ggc gcg ggt ggt gcc gac gtg ctt 4284
Ala Gln Ala Asn Val Leu Arg Gly Ala Gly Gly Ala Asp Val Leu
    1415                1420                1425

gcg ggc ggc gag ggc gac gat gtg ctg ctg ggc ggc gag ggc gat 4329
Ala Gly Gly Glu Gly Asp Asp Val Leu Leu Gly Gly Glu Gly Asp
    1430                1435                1440

gac cag ctg tcg ggc gac gcc gga cgc gac cgc ttg tac ggc gaa 4374
Asp Gln Leu Ser Gly Asp Ala Gly Arg Asp Arg Leu Tyr Gly Glu
    1445                1450                1455

gcc ggt gac gac tgg ttc ttc cag gat gcc gcc aat gcc ggc aat 4419
Ala Gly Asp Asp Trp Phe Phe Gln Asp Ala Ala Asn Ala Gly Asn
    1460                1465                1470

ctg ctc gac ggt ggt gac ggc aac gat acc gtg gat ttc agc ggc 4464
Leu Leu Asp Gly Gly Asp Gly Asn Asp Thr Val Asp Phe Ser Gly
    1475                1480                1485

ccg ggc cgg ggc ctc gac gcc ggc gca aag ggc gta ttc ctg agc 4509
Pro Gly Arg Gly Leu Asp Ala Gly Ala Lys Gly Val Phe Leu Ser
    1490                1495                1500

ctg ggc aag ggg ttc gcc agc ctg atg gac gaa ccc gaa acc agc 4554
Leu Gly Lys Gly Phe Ala Ser Leu Met Asp Glu Pro Glu Thr Ser
    1505                1510                1515

aac gtg ttg cgc cat atc gag aac gcc gtg ggc agc gtg cgt gat 4599
Asn Val Leu Arg His Ile Glu Asn Ala Val Gly Ser Val Arg Asp
    1520                1525                1530

gac gtg ctg atc ggc gac gca ggc gcc aac gtc ctc aat ggc ctg 4644
Asp Val Leu Ile Gly Asp Ala Gly Ala Asn Val Leu Asn Gly Leu
    1535                1540                1545

gcg ggc aac gac gtg ttg tcc ggc ggc gcc ggc gac gat gtg ctg 4689
Ala Gly Asn Asp Val Leu Ser Gly Gly Ala Gly Asp Asp Val Leu
    1550                1555                1560

ctg ggc gac gag ggc tcg gac ctg ctc agc ggc gat gcg ggc aac 4734
Leu Gly Asp Glu Gly Ser Asp Leu Leu Ser Gly Asp Ala Gly Asn
    1565                1570                1575

gac gat ctg ttc ggc ggg cag ggc gat gat acc tat ctg ttc ggg 4779
Asp Asp Leu Phe Gly Gly Gln Gly Asp Asp Thr Tyr Leu Phe Gly
    1580                1585                1590

gcc ggg tac gga cat gac acg atc tac gaa tcg ggc ggc ggc cat 4824
Ala Gly Tyr Gly His Asp Thr Ile Tyr Glu Ser Gly Gly Gly His
    1595                1600                1605

gac acc atc cgt atc aac gcg ggg gcg gac cag ctg tgg ttt gcg 4869
Asp Thr Ile Arg Ile Asn Ala Gly Ala Asp Gln Leu Trp Phe Ala
    1610                1615                1620

cgc cag ggc aac gac ctg gag atc cgc att ctt ggc acc gac gat 4914
Arg Gln Gly Asn Asp Leu Glu Ile Arg Ile Leu Gly Thr Asp Asp
    1625                1630                1635

gca ctt acc gtg cac gac tgg tat cgc gac gcc gat cac cgg gtg 4959
Ala Leu Thr Val His Asp Trp Tyr Arg Asp Ala Asp His Arg Val
    1640                1645                1650

gaa gcc atc cat gcc gcc aac cag gcc ata gac ccg gcc ggc atc 5004
Glu Ala Ile His Ala Ala Asn Gln Ala Ile Asp Pro Ala Gly Ile
    1655                1660                1665

gaa aag ctg gtc gag gca atg gcg cag tac ccg gac ccc ggc gcg 5049
Glu Lys Leu Val Glu Ala Met Ala Gln Tyr Pro Asp Pro Gly Ala
    1670                1675                1680

gcg gcg gct gcc ccg ccg gcg gcg cgc gtg ccg gac acg ctg atg 5094
Ala Ala Ala Ala Pro Pro Ala Ala Arg Val Pro Asp Thr Leu Met
    1685                1690                1695

cag tcc ctg gct gtc aac tgg cgc tga 5121
Gln Ser Leu Ala Val Asn Trp Arg
    1700                1705

<210> 6

<211>  1706
<212>  PRT
<213>  Bordetella parapertussis (wild type CyaA)

<400>  6

Met Gln Gln Ser His Gln Ala Gly Tyr Ala Asn Ala Ala Asp Arg Glu
1               5                   10                  15

Ser Gly Ile Pro Ala Ala Val Leu Asp Gly Ile Lys Ala Val Ala Lys
            20                  25                  30

Glu Lys Asn Ala Thr Leu Met Phe Arg Leu Val Asn Pro His Ser Thr
        35                  40                  45

Ser Leu Ile Ala Glu Gly Val Ala Thr Lys Gly Leu Gly Val His Ala
    50                  55                  60

Lys Ser Ser Asp Trp Gly Leu Gln Ala Gly Tyr Ile Pro Val Asn Pro
65                  70                  75                  80

Asn Leu Ser Lys Leu Phe Gly Arg Ala Pro Glu Val Ile Ala Arg Ala
                85                  90                  95

Asp Asn Asp Val Asn Ser Ser Leu Ala His Gly His Thr Ala Val Asp
            100                 105                 110

Leu Thr Leu Ser Lys Glu Arg Leu Asp Tyr Leu Arg Gln Ala Gly Leu
        115                 120                 125

Val Thr Gly Met Ala Asp Gly Val Val Ala Ser Asn His Ala Gly Tyr
    130                 135                 140

Glu Gln Phe Glu Phe Arg Val Lys Glu Thr Ser Asp Gly Arg Tyr Ala
145                 150                 155                 160

Val Gln Tyr Arg Arg Lys Gly Gly Asp Asp Phe Glu Ala Val Lys Val
                165                 170                 175

Ile Gly Asn Ala Ala Gly Ile Pro Leu Thr Ala Asp Ile Asp Met Phe
            180                 185                 190

Ala Ile Met Pro His Leu Ser Asn Phe Arg Asp Ser Ala Arg Ser Ser
        195                 200                 205

Val Thr Ser Gly Asp Ser Val Thr Asp Tyr Leu Ala Arg Thr Arg Arg
    210                 215                 220

Ala Ala Ser Glu Ala Thr Gly Gly Leu Asp Arg Glu Arg Ile Asp Leu

225                 230                 235                 240

Leu Trp Lys Ile Ala Arg Ala Gly Ala Arg Ser Ala Val Gly Thr Glu
                245                 250                 255

Ala Arg Arg Gln Phe Arg Tyr Asp Gly Asp Met Asn Ile Gly Val Ile
            260                 265                 270

Thr Asp Phe Glu Leu Glu Val Arg Asn Ala Leu Asn Arg Arg Ala His
        275                 280                 285

Ala Val Gly Ala Gln Asp Val Val Gln His Gly Thr Glu Gln Asn Asn
    290                 295                 300

Pro Phe Pro Glu Ala Asp Glu Lys Ile Phe Val Val Ser Ala Thr Gly
305                 310                 315                 320

Glu Ser Gln Met Leu Thr Arg Gly Gln Leu Lys Glu Tyr Ile Gly Gln
                325                 330                 335

Gln Arg Gly Glu Gly Tyr Val Phe Tyr Glu Asn Arg Ala Tyr Gly Val
            340                 345                 350

Ala Gly Lys Ser Leu Phe Asp Asp Gly Leu Gly Ala Ala Pro Gly Val
        355                 360                 365

Pro Gly Gly Arg Ser Lys Ser Ser Pro Asp Val Leu Glu Thr Val Pro
    370                 375                 380

Ala Ser Pro Gly Leu Arg Arg Pro Ser Leu Gly Ala Val Glu Arg Gln
385                 390                 395                 400

Asp Ser Gly Tyr Asp Ser Leu Asp Gly Val Gly Ser Arg Ser Phe Ser
                405                 410                 415

Leu Gly Glu Val Ser Asp Met Ala Ala Val Glu Ala Ala Glu Leu Glu
            420                 425                 430

Met Thr Arg Gln Val Leu His Ala Gly Ala Arg Gln Asp Asp Ala Glu
        435                 440                 445

Pro Gly Val Ser Gly Ala Ser Ala His Trp Gly Gln Arg Ala Leu Gln
    450                 455                 460

Gly Ala Gln Ala Val Ala Ala Ala Gln Arg Leu Val His Ala Ile Ala
465                 470                 475                 480

Leu Met Thr Gln Phe Gly Arg Ala Gly Ser Thr Asn Thr Pro Gln Glu
                485                 490                 495

Ala Ala Ser Leu Ser Ala Ala Val Phe Gly Leu Gly Glu Ala Ser Ser
            500                 505                 510

Ala Val Ala Glu Thr Val Ser Gly Phe Phe Arg Gly Ser Ser Arg Trp
        515                 520                 525

Ala Gly Gly Phe Gly Val Ala Gly Gly Ala Met Ala Leu Gly Gly Gly
    530                 535                 540

Ile Ala Ala Ala Val Gly Ala Gly Met Ser Leu Thr Asp Asp Ala Pro
545                 550                 555                 560

Ala Gly Gln Lys Ala Ala Val Gly Ala Glu Ile Ala Leu Gln Leu Thr
                565                 570                 575

Gly Gly Thr Val Glu Leu Ala Ser Ser Ile Ala Leu Ala Leu Ala Ala
            580                 585                 590

Ala Arg Gly Val Thr Ser Gly Leu Gln Val Ala Gly Ala Ser Ala Gly
        595                 600                 605

Ala Ala Ala Gly Ala Leu Ala Ala Ala Leu Ser Pro Met Glu Ile Tyr
    610                 615                 620

Gly Leu Val Gln Gln Ser His Tyr Ala Asp Gln Leu Asp Lys Leu Ala
625                 630                 635                 640

Gln Glu Ser Ser Ala Tyr Gly Tyr Glu Gly Asp Ala Leu Leu Ala Gln
                645                 650                 655

Leu Tyr Arg Asp Lys Thr Ala Ala Glu Gly Ala Val Ala Gly Val Ser
            660                 665                 670

Ala Val Leu Ser Thr Val Gly Ala Ala Val Ser Ile Ala Ala Ala Ala
        675                 680                 685

Ser Val Val Gly Ala Pro Val Ala Val Val Thr Ser Leu Leu Thr Gly
    690                 695                 700

Ala Leu Asn Gly Ile Leu Arg Gly Val Gln Gln Pro Ile Ile Glu Lys
705                 710                 715                 720

Leu Ala Asn Asp Tyr Ala Arg Lys Ile Asp Glu Leu Gly Gly Pro Gln
                725                 730                 735

Ala Tyr Phe Glu Lys Asn Leu Gln Ala Arg His Glu Gln Leu Ala Asn
        740                 745                 750

Ser Asp Gly Leu Arg Lys Met Leu Ala Asp Leu Gln Ala Gly Trp Asn
        755                 760                 765

Ala Ser Ser Val Ile Gly Val Gln Thr Thr Glu Ile Ser Lys Ser Ala
    770                 775                 780

Leu Glu Leu Ala Ala Ile Thr Gly Asn Ala Asp Asn Leu Lys Ser Ala
785                 790                 795                 800

Asp Val Phe Val Asp Arg Phe Ile Gln Gly Glu Arg Val Ala Gly Gln
                805                 810                 815

Pro Val Val Leu Asp Val Ala Ala Gly Gly Ile Asp Ile Ala Ser Arg
            820                 825                 830

Lys Gly Glu Arg Pro Ala Leu Thr Phe Ile Thr Pro Leu Ala Ala Pro
        835                 840                 845

Gly Glu Glu Gln Arg Arg Arg Thr Lys Thr Gly Lys Ser Glu Phe Thr
    850                 855                 860

Thr Phe Val Glu Ile Val Gly Lys Gln Asp Arg Trp Arg Ile Arg Asp
865                 870                 875                 880

Gly Ala Ala Asp Thr Thr Ile Asp Leu Ala Lys Val Val Ser Gln Leu
                885                 890                 895

Val Asp Ala Asn Gly Val Leu Lys His Ser Ile Lys Leu Glu Val Ile
            900                 905                 910

Gly Gly Asp Gly Asp Asp Val Val Leu Ala Asn Ala Ser Arg Ile His
        915                 920                 925

Tyr Asp Gly Gly Ala Gly Thr Asn Thr Val Ser Tyr Ala Ala Leu Gly
    930                 935                 940

Arg Gln Asp Ser Ile Thr Val Ser Ala Asp Gly Glu Arg Phe Asn Val
945                 950                 955                 960

Arg Lys Gln Leu Asn Asn Ala Asn Val Tyr Arg Glu Gly Val Ala Thr
                965                 970                 975

Gln Lys Thr Ala Tyr Gly Lys Arg Thr Glu Asn Val Gln Tyr Arg His
            980                 985                 990

Val Glu Leu Ala Arg Val Gly Gln Leu Val Glu Val Asp Thr Leu Glu
        995                 1000                1005

His Val Gln His Ile Ile Gly Gly Ala Gly Asn Asp Ser Ile Thr
    1010                1015                1020

Gly Asn Ala His Asp Asn Phe Leu Ala Gly Gly Ala Gly Asp Asp
    1025                1030                1035

Arg Leu Asp Gly Gly Ala Gly Asn Asp Thr Leu Val Gly Gly Glu
    1040                1045                1050

Gly His Asn Thr Val Val Gly Gly Ala Gly Asp Asp Val Phe Leu
    1055                1060                1065

Gln Asp Leu Gly Val Trp Ser Asn Gln Leu Asp Gly Gly Ala Gly
    1070                1075                1080

Val Asp Thr Val Lys Tyr Asn Val His Gln Pro Ser Glu Glu Arg
    1085                1090                1095

Leu Glu Arg Met Gly Asp Thr Gly Ile His Ala Asp Leu Gln Lys
    1100                1105                1110

Gly Thr Val Glu Lys Trp Pro Ala Leu Asn Leu Phe Ser Val Asp
    1115                1120                1125

His Val Lys Asn Ile Glu Asn Leu His Gly Ser Ser Leu Asn Asp
    1130                1135                1140

Ser Ile Ala Gly Asp Asp Arg Asp Asn Glu Leu Trp Gly Asp Asp
    1145                1150                1155

Gly Asn Asp Thr Ile His Gly Arg Gly Gly Asp Asp Ile Leu Arg
    1160                1165                1170

Gly Gly Leu Gly Leu Asp Thr Leu Tyr Gly Glu Asp Gly Asn Asp
    1175                1180                1185

Ile Phe Leu Gln Asp Asp Glu Thr Val Ser Asp Asp Ile Asp Gly
    1190                1195                1200

Gly Ala Gly Leu Asp Thr Val Asp Tyr Ser Ala Met Ile His Ala
    1205                1210                1215

Gly Lys Ile Val Ala Pro His Glu Tyr Gly Phe Gly Ile Glu Ala

1220                1225                1230

Asp Leu Ser Glu Gly Trp Val Arg Lys Ala Ala Arg Arg Gly Met
    1235                1240                1245

Gly Tyr Tyr Asp Ser Val Arg Ser Val Glu Asn Val Ile Gly Thr
    1250                1255                1260

Ser Met Lys Asp Val Leu Ile Gly Asp Ala Gln Ala Asn Thr Leu
    1265                1270                1275

Met Gly Gln Gly Gly Asp Asp Thr Val Arg Gly Gly Asp Gly Asp
    1280                1285                1290

Asp Leu Leu Phe Gly Gly Asp Gly Asn Asp Met Leu Tyr Gly Asp
    1295                1300                1305

Ala Gly Asn Asp Thr Leu Tyr Gly Gly Leu Gly Asp Asp Thr Leu
    1310                1315                1320

Glu Gly Gly Ala Gly Asn Asp Trp Phe Gly Gln Thr Pro Ala Arg
    1325                1330                1335

Glu His Asp Val Leu Arg Gly Gly Ala Gly Val Asp Thr Val Asp
    1340                1345                1350

Tyr Ser Gln Ala Gly Ala His Ala Gly Val Ala Thr Gly Arg Ile
    1355                1360                1365

Gly Leu Gly Ile Leu Ala Asp Leu Gly Ala Gly Arg Val Asp Lys
    1370                1375                1380

Leu Gly Glu Ala Gly Ser Ser Ala Tyr Asp Thr Val Ser Gly Ile
    1385                1390                1395

Glu Asn Val Val Gly Thr Glu Leu Ala Asp Arg Ile Thr Gly Asp
    1400                1405                1410

Ala Gln Ala Asn Val Leu Arg Gly Ala Gly Gly Ala Asp Val Leu
    1415                1420                1425

Ala Gly Gly Glu Gly Asp Asp Val Leu Leu Gly Gly Glu Gly Asp
    1430                1435                1440

Asp Gln Leu Ser Gly Asp Ala Gly Arg Asp Arg Leu Tyr Gly Glu
    1445                1450                1455

Ala Gly Asp Asp Trp Phe Phe Gln Asp Ala Ala Asn Ala Gly Asn
    1460                1465                1470

Leu Leu Asp Gly Gly Asp Gly Asn Asp Thr Val Asp Phe Ser Gly
    1475                1480                1485

Pro Gly Arg Gly Leu Asp Ala Gly Ala Lys Gly Val Phe Leu Ser
    1490                1495                1500

Leu Gly Lys Gly Phe Ala Ser Leu Met Asp Glu Pro Glu Thr Ser
    1505                1510                1515

Asn Val Leu Arg His Ile Glu Asn Ala Val Gly Ser Val Arg Asp
    1520                1525                1530

Asp Val Leu Ile Gly Asp Ala Gly Ala Asn Val Leu Asn Gly Leu
    1535                1540                1545

Ala Gly Asn Asp Val Leu Ser Gly Gly Ala Gly Asp Asp Val Leu
    1550                1555                1560

Leu Gly Asp Glu Gly Ser Asp Leu Leu Ser Gly Asp Ala Gly Asn
    1565                1570                1575

Asp Asp Leu Phe Gly Gly Gln Gly Asp Asp Thr Tyr Leu Phe Gly
    1580                1585                1590

Ala Gly Tyr Gly His Asp Thr Ile Tyr Glu Ser Gly Gly Gly His
    1595                1600                1605

Asp Thr Ile Arg Ile Asn Ala Gly Ala Asp Gln Leu Trp Phe Ala
    1610                1615                1620

Arg Gln Gly Asn Asp Leu Glu Ile Arg Ile Leu Gly Thr Asp Asp
    1625                1630                1635

Ala Leu Thr Val His Asp Trp Tyr Arg Asp Ala Asp His Arg Val
    1640                1645                1650

Glu Ala Ile His Ala Ala Asn Gln Ala Ile Asp Pro Ala Gly Ile
    1655                1660                1665

Glu Lys Leu Val Glu Ala Met Ala Gln Tyr Pro Asp Pro Gly Ala
    1670                1675                1680

Ala Ala Ala Ala Pro Pro Ala Ala Arg Val Pro Asp Thr Leu Met
    1685                1690                1695

Gln Ser Leu Ala Val Asn Trp Arg
    1700                1705

<210>  7
<211>  5118
<212>  DNA
<213>  Bordetella bronchiseptica (wild type CyaA)

<220>
<221>  CDS
<222>  (1)..(5118)

<400>  7
atg cag caa tcg cat cag gct ggt tac gca aac gcc gcc gac cgg gag      48
Met Gln Gln Ser His Gln Ala Gly Tyr Ala Asn Ala Ala Asp Arg Glu
1               5                   10                  15

tct ggc atc ccc gca gcc gta ctc gat ggc atc aag gcc gtg gcg aag      96
Ser Gly Ile Pro Ala Ala Val Leu Asp Gly Ile Lys Ala Val Ala Lys
            20                  25                  30

gaa aaa aac gcc aca ttg atg ttc cgc ctg gtc aac ccc cat tcc acc      144
Glu Lys Asn Ala Thr Leu Met Phe Arg Leu Val Asn Pro His Ser Thr
        35                  40                  45

agc ctg att gcc gaa ggg gtg gcc acc aaa gga ttg ggc gtg cac gcc      192
Ser Leu Ile Ala Glu Gly Val Ala Thr Lys Gly Leu Gly Val His Ala
    50                  55                  60

aag tcg tcc gat tgg ggg ttg cag gcg ggc tac att ccc gtc aac ccg      240
Lys Ser Ser Asp Trp Gly Leu Gln Ala Gly Tyr Ile Pro Val Asn Pro
65                  70                  75                  80

aat ctt tcc aaa ctg ttc ggc cgt gcg ccc gag gtg atc gcg cgg gcc      288
Asn Leu Ser Lys Leu Phe Gly Arg Ala Pro Glu Val Ile Ala Arg Ala
                85                  90                  95

gac aac gac gtc aac agc agc ctg gcg cat ggc cat acc gcg gtc gac      336
Asp Asn Asp Val Asn Ser Ser Leu Ala His Gly His Thr Ala Val Asp
            100                 105                 110

ctg acg ctg tcg aaa gag cgg ctt gac tat ctg cgg caa gcg ggc ctg      384
Leu Thr Leu Ser Lys Glu Arg Leu Asp Tyr Leu Arg Gln Ala Gly Leu
        115                 120                 125

gtc acc ggc atg gcc gat ggc gtg gtc gcg agc aac cac gca ggc tac      432
Val Thr Gly Met Ala Asp Gly Val Val Ala Ser Asn His Ala Gly Tyr
    130                 135                 140

gag cag ttc gag ttt cgc gtg aag gaa acc tcg gac ggg cgc tat gcc      480
Glu Gln Phe Glu Phe Arg Val Lys Glu Thr Ser Asp Gly Arg Tyr Ala
145                 150                 155                 160

gtg cag tat cgc cgc aag ggc ggc gac gat ttc gag gcg gtc aag gtg      528
Val Gln Tyr Arg Arg Lys Gly Gly Asp Asp Phe Glu Ala Val Lys Val
                165                 170                 175

atc ggc aat gcc gcc ggt att cca ctg acg gcg gat atc gac atg ttc      576
Ile Gly Asn Ala Ala Gly Ile Pro Leu Thr Ala Asp Ile Asp Met Phe
            180                 185                 190

gcc atc atg ccg cat ctg tcc aac ttc cgc gac tcg gcg cgc agt tcg 624
Ala Ile Met Pro His Leu Ser Asn Phe Arg Asp Ser Ala Arg Ser Ser
        195                 200                 205

gtg acc agc ggc gat tcg gtg acc gat tac ctg gcg cgc acg cgg cgg 672
Val Thr Ser Gly Asp Ser Val Thr Asp Tyr Leu Ala Arg Thr Arg Arg
    210                 215                 220

gcc gcc agc gag gcc acg ggc ggc ctg gat cgc gaa cgc atc gac ttg 720
Ala Ala Ser Glu Ala Thr Gly Gly Leu Asp Arg Glu Arg Ile Asp Leu
225                 230                 235                 240

ttg tgg aaa atc gct cgc gcc ggc gcc cgt tcc gca gtg ggc acc gag 768
Leu Trp Lys Ile Ala Arg Ala Gly Ala Arg Ser Ala Val Gly Thr Glu
                245                 250                 255

gcg cgt cgc cag ttc cgc tac gac ggc gac atg aat atc ggc gtg atc 816
Ala Arg Arg Gln Phe Arg Tyr Asp Gly Asp Met Asn Ile Gly Val Ile
            260                 265                 270

acc gat ttc gag ctg gaa gtg cgc aat gcg ctg aac agg cgg gcg cac 864
Thr Asp Phe Glu Leu Glu Val Arg Asn Ala Leu Asn Arg Arg Ala His
        275                 280                 285

gcg gtc ggc agg cag gac gtg gtc cag cat ggc act gag cag aac aat 912
Ala Val Gly Arg Gln Asp Val Val Gln His Gly Thr Glu Gln Asn Asn
    290                 295                 300

cct ttc ccg gag gca gat gag aag att ttc gtc gta tcg gcc acc ggt 960
Pro Phe Pro Glu Ala Asp Glu Lys Ile Phe Val Val Ser Ala Thr Gly
305                 310                 315                 320

gaa agc cag atg ctc acg cgc ggg caa ctg aag gaa tac att ggc cag 1008
Glu Ser Gln Met Leu Thr Arg Gly Gln Leu Lys Glu Tyr Ile Gly Gln
                325                 330                 335

cag cgc ggc gag ggc tat gtc ttc tac gag aac cgt gcg tac ggc gtg 1056
Gln Arg Gly Glu Gly Tyr Val Phe Tyr Glu Asn Arg Ala Tyr Gly Val
            340                 345                 350

gcg ggg aaa agc ctg ttc gac gat ggg ctg gga gcc gcg ccc ggc gtg 1104
Ala Gly Lys Ser Leu Phe Asp Asp Gly Leu Gly Ala Ala Pro Gly Val
        355                 360                 365

ccg ggg cga cgt tcg aag tcc tcg ccg gat gta ctg gaa acg gtg ccg 1152
Pro Gly Arg Arg Ser Lys Ser Ser Pro Asp Val Leu Glu Thr Val Pro
    370                 375                 380

gcg tca ccc gga ttg cgg cgg ccg tcg ctg ggc gca gtg gaa cgc cag 1200
Ala Ser Pro Gly Leu Arg Arg Pro Ser Leu Gly Ala Val Glu Arg Gln
385                 390                 395                 400

gat tcc ggc tat gac agc ctt gat ggg gtg gga tcg cga tcg ttc tcg 1248
Asp Ser Gly Tyr Asp Ser Leu Asp Gly Val Gly Ser Arg Ser Phe Ser
                405                 410                 415

ttg ggc gag gtg tcc gac atg gcc gcc gtg gaa gcg gcg gaa ctg gaa 1296
Leu Gly Glu Val Ser Asp Met Ala Ala Val Glu Ala Ala Glu Leu Glu
            420                 425                 430

atg acc cgg caa gtc ttg cac gcc ggg gcg cgg cag gac gat gcc gag 1344
Met Thr Arg Gln Val Leu His Ala Gly Ala Arg Gln Asp Asp Ala Glu

435                 440                 445

ccg ggc gtg agc ggt gcg tcg gcg cac tgg ggg cag cgg gcg ctg cag        1392
Pro Gly Val Ser Gly Ala Ser Ala His Trp Gly Gln Arg Ala Leu Gln
    450                 455                 460

ggc gcc cag gcg gtg gcg gcg gcg cag cgg ctg gtt cat gcc att gcc        1440
Gly Ala Gln Ala Val Ala Ala Ala Gln Arg Leu Val His Ala Ile Ala
465                 470                 475                 480

ctg atg acg caa ttc ggc cgg gcc ggt tcc acc aac acg ccg cag gaa        1488
Leu Met Thr Gln Phe Gly Arg Ala Gly Ser Thr Asn Thr Pro Gln Glu
                485                 490                 495

gcg gcc tcg ttg tcg gcg gcc gtg ttc ggc ttg ggc gag gcc agc agc        1536
Ala Ala Ser Leu Ser Ala Ala Val Phe Gly Leu Gly Glu Ala Ser Ser
            500                 505                 510

gcc gtg gcc gaa acc gtg agc ggt ttt ttc cgc ggg tct tcg cgc tgg        1584
Ala Val Ala Glu Thr Val Ser Gly Phe Phe Arg Gly Ser Ser Arg Trp
        515                 520                 525

gcc ggc ggt ttc ggc gtg gct ggc ggc gcg atg gcg ctg gga ggc ggc        1632
Ala Gly Gly Phe Gly Val Ala Gly Gly Ala Met Ala Leu Gly Gly Gly
    530                 535                 540

atc ggc gcc gtt ggc gcc ggg atg tcg ttg acc gat gac gcg ccg gcc        1680
Ile Gly Ala Val Gly Ala Gly Met Ser Leu Thr Asp Asp Ala Pro Ala
545                 550                 555                 560

gga cag aag gcc gcc gcc ggc gcc gag atc gcg ctg cag ttg aca ggt        1728
Gly Gln Lys Ala Ala Ala Gly Ala Glu Ile Ala Leu Gln Leu Thr Gly
                565                 570                 575

gga acg gtc gag ctg gct tct tcc atc gcg ttg gcg ctg gcc gcg gcg        1776
Gly Thr Val Glu Leu Ala Ser Ser Ile Ala Leu Ala Leu Ala Ala Ala
            580                 585                 590

cgc ggc gtg acc agc ggc ttg cag gtg gcg ggg gcg tcg gcc ggg gcg        1824
Arg Gly Val Thr Ser Gly Leu Gln Val Ala Gly Ala Ser Ala Gly Ala
        595                 600                 605

gct gcc ggc gca ttg gcc gcg gcg ctc agt ccc atg gag atc tac ggc        1872
Ala Ala Gly Ala Leu Ala Ala Ala Leu Ser Pro Met Glu Ile Tyr Gly
    610                 615                 620

ctg gtg cag caa tcg cac tat gcg gat cag ctg gac aag ctg gcg cag        1920
Leu Val Gln Gln Ser His Tyr Ala Asp Gln Leu Asp Lys Leu Ala Gln
625                 630                 635                 640

gaa tcg agc gca tac ggt tac gag ggc gac gcc ttg ctg gcc cag ctg        1968
Glu Ser Ser Ala Tyr Gly Tyr Glu Gly Asp Ala Leu Leu Ala Gln Leu
                645                 650                 655

tat cgc gac aag acg gcc gcc gag ggc gcc gtc gcc ggc gtc tcc gcc        2016
Tyr Arg Asp Lys Thr Ala Ala Glu Gly Ala Val Ala Gly Val Ser Ala
            660                 665                 670

gtc ctg agc acg gtg ggg gct gcg gtg tcg atc gcc gcg gcg gcc agc        2064
Val Leu Ser Thr Val Gly Ala Ala Val Ser Ile Ala Ala Ala Ala Ser
        675                 680                 685

gtg gta ggc gcc ccg gtg gcg gtg gtc act tcc ttg ttg acc ggg gct        2112

Val Val Gly Ala Pro Val Ala Val Val Thr Ser Leu Leu Thr Gly Ala
    690                 695                 700

ctc aac ggc atc ctg cgc ggc gtg cag cag ccc atc atc gaa aag ctg      2160
Leu Asn Gly Ile Leu Arg Gly Val Gln Gln Pro Ile Ile Glu Lys Leu
705                 710                 715                 720

gcc aat gat tac gct cgc aag atc gac gag ctg ggc ggg ccg caa gcg      2208
Ala Asn Asp Tyr Ala Arg Lys Ile Asp Glu Leu Gly Gly Pro Gln Ala
                725                 730                 735

tac ttc gag aaa aac ctg cag gcg cgt cac gaa caa ctg gcc aat tcg      2256
Tyr Phe Glu Lys Asn Leu Gln Ala Arg His Glu Gln Leu Ala Asn Ser
            740                 745                 750

gac ggc cta cgg aaa atg ctg gcc gac ctg cag gcc ggg tgg aac gcc      2304
Asp Gly Leu Arg Lys Met Leu Ala Asp Leu Gln Ala Gly Trp Asn Ala
        755                 760                 765

agc agc gtg atc ggg gtg cag acg aca gag att tcc aag tcg gcg ctc      2352
Ser Ser Val Ile Gly Val Gln Thr Thr Glu Ile Ser Lys Ser Ala Leu
    770                 775                 780

gaa ctg gcc gcc att acc ggc aac gcg gac aac ctg aaa tcc gcc gac      2400
Glu Leu Ala Ala Ile Thr Gly Asn Ala Asp Asn Leu Lys Ser Ala Asp
785                 790                 795                 800

gtg ttc gtg gac cgc ttc atc cag ggc gag cgg gtg gcc ggc cag ccg      2448
Val Phe Val Asp Arg Phe Ile Gln Gly Glu Arg Val Ala Gly Gln Pro
                805                 810                 815

gtg gta ctc gac gtc gcc gcc ggc ggc atc gat atc gcc agc cgc aag      2496
Val Val Leu Asp Val Ala Ala Gly Gly Ile Asp Ile Ala Ser Arg Lys
            820                 825                 830

ggc gag cgg ccg gcg ctg acg ttc atc acg ccg ctg gcc gcg cca gga      2544
Gly Glu Arg Pro Ala Leu Thr Phe Ile Thr Pro Leu Ala Ala Pro Gly
        835                 840                 845

gaa gag cag cgc cgg cgc acg aaa acg ggc aag agc gaa ttc acc aca      2592
Glu Glu Gln Arg Arg Arg Thr Lys Thr Gly Lys Ser Glu Phe Thr Thr
    850                 855                 860

ttc gtc gag atc gtg ggc aag cag gac cgc tgg cgc atc cgg gac ggc      2640
Phe Val Glu Ile Val Gly Lys Gln Asp Arg Trp Arg Ile Arg Asp Gly
865                 870                 875                 880

gcg gcc gac acc acc atc gat ctg gcc aag gtg gtg tcg caa ctg gtc      2688
Ala Ala Asp Thr Thr Ile Asp Leu Ala Lys Val Val Ser Gln Leu Val
                885                 890                 895

gac gcc aat ggc gtg ctc aag cac agc atc aaa ctg gag gtg atc ggc      2736
Asp Ala Asn Gly Val Leu Lys His Ser Ile Lys Leu Glu Val Ile Gly
            900                 905                 910

gga gat ggc gat gat gtc gtg ctt gcc aat gct tcg cgc atc cat tac      2784
Gly Asp Gly Asp Asp Val Val Leu Ala Asn Ala Ser Arg Ile His Tyr
        915                 920                 925

gac ggc ggc gcg gga acc aac acg gtc agc tat gcc gcc ctg ggc cga      2832
Asp Gly Gly Ala Gly Thr Asn Thr Val Ser Tyr Ala Ala Leu Gly Arg
    930                 935                 940

cag gat tcc att acc gtg tcc gcc gac ggg gaa cgt ttc aac gtg cgc 2880
Gln Asp Ser Ile Thr Val Ser Ala Asp Gly Glu Arg Phe Asn Val Arg
945                 950                 955                 960

aag cag ttg aac aac gcc aac gtg tat cgc gaa ggc gtg gct acc cag 2928
Lys Gln Leu Asn Asn Ala Asn Val Tyr Arg Glu Gly Val Ala Thr Gln
                965                 970                 975

aaa acc gcc tac ggc aag cgc acg gag aat gtc caa tac cgc cat gtc 2976
Lys Thr Ala Tyr Gly Lys Arg Thr Glu Asn Val Gln Tyr Arg His Val
            980                 985                 990

gag ctg gcc cgt gtc ggg caa ctg gtg gag gtc gac acg ctc gag cat 3024
Glu Leu Ala Arg Val Gly Gln Leu Val Glu Val Asp Thr Leu Glu His
        995                 1000                1005

gtg cag cac atc atc ggc ggg gcc ggc aac gat tcg atc acc ggc 3069
Val Gln His Ile Ile Gly Gly Ala Gly Asn Asp Ser Ile Thr Gly
    1010                1015                1020

aat gcg cac gac aac ttc ctg gcc ggc ggg gcg ggc gac gac agg 3114
Asn Ala His Asp Asn Phe Leu Ala Gly Gly Ala Gly Asp Asp Arg
    1025                1030                1035

ctg gat ggc ggc gcc ggc aac gac aca ctg gtc ggc ggc gag ggc 3159
Leu Asp Gly Gly Ala Gly Asn Asp Thr Leu Val Gly Gly Glu Gly
    1040                1045                1050

cac aac acg gtc gtc ggc ggc gct ggc gac gac gta ttc ctg cag 3204
His Asn Thr Val Val Gly Gly Ala Gly Asp Asp Val Phe Leu Gln
    1055                1060                1065

gac ctg ggg gta tgg agc aac cag ctc gat ggc ggc gcg ggc gtc 3249
Asp Leu Gly Val Trp Ser Asn Gln Leu Asp Gly Gly Ala Gly Val
    1070                1075                1080

gat acc gtg aag tac aac gtg cac cag cct tcc gag gaa cgc ctc 3294
Asp Thr Val Lys Tyr Asn Val His Gln Pro Ser Glu Glu Arg Leu
    1085                1090                1095

gaa cgc atg ggc gac acg ggc atc cat gcc gat ctt caa aag ggc 3339
Glu Arg Met Gly Asp Thr Gly Ile His Ala Asp Leu Gln Lys Gly
    1100                1105                1110

acg gtc gag aag tgg ccg gcc ctg aac ctg ttc agc gtc gac cat 3384
Thr Val Glu Lys Trp Pro Ala Leu Asn Leu Phe Ser Val Asp His
    1115                1120                1125

gtc aag aat atc gag aat ctg cac ggc tcc agc ctg aac gac agc 3429
Val Lys Asn Ile Glu Asn Leu His Gly Ser Ser Leu Asn Asp Ser
    1130                1135                1140

atc gcc ggc gac gac cgg gac aac gag ctc tgg ggc gac gat ggc 3474
Ile Ala Gly Asp Asp Arg Asp Asn Glu Leu Trp Gly Asp Asp Gly
    1145                1150                1155

aac gac acg ata cac ggc cgg ggc ggc gac gat atc ctg cgc ggc 3519
Asn Asp Thr Ile His Gly Arg Gly Gly Asp Asp Ile Leu Arg Gly
    1160                1165                1170

ggc ctg ggc ctg gac acg ctg tat ggc gag gac ggc aac gac atc 3564
Gly Leu Gly Leu Asp Thr Leu Tyr Gly Glu Asp Gly Asn Asp Ile
    1175                1180                1185

ttc ctg cag gac gac gag acc gtc agc gat gac atc gac ggt ggc 3609
Phe Leu Gln Asp Asp Glu Thr Val Ser Asp Asp Ile Asp Gly Gly
    1190                1195                1200

gcg gga ctg gac acc gtc gac tat tcc gcc atg atc cat gca ggc 3654
Ala Gly Leu Asp Thr Val Asp Tyr Ser Ala Met Ile His Ala Gly
    1205                1210                1215

aag atc gtt gcg ccg cat gaa tac ggc ttc ggg atc gag gcg gac 3699
Lys Ile Val Ala Pro His Glu Tyr Gly Phe Gly Ile Glu Ala Asp
    1220                1225                1230

ctg tcc gaa ggg tgg gtg cgc aag gcg gcc cgg cgc ggc atg gac 3744
Leu Ser Glu Gly Trp Val Arg Lys Ala Ala Arg Arg Gly Met Asp
    1235                1240                1245

tac tac gac agt gtc cgc agt gtc gaa aac gtc atc ggc acg agc 3789
Tyr Tyr Asp Ser Val Arg Ser Val Glu Asn Val Ile Gly Thr Ser
    1250                1255                1260

atg aag gat gtg ctc atc ggc gac gcg caa gcc aat acc ctg atg 3834
Met Lys Asp Val Leu Ile Gly Asp Ala Gln Ala Asn Thr Leu Met
    1265                1270                1275

ggc cag ggc ggc gac gat acc gtg cgc ggc ggc gac ggc gat gat 3879
Gly Gln Gly Gly Asp Asp Thr Val Arg Gly Gly Asp Gly Asp Asp
    1280                1285                1290

ctg ctg ttc ggc ggc gac ggc aac gac atg ctg tat gga gac gcc 3924
Leu Leu Phe Gly Gly Asp Gly Asn Asp Met Leu Tyr Gly Asp Ala
    1295                1300                1305

ggc aac gac acc ctc tac gga ggg ctg ggc gac gat acc ctt gaa 3969
Gly Asn Asp Thr Leu Tyr Gly Gly Leu Gly Asp Asp Thr Leu Glu
    1310                1315                1320

ggc ggc gcg ggc aac gat tgg ttc ggc cag acg ccg gcg cgc gag 4014
Gly Gly Ala Gly Asn Asp Trp Phe Gly Gln Thr Pro Ala Arg Glu
    1325                1330                1335

cat gac gtg ctg cgc ggc ggg gct ggg gtg gat acc gtg gat tac 4059
His Asp Val Leu Arg Gly Gly Ala Gly Val Asp Thr Val Asp Tyr
    1340                1345                1350

agc cag gcg ggc gcg cat gcc ggc gtt gcc acg ggt cgc atc ggg 4104
Ser Gln Ala Gly Ala His Ala Gly Val Ala Thr Gly Arg Ile Gly
    1355                1360                1365

ctg ggt att ctg gcg gac ctg ggc gcc ggc cgc gtc gac aag ctg 4149
Leu Gly Ile Leu Ala Asp Leu Gly Ala Gly Arg Val Asp Lys Leu
    1370                1375                1380

ggc gag gcc ggc agc agc gcc tac gat acg gtt tcc ggc atc gaa 4194
Gly Glu Ala Gly Ser Ser Ala Tyr Asp Thr Val Ser Gly Ile Glu
    1385                1390                1395

aat gtg gtg ggc acg gaa ctg gcc gac cgc atc acg ggc gat gcg 4239
Asn Val Val Gly Thr Glu Leu Ala Asp Arg Ile Thr Gly Asp Ala
    1400                1405                1410

cag gcc aac gta ctg cgc ggc gcg ggt ggc gcc gac gtg ctt gcg 4284
Gln Ala Asn Val Leu Arg Gly Ala Gly Gly Ala Asp Val Leu Ala

1415                1420                1425

ggc ggc gag ggc gac gat gtg ctg ctg ggc ggc gac ggc gac gac      4329
Gly Gly Glu Gly Asp Asp Val Leu Leu Gly Gly Asp Gly Asp Asp
    1430                1435                1440

cag ctg tcg ggc gac gcc gga cgc gac cgc ttg tac ggc gaa gcc      4374
Gln Leu Ser Gly Asp Ala Gly Arg Asp Arg Leu Tyr Gly Glu Ala
    1445                1450                1455

ggt gac gac tgg ttc ttc cag gat gcc gcc aat gcc ggc aat ctg      4419
Gly Asp Asp Trp Phe Phe Gln Asp Ala Ala Asn Ala Gly Asn Leu
    1460                1465                1470

ctc gac ggt ggt gac ggc aac gat acc gtg gat ttc agc ggc ccg      4464
Leu Asp Gly Gly Asp Gly Asn Asp Thr Val Asp Phe Ser Gly Pro
    1475                1480                1485

ggc cgg ggc ctc gac gcc ggc gca aag ggc gta ttc ctg agc ctg      4509
Gly Arg Gly Leu Asp Ala Gly Ala Lys Gly Val Phe Leu Ser Leu
    1490                1495                1500

ggc aag ggg ttc gcc agc ctg atg gac gaa ccc gaa acc agc aac      4554
Gly Lys Gly Phe Ala Ser Leu Met Asp Glu Pro Glu Thr Ser Asn
    1505                1510                1515

gtg ttg cgc cat atc gag aac gcc gtg ggc agc gtg cgt gat gac      4599
Val Leu Arg His Ile Glu Asn Ala Val Gly Ser Val Arg Asp Asp
    1520                1525                1530

gtg ctg atc ggc gac gca ggc gcc aac gtc ctc aat ggc ctg gcg      4644
Val Leu Ile Gly Asp Ala Gly Ala Asn Val Leu Asn Gly Leu Ala
    1535                1540                1545

ggc aac gac gtg ctg tcc ggc ggc gcc ggc gac gat gtg ctg ctg      4689
Gly Asn Asp Val Leu Ser Gly Gly Ala Gly Asp Asp Val Leu Leu
    1550                1555                1560

ggc gac gag ggc tcg gac ctg ctc agc ggc gat gcg ggc aac gac      4734
Gly Asp Glu Gly Ser Asp Leu Leu Ser Gly Asp Ala Gly Asn Asp
    1565                1570                1575

gat ctg ttc ggc ggg cag ggc gat gat acc tat ctg ttc ggg gcc      4779
Asp Leu Phe Gly Gly Gln Gly Asp Asp Thr Tyr Leu Phe Gly Ala
    1580                1585                1590

ggg tac gga cat gac acg atc tac gaa tcg ggc ggc ggc cat gac      4824
Gly Tyr Gly His Asp Thr Ile Tyr Glu Ser Gly Gly Gly His Asp
    1595                1600                1605

acc atc cgt atc aac gcg ggg gcg gac cag ctg tgg ttt gcg cgc      4869
Thr Ile Arg Ile Asn Ala Gly Ala Asp Gln Leu Trp Phe Ala Arg
    1610                1615                1620

cag ggc aac gac ctg gag atc cgc att ctt ggc acc gac gat gca      4914
Gln Gly Asn Asp Leu Glu Ile Arg Ile Leu Gly Thr Asp Asp Ala
    1625                1630                1635

ctt acc gtg cac gac tgg tat cgc gac gcc gat cac cgg gtg gaa      4959
Leu Thr Val His Asp Trp Tyr Arg Asp Ala Asp His Arg Val Glu
    1640                1645                1650

gcc atc cat gcc gcc aac cag gcc ata gac ccg gcc ggc atc gaa      5004

Ala Ile His Ala Ala Asn Gln Ala Ile Asp Pro Ala Gly Ile Glu
    1655                1660                1665

aag ctg gtc gag gca atg gcg cag tac ccg gac ccc ggc gcg gcg 5049
Lys Leu Val Glu Ala Met Ala Gln Tyr Pro Asp Pro Gly Ala Ala
    1670                1675                1680

gcg gct gcc ccg ccg gcg gcg cgc gtg ccg gac acg ctg atg cag 5094
Ala Ala Ala Pro Pro Ala Ala Arg Val Pro Asp Thr Leu Met Gln
    1685                1690                1695

tcc ctg gct gtc aac tgg cgc tga 5118
Ser Leu Ala Val Asn Trp Arg
    1700                1705

<210> 8
<211> 1705
<212> PRT
<213> Bordetella bronchiseptica (wild type CyaA)

<400> 8
   Met Gln Gln Ser His Gln Ala Gly Tyr Ala Asn Ala Ala Asp Arg Glu
   1 5 10 15
   Ser Gly Ile Pro Ala Ala Val Leu Asp Gly Ile Lys Ala Val Ala Lys
   20 25 30
   Glu Lys Asn Ala Thr Leu Met Phe Arg Leu Val Asn Pro His Ser Thr
   35 40 45
   Ser Leu Ile Ala Glu Gly Val Ala Thr Lys Gly Leu Gly Val His Ala
   50 55 60
   Lys Ser Ser Asp Trp Gly Leu Gln Ala Gly Tyr Ile Pro Val Asn Pro
   65 70 75 80
   Asn Leu Ser Lys Leu Phe Gly Arg Ala Pro Glu Val Ile Ala Arg Ala
   85 90 95
   Asp Asn Asp Val Asn Ser Ser Leu Ala His Gly His Thr Ala Val Asp
   100 105 110
   Leu Thr Leu Ser Lys Glu Arg Leu Asp Tyr Leu Arg Gln Ala Gly Leu
   115 120 125
   Val Thr Gly Met Ala Asp Gly Val Val Ala Ser Asn His Ala Gly Tyr
   130 135 140
   Glu Gln Phe Glu Phe Arg Val Lys Glu Thr Ser Asp Gly Arg Tyr Ala
   145 150 155 160
   Val Gln Tyr Arg Arg Lys Gly Gly Asp Asp Phe Glu Ala Val Lys Val
   165 170 175
   Ile Gly Asn Ala Ala Gly Ile Pro Leu Thr Ala Asp Ile Asp Met Phe
   180 185 190
   Ala Ile Met Pro His Leu Ser Asn Phe Arg Asp Ser Ala Arg Ser Ser
   195 200 205
   Val Thr Ser Gly Asp Ser Val Thr Asp Tyr Leu Ala Arg Thr Arg Arg
   210 215 220
   Ala Ala Ser Glu Ala Thr Gly Gly Leu Asp Arg Glu Arg Ile Asp Leu
   225 230 235 240
   Leu Trp Lys Ile Ala Arg Ala Gly Ala Arg Ser Ala Val Gly Thr Glu
   245 250 255
   Ala Arg Arg Gln Phe Arg Tyr Asp Gly Asp Met Asn Ile Gly Val Ile
   260 265 270
   Thr Asp Phe Glu Leu Glu Val Arg Asn Ala Leu Asn Arg Arg Ala His
   275 280 285
   Ala Val Gly Arg Gln Asp Val Val Gln His Gly Thr Glu Gln Asn Asn
   290 295 300
   Pro Phe Pro Glu Ala Asp Glu Lys Ile Phe Val Val Ser Ala Thr Gly
   305 310 315 320
   Glu Ser Gln Met Leu Thr Arg Gly Gln Leu Lys Glu Tyr Ile Gly Gln
   325 330 335
   Gln Arg Gly Glu Gly Tyr Val Phe Tyr Glu Asn Arg Ala Tyr Gly Val
   340 345 350
   Ala Gly Lys Ser Leu Phe Asp Asp Gly Leu Gly Ala Ala Pro Gly Val
   355 360 365
   Pro Gly Arg Arg Ser Lys Ser Ser Pro Asp Val Leu Glu Thr Val Pro
   370 375 380
   Ala Ser Pro Gly Leu Arg Arg Pro Ser Leu Gly Ala Val Glu Arg Gln
   385 390 395 400
   Asp Ser Gly Tyr Asp Ser Leu Asp Gly Val Gly Ser Arg Ser Phe Ser
   405 410 415
   Leu Gly Glu Val Ser Asp Met Ala Ala Val Glu Ala Ala Glu Leu Glu
           420                 425                 430
   Met Thr Arg Gln Val Leu His Ala Gly Ala Arg Gln Asp Asp Ala Glu
       435                 440                 445
   Pro Gly Val Ser Gly Ala Ser Ala His Trp Gly Gln Arg Ala Leu Gln
       450                 455                 460
   Gly Ala Gln Ala Val Ala Ala Ala Gln Arg Leu Val His Ala Ile Ala
   465                 470                 475                 480
   Leu Met Thr Gln Phe Gly Arg Ala Gly Ser Thr Asn Thr Pro Gln Glu
                   485                 490                 495
   Ala Ala Ser Leu Ser Ala Ala Val Phe Gly Leu Gly Glu Ala Ser Ser
           500                 505                 510
   Ala Val Ala Glu Thr Val Ser Gly Phe Phe Arg Gly Ser Ser Arg Trp
       515                 520                 525
   Ala Gly Gly Phe Gly Val Ala Gly Gly Ala Met Ala Leu Gly Gly Gly
       530                 535                 540
   Ile Gly Ala Val Gly Ala Gly Met Ser Leu Thr Asp Asp Ala Pro Ala
   545                 550                 555                 560
   Gly Gln Lys Ala Ala Ala Gly Ala Glu Ile Ala Leu Gln Leu Thr Gly
                   565                 570                 575
   Gly Thr Val Glu Leu Ala Ser Ser Ile Ala Leu Ala Leu Ala Ala Ala
           580                 585                 590
   Arg Gly Val Thr Ser Gly Leu Gln Val Ala Gly Ala Ser Ala Gly Ala
       595                 600                 605
   Ala Ala Gly Ala Leu Ala Ala Ala Leu Ser Pro Met Glu Ile Tyr Gly
       610                 615                 620
   Leu Val Gln Gln Ser His Tyr Ala Asp Gln Leu Asp Lys Leu Ala Gln
   625                 630                 635                 640
   Glu Ser Ser Ala Tyr Gly Tyr Glu Gly Asp Ala Leu Leu Ala Gln Leu
                   645                 650                 655
   Tyr Arg Asp Lys Thr Ala Ala Glu Gly Ala Val Ala Gly Val Ser Ala
           660                 665                 670
   Val Leu Ser Thr Val Gly Ala Ala Val Ser Ile Ala Ala Ala Ala Ser
           675                 680                 685
   Val Val Gly Ala Pro Val Ala Val Val Thr Ser Leu Leu Thr Gly Ala
       690                 695                 700
   Leu Asn Gly Ile Leu Arg Gly Val Gln Gln Pro Ile Ile Glu Lys Leu
   705                 710                 715                 720
   Ala Asn Asp Tyr Ala Arg Lys Ile Asp Glu Leu Gly Gly Pro Gln Ala
                   725                 730                 735
   Tyr Phe Glu Lys Asn Leu Gln Ala Arg His Glu Gln Leu Ala Asn Ser
               740                 745                 750
   Asp Gly Leu Arg Lys Met Leu Ala Asp Leu Gln Ala Gly Trp Asn Ala
           755                 760                 765
   Ser Ser Val Ile Gly Val Gln Thr Thr Glu Ile Ser Lys Ser Ala Leu
       770                 775                 780
   Glu Leu Ala Ala Ile Thr Gly Asn Ala Asp Asn Leu Lys Ser Ala Asp
   785                 790                 795                 800
   Val Phe Val Asp Arg Phe Ile Gln Gly Glu Arg Val Ala Gly Gln Pro
                   805                 810                 815
   Val Val Leu Asp Val Ala Ala Gly Gly Ile Asp Ile Ala Ser Arg Lys
               820                 825                 830
   Gly Glu Arg Pro Ala Leu Thr Phe Ile Thr Pro Leu Ala Ala Pro Gly
           835                 840                 845
   Glu Glu Gln Arg Arg Arg Thr Lys Thr Gly Lys Ser Glu Phe Thr Thr
       850                 855                 860
   Phe Val Glu Ile Val Gly Lys Gln Asp Arg Trp Arg Ile Arg Asp Gly
   865                 870                 875                 880
   Ala Ala Asp Thr Thr Ile Asp Leu Ala Lys Val Val Ser Gln Leu Val
                   885                 890                 895
   Asp Ala Asn Gly Val Leu Lys His Ser Ile Lys Leu Glu Val Ile Gly
               900                 905                 910
   Gly Asp Gly Asp Asp Val Val Leu Ala Asn Ala Ser Arg Ile His Tyr
   915 920 925
   Asp Gly Gly Ala Gly Thr Asn Thr Val Ser Tyr Ala Ala Leu Gly Arg
   930 935 940
   Gln Asp Ser Ile Thr Val Ser Ala Asp Gly Glu Arg Phe Asn Val Arg
   945 950 955 960
   Lys Gln Leu Asn Asn Ala Asn Val Tyr Arg Glu Gly Val Ala Thr Gln
   965 970 975
   Lys Thr Ala Tyr Gly Lys Arg Thr Glu Asn Val Gln Tyr Arg His Val
   980 985 990
   Glu Leu Ala Arg Val Gly Gln Leu Val Glu Val Asp Thr Leu Glu His
   995 1000 1005
   Val Gln His Ile Ile Gly Gly Ala Gly Asn Asp Ser Ile Thr Gly
   1010 1015 1020
   Asn Ala His Asp Asn Phe Leu Ala Gly Gly Ala Gly Asp Asp Arg
   1025 1030 1035
   Leu Asp Gly Gly Ala Gly Asn Asp Thr Leu Val Gly Gly Glu Gly
   1040 1045 1050
   His Asn Thr Val Val Gly Gly Ala Gly Asp Asp Val Phe Leu Gln
   1055 1060 1065
   Asp Leu Gly Val Trp Ser Asn Gln Leu Asp Gly Gly Ala Gly Val
   1070 1075 1080
   Asp Thr Val Lys Tyr Asn Val His Gln Pro Ser Glu Glu Arg Leu
   1085 1090 1095
   Glu Arg Met Gly Asp Thr Gly Ile His Ala Asp Leu Gln Lys Gly
   1100 1105 1110
   Thr Val Glu Lys Trp Pro Ala Leu Asn Leu Phe Ser Val Asp His
   1115 1120 1125
   Val Lys Asn Ile Glu Asn Leu His Gly Ser Ser Leu Asn Asp Ser
   1130 1135 1140
   Ile Ala Gly Asp Asp Arg Asp Asn Glu Leu Trp Gly Asp Asp Gly
   1145 1150 1155
   Asn Asp Thr Ile His Gly Arg Gly Gly Asp Asp Ile Leu Arg Gly
       1160                1165                1170
   Gly Leu Gly Leu Asp Thr Leu Tyr Gly Glu Asp Gly Asn Asp Ile
       1175                1180                1185
   Phe Leu Gln Asp Asp Glu Thr Val Ser Asp Asp Ile Asp Gly Gly
       1190                1195                1200
   Ala Gly Leu Asp Thr Val Asp Tyr Ser Ala Met Ile His Ala Gly
       1205                1210                1215
   Lys Ile Val Ala Pro His Glu Tyr Gly Phe Gly Ile Glu Ala Asp
       1220                1225                1230
   Leu Ser Glu Gly Trp Val Arg Lys Ala Ala Arg Arg Gly Met Asp
       1235                1240                1245
   Tyr Tyr Asp Ser Val Arg Ser Val Glu Asn Val Ile Gly Thr Ser
       1250                1255                1260
   Met Lys Asp Val Leu Ile Gly Asp Ala Gln Ala Asn Thr Leu Met
       1265                1270                1275
   Gly Gln Gly Gly Asp Asp Thr Val Arg Gly Gly Asp Gly Asp Asp
       1280                1285                1290
   Leu Leu Phe Gly Gly Asp Gly Asn Asp Met Leu Tyr Gly Asp Ala
       1295                1300                1305
   Gly Asn Asp Thr Leu Tyr Gly Gly Leu Gly Asp Asp Thr Leu Glu
       1310                1315                1320
   Gly Gly Ala Gly Asn Asp Trp Phe Gly Gln Thr Pro Ala Arg Glu
       1325                1330                1335
   His Asp Val Leu Arg Gly Gly Ala Gly Val Asp Thr Val Asp Tyr
       1340                1345                1350
   Ser Gln Ala Gly Ala His Ala Gly Val Ala Thr Gly Arg Ile Gly
       1355                1360                1365
   Leu Gly Ile Leu Ala Asp Leu Gly Ala Gly Arg Val Asp Lys Leu
       1370                1375                1380
   Gly Glu Ala Gly Ser Ser Ala Tyr Asp Thr Val Ser Gly Ile Glu
       1385                1390                1395
   Asn Val Val Gly Thr Glu Leu Ala Asp Arg Ile Thr Gly Asp Ala
       1400                1405                1410
   Gln Ala Asn Val Leu Arg Gly Ala Gly Gly Ala Asp Val Leu Ala
       1415                1420                1425
   Gly Gly Glu Gly Asp Asp Val Leu Leu Gly Gly Asp Gly Asp Asp
       1430                1435                1440
   Gln Leu Ser Gly Asp Ala Gly Arg Asp Arg Leu Tyr Gly Glu Ala
       1445                1450                1455
   Gly Asp Asp Trp Phe Phe Gln Asp Ala Ala Asn Ala Gly Asn Leu
       1460                1465                1470
   Leu Asp Gly Gly Asp Gly Asn Asp Thr Val Asp Phe Ser Gly Pro
       1475                1480                1485
   Gly Arg Gly Leu Asp Ala Gly Ala Lys Gly Val Phe Leu Ser Leu
       1490                1495                1500
   Gly Lys Gly Phe Ala Ser Leu Met Asp Glu Pro Glu Thr Ser Asn
       1505                1510                1515
   Val Leu Arg His Ile Glu Asn Ala Val Gly Ser Val Arg Asp Asp
       1520                1525                1530
   Val Leu Ile Gly Asp Ala Gly Ala Asn Val Leu Asn Gly Leu Ala
       1535                1540                1545
   Gly Asn Asp Val Leu Ser Gly Gly Ala Gly Asp Asp Val Leu Leu
       1550                1555                1560
   Gly Asp Glu Gly Ser Asp Leu Leu Ser Gly Asp Ala Gly Asn Asp
       1565                1570                1575
   Asp Leu Phe Gly Gly Gln Gly Asp Asp Thr Tyr Leu Phe Gly Ala
       1580                1585                1590
   Gly Tyr Gly His Asp Thr Ile Tyr Glu Ser Gly Gly Gly His Asp
       1595                1600                1605
   Thr Ile Arg Ile Asn Ala Gly Ala Asp Gln Leu Trp Phe Ala Arg
       1610                1615                1620
   Gln Gly Asn Asp Leu Glu Ile Arg Ile Leu Gly Thr Asp Asp Ala
       1625                1630                1635
   Leu Thr Val His Asp Trp Tyr Arg Asp Ala Asp His Arg Val Glu
   1640                1645                1650
   Ala Ile His Ala Ala Asn Gln Ala Ile Asp Pro Ala Gly Ile Glu
   1655                1660                1665
   Lys Leu Val Glu Ala Met Ala Gln Tyr Pro Asp Pro Gly Ala Ala
   1670                1675                1680
   Ala Ala Ala Pro Pro Ala Ala Arg Val Pro Asp Thr Leu Met Gln
   1685                1690                1695
   Ser Leu Ala Val Asn Trp Arg
   1700                1705

<210> 9
<211> 4842
<212> DNA
<213> Artificial Sequence

<220>
<223> Nucleotide sequence encoding polypeptide 1-227 + 321-1706 of SEQ
ID NO:2

<220>
<221> CDS
<222> (1)..(4842)

<400> 9
atg cag cag tcc cat cag gcc ggt tac gca aac gca gcc gat cgt gaa 48
Met Gln Gln Ser His Gln Ala Gly Tyr Ala Asn Ala Ala Asp Arg Glu
1               5                   10                  15

tct ggt atc ccg gca gcg gtt ctg gat ggt att aag gca gtt gcc aaa 96
Ser Gly Ile Pro Ala Ala Val Leu Asp Gly Ile Lys Ala Val Ala Lys
            20                  25                  30

gag aag aac gct acc ctg atg ttc cgt ctg gtc aac cca cac tct act 144
Glu Lys Asn Ala Thr Leu Met Phe Arg Leu Val Asn Pro His Ser Thr
        35                  40                  45

tcc ctg atc gcg gaa ggt gtg gcg acc aaa ggc ctg ggt gtg cac gcc 192
Ser Leu Ile Ala Glu Gly Val Ala Thr Lys Gly Leu Gly Val His Ala
    50                  55                  60

aaa agc agc gac tgg ggt ctg cag gca ggt tat att ccg gtg aac ccg 240
Lys Ser Ser Asp Trp Gly Leu Gln Ala Gly Tyr Ile Pro Val Asn Pro
65                  70                  75                  80

aat ctg tct aaa ctg ttc ggt cgt gcc ccg gag gtg atc gca cgt gca 288
Asn Leu Ser Lys Leu Phe Gly Arg Ala Pro Glu Val Ile Ala Arg Ala
                85                  90                  95

gac aac gat gtc aac tct tct ctg gcg cat ggt cac acc gcg gtt gat 336
Asp Asn Asp Val Asn Ser Ser Leu Ala His Gly His Thr Ala Val Asp

            100                 105                 110

ctg acc ctg tcc aaa gaa cgc ctg gat tac ctg cgt cag gcg ggc ctg 384
Leu Thr Leu Ser Lys Glu Arg Leu Asp Tyr Leu Arg Gln Ala Gly Leu
        115                 120                 125

gtg acg ggc atg gcc gac ggt gtt gta gcg agc aac cac gcg ggt tat 432
Val Thr Gly Met Ala Asp Gly Val Val Ala Ser Asn His Ala Gly Tyr
    130                 135                 140

gaa caa ttt gag ttt cgc gtt aaa gag acc tct gac ggt cgt tac gcg 480
Glu Gln Phe Glu Phe Arg Val Lys Glu Thr Ser Asp Gly Arg Tyr Ala
145                 150                 155                 160

gtg cag tac cgc cgc aaa ggt ggc gat gac ttt gaa gct gtc aaa gtg 528
Val Gln Tyr Arg Arg Lys Gly Gly Asp Asp Phe Glu Ala Val Lys Val
                165                 170                 175

atc ggt aac gct gct gga att ccg ctg acc gcg gat atc gac atg ttt 576
Ile Gly Asn Ala Ala Gly Ile Pro Leu Thr Ala Asp Ile Asp Met Phe
            180                 185                 190

gcc atc atg cct cac ctg tcc aac ttt cgc gat tcc gca cgc agc tct 624
Ala Ile Met Pro His Leu Ser Asn Phe Arg Asp Ser Ala Arg Ser Ser
        195                 200                 205

gtg acg tct ggt gat tcc gtc act gac tac ctg gct cgt acc cgt cgt 672
Val Thr Ser Gly Asp Ser Val Thr Asp Tyr Leu Ala Arg Thr Arg Arg
    210                 215                 220

gcg gcc tct gaa tcc cag atg ctg act cgt ggc cag ctg aaa gaa tac 720
Ala Ala Ser Glu Ser Gln Met Leu Thr Arg Gly Gln Leu Lys Glu Tyr
225                 230                 235                 240

atc ggc caa cag cgt ggc gaa ggc tac gtt ttc tac gag aat cgt gct 768
Ile Gly Gln Gln Arg Gly Glu Gly Tyr Val Phe Tyr Glu Asn Arg Ala
                245                 250                 255

tac ggc gtt gcg ggc aaa tcc ctg ttc gac gat ggt ctg ggc gct gca 816
Tyr Gly Val Ala Gly Lys Ser Leu Phe Asp Asp Gly Leu Gly Ala Ala
            260                 265                 270

ccg ggc gtt ccg agc ggc cgt tct aaa ttc agc ccg gac gta ctg gaa 864
Pro Gly Val Pro Ser Gly Arg Ser Lys Phe Ser Pro Asp Val Leu Glu
        275                 280                 285

act gtg ccg gct tcc ccg ggc ctg cgc cgt ccg tcc ctg ggc gca gtc 912
Thr Val Pro Ala Ser Pro Gly Leu Arg Arg Pro Ser Leu Gly Ala Val
    290                 295                 300

gaa cgt cag gac tcc ggc tac gat tcc ctg gat ggc gtt ggc tcc cgc 960
Glu Arg Gln Asp Ser Gly Tyr Asp Ser Leu Asp Gly Val Gly Ser Arg
305                 310                 315                 320

tcc ttc tcc ctg ggc gag gtt tct gac atg gct gcg gtg gaa gcg gca 1008
Ser Phe Ser Leu Gly Glu Val Ser Asp Met Ala Ala Val Glu Ala Ala
                325                 330                 335

gag ctg gaa atg acc cgt caa gtg ctg cac gcg ggc gca cgt cag gac 1056
Glu Leu Glu Met Thr Arg Gln Val Leu His Ala Gly Ala Arg Gln Asp
            340                 345                 350

gat gct gag ccg ggc gtg tcc ggt gcg tct gca cac tgg ggt caa cgt 1104

Asp Ala Glu Pro Gly Val Ser Gly Ala Ser Ala His Trp Gly Gln Arg
        355                 360                 365

gcc ctg cag ggt gct caa gct gtc gcg gca gcg cag cgt ctg gta cat        1152
Ala Leu Gln Gly Ala Gln Ala Val Ala Ala Ala Gln Arg Leu Val His
    370                 375                 380

gcg atc gca ctg atg acc cag ttc ggt cgc gca ggt tct acc aac act        1200
Ala Ile Ala Leu Met Thr Gln Phe Gly Arg Ala Gly Ser Thr Asn Thr
385                 390                 395                 400

cct cag gaa gca gct agc ctg tct gct gct gtt ttc ggt ctg ggc gaa        1248
Pro Gln Glu Ala Ala Ser Leu Ser Ala Ala Val Phe Gly Leu Gly Glu
                405                 410                 415

gcg tct tct gcg gtt gca gaa acc gtg agc ggt ttc ttc cgt ggt tct        1296
Ala Ser Ser Ala Val Ala Glu Thr Val Ser Gly Phe Phe Arg Gly Ser
            420                 425                 430

agc cgc tgg gcg ggt ggt ttt ggt gta gca ggt ggc gct atg gcc ctg        1344
Ser Arg Trp Ala Gly Gly Phe Gly Val Ala Gly Gly Ala Met Ala Leu
        435                 440                 445

ggt ggc ggt atc gcg gct gct gtc ggc gca ggt atg agc ctg acc gac        1392
Gly Gly Gly Ile Ala Ala Ala Val Gly Ala Gly Met Ser Leu Thr Asp
    450                 455                 460

gat gct cct gcg ggc cag aaa gcc gca gct ggc gct gaa atc gcg ctg        1440
Asp Ala Pro Ala Gly Gln Lys Ala Ala Ala Gly Ala Glu Ile Ala Leu
465                 470                 475                 480

cag ctg act ggt ggt acc gtt gaa ctg gct agc tct atc gcg ctg gct        1488
Gln Leu Thr Gly Gly Thr Val Glu Leu Ala Ser Ser Ile Ala Leu Ala
                485                 490                 495

ctg gca gcg gca cgt ggc gtg act tct ggc ctg caa gtc gcc ggt gcc        1536
Leu Ala Ala Ala Arg Gly Val Thr Ser Gly Leu Gln Val Ala Gly Ala
            500                 505                 510

tct gcg ggc gct gct gcg ggc gct ctg gca gct gca ctg tcc ccc atg        1584
Ser Ala Gly Ala Ala Ala Gly Ala Leu Ala Ala Ala Leu Ser Pro Met
        515                 520                 525

gaa atc tac ggt ctg gta cag cag tct cac tac gca gac cag ctg gat        1632
Glu Ile Tyr Gly Leu Val Gln Gln Ser His Tyr Ala Asp Gln Leu Asp
    530                 535                 540

aaa ctg gcg cag gag tct tct gca tac ggt tac gaa ggc gac gca ctg        1680
Lys Leu Ala Gln Glu Ser Ser Ala Tyr Gly Tyr Glu Gly Asp Ala Leu
545                 550                 555                 560

ctg gcg cag ctg tat cgt gac aaa act gct gct gaa ggt gct gtg gca        1728
Leu Ala Gln Leu Tyr Arg Asp Lys Thr Ala Ala Glu Gly Ala Val Ala
                565                 570                 575

ggc gtt tct gcg gta ctg tct acc gtt ggc gct gcg gtt tct att gcg        1776
Gly Val Ser Ala Val Leu Ser Thr Val Gly Ala Ala Val Ser Ile Ala
            580                 585                 590

gct gcc gca tcc gtt gta ggt gcg ccg gtt gct gta gtt acc tct ctg        1824
Ala Ala Ala Ser Val Val Gly Ala Pro Val Ala Val Val Thr Ser Leu
        595                 600                 605

ctg act ggt gca ctg aac ggt atc ctg cgt ggt gtt cag cag cct att     1872
Leu Thr Gly Ala Leu Asn Gly Ile Leu Arg Gly Val Gln Gln Pro Ile
    610                 615                 620

atc gaa aaa ctg gcg aat gat tat gcc cgt aaa atc gac gaa ctg ggc     1920
Ile Glu Lys Leu Ala Asn Asp Tyr Ala Arg Lys Ile Asp Glu Leu Gly
625                 630                 635                 640

ggt ccg cag gct tat ttt gaa aaa aac ctg cag gcg cgc cac gaa cag     1968
Gly Pro Gln Ala Tyr Phe Glu Lys Asn Leu Gln Ala Arg His Glu Gln
                645                 650                 655

ctg gca aac agc gac ggc ctg cgc aaa atg ctg gca gac ctg caa gct     2016
Leu Ala Asn Ser Asp Gly Leu Arg Lys Met Leu Ala Asp Leu Gln Ala
            660                 665                 670

ggt tgg aac gcg tct tct gtg atc ggc gtg cag acc acc gaa att agc     2064
Gly Trp Asn Ala Ser Ser Val Ile Gly Val Gln Thr Thr Glu Ile Ser
        675                 680                 685

aaa tct gcg ctg gaa ctg gct gca att act ggc aac gcg gat aac ctg     2112
Lys Ser Ala Leu Glu Leu Ala Ala Ile Thr Gly Asn Ala Asp Asn Leu
    690                 695                 700

aaa agc gtt gat gtt ttt gtc gat cgc ttc gtt cag ggc gag cgc gtt     2160
Lys Ser Val Asp Val Phe Val Asp Arg Phe Val Gln Gly Glu Arg Val
705                 710                 715                 720

gct ggt cag ccg gtt gtt ctg gac gtt gcg gca ggc ggc atc gac atc     2208
Ala Gly Gln Pro Val Val Leu Asp Val Ala Ala Gly Gly Ile Asp Ile
                725                 730                 735

gct agc cgc aag ggc gag cgt ccg gct ctg act ttc att acc ccg ctg     2256
Ala Ser Arg Lys Gly Glu Arg Pro Ala Leu Thr Phe Ile Thr Pro Leu
            740                 745                 750

gca gct ccg ggt gaa gaa cag cgt cgc cgt acc aag act ggt aaa agc     2304
Ala Ala Pro Gly Glu Glu Gln Arg Arg Arg Thr Lys Thr Gly Lys Ser
        755                 760                 765

gag ttc acc acc ttc gtg gaa atc gta ggt aaa cag gac cgc tgg cgt     2352
Glu Phe Thr Thr Phe Val Glu Ile Val Gly Lys Gln Asp Arg Trp Arg
    770                 775                 780

att cgc gac ggt gcg gcg gac acc acc atc gac ctg gca aaa gtt gtt     2400
Ile Arg Asp Gly Ala Ala Asp Thr Thr Ile Asp Leu Ala Lys Val Val
785                 790                 795                 800

tcc caa ctg gta gat gct aac ggc gtg ctg aaa cat tct att aaa ctg     2448
Ser Gln Leu Val Asp Ala Asn Gly Val Leu Lys His Ser Ile Lys Leu
                805                 810                 815

gat gta atc ggc ggt gac ggc gat gac gtt gta ctg gcg aac gcg tct     2496
Asp Val Ile Gly Gly Asp Gly Asp Asp Val Val Leu Ala Asn Ala Ser
            820                 825                 830

cgt atc cac tat gac ggc ggc gca ggt acc aac acg gtt tcc tac gca     2544
Arg Ile His Tyr Asp Gly Gly Ala Gly Thr Asn Thr Val Ser Tyr Ala
        835                 840                 845

gcg ctg ggc cgc cag gat tcc atc act gtt agc gcc gat ggc gaa cgt     2592
Ala Leu Gly Arg Gln Asp Ser Ile Thr Val Ser Ala Asp Gly Glu Arg
    850                 855                 860

ttc aac gtg cgt aaa cag ctg aat aac gca aac gtc tat cgt gag ggt 2640
Phe Asn Val Arg Lys Gln Leu Asn Asn Ala Asn Val Tyr Arg Glu Gly
865                 870                 875                 880

gta gct acc cag acc acc gcg tac ggt aag cgt acc gaa aac gtt cag 2688
Val Ala Thr Gln Thr Thr Ala Tyr Gly Lys Arg Thr Glu Asn Val Gln
                885                 890                 895

tat cgt cac gtg gaa ctg gca cgt gtg ggt cag ctg gta gag gtg gat 2736
Tyr Arg His Val Glu Leu Ala Arg Val Gly Gln Leu Val Glu Val Asp
            900                 905                 910

acg ctg gaa cac gtg cag cac atc atc ggt ggc gcc ggt aac gat tcc 2784
Thr Leu Glu His Val Gln His Ile Ile Gly Gly Ala Gly Asn Asp Ser
        915                 920                 925

att act ggt aac gct cac gac aac ttc ctg gca ggt ggt tct ggt gat 2832
Ile Thr Gly Asn Ala His Asp Asn Phe Leu Ala Gly Gly Ser Gly Asp
    930                 935                 940

gat cgt ctg gac ggt ggt gcg ggt aac gat acg ctg gta ggc ggc gaa 2880
Asp Arg Leu Asp Gly Gly Ala Gly Asn Asp Thr Leu Val Gly Gly Glu
945                 950                 955                 960

ggt cag aac acc gta att ggt ggt gcc ggt gac gac gtt ttc ctg cag 2928
Gly Gln Asn Thr Val Ile Gly Gly Ala Gly Asp Asp Val Phe Leu Gln
                965                 970                 975

gat ctg ggc gtt tgg agc aac cag ctg gat ggc ggt gcc ggt gtt gac 2976
Asp Leu Gly Val Trp Ser Asn Gln Leu Asp Gly Gly Ala Gly Val Asp
            980                 985                 990

acg gtt aag tat aac gtt cac cag ccg tct gaa gag cgc ctg gaa cgt 3024
Thr Val Lys Tyr Asn Val His Gln Pro Ser Glu Glu Arg Leu Glu Arg
        995                 1000                1005

atg ggt gac act ggt att cat gcc gat ctg cag aaa ggc act gtt 3069
Met Gly Asp Thr Gly Ile His Ala Asp Leu Gln Lys Gly Thr Val
    1010                1015                1020

gaa aaa tgg cct gcg ctg aac ctg ttc tct gtg gac cat gtt aag 3114
Glu Lys Trp Pro Ala Leu Asn Leu Phe Ser Val Asp His Val Lys
    1025                1030                1035

aac att gaa aac ctg cat ggc tct cgc ctg aac gac cgt atc gct 3159
Asn Ile Glu Asn Leu His Gly Ser Arg Leu Asn Asp Arg Ile Ala
    1040                1045                1050

ggt gac gac cag gat aac gaa ctg tgg ggt cat gac ggc aat gat 3204
Gly Asp Asp Gln Asp Asn Glu Leu Trp Gly His Asp Gly Asn Asp
    1055                1060                1065

acc att cgc ggt cgt ggc ggc gac gat att ctg cgt ggc ggt ctg 3249
Thr Ile Arg Gly Arg Gly Gly Asp Asp Ile Leu Arg Gly Gly Leu
    1070                1075                1080

ggc ctg gat acc ctg tat ggt gaa gat ggt aac gat att ttc ctg 3294
Gly Leu Asp Thr Leu Tyr Gly Glu Asp Gly Asn Asp Ile Phe Leu
    1085                1090                1095

caa gac gat gaa acg gta tct gat gac atc gac ggt ggt gca ggc 3339
Gln Asp Asp Glu Thr Val Ser Asp Asp Ile Asp Gly Gly Ala Gly

1100                1105                1110

ctg gac acc gta gat tac agc gct atg atc cat ccg ggt cgt atc        3384
Leu Asp Thr Val Asp Tyr Ser Ala Met Ile His Pro Gly Arg Ile
    1115                1120                1125

gta gct ccg cac gag tac ggc ttc ggt atc gag gcg gac ctg tct        3429
Val Ala Pro His Glu Tyr Gly Phe Gly Ile Glu Ala Asp Leu Ser
    1130                1135                1140

cgt gaa tgg gtt cgt aaa gcg tcc gcg ctg ggt gtg gac tac tac        3474
Arg Glu Trp Val Arg Lys Ala Ser Ala Leu Gly Val Asp Tyr Tyr
    1145                1150                1155

gat aac gtt cgt aac gtg gaa aac gtg atc ggt acc tcc atg aaa        3519
Asp Asn Val Arg Asn Val Glu Asn Val Ile Gly Thr Ser Met Lys
    1160                1165                1170

gac gtg ctg att ggc gac gca cag gcc aac act ctg atg ggc cag        3564
Asp Val Leu Ile Gly Asp Ala Gln Ala Asn Thr Leu Met Gly Gln
    1175                1180                1185

ggc ggt gat gac acg gtt cgc ggc ggc gac ggt gat gac ctg ctg        3609
Gly Gly Asp Asp Thr Val Arg Gly Gly Asp Gly Asp Asp Leu Leu
    1190                1195                1200

ttc ggt ggt gac ggc aac gac atg ctg tac ggc gac gcg ggc aac        3654
Phe Gly Gly Asp Gly Asn Asp Met Leu Tyr Gly Asp Ala Gly Asn
    1205                1210                1215

gac acc ctg tac ggc ggt ctg ggt gac gac act ctg gaa ggt ggt        3699
Asp Thr Leu Tyr Gly Gly Leu Gly Asp Asp Thr Leu Glu Gly Gly
    1220                1225                1230

gca ggt aac gac tgg ttc ggc caa acc cag gca cgc gaa cac gac        3744
Ala Gly Asn Asp Trp Phe Gly Gln Thr Gln Ala Arg Glu His Asp
    1235                1240                1245

gtg ctg cgt ggt ggc gac ggc gta gac acc gtg gat tac tcc caa        3789
Val Leu Arg Gly Gly Asp Gly Val Asp Thr Val Asp Tyr Ser Gln
    1250                1255                1260

act ggc gct cac gcg ggt atc gcg gcc ggt cgt atc ggt ctg ggc        3834
Thr Gly Ala His Ala Gly Ile Ala Ala Gly Arg Ile Gly Leu Gly
    1265                1270                1275

att ctg gcc gat ctg ggt gcc ggc cgt gtc gac aaa ctg ggt gag        3879
Ile Leu Ala Asp Leu Gly Ala Gly Arg Val Asp Lys Leu Gly Glu
    1280                1285                1290

gct ggc tct agc gcc tat gat act gtt tcc ggc atc gaa aac gtg        3924
Ala Gly Ser Ser Ala Tyr Asp Thr Val Ser Gly Ile Glu Asn Val
    1295                1300                1305

gta ggc act gaa ctg gca gat cgt atc acg ggt gac gcg caa gcg        3969
Val Gly Thr Glu Leu Ala Asp Arg Ile Thr Gly Asp Ala Gln Ala
    1310                1315                1320

aac gtt ctg cgt ggt gct ggc ggt gca gac gtg ctg gcg ggt ggt        4014
Asn Val Leu Arg Gly Ala Gly Gly Ala Asp Val Leu Ala Gly Gly
    1325                1330                1335

gaa ggc gac gac gta ctg ctg ggc ggt gac ggt gac gat cag ctg        4059

Glu Gly Asp Asp Val Leu Leu Gly Gly Asp Gly Asp Asp Gln Leu
    1340                1345                1350

agc ggt gat gcg ggt cgt gac cgt ctg tac ggt gaa gca ggt gac    4104
Ser Gly Asp Ala Gly Arg Asp Arg Leu Tyr Gly Glu Ala Gly Asp
    1355                1360                1365

gat tgg ttc ttc cag gac gct gct aac gct ggc aac ctg ctg gac    4149
Asp Trp Phe Phe Gln Asp Ala Ala Asn Ala Gly Asn Leu Leu Asp
    1370                1375                1380

ggc ggc gat ggc cgt gac act gtt gac ttc agc ggt ccg ggt cgc    4194
Gly Gly Asp Gly Arg Asp Thr Val Asp Phe Ser Gly Pro Gly Arg
    1385                1390                1395

ggt ctg gac gca ggc gcg aaa ggc gtt ttc ctg agc ctg ggt aag    4239
Gly Leu Asp Ala Gly Ala Lys Gly Val Phe Leu Ser Leu Gly Lys
    1400                1405                1410

ggt ttc gca tct ctg atg gac gaa cca gaa acc agc aat gtc ctg    4284
Gly Phe Ala Ser Leu Met Asp Glu Pro Glu Thr Ser Asn Val Leu
    1415                1420                1425

cgt aac atc gaa aac gct gtc ggt tcc gca cgc gat gat gtt ctg    4329
Arg Asn Ile Glu Asn Ala Val Gly Ser Ala Arg Asp Asp Val Leu
    1430                1435                1440

att ggc gat gct ggt gcc aat gtt ctg aac ggt ctg gcg ggt aat    4374
Ile Gly Asp Ala Gly Ala Asn Val Leu Asn Gly Leu Ala Gly Asn
    1445                1450                1455

gat gta ctg tct ggt ggc gct ggt gat gat gtc ctg ctg ggt gat    4419
Asp Val Leu Ser Gly Gly Ala Gly Asp Asp Val Leu Leu Gly Asp
    1460                1465                1470

gag ggc agc gat ctg ctg tcc ggc gat gcc ggc aac gac gat ctg    4464
Glu Gly Ser Asp Leu Leu Ser Gly Asp Ala Gly Asn Asp Asp Leu
    1475                1480                1485

ttc ggt ggc caa ggt gat gat act tac ctg ttc ggc gtt ggc tat    4509
Phe Gly Gly Gln Gly Asp Asp Thr Tyr Leu Phe Gly Val Gly Tyr
    1490                1495                1500

ggt cat gac acc atc tat gag tct ggt ggc ggc cac gat acg att    4554
Gly His Asp Thr Ile Tyr Glu Ser Gly Gly Gly His Asp Thr Ile
    1505                1510                1515

cgt att aat gca ggt gct gac caa ctg tgg ttt gca cgt cag ggt    4599
Arg Ile Asn Ala Gly Ala Asp Gln Leu Trp Phe Ala Arg Gln Gly
    1520                1525                1530

aac gat ctg gaa att cgc atc ctg ggt act gat gac gct ctg acc    4644
Asn Asp Leu Glu Ile Arg Ile Leu Gly Thr Asp Asp Ala Leu Thr
    1535                1540                1545

gta cac gac tgg tac cgc gat gcg gat cac cgc gta gaa atc atc    4689
Val His Asp Trp Tyr Arg Asp Ala Asp His Arg Val Glu Ile Ile
    1550                1555                1560

cat gcg gct aac caa gct gta gac cag gct ggt att gaa aaa ctg    4734
His Ala Ala Asn Gln Ala Val Asp Gln Ala Gly Ile Glu Lys Leu
    1565                1570                1575

gta gaa  gcg atg gca cag tat  ccg gac cca ggt gcg  gct gct gca          4779
Val Glu  Ala Met Ala Gln Tyr  Pro Asp Pro Gly Ala  Ala Ala Ala
    1580                 1585                 1590

gca cct  cca gcg gct cgt gtg  ccg gat acc ctg atg  cag agc ctg          4824
Ala Pro  Pro Ala Ala Arg Val  Pro Asp Thr Leu Met  Gln Ser Leu
    1595                 1600                 1605

gcc gtc  aat tgg cgt taa                                                4842
Ala Val  Asn Trp Arg
    1610

<210>  10
<211>  1613
<212>  PRT
<213>  Artificial Sequence

<220>
<223>  Synthetic Construct

<400>  10

Met Gln Gln Ser His Gln Ala Gly Tyr Ala Asn Ala Ala Asp Arg Glu
1               5                   10                  15

Ser Gly Ile Pro Ala Ala Val Leu Asp Gly Ile Lys Ala Val Ala Lys
            20                  25                  30

Glu Lys Asn Ala Thr Leu Met Phe Arg Leu Val Asn Pro His Ser Thr
        35                  40                  45

Ser Leu Ile Ala Glu Gly Val Ala Thr Lys Gly Leu Gly Val His Ala
    50                  55                  60

Lys Ser Ser Asp Trp Gly Leu Gln Ala Gly Tyr Ile Pro Val Asn Pro
65                  70                  75                  80

Asn Leu Ser Lys Leu Phe Gly Arg Ala Pro Glu Val Ile Ala Arg Ala
                85                  90                  95

Asp Asn Asp Val Asn Ser Ser Leu Ala His Gly His Thr Ala Val Asp
            100                 105                 110

Leu Thr Leu Ser Lys Glu Arg Leu Asp Tyr Leu Arg Gln Ala Gly Leu
        115                 120                 125

Val Thr Gly Met Ala Asp Gly Val Val Ala Ser Asn His Ala Gly Tyr
    130                 135                 140

Glu Gln Phe Glu Phe Arg Val Lys Glu Thr Ser Asp Gly Arg Tyr Ala
145                 150                 155                 160

Val Gln Tyr Arg Arg Lys Gly Gly Asp Asp Phe Glu Ala Val Lys Val
                165                 170                 175

Ile Gly Asn Ala Ala Gly Ile Pro Leu Thr Ala Asp Ile Asp Met Phe
            180                 185                 190

Ala Ile Met Pro His Leu Ser Asn Phe Arg Asp Ser Ala Arg Ser Ser
        195                 200                 205

Val Thr Ser Gly Asp Ser Val Thr Asp Tyr Leu Ala Arg Thr Arg Arg
    210                 215                 220

Ala Ala Ser Glu Ser Gln Met Leu Thr Arg Gly Gln Leu Lys Glu Tyr
225                 230                 235                 240

Ile Gly Gln Gln Arg Gly Glu Gly Tyr Val Phe Tyr Glu Asn Arg Ala
                245                 250                 255

Tyr Gly Val Ala Gly Lys Ser Leu Phe Asp Asp Gly Leu Gly Ala Ala
            260                 265                 270

Pro Gly Val Pro Ser Gly Arg Ser Lys Phe Ser Pro Asp Val Leu Glu
        275                 280                 285

Thr Val Pro Ala Ser Pro Gly Leu Arg Arg Pro Ser Leu Gly Ala Val
    290                 295                 300

Glu Arg Gln Asp Ser Gly Tyr Asp Ser Leu Asp Gly Val Gly Ser Arg
305                 310                 315                 320

Ser Phe Ser Leu Gly Glu Val Ser Asp Met Ala Ala Val Glu Ala Ala
                325                 330                 335

Glu Leu Glu Met Thr Arg Gln Val Leu His Ala Gly Ala Arg Gln Asp
            340                 345                 350

Asp Ala Glu Pro Gly Val Ser Gly Ala Ser Ala His Trp Gly Gln Arg
        355                 360                 365

Ala Leu Gln Gly Ala Gln Ala Val Ala Ala Ala Gln Arg Leu Val His
    370                 375                 380

Ala Ile Ala Leu Met Thr Gln Phe Gly Arg Ala Gly Ser Thr Asn Thr
385                 390                 395                 400

Pro Gln Glu Ala Ala Ser Leu Ser Ala Ala Val Phe Gly Leu Gly Glu
                405                 410                 415

Ala Ser Ser Ala Val Ala Glu Thr Val Ser Gly Phe Phe Arg Gly Ser
            420                 425                 430

Ser Arg Trp Ala Gly Gly Phe Gly Val Ala Gly Gly Ala Met Ala Leu
        435                 440                 445

Gly Gly Gly Ile Ala Ala Ala Val Gly Ala Gly Met Ser Leu Thr Asp
    450                 455                 460

Asp Ala Pro Ala Gly Gln Lys Ala Ala Ala Gly Ala Glu Ile Ala Leu
465                 470                 475                 480

Gln Leu Thr Gly Gly Thr Val Glu Leu Ala Ser Ser Ile Ala Leu Ala
                485                 490                 495

Leu Ala Ala Ala Arg Gly Val Thr Ser Gly Leu Gln Val Ala Gly Ala
            500                 505                 510

Ser Ala Gly Ala Ala Ala Gly Ala Leu Ala Ala Ala Leu Ser Pro Met
        515                 520                 525

Glu Ile Tyr Gly Leu Val Gln Gln Ser His Tyr Ala Asp Gln Leu Asp
    530                 535                 540

Lys Leu Ala Gln Glu Ser Ser Ala Tyr Gly Tyr Glu Gly Asp Ala Leu
545                 550                 555                 560

Leu Ala Gln Leu Tyr Arg Asp Lys Thr Ala Ala Glu Gly Ala Val Ala
                565                 570                 575

Gly Val Ser Ala Val Leu Ser Thr Val Gly Ala Ala Val Ser Ile Ala
            580                 585                 590

Ala Ala Ala Ser Val Val Gly Ala Pro Val Ala Val Val Thr Ser Leu
        595                 600                 605

Leu Thr Gly Ala Leu Asn Gly Ile Leu Arg Gly Val Gln Gln Pro Ile
    610                 615                 620

Ile Glu Lys Leu Ala Asn Asp Tyr Ala Arg Lys Ile Asp Glu Leu Gly
625                 630                 635                 640

Gly Pro Gln Ala Tyr Phe Glu Lys Asn Leu Gln Ala Arg His Glu Gln
                645                 650                 655

Leu Ala Asn Ser Asp Gly Leu Arg Lys Met Leu Ala Asp Leu Gln Ala
            660                 665                 670

Gly Trp Asn Ala Ser Ser Val Ile Gly Val Gln Thr Thr Glu Ile Ser
        675                 680                 685

Lys Ser Ala Leu Glu Leu Ala Ala Ile Thr Gly Asn Ala Asp Asn Leu
    690                 695                 700

Lys Ser Val Asp Val Phe Val Asp Arg Phe Val Gln Gly Glu Arg Val
705                 710                 715                 720

Ala Gly Gln Pro Val Val Leu Asp Val Ala Ala Gly Gly Ile Asp Ile
                725                 730                 735

Ala Ser Arg Lys Gly Glu Arg Pro Ala Leu Thr Phe Ile Thr Pro Leu
            740                 745                 750

Ala Ala Pro Gly Glu Glu Gln Arg Arg Arg Thr Lys Thr Gly Lys Ser
        755                 760                 765

Glu Phe Thr Thr Phe Val Glu Ile Val Gly Lys Gln Asp Arg Trp Arg
    770                 775                 780

Ile Arg Asp Gly Ala Ala Asp Thr Thr Ile Asp Leu Ala Lys Val Val
785                 790                 795                 800

Ser Gln Leu Val Asp Ala Asn Gly Val Leu Lys His Ser Ile Lys Leu
                805                 810                 815

Asp Val Ile Gly Gly Asp Gly Asp Asp Val Val Leu Ala Asn Ala Ser
            820                 825                 830

Arg Ile His Tyr Asp Gly Gly Ala Gly Thr Asn Thr Val Ser Tyr Ala
        835                 840                 845

Ala Leu Gly Arg Gln Asp Ser Ile Thr Val Ser Ala Asp Gly Glu Arg
    850                 855                 860

Phe Asn Val Arg Lys Gln Leu Asn Asn Ala Asn Val Tyr Arg Glu Gly
865                 870                 875                 880

Val Ala Thr Gln Thr Thr Ala Tyr Gly Lys Arg Thr Glu Asn Val Gln
                885                 890                 895

Tyr Arg His Val Glu Leu Ala Arg Val Gly Gln Leu Val Glu Val Asp
            900                 905                 910

Thr Leu Glu His Val Gln His Ile Ile Gly Gly Ala Gly Asn Asp Ser

915                 920                 925

Ile Thr Gly Asn Ala His Asp Asn Phe Leu Ala Gly Gly Ser Gly Asp
    930                 935                 940

Asp Arg Leu Asp Gly Gly Ala Gly Asn Asp Thr Leu Val Gly Gly Glu
945                 950                 955                 960

Gly Gln Asn Thr Val Ile Gly Gly Ala Gly Asp Asp Val Phe Leu Gln
                965                 970                 975

Asp Leu Gly Val Trp Ser Asn Gln Leu Asp Gly Gly Ala Gly Val Asp
            980                 985                 990

Thr Val Lys Tyr Asn Val His Gln  Pro Ser Glu Glu Arg  Leu Glu Arg
        995                 1000                 1005

Met Gly  Asp Thr Gly Ile His  Ala Asp Leu Gln Lys  Gly Thr Val
    1010                 1015                 1020

Glu Lys  Trp Pro Ala Leu Asn  Leu Phe Ser Val Asp  His Val Lys
    1025                 1030                 1035

Asn Ile  Glu Asn Leu His Gly  Ser Arg Leu Asn Asp  Arg Ile Ala
    1040                 1045                 1050

Gly Asp  Asp Gln Asp Asn Glu  Leu Trp Gly His Asp  Gly Asn Asp
    1055                 1060                 1065

Thr Ile  Arg Gly Arg Gly Gly  Asp Asp Ile Leu Arg  Gly Gly Leu
    1070                 1075                 1080

Gly Leu  Asp Thr Leu Tyr Gly  Glu Asp Gly Asn Asp  Ile Phe Leu
    1085                 1090                 1095

Gln Asp  Asp Glu Thr Val Ser  Asp Asp Ile Asp Gly  Gly Ala Gly
    1100                 1105                 1110

Leu Asp  Thr Val Asp Tyr Ser  Ala Met Ile His Pro  Gly Arg Ile
    1115                 1120                 1125

Val Ala  Pro His Glu Tyr Gly  Phe Gly Ile Glu Ala  Asp Leu Ser
    1130                 1135                 1140

Arg Glu  Trp Val Arg Lys Ala  Ser Ala Leu Gly Val  Asp Tyr Tyr
    1145                 1150                 1155

Asp Asn Val Arg Asn Val Glu Asn Val Ile Gly Thr Ser Met Lys
    1160                1165                1170

Asp Val Leu Ile Gly Asp Ala Gln Ala Asn Thr Leu Met Gly Gln
    1175                1180                1185

Gly Gly Asp Asp Thr Val Arg Gly Gly Asp Gly Asp Asp Leu Leu
    1190                1195                1200

Phe Gly Gly Asp Gly Asn Asp Met Leu Tyr Gly Asp Ala Gly Asn
    1205                1210                1215

Asp Thr Leu Tyr Gly Gly Leu Gly Asp Asp Thr Leu Glu Gly Gly
    1220                1225                1230

Ala Gly Asn Asp Trp Phe Gly Gln Thr Gln Ala Arg Glu His Asp
    1235                1240                1245

Val Leu Arg Gly Gly Asp Gly Val Asp Thr Val Asp Tyr Ser Gln
    1250                1255                1260

Thr Gly Ala His Ala Gly Ile Ala Ala Gly Arg Ile Gly Leu Gly
    1265                1270                1275

Ile Leu Ala Asp Leu Gly Ala Gly Arg Val Asp Lys Leu Gly Glu
    1280                1285                1290

Ala Gly Ser Ser Ala Tyr Asp Thr Val Ser Gly Ile Glu Asn Val
    1295                1300                1305

Val Gly Thr Glu Leu Ala Asp Arg Ile Thr Gly Asp Ala Gln Ala
    1310                1315                1320

Asn Val Leu Arg Gly Ala Gly Gly Ala Asp Val Leu Ala Gly Gly
    1325                1330                1335

Glu Gly Asp Asp Val Leu Leu Gly Gly Asp Gly Asp Asp Gln Leu
    1340                1345                1350

Ser Gly Asp Ala Gly Arg Asp Arg Leu Tyr Gly Glu Ala Gly Asp
    1355                1360                1365

Asp Trp Phe Phe Gln Asp Ala Ala Asn Ala Gly Asn Leu Leu Asp
    1370                1375                1380

Gly Gly Asp Gly Arg Asp Thr Val Asp Phe Ser Gly Pro Gly Arg
    1385                1390                1395

Gly Leu Asp Ala Gly Ala Lys Gly Val Phe Leu Ser Leu Gly Lys
1400                1405                1410

Gly Phe Ala Ser Leu Met Asp Glu Pro Glu Thr Ser Asn Val Leu
1415                1420                1425

Arg Asn Ile Glu Asn Ala Val Gly Ser Ala Arg Asp Asp Val Leu
1430                1435                1440

Ile Gly Asp Ala Gly Ala Asn Val Leu Asn Gly Leu Ala Gly Asn
1445                1450                1455

Asp Val Leu Ser Gly Gly Ala Gly Asp Asp Val Leu Leu Gly Asp
1460                1465                1470

Glu Gly Ser Asp Leu Leu Ser Gly Asp Ala Gly Asn Asp Asp Leu
1475                1480                1485

Phe Gly Gly Gln Gly Asp Asp Thr Tyr Leu Phe Gly Val Gly Tyr
1490                1495                1500

Gly His Asp Thr Ile Tyr Glu Ser Gly Gly Gly His Asp Thr Ile
1505                1510                1515

Arg Ile Asn Ala Gly Ala Asp Gln Leu Trp Phe Ala Arg Gln Gly
1520                1525                1530

Asn Asp Leu Glu Ile Arg Ile Leu Gly Thr Asp Asp Ala Leu Thr
1535                1540                1545

Val His Asp Trp Tyr Arg Asp Ala Asp His Arg Val Glu Ile Ile
1550                1555                1560

His Ala Ala Asn Gln Ala Val Asp Gln Ala Gly Ile Glu Lys Leu
1565                1570                1575

Val Glu Ala Met Ala Gln Tyr Pro Asp Pro Gly Ala Ala Ala Ala
1580                1585                1590

Ala Pro Pro Ala Ala Arg Val Pro Asp Thr Leu Met Gln Ser Leu
1595                1600                1605

Ala Val Asn Trp Arg
    1610

<210>  11
<211>  4512

<212>  DNA
<213>  Artificial Sequence

<220>
<223>  Nucleotide sequence encoding polypeptide 1-183 + 387-1706 of SEQ
       ID NO:2

<220>
<221>  CDS
<222>  (1)..(4512)

<400>  11
atg cag cag tcc cat cag gcc ggt tac gca aac gca gcc gat cgt gaa        48
Met Gln Gln Ser His Gln Ala Gly Tyr Ala Asn Ala Ala Asp Arg Glu
1               5                   10                  15

tct ggt atc ccg gca gcg gtt ctg gat ggt att aag gca gtt gcc aaa        96
Ser Gly Ile Pro Ala Ala Val Leu Asp Gly Ile Lys Ala Val Ala Lys
            20                  25                  30

gag aag aac gct acc ctg atg ttc cgt ctg gtc aac cca cac tct act       144
Glu Lys Asn Ala Thr Leu Met Phe Arg Leu Val Asn Pro His Ser Thr
        35                  40                  45

tcc ctg atc gcg gaa ggt gtg gcg acc aaa ggc ctg ggt gtg cac gcc       192
Ser Leu Ile Ala Glu Gly Val Ala Thr Lys Gly Leu Gly Val His Ala
    50                  55                  60

aaa agc agc gac tgg ggt ctg cag gca ggt tat att ccg gtg aac ccg       240
Lys Ser Ser Asp Trp Gly Leu Gln Ala Gly Tyr Ile Pro Val Asn Pro
65                  70                  75                  80

aat ctg tct aaa ctg ttc ggt cgt gcc ccg gag gtg atc gca cgt gca       288
Asn Leu Ser Lys Leu Phe Gly Arg Ala Pro Glu Val Ile Ala Arg Ala
                85                  90                  95

gac aac gat gtc aac tct tct ctg gcg cat ggt cac acc gcg gtt gat       336
Asp Asn Asp Val Asn Ser Ser Leu Ala His Gly His Thr Ala Val Asp
            100                 105                 110

ctg acc ctg tcc aaa gaa cgc ctg gat tac ctg cgt cag gcg ggc ctg       384
Leu Thr Leu Ser Lys Glu Arg Leu Asp Tyr Leu Arg Gln Ala Gly Leu
        115                 120                 125

gtg acg ggc atg gcc gac ggt gtt gta gcg agc aac cac gcg ggt tat       432
Val Thr Gly Met Ala Asp Gly Val Val Ala Ser Asn His Ala Gly Tyr
    130                 135                 140

gaa caa ttt gag ttt cgc gtt aaa gag acc tct gac ggt cgt tac gcg       480
Glu Gln Phe Glu Phe Arg Val Lys Glu Thr Ser Asp Gly Arg Tyr Ala
145                 150                 155                 160

gtg cag tac cgc cgc aaa ggt ggc gat gac ttt gaa gct gtc aaa gtg       528
Val Gln Tyr Arg Arg Lys Gly Gly Asp Asp Phe Glu Ala Val Lys Val
                165                 170                 175

atc ggt aac gct gct gga att ccg ggc ctg cgc cgt ccg tcc ctg ggc       576
Ile Gly Asn Ala Ala Gly Ile Pro Gly Leu Arg Arg Pro Ser Leu Gly
            180                 185                 190

gca gtc gaa cgt cag gac tcc ggc tac gat tcc ctg gat ggc gtt ggc       624
Ala Val Glu Arg Gln Asp Ser Gly Tyr Asp Ser Leu Asp Gly Val Gly

195                 200                 205

tcc cgc tcc ttc tcc ctg ggc gag gtt tct gac atg gct gcg gtg gaa        672
Ser Arg Ser Phe Ser Leu Gly Glu Val Ser Asp Met Ala Ala Val Glu
    210                 215                 220

gcg gca gag ctg gaa atg acc cgt caa gtg ctg cac gcg ggc gca cgt        720
Ala Ala Glu Leu Glu Met Thr Arg Gln Val Leu His Ala Gly Ala Arg
225                 230                 235                 240

cag gac gat gct gag ccg ggc gtg tcc ggt gcg tct gca cac tgg ggt        768
Gln Asp Asp Ala Glu Pro Gly Val Ser Gly Ala Ser Ala His Trp Gly
                245                 250                 255

caa cgt gcc ctg cag ggt gct caa gct gtc gcg gca gcg cag cgt ctg        816
Gln Arg Ala Leu Gln Gly Ala Gln Ala Val Ala Ala Ala Gln Arg Leu
            260                 265                 270

gta cat gcg atc gca ctg atg acc cag ttc ggt cgc gca ggt tct acc        864
Val His Ala Ile Ala Leu Met Thr Gln Phe Gly Arg Ala Gly Ser Thr
        275                 280                 285

aac act cct cag gaa gca gct agc ctg tct gct gct gtt ttc ggt ctg        912
Asn Thr Pro Gln Glu Ala Ala Ser Leu Ser Ala Ala Val Phe Gly Leu
    290                 295                 300

ggc gaa gcg tct tct gcg gtt gca gaa acc gtg agc ggt ttc ttc cgt        960
Gly Glu Ala Ser Ser Ala Val Ala Glu Thr Val Ser Gly Phe Phe Arg
305                 310                 315                 320

ggt tct agc cgc tgg gcg ggt ggt ttt ggt gta gca ggt ggc gct atg       1008
Gly Ser Ser Arg Trp Ala Gly Gly Phe Gly Val Ala Gly Gly Ala Met
                325                 330                 335

gcc ctg ggt ggc ggt atc gcg gct gct gtc ggc gca ggt atg agc ctg       1056
Ala Leu Gly Gly Gly Ile Ala Ala Ala Val Gly Ala Gly Met Ser Leu
            340                 345                 350

acc gac gat gct cct gcg ggc cag aaa gcc gca gct ggc gct gaa atc       1104
Thr Asp Asp Ala Pro Ala Gly Gln Lys Ala Ala Ala Gly Ala Glu Ile
        355                 360                 365

gcg ctg cag ctg act ggt ggt acc gtt gaa ctg gct agc tct atc gcg       1152
Ala Leu Gln Leu Thr Gly Gly Thr Val Glu Leu Ala Ser Ser Ile Ala
    370                 375                 380

ctg gct ctg gca gcg gca cgt ggc gtg act tct ggc ctg caa gtc gcc       1200
Leu Ala Leu Ala Ala Ala Arg Gly Val Thr Ser Gly Leu Gln Val Ala
385                 390                 395                 400

ggt gcc tct gcg ggc gct gct gcg ggc gct ctg gca gct gca ctg tcc       1248
Gly Ala Ser Ala Gly Ala Ala Ala Gly Ala Leu Ala Ala Ala Leu Ser
                405                 410                 415

ccc atg gaa atc tac ggt ctg gta cag cag tct cac tac gca gac cag       1296
Pro Met Glu Ile Tyr Gly Leu Val Gln Gln Ser His Tyr Ala Asp Gln
            420                 425                 430

ctg gat aaa ctg gcg cag gag tct tct gca tac ggt tac gaa ggc gac       1344
Leu Asp Lys Leu Ala Gln Glu Ser Ser Ala Tyr Gly Tyr Glu Gly Asp
        435                 440                 445

gca ctg ctg gcg cag ctg tat cgt gac aaa act gct gct gaa ggt gct       1392

Ala Leu Leu Ala Gln Leu Tyr Arg Asp Lys Thr Ala Ala Glu Gly Ala
    450                 455                 460

gtg gca ggc gtt tct gcg gta ctg tct acc gtt ggc gct gcg gtt tct 1440
Val Ala Gly Val Ser Ala Val Leu Ser Thr Val Gly Ala Ala Val Ser
465                 470                 475                 480

att gcg gct gcc gca tcc gtt gta ggt gcg ccg gtt gct gta gtt acc 1488
Ile Ala Ala Ala Ala Ser Val Val Gly Ala Pro Val Ala Val Val Thr
                485                 490                 495

tct ctg ctg act ggt gca ctg aac ggt atc ctg cgt ggt gtt cag cag 1536
Ser Leu Leu Thr Gly Ala Leu Asn Gly Ile Leu Arg Gly Val Gln Gln
            500                 505                 510

cct att atc gaa aaa ctg gcg aat gat tat gcc cgt aaa atc gac gaa 1584
Pro Ile Ile Glu Lys Leu Ala Asn Asp Tyr Ala Arg Lys Ile Asp Glu
        515                 520                 525

ctg ggc ggt ccg cag gct tat ttt gaa aaa aac ctg cag gcg cgc cac 1632
Leu Gly Gly Pro Gln Ala Tyr Phe Glu Lys Asn Leu Gln Ala Arg His
    530                 535                 540

gaa cag ctg gca aac agc gac ggc ctg cgc aaa atg ctg gca gac ctg 1680
Glu Gln Leu Ala Asn Ser Asp Gly Leu Arg Lys Met Leu Ala Asp Leu
545                 550                 555                 560

caa gct ggt tgg aac gcg tct tct gtg atc ggc gtg cag acc acc gaa 1728
Gln Ala Gly Trp Asn Ala Ser Ser Val Ile Gly Val Gln Thr Thr Glu
                565                 570                 575

att agc aaa tct gcg ctg gaa ctg gct gca att act ggc aac gcg gat 1776
Ile Ser Lys Ser Ala Leu Glu Leu Ala Ala Ile Thr Gly Asn Ala Asp
            580                 585                 590

aac ctg aaa agc gtt gat gtt ttt gtc gat cgc ttc gtt cag ggc gag 1824
Asn Leu Lys Ser Val Asp Val Phe Val Asp Arg Phe Val Gln Gly Glu
        595                 600                 605

cgc gtt gct ggt cag ccg gtt gtt ctg gac gtt gcg gca ggc ggc atc 1872
Arg Val Ala Gly Gln Pro Val Val Leu Asp Val Ala Ala Gly Gly Ile
    610                 615                 620

gac atc gct agc cgc aag ggc gag cgt ccg gct ctg act ttc att acc 1920
Asp Ile Ala Ser Arg Lys Gly Glu Arg Pro Ala Leu Thr Phe Ile Thr
625                 630                 635                 640

ccg ctg gca gct ccg ggt gaa gaa cag cgt cgc cgt acc aag act ggt 1968
Pro Leu Ala Ala Pro Gly Glu Glu Gln Arg Arg Arg Thr Lys Thr Gly
                645                 650                 655

aaa agc gag ttc acc acc ttc gtg gaa atc gta ggt aaa cag gac cgc 2016
Lys Ser Glu Phe Thr Thr Phe Val Glu Ile Val Gly Lys Gln Asp Arg
            660                 665                 670

tgg cgt att cgc gac ggt gcg gcg gac acc acc atc gac ctg gca aaa 2064
Trp Arg Ile Arg Asp Gly Ala Ala Asp Thr Thr Ile Asp Leu Ala Lys
        675                 680                 685

gtt gtt tcc caa ctg gta gat gct aac ggc gtg ctg aaa cat tct att 2112
Val Val Ser Gln Leu Val Asp Ala Asn Gly Val Leu Lys His Ser Ile
    690                 695                 700

aaa ctg gat gta atc ggc ggt gac ggc gat gac gtt gta ctg gcg aac 2160
Lys Leu Asp Val Ile Gly Gly Asp Gly Asp Asp Val Val Leu Ala Asn
705                 710                 715                 720

gcg tct cgt atc cac tat gac ggc ggc gca ggt acc aac acg gtt tcc 2208
Ala Ser Arg Ile His Tyr Asp Gly Gly Ala Gly Thr Asn Thr Val Ser
                725                 730                 735

tac gca gcg ctg ggc cgc cag gat tcc atc act gtt agc gcc gat ggc 2256
Tyr Ala Ala Leu Gly Arg Gln Asp Ser Ile Thr Val Ser Ala Asp Gly
            740                 745                 750

gaa cgt ttc aac gtg cgt aaa cag ctg aat aac gca aac gtc tat cgt 2304
Glu Arg Phe Asn Val Arg Lys Gln Leu Asn Asn Ala Asn Val Tyr Arg
        755                 760                 765

gag ggt gta gct acc cag acc acc gcg tac ggt aag cgt acc gaa aac 2352
Glu Gly Val Ala Thr Gln Thr Thr Ala Tyr Gly Lys Arg Thr Glu Asn
    770                 775                 780

gtt cag tat cgt cac gtg gaa ctg gca cgt gtg ggt cag ctg gta gag 2400
Val Gln Tyr Arg His Val Glu Leu Ala Arg Val Gly Gln Leu Val Glu
785                 790                 795                 800

gtg gat acg ctg gaa cac gtg cag cac atc atc ggt ggc gcc ggt aac 2448
Val Asp Thr Leu Glu His Val Gln His Ile Ile Gly Gly Ala Gly Asn
                805                 810                 815

gat tcc att act ggt aac gct cac gac aac ttc ctg gca ggt ggt tct 2496
Asp Ser Ile Thr Gly Asn Ala His Asp Asn Phe Leu Ala Gly Gly Ser
            820                 825                 830

ggt gat gat cgt ctg gac ggt ggt gcg ggt aac gat acg ctg gta ggc 2544
Gly Asp Asp Arg Leu Asp Gly Gly Ala Gly Asn Asp Thr Leu Val Gly
        835                 840                 845

ggc gaa ggt cag aac acc gta att ggt ggt gcc ggt gac gac gtt ttc 2592
Gly Glu Gly Gln Asn Thr Val Ile Gly Gly Ala Gly Asp Asp Val Phe
    850                 855                 860

ctg cag gat ctg ggc gtt tgg agc aac cag ctg gat ggc ggt gcc ggt 2640
Leu Gln Asp Leu Gly Val Trp Ser Asn Gln Leu Asp Gly Gly Ala Gly
865                 870                 875                 880

gtt gac acg gtt aag tat aac gtt cac cag ccg tct gaa gag cgc ctg 2688
Val Asp Thr Val Lys Tyr Asn Val His Gln Pro Ser Glu Glu Arg Leu
                885                 890                 895

gaa cgt atg ggt gac act ggt att cat gcc gat ctg cag aaa ggc act 2736
Glu Arg Met Gly Asp Thr Gly Ile His Ala Asp Leu Gln Lys Gly Thr
            900                 905                 910

gtt gaa aaa tgg cct gcg ctg aac ctg ttc tct gtg gac cat gtt aag 2784
Val Glu Lys Trp Pro Ala Leu Asn Leu Phe Ser Val Asp His Val Lys
        915                 920                 925

aac att gaa aac ctg cat ggc tct cgc ctg aac gac cgt atc gct ggt 2832
Asn Ile Glu Asn Leu His Gly Ser Arg Leu Asn Asp Arg Ile Ala Gly
    930                 935                 940

gac gac cag gat aac gaa ctg tgg ggt cat gac ggc aat gat acc att 2880
Asp Asp Gln Asp Asn Glu Leu Trp Gly His Asp Gly Asn Asp Thr Ile
945                 950                 955                 960

cgc ggt cgt ggc ggc gac gat att ctg cgt ggc ggt ctg ggc ctg gat 2928
Arg Gly Arg Gly Gly Asp Asp Ile Leu Arg Gly Gly Leu Gly Leu Asp
                965                 970                 975

acc ctg tat ggt gaa gat ggt aac gat att ttc ctg caa gac gat gaa 2976
Thr Leu Tyr Gly Glu Asp Gly Asn Asp Ile Phe Leu Gln Asp Asp Glu
            980                 985                 990

acg gta tct gat gac atc gac ggt ggt gca ggc ctg gac acc gta gat 3024
Thr Val Ser Asp Asp Ile Asp Gly Gly Ala Gly Leu Asp Thr Val Asp
        995                 1000                1005

tac agc gct atg atc cat ccg ggt cgt atc gta gct ccg cac gag 3069
Tyr Ser Ala Met Ile His Pro Gly Arg Ile Val Ala Pro His Glu
    1010                1015                1020

tac ggc ttc ggt atc gag gcg gac ctg tct cgt gaa tgg gtt cgt 3114
Tyr Gly Phe Gly Ile Glu Ala Asp Leu Ser Arg Glu Trp Val Arg
    1025                1030                1035

aaa gcg tcc gcg ctg ggt gtg gac tac tac gat aac gtt cgt aac 3159
Lys Ala Ser Ala Leu Gly Val Asp Tyr Tyr Asp Asn Val Arg Asn
    1040                1045                1050

gtg gaa aac gtg atc ggt acc tcc atg aaa gac gtg ctg att ggc 3204
Val Glu Asn Val Ile Gly Thr Ser Met Lys Asp Val Leu Ile Gly
    1055                1060                1065

gac gca cag gcc aac act ctg atg ggc cag ggc ggt gat gac acg 3249
Asp Ala Gln Ala Asn Thr Leu Met Gly Gln Gly Gly Asp Asp Thr
    1070                1075                1080

gtt cgc ggc ggc gac ggt gat gac ctg ctg ttc ggt ggt gac ggc 3294
Val Arg Gly Gly Asp Gly Asp Asp Leu Leu Phe Gly Gly Asp Gly
    1085                1090                1095

aac gac atg ctg tac ggc gac gcg ggc aac gac acc ctg tac ggc 3339
Asn Asp Met Leu Tyr Gly Asp Ala Gly Asn Asp Thr Leu Tyr Gly
    1100                1105                1110

ggt ctg ggt gac gac act ctg gaa ggt ggt gca ggt aac gac tgg 3384
Gly Leu Gly Asp Asp Thr Leu Glu Gly Gly Ala Gly Asn Asp Trp
    1115                1120                1125

ttc ggc caa acc cag gca cgc gaa cac gac gtg ctg cgt ggt ggc 3429
Phe Gly Gln Thr Gln Ala Arg Glu His Asp Val Leu Arg Gly Gly
    1130                1135                1140

gac ggc gta gac acc gtg gat tac tcc caa act ggc gct cac gcg 3474
Asp Gly Val Asp Thr Val Asp Tyr Ser Gln Thr Gly Ala His Ala
    1145                1150                1155

ggt atc gcg gcc ggt cgt atc ggt ctg ggc att ctg gcc gat ctg 3519
Gly Ile Ala Ala Gly Arg Ile Gly Leu Gly Ile Leu Ala Asp Leu
    1160                1165                1170

ggt gcc ggc cgt gtc gac aaa ctg ggt gag gct ggc tct agc gcc 3564
Gly Ala Gly Arg Val Asp Lys Leu Gly Glu Ala Gly Ser Ser Ala
    1175                1180                1185

tat gat act gtt tcc ggc atc gaa aac gtg gta ggc act gaa ctg 3609
Tyr Asp Thr Val Ser Gly Ile Glu Asn Val Val Gly Thr Glu Leu

1190                    1195                    1200

gca gat cgt atc acg ggt gac gcg caa gcg aac gtt ctg cgt ggt          3654
Ala Asp Arg Ile Thr Gly Asp Ala Gln Ala Asn Val Leu Arg Gly
    1205                    1210                    1215

gct ggc ggt gca gac gtg ctg gcg ggt ggt gaa ggc gac gac gta          3699
Ala Gly Gly Ala Asp Val Leu Ala Gly Gly Glu Gly Asp Asp Val
    1220                    1225                    1230

ctg ctg ggc ggt gac ggt gac gat cag ctg agc ggt gat gcg ggt          3744
Leu Leu Gly Gly Asp Gly Asp Asp Gln Leu Ser Gly Asp Ala Gly
    1235                    1240                    1245

cgt gac cgt ctg tac ggt gaa gca ggt gac gat tgg ttc ttc cag          3789
Arg Asp Arg Leu Tyr Gly Glu Ala Gly Asp Asp Trp Phe Phe Gln
    1250                    1255                    1260

gac gct gct aac gct ggc aac ctg ctg gac ggc ggc gat ggc cgt          3834
Asp Ala Ala Asn Ala Gly Asn Leu Leu Asp Gly Gly Asp Gly Arg
    1265                    1270                    1275

gac act gtt gac ttc agc ggt ccg ggt cgc ggt ctg gac gca ggc          3879
Asp Thr Val Asp Phe Ser Gly Pro Gly Arg Gly Leu Asp Ala Gly
    1280                    1285                    1290

gcg aaa ggc gtt ttc ctg agc ctg ggt aag ggt ttc gca tct ctg          3924
Ala Lys Gly Val Phe Leu Ser Leu Gly Lys Gly Phe Ala Ser Leu
    1295                    1300                    1305

atg gac gaa cca gaa acc agc aat gtc ctg cgt aac atc gaa aac          3969
Met Asp Glu Pro Glu Thr Ser Asn Val Leu Arg Asn Ile Glu Asn
    1310                    1315                    1320

gct gtc ggt tcc gca cgc gat gat gtt ctg att ggc gat gct ggt          4014
Ala Val Gly Ser Ala Arg Asp Asp Val Leu Ile Gly Asp Ala Gly
    1325                    1330                    1335

gcc aat gtt ctg aac ggt ctg gcg ggt aat gat gta ctg tct ggt          4059
Ala Asn Val Leu Asn Gly Leu Ala Gly Asn Asp Val Leu Ser Gly
    1340                    1345                    1350

ggc gct ggt gat gat gtc ctg ctg ggt gat gag ggc agc gat ctg          4104
Gly Ala Gly Asp Asp Val Leu Leu Gly Asp Glu Gly Ser Asp Leu
    1355                    1360                    1365

ctg tcc ggc gat gcc ggc aac gac gat ctg ttc ggt ggc caa ggt          4149
Leu Ser Gly Asp Ala Gly Asn Asp Asp Leu Phe Gly Gly Gln Gly
    1370                    1375                    1380

gat gat act tac ctg ttc ggc gtt ggc tat ggt cat gac acc atc          4194
Asp Asp Thr Tyr Leu Phe Gly Val Gly Tyr Gly His Asp Thr Ile
    1385                    1390                    1395

tat gag tct ggt ggc ggc cac gat acg att cgt att aat gca ggt          4239
Tyr Glu Ser Gly Gly Gly His Asp Thr Ile Arg Ile Asn Ala Gly
    1400                    1405                    1410

gct gac caa ctg tgg ttt gca cgt cag ggt aac gat ctg gaa att          4284
Ala Asp Gln Leu Trp Phe Ala Arg Gln Gly Asn Asp Leu Glu Ile
    1415                    1420                    1425

cgc atc ctg ggt act gat gac gct ctg acc gta cac gac tgg tac          4329

Arg Ile Leu Gly Thr Asp Asp Ala Leu Thr Val His Asp Trp Tyr
    1430                1435                1440

cgc gat gcg gat cac cgc gta gaa atc atc cat gcg gct aac caa 4374
Arg Asp Ala Asp His Arg Val Glu Ile Ile His Ala Ala Asn Gln
    1445                1450                1455

gct gta gac cag gct ggt att gaa aaa ctg gta gaa gcg atg gca 4419
Ala Val Asp Gln Ala Gly Ile Glu Lys Leu Val Glu Ala Met Ala
    1460                1465                1470

cag tat ccg gac cca ggt gcg gct gct gca gca cct cca gcg gct 4464
Gln Tyr Pro Asp Pro Gly Ala Ala Ala Ala Ala Pro Pro Ala Ala
    1475                1480                1485

cgt gtg ccg gat acc ctg atg cag agc ctg gcc gtc aat tgg cgt 4509
Arg Val Pro Asp Thr Leu Met Gln Ser Leu Ala Val Asn Trp Arg
    1490                1495                1500

taa 4512

<210> 12
<211> 1503
<212> PRT
<213> Artificial Sequence

<220>
<223> Synthetic Construct

<400> 12

Met Gln Gln Ser His Gln Ala Gly Tyr Ala Asn Ala Ala Asp Arg Glu
1               5                   10                  15

Ser Gly Ile Pro Ala Ala Val Leu Asp Gly Ile Lys Ala Val Ala Lys
            20                  25                  30

Glu Lys Asn Ala Thr Leu Met Phe Arg Leu Val Asn Pro His Ser Thr
        35                  40                  45

Ser Leu Ile Ala Glu Gly Val Ala Thr Lys Gly Leu Gly Val His Ala
    50                  55                  60

Lys Ser Ser Asp Trp Gly Leu Gln Ala Gly Tyr Ile Pro Val Asn Pro
65                  70                  75                  80

Asn Leu Ser Lys Leu Phe Gly Arg Ala Pro Glu Val Ile Ala Arg Ala
                85                  90                  95

Asp Asn Asp Val Asn Ser Ser Leu Ala His Gly His Thr Ala Val Asp
            100                 105                 110

Leu Thr Leu Ser Lys Glu Arg Leu Asp Tyr Leu Arg Gln Ala Gly Leu
        115                 120                 125

Val Thr Gly Met Ala Asp Gly Val Val Ala Ser Asn His Ala Gly Tyr
    130                 135                 140

Glu Gln Phe Glu Phe Arg Val Lys Glu Thr Ser Asp Gly Arg Tyr Ala
145                 150                 155                 160

Val Gln Tyr Arg Arg Lys Gly Gly Asp Asp Phe Glu Ala Val Lys Val
                165                 170                 175

Ile Gly Asn Ala Ala Gly Ile Pro Gly Leu Arg Arg Pro Ser Leu Gly
            180                 185                 190

Ala Val Glu Arg Gln Asp Ser Gly Tyr Asp Ser Leu Asp Gly Val Gly
        195                 200                 205

Ser Arg Ser Phe Ser Leu Gly Glu Val Ser Asp Met Ala Ala Val Glu
    210                 215                 220

Ala Ala Glu Leu Glu Met Thr Arg Gln Val Leu His Ala Gly Ala Arg
225                 230                 235                 240

Gln Asp Asp Ala Glu Pro Gly Val Ser Gly Ala Ser Ala His Trp Gly
                245                 250                 255

Gln Arg Ala Leu Gln Gly Ala Gln Ala Val Ala Ala Ala Gln Arg Leu
            260                 265                 270

Val His Ala Ile Ala Leu Met Thr Gln Phe Gly Arg Ala Gly Ser Thr
        275                 280                 285

Asn Thr Pro Gln Glu Ala Ala Ser Leu Ser Ala Ala Val Phe Gly Leu
    290                 295                 300

Gly Glu Ala Ser Ser Ala Val Ala Glu Thr Val Ser Gly Phe Phe Arg
305                 310                 315                 320

Gly Ser Ser Arg Trp Ala Gly Gly Phe Gly Val Ala Gly Gly Ala Met
                325                 330                 335

Ala Leu Gly Gly Gly Ile Ala Ala Ala Val Gly Ala Gly Met Ser Leu
            340                 345                 350

Thr Asp Asp Ala Pro Ala Gly Gln Lys Ala Ala Ala Gly Ala Glu Ile
        355                 360                 365

Ala Leu Gln Leu Thr Gly Gly Thr Val Glu Leu Ala Ser Ser Ile Ala
    370                 375                 380

Leu Ala Leu Ala Ala Ala Arg Gly Val Thr Ser Gly Leu Gln Val Ala
385                 390                 395                 400

Gly Ala Ser Ala Gly Ala Ala Ala Gly Ala Leu Ala Ala Ala Leu Ser
                405                 410                 415

Pro Met Glu Ile Tyr Gly Leu Val Gln Gln Ser His Tyr Ala Asp Gln
            420                 425                 430

Leu Asp Lys Leu Ala Gln Glu Ser Ser Ala Tyr Gly Tyr Glu Gly Asp
        435                 440                 445

Ala Leu Leu Ala Gln Leu Tyr Arg Asp Lys Thr Ala Ala Glu Gly Ala
    450                 455                 460

Val Ala Gly Val Ser Ala Val Leu Ser Thr Val Gly Ala Ala Val Ser
465                 470                 475                 480

Ile Ala Ala Ala Ala Ser Val Val Gly Ala Pro Val Ala Val Val Thr
                485                 490                 495

Ser Leu Leu Thr Gly Ala Leu Asn Gly Ile Leu Arg Gly Val Gln Gln
            500                 505                 510

Pro Ile Ile Glu Lys Leu Ala Asn Asp Tyr Ala Arg Lys Ile Asp Glu
        515                 520                 525

Leu Gly Gly Pro Gln Ala Tyr Phe Glu Lys Asn Leu Gln Ala Arg His
    530                 535                 540

Glu Gln Leu Ala Asn Ser Asp Gly Leu Arg Lys Met Leu Ala Asp Leu
545                 550                 555                 560

Gln Ala Gly Trp Asn Ala Ser Ser Val Ile Gly Val Gln Thr Thr Glu
                565                 570                 575

Ile Ser Lys Ser Ala Leu Glu Leu Ala Ala Ile Thr Gly Asn Ala Asp
            580                 585                 590

Asn Leu Lys Ser Val Asp Val Phe Val Asp Arg Phe Val Gln Gly Glu
        595                 600                 605

Arg Val Ala Gly Gln Pro Val Val Leu Asp Val Ala Ala Gly Gly Ile
    610                 615                 620

Asp Ile Ala Ser Arg Lys Gly Glu Arg Pro Ala Leu Thr Phe Ile Thr

625                 630                 635                 640

Pro Leu Ala Ala Pro Gly Glu Glu Gln Arg Arg Arg Thr Lys Thr Gly
                645                 650                 655

Lys Ser Glu Phe Thr Thr Phe Val Glu Ile Val Gly Lys Gln Asp Arg
            660                 665                 670

Trp Arg Ile Arg Asp Gly Ala Ala Asp Thr Thr Ile Asp Leu Ala Lys
        675                 680                 685

Val Val Ser Gln Leu Val Asp Ala Asn Gly Val Leu Lys His Ser Ile
    690                 695                 700

Lys Leu Asp Val Ile Gly Gly Asp Gly Asp Asp Val Val Leu Ala Asn
705                 710                 715                 720

Ala Ser Arg Ile His Tyr Asp Gly Gly Ala Gly Thr Asn Thr Val Ser
                725                 730                 735

Tyr Ala Ala Leu Gly Arg Gln Asp Ser Ile Thr Val Ser Ala Asp Gly
            740                 745                 750

Glu Arg Phe Asn Val Arg Lys Gln Leu Asn Asn Ala Asn Val Tyr Arg
        755                 760                 765

Glu Gly Val Ala Thr Gln Thr Thr Ala Tyr Gly Lys Arg Thr Glu Asn
    770                 775                 780

Val Gln Tyr Arg His Val Glu Leu Ala Arg Val Gly Gln Leu Val Glu
785                 790                 795                 800

Val Asp Thr Leu Glu His Val Gln His Ile Ile Gly Gly Ala Gly Asn
                805                 810                 815

Asp Ser Ile Thr Gly Asn Ala His Asp Asn Phe Leu Ala Gly Gly Ser
            820                 825                 830

Gly Asp Asp Arg Leu Asp Gly Gly Ala Gly Asn Asp Thr Leu Val Gly
        835                 840                 845

Gly Glu Gly Gln Asn Thr Val Ile Gly Gly Ala Gly Asp Asp Val Phe
    850                 855                 860

Leu Gln Asp Leu Gly Val Trp Ser Asn Gln Leu Asp Gly Gly Ala Gly
865                 870                 875                 880

Val Asp Thr Val Lys Tyr Asn Val His Gln Pro Ser Glu Glu Arg Leu
                885                 890                 895

Glu Arg Met Gly Asp Thr Gly Ile His Ala Asp Leu Gln Lys Gly Thr
            900                 905                 910

Val Glu Lys Trp Pro Ala Leu Asn Leu Phe Ser Val Asp His Val Lys
        915                 920                 925

Asn Ile Glu Asn Leu His Gly Ser Arg Leu Asn Asp Arg Ile Ala Gly
    930                 935                 940

Asp Asp Gln Asp Asn Glu Leu Trp Gly His Asp Gly Asn Asp Thr Ile
945                 950                 955                 960

Arg Gly Arg Gly Gly Asp Asp Ile Leu Arg Gly Gly Leu Gly Leu Asp
                965                 970                 975

Thr Leu Tyr Gly Glu Asp Gly Asn Asp Ile Phe Leu Gln Asp Asp Glu
            980                 985                 990

Thr Val Ser Asp Asp Ile Asp Gly  Gly Ala Gly Leu Asp  Thr Val Asp
        995                 1000                1005

Tyr Ser  Ala Met Ile His Pro  Gly Arg Ile Val Ala  Pro His Glu
    1010                1015                1020

Tyr Gly  Phe Gly Ile Glu Ala  Asp Leu Ser Arg Glu  Trp Val Arg
    1025                1030                1035

Lys Ala  Ser Ala Leu Gly Val  Asp Tyr Tyr Asp Asn  Val Arg Asn
    1040                1045                1050

Val Glu  Asn Val Ile Gly Thr  Ser Met Lys Asp Val  Leu Ile Gly
    1055                1060                1065

Asp Ala  Gln Ala Asn Thr Leu  Met Gly Gln Gly Gly  Asp Asp Thr
    1070                1075                1080

Val Arg  Gly Gly Asp Gly Asp  Asp Leu Leu Phe Gly  Gly Asp Gly
    1085                1090                1095

Asn Asp  Met Leu Tyr Gly Asp  Ala Gly Asn Asp Thr  Leu Tyr Gly
    1100                1105                1110

Gly Leu  Gly Asp Asp Thr Leu  Glu Gly Gly Ala Gly  Asn Asp Trp
    1115                1120                1125

Phe Gly Gln Thr Gln Ala Arg Glu His Asp Val Leu Arg Gly Gly
    1130                1135                1140

Asp Gly Val Asp Thr Val Asp Tyr Ser Gln Thr Gly Ala His Ala
    1145                1150                1155

Gly Ile Ala Ala Gly Arg Ile Gly Leu Gly Ile Leu Ala Asp Leu
    1160                1165                1170

Gly Ala Gly Arg Val Asp Lys Leu Gly Glu Ala Gly Ser Ser Ala
    1175                1180                1185

Tyr Asp Thr Val Ser Gly Ile Glu Asn Val Val Gly Thr Glu Leu
    1190                1195                1200

Ala Asp Arg Ile Thr Gly Asp Ala Gln Ala Asn Val Leu Arg Gly
    1205                1210                1215

Ala Gly Gly Ala Asp Val Leu Ala Gly Gly Glu Gly Asp Asp Val
    1220                1225                1230

Leu Leu Gly Gly Asp Gly Asp Asp Gln Leu Ser Gly Asp Ala Gly
    1235                1240                1245

Arg Asp Arg Leu Tyr Gly Glu Ala Gly Asp Asp Trp Phe Phe Gln
    1250                1255                1260

Asp Ala Ala Asn Ala Gly Asn Leu Leu Asp Gly Gly Asp Gly Arg
    1265                1270                1275

Asp Thr Val Asp Phe Ser Gly Pro Gly Arg Gly Leu Asp Ala Gly
    1280                1285                1290

Ala Lys Gly Val Phe Leu Ser Leu Gly Lys Gly Phe Ala Ser Leu
    1295                1300                1305

Met Asp Glu Pro Glu Thr Ser Asn Val Leu Arg Asn Ile Glu Asn
    1310                1315                1320

Ala Val Gly Ser Ala Arg Asp Asp Val Leu Ile Gly Asp Ala Gly
    1325                1330                1335

Ala Asn Val Leu Asn Gly Leu Ala Gly Asn Asp Val Leu Ser Gly
    1340                1345                1350

Gly Ala Gly Asp Asp Val Leu Leu Gly Asp Glu Gly Ser Asp Leu
    1355                1360                1365

Leu Ser Gly Asp Ala Gly Asn Asp Asp Leu Phe Gly Gly Gln Gly
    1370                1375                1380

Asp Asp Thr Tyr Leu Phe Gly Val Gly Tyr Gly His Asp Thr Ile
    1385                1390                1395

Tyr Glu Ser Gly Gly Gly His Asp Thr Ile Arg Ile Asn Ala Gly
    1400                1405                1410

Ala Asp Gln Leu Trp Phe Ala Arg Gln Gly Asn Asp Leu Glu Ile
    1415                1420                1425

Arg Ile Leu Gly Thr Asp Asp Ala Leu Thr Val His Asp Trp Tyr
    1430                1435                1440

Arg Asp Ala Asp His Arg Val Glu Ile Ile His Ala Ala Asn Gln
    1445                1450                1455

Ala Val Asp Gln Ala Gly Ile Glu Lys Leu Val Glu Ala Met Ala
    1460                1465                1470

Gln Tyr Pro Asp Pro Gly Ala Ala Ala Ala Ala Pro Pro Ala Ala
    1475                1480                1485

Arg Val Pro Asp Thr Leu Met Gln Ser Leu Ala Val Asn Trp Arg
    1490                1495                1500

<210> 13
<211> 1613
<212> PRT
<213> Artificial Sequence

<220>
<223> Polypeptide 1-227 + 321-1706 of SEQ ID NO: 4

<400> 13

Met Gln Gln Ser His Gln Ala Gly Tyr Ala Asn Ala Ala Asp Arg Glu
1               5                   10                  15

Ser Gly Ile Pro Ala Ala Val Leu Asp Gly Ile Lys Ala Val Ala Lys
            20                  25                  30

Glu Lys Asn Ala Thr Leu Met Phe Arg Leu Val Asn Pro His Ser Thr
        35                  40                  45

Ser Leu Ile Ala Glu Gly Val Ala Thr Lys Gly Leu Gly Val His Ala
    50                  55                  60

Lys Ser Ser Asp Trp Gly Leu Gln Ala Gly Tyr Ile Pro Val Asn Pro
65                  70                  75                  80

Asn Leu Ser Lys Leu Phe Gly Arg Ala Pro Glu Val Ile Ala Arg Ala
                85                  90                  95

Asp Asn Asp Val Asn Ser Ser Leu Ala His Gly His Thr Ala Val Asp
            100                 105                 110

Leu Thr Leu Ser Lys Glu Arg Leu Asp Tyr Leu Arg Gln Ala Gly Leu
        115                 120                 125

Val Thr Gly Met Ala Asp Gly Val Val Ala Ser Asn His Ala Gly Tyr
    130                 135                 140

Glu Gln Phe Glu Phe Arg Val Lys Glu Thr Ser Asp Gly Arg Tyr Ala
145                 150                 155                 160

Val Gln Tyr Arg Arg Lys Gly Gly Asp Asp Phe Glu Ala Val Lys Val
                165                 170                 175

Ile Gly Asn Ala Ala Gly Ile Pro Leu Thr Ala Asp Ile Asp Met Phe
            180                 185                 190

Ala Ile Met Pro His Leu Ser Asn Phe Arg Asp Ser Ala Arg Ser Ser
        195                 200                 205

Val Thr Ser Gly Asp Ser Val Thr Asp Tyr Leu Ala Arg Thr Arg Arg
    210                 215                 220

Ala Ala Ser Glu Ser Gln Met Leu Thr Arg Gly Gln Leu Lys Glu Tyr
225                 230                 235                 240

Ile Gly Gln Gln Arg Gly Glu Gly Tyr Val Phe Tyr Glu Asn Arg Ala
                245                 250                 255

Tyr Gly Val Ala Gly Lys Ser Leu Phe Asp Asp Gly Leu Gly Ala Ala
            260                 265                 270

Pro Gly Val Pro Ser Gly Arg Ser Lys Phe Ser Pro Asp Val Leu Glu
        275                 280                 285

Thr Val Pro Ala Ser Pro Gly Leu Arg Arg Pro Ser Leu Gly Ala Val
    290                 295                 300

Glu Arg Gln Asp Ser Gly Tyr Asp Ser Leu Asp Gly Val Gly Ser Arg
305                 310                 315                 320

Ser Phe Ser Leu Gly Glu Val Ser Asp Met Ala Ala Val Glu Ala Ala
                325                 330                 335

Glu Leu Glu Met Thr Arg Gln Val Leu His Ala Gly Ala Arg Gln Asp
            340                 345                 350

Asp Ala Glu Pro Gly Val Ser Gly Ala Ser Ala His Trp Gly Gln Arg
        355                 360                 365

Ala Leu Gln Gly Ala Gln Ala Val Ala Ala Ala Gln Arg Leu Val His
    370                 375                 380

Ala Ile Ala Leu Met Thr Gln Phe Gly Arg Ala Gly Ser Thr Asn Thr
385                 390                 395                 400

Pro Gln Glu Ala Ala Ser Leu Ser Ala Ala Val Phe Gly Leu Gly Glu
                405                 410                 415

Ala Ser Ser Ala Val Ala Glu Thr Val Ser Gly Phe Phe Arg Gly Ser
            420                 425                 430

Ser Arg Trp Ala Gly Gly Phe Gly Val Ala Gly Gly Ala Met Ala Leu
        435                 440                 445

Gly Gly Gly Ile Ala Ala Ala Val Gly Ala Gly Met Ser Leu Thr Asp
    450                 455                 460

Asp Ala Pro Ala Gly Gln Lys Ala Ala Ala Gly Ala Glu Ile Ala Leu
465                 470                 475                 480

Gln Leu Thr Gly Gly Thr Val Glu Leu Ala Ser Ser Ile Ala Leu Ala
                485                 490                 495

Leu Ala Ala Ala Arg Gly Val Thr Ser Gly Leu Gln Val Ala Gly Ala
            500                 505                 510

Ser Ala Gly Ala Ala Ala Gly Ala Leu Ala Ala Ala Leu Ser Pro Met
        515                 520                 525

Glu Ile Tyr Gly Leu Val Gln Gln Ser His Tyr Ala Asp Gln Leu Asp
    530                 535                 540

Lys Leu Ala Gln Glu Ser Ser Ala Tyr Gly Tyr Glu Gly Asp Ala Leu
545                 550                 555                 560

Leu Ala Gln Leu Tyr Arg Asp Lys Thr Ala Ala Glu Gly Ala Val Ala

                565                 570                 575

Gly Val Ser Ala Val Leu Ser Thr Val Gly Ala Ala Val Ser Ile Ala
            580                 585                 590

Ala Ala Ala Ser Val Val Gly Ala Pro Val Ala Val Val Thr Ser Leu
        595                 600                 605

Leu Thr Gly Ala Leu Asn Gly Ile Leu Arg Gly Val Gln Gln Pro Ile
    610                 615                 620

Ile Glu Lys Leu Ala Asn Asp Tyr Ala Arg Lys Ile Asp Glu Leu Gly
625                 630                 635                 640

Gly Pro Gln Ala Tyr Phe Glu Lys Asn Leu Gln Ala Arg His Glu Gln
                645                 650                 655

Leu Ala Asn Ser Asp Gly Leu Arg Lys Met Leu Ala Asp Leu Gln Ala
            660                 665                 670

Gly Trp Asn Ala Ser Ser Val Ile Gly Val Gln Thr Thr Glu Ile Ser
        675                 680                 685

Lys Ser Ala Leu Glu Leu Ala Ala Ile Thr Gly Asn Ala Asp Asn Leu
    690                 695                 700

Lys Ser Val Asp Val Phe Val Asp Arg Phe Val Gln Gly Glu Arg Val
705                 710                 715                 720

Ala Gly Gln Pro Val Val Leu Asp Val Ala Ala Gly Gly Ile Asp Ile
                725                 730                 735

Ala Ser Arg Lys Gly Glu Arg Pro Ala Leu Thr Phe Ile Thr Pro Leu
            740                 745                 750

Ala Ala Pro Gly Glu Glu Gln Arg Arg Arg Thr Lys Thr Gly Lys Ser
        755                 760                 765

Glu Phe Thr Thr Phe Val Glu Ile Val Gly Lys Gln Asp Arg Trp Arg
    770                 775                 780

Ile Arg Asp Gly Ala Ala Asp Thr Thr Ile Asp Leu Ala Lys Val Val
785                 790                 795                 800

Ser Gln Leu Val Asp Ala Asn Gly Val Leu Lys His Ser Ile Lys Leu
                805                 810                 815

Asp Val Ile Gly Gly Asp Gly Asp Asp Val Val Leu Ala Asn Ala Ser
            820                 825                 830

Arg Ile His Tyr Asp Gly Gly Ala Gly Thr Asn Thr Val Ser Tyr Ala
        835                 840                 845

Ala Leu Gly Arg Gln Asp Ser Ile Thr Val Ser Ala Asp Gly Glu Arg
    850                 855                 860

Phe Asn Val Arg Lys Gln Leu Asn Asn Ala Asn Val Tyr Arg Glu Gly
865                 870                 875                 880

Val Ala Thr Gln Thr Thr Ala Tyr Gly Lys Arg Thr Glu Asn Val Gln
                885                 890                 895

Tyr Arg His Val Glu Leu Ala Arg Val Gly Gln Leu Val Glu Val Asp
            900                 905                 910

Thr Leu Glu His Val Gln His Ile Ile Gly Gly Ala Gly Asn Asp Ser
        915                 920                 925

Ile Thr Gly Asn Ala His Asp Asn Phe Leu Ala Gly Gly Ser Gly Asp
    930                 935                 940

Asp Arg Leu Asp Gly Gly Ala Gly Asn Asp Thr Leu Val Gly Gly Glu
945                 950                 955                 960

Gly Gln Asn Thr Val Ile Gly Gly Ala Gly Asp Asp Val Phe Leu Gln
                965                 970                 975

Asp Leu Gly Val Trp Ser Asn Gln Leu Asp Gly Gly Ala Gly Val Asp
            980                 985                 990

Thr Val Lys Tyr Asn Val His Gln Pro Ser Glu Glu Arg Leu Glu Arg
        995                 1000                1005

Met Gly Asp Thr Gly Ile His Ala Asp Leu Gln Lys Gly Thr Val
    1010                1015                1020

Glu Lys Trp Pro Ala Leu Asn Leu Phe Ser Val Asp His Val Lys
    1025                1030                1035

Asn Ile Glu Asn Leu His Gly Ser Arg Leu Asn Asp Arg Ile Ala
    1040                1045                1050

Gly Asp Asp Gln Asp Asn Glu Leu Trp Gly His Asp Gly Asn Asp
    1055                1060                1065

Thr Ile Arg Gly Arg Gly Gly Asp Asp Ile Leu Arg Gly Gly Leu
    1070                1075                1080

Gly Leu Asp Thr Leu Tyr Gly Glu Asp Gly Asn Asp Ile Phe Leu
    1085                1090                1095

Gln Asp Asp Glu Thr Val Ser Asp Asp Ile Asp Gly Gly Ala Gly
    1100                1105                1110

Leu Asp Thr Val Asp Tyr Ser Ala Met Ile His Pro Gly Arg Ile
    1115                1120                1125

Val Ala Pro His Glu Tyr Gly Phe Gly Ile Glu Ala Asp Leu Ser
    1130                1135                1140

Arg Glu Trp Val Arg Lys Ala Ser Ala Leu Gly Val Asp Tyr Tyr
    1145                1150                1155

Asp Asn Val Arg Asn Val Glu Asn Val Ile Gly Thr Ser Met Lys
    1160                1165                1170

Asp Val Leu Ile Gly Asp Ala Gln Ala Asn Thr Leu Met Gly Gln
    1175                1180                1185

Gly Gly Asp Asp Thr Val Arg Gly Gly Asp Gly Asp Asp Leu Leu
    1190                1195                1200

Phe Gly Gly Asp Gly Asn Asp Met Leu Tyr Gly Asp Ala Gly Asn
    1205                1210                1215

Asp Thr Leu Tyr Gly Gly Leu Gly Asp Asp Thr Leu Glu Gly Gly
    1220                1225                1230

Ala Gly Asn Asp Trp Phe Gly Gln Thr Gln Ala Arg Glu His Asp
    1235                1240                1245

Val Leu Arg Gly Gly Asp Gly Val Asp Thr Val Asp Tyr Ser Gln
    1250                1255                1260

Thr Gly Ala His Ala Gly Ile Ala Ala Gly Arg Ile Gly Leu Gly
    1265                1270                1275

Ile Leu Ala Asp Leu Gly Ala Gly Arg Val Asp Lys Leu Gly Glu
    1280                1285                1290

Ala Gly Ser Ser Ala Tyr Asp Thr Val Ser Gly Ile Glu Asn Val
    1295                1300                1305

Val Gly Thr Glu Leu Ala Asp Arg Ile Thr Gly Asp Ala Gln Ala
    1310                1315                1320

Asn Val Leu Arg Gly Ala Gly Gly Ala Asp Val Leu Ala Gly Gly
    1325                1330                1335

Glu Gly Asp Asp Val Leu Leu Gly Gly Asp Gly Asp Asp Gln Leu
    1340                1345                1350

Ser Gly Asp Ala Gly Arg Asp Arg Leu Tyr Gly Glu Ala Gly Asp
    1355                1360                1365

Asp Trp Phe Phe Gln Asp Ala Ala Asn Ala Gly Asn Leu Leu Asp
    1370                1375                1380

Gly Gly Asp Gly Arg Asp Thr Val Asp Phe Ser Gly Pro Gly Arg
    1385                1390                1395

Gly Leu Asp Ala Gly Ala Lys Gly Val Phe Leu Ser Leu Gly Lys
    1400                1405                1410

Gly Phe Ala Ser Leu Met Asp Glu Pro Glu Thr Ser Asn Val Leu
    1415                1420                1425

Arg Asn Ile Glu Asn Ala Val Gly Ser Ala Arg Asp Asp Val Leu
    1430                1435                1440

Ile Gly Asp Ala Gly Ala Asn Val Leu Asn Gly Leu Ala Gly Asn
    1445                1450                1455

Asp Val Leu Ser Gly Gly Ala Gly Asp Asp Val Leu Leu Gly Asp
    1460                1465                1470

Glu Gly Ser Asp Leu Leu Ser Gly Asp Ala Gly Asn Asp Asp Leu
    1475                1480                1485

Phe Gly Gly Gln Gly Asp Asp Thr Tyr Leu Phe Gly Val Gly Tyr
    1490                1495                1500

Gly His Asp Thr Ile Tyr Glu Ser Gly Gly Gly His Asp Thr Ile
    1505                1510                1515

Arg Ile Asn Ala Gly Ala Asp Gln Leu Trp Phe Ala Arg Gln Gly
    1520                1525                1530

Asn Asp Leu Glu Ile Arg Ile Leu Gly Thr Asp Asp Ala Leu Thr

1535                    1540                    1545

Val His  Asp Trp Tyr Arg Asp  Ala Asp His Arg Val  Glu Ile Ile
    1550                    1555                    1560

His Ala  Ala Asn Gln Ala Val  Asp Gln Ala Gly Ile  Glu Lys Leu
    1565                    1570                    1575

Val Glu  Ala Met Ala Gln Tyr  Pro Asp Pro Gly Ala  Ala Ala Ala
    1580                    1585                    1590

Ala Pro  Pro Ala Ala Arg Val  Pro Asp Thr Leu Met  Gln Ser Leu
    1595                    1600                    1605

Ala Val  Asn Trp Arg
    1610

<210>  14
<211>  1503
<212>  PRT
<213>  Artificial Sequence

<220>
<223>  Polypeptide 1-183 + 387-1706 of SEQ ID NO:4

<400>  14

Met Gln Gln Ser His Gln Ala Gly Tyr Ala Asn Ala Ala Asp Arg Glu
1               5                   10                  15

Ser Gly Ile Pro Ala Ala Val Leu Asp Gly Ile Lys Ala Val Ala Lys
            20                  25                  30

Glu Lys Asn Ala Thr Leu Met Phe Arg Leu Val Asn Pro His Ser Thr
        35                  40                  45

Ser Leu Ile Ala Glu Gly Val Ala Thr Lys Gly Leu Gly Val His Ala
    50                  55                  60

Lys Ser Ser Asp Trp Gly Leu Gln Ala Gly Tyr Ile Pro Val Asn Pro
65                  70                  75                  80

Asn Leu Ser Lys Leu Phe Gly Arg Ala Pro Glu Val Ile Ala Arg Ala
                85                  90                  95

Asp Asn Asp Val Asn Ser Ser Leu Ala His Gly His Thr Ala Val Asp
            100                 105                 110

Leu Thr Leu Ser Lys Glu Arg Leu Asp Tyr Leu Arg Gln Ala Gly Leu
        115                 120                 125

Val Thr Gly Met Ala Asp Gly Val Val Ala Ser Asn His Ala Gly Tyr
    130                 135                 140

Glu Gln Phe Glu Phe Arg Val Lys Glu Thr Ser Asp Gly Arg Tyr Ala
145                 150                 155                 160

Val Gln Tyr Arg Arg Lys Gly Gly Asp Asp Phe Glu Ala Val Lys Val
                165                 170                 175

Ile Gly Asn Ala Ala Gly Ile Pro Gly Leu Arg Arg Pro Ser Leu Gly
            180                 185                 190

Ala Val Glu Arg Gln Asp Ser Gly Tyr Asp Ser Leu Asp Gly Val Gly
        195                 200                 205

Ser Arg Ser Phe Ser Leu Gly Glu Val Ser Asp Met Ala Ala Val Glu
    210                 215                 220

Ala Ala Glu Leu Glu Met Thr Arg Gln Val Leu His Ala Gly Ala Arg
225                 230                 235                 240

Gln Asp Asp Ala Glu Pro Gly Val Ser Gly Ala Ser Ala His Trp Gly
                245                 250                 255

Gln Arg Ala Leu Gln Gly Ala Gln Ala Val Ala Ala Ala Gln Arg Leu
            260                 265                 270

Val His Ala Ile Ala Leu Met Thr Gln Phe Gly Arg Ala Gly Ser Thr
        275                 280                 285

Asn Thr Pro Gln Glu Ala Ala Ser Leu Ser Ala Ala Val Phe Gly Leu
    290                 295                 300

Gly Glu Ala Ser Ser Ala Val Ala Glu Thr Val Ser Gly Phe Phe Arg
305                 310                 315                 320

Gly Ser Ser Arg Trp Ala Gly Gly Phe Gly Val Ala Gly Gly Ala Met
                325                 330                 335

Ala Leu Gly Gly Gly Ile Ala Ala Ala Val Gly Ala Gly Met Ser Leu
            340                 345                 350

Thr Asp Asp Ala Pro Ala Gly Gln Lys Ala Ala Ala Gly Ala Glu Ile
        355                 360                 365

Ala Leu Gln Leu Thr Gly Gly Thr Val Glu Leu Ala Ser Ser Ile Ala

370                 375                 380

Leu Ala Leu Ala Ala Ala Arg Gly Val Thr Ser Gly Leu Gln Val Ala
385                 390                 395                 400

Gly Ala Ser Ala Gly Ala Ala Ala Gly Ala Leu Ala Ala Ala Leu Ser
                405                 410                 415

Pro Met Glu Ile Tyr Gly Leu Val Gln Gln Ser His Tyr Ala Asp Gln
            420                 425                 430

Leu Asp Lys Leu Ala Gln Glu Ser Ser Ala Tyr Gly Tyr Glu Gly Asp
        435                 440                 445

Ala Leu Leu Ala Gln Leu Tyr Arg Asp Lys Thr Ala Ala Glu Gly Ala
    450                 455                 460

Val Ala Gly Val Ser Ala Val Leu Ser Thr Val Gly Ala Ala Val Ser
465                 470                 475                 480

Ile Ala Ala Ala Ala Ser Val Val Gly Ala Pro Val Ala Val Val Thr
                485                 490                 495

Ser Leu Leu Thr Gly Ala Leu Asn Gly Ile Leu Arg Gly Val Gln Gln
            500                 505                 510

Pro Ile Ile Glu Lys Leu Ala Asn Asp Tyr Ala Arg Lys Ile Asp Glu
        515                 520                 525

Leu Gly Gly Pro Gln Ala Tyr Phe Glu Lys Asn Leu Gln Ala Arg His
    530                 535                 540

Glu Gln Leu Ala Asn Ser Asp Gly Leu Arg Lys Met Leu Ala Asp Leu
545                 550                 555                 560

Gln Ala Gly Trp Asn Ala Ser Ser Val Ile Gly Val Gln Thr Thr Glu
                565                 570                 575

Ile Ser Lys Ser Ala Leu Glu Leu Ala Ala Ile Thr Gly Asn Ala Asp
            580                 585                 590

Asn Leu Lys Ser Val Asp Val Phe Val Asp Arg Phe Val Gln Gly Glu
        595                 600                 605

Arg Val Ala Gly Gln Pro Val Val Leu Asp Val Ala Ala Gly Gly Ile
    610                 615                 620

Asp Ile Ala Ser Arg Lys Gly Glu Arg Pro Ala Leu Thr Phe Ile Thr
625                 630                 635                 640

Pro Leu Ala Ala Pro Gly Glu Glu Gln Arg Arg Arg Thr Lys Thr Gly
                645                 650                 655

Lys Ser Glu Phe Thr Thr Phe Val Glu Ile Val Gly Lys Gln Asp Arg
            660                 665                 670

Trp Arg Ile Arg Asp Gly Ala Ala Asp Thr Thr Ile Asp Leu Ala Lys
        675                 680                 685

Val Val Ser Gln Leu Val Asp Ala Asn Gly Val Leu Lys His Ser Ile
    690                 695                 700

Lys Leu Asp Val Ile Gly Gly Asp Gly Asp Asp Val Val Leu Ala Asn
705                 710                 715                 720

Ala Ser Arg Ile His Tyr Asp Gly Gly Ala Gly Thr Asn Thr Val Ser
                725                 730                 735

Tyr Ala Ala Leu Gly Arg Gln Asp Ser Ile Thr Val Ser Ala Asp Gly
            740                 745                 750

Glu Arg Phe Asn Val Arg Lys Gln Leu Asn Asn Ala Asn Val Tyr Arg
        755                 760                 765

Glu Gly Val Ala Thr Gln Thr Thr Ala Tyr Gly Lys Arg Thr Glu Asn
    770                 775                 780

Val Gln Tyr Arg His Val Glu Leu Ala Arg Val Gly Gln Leu Val Glu
785                 790                 795                 800

Val Asp Thr Leu Glu His Val Gln His Ile Ile Gly Gly Ala Gly Asn
                805                 810                 815

Asp Ser Ile Thr Gly Asn Ala His Asp Asn Phe Leu Ala Gly Gly Ser
            820                 825                 830

Gly Asp Asp Arg Leu Asp Gly Gly Ala Gly Asn Asp Thr Leu Val Gly
        835                 840                 845

Gly Glu Gly Gln Asn Thr Val Ile Gly Gly Ala Gly Asp Asp Val Phe
    850                 855                 860

Leu Gln Asp Leu Gly Val Trp Ser Asn Gln Leu Asp Gly Gly Ala Gly
865                 870                 875                 880

Val Asp Thr Val Lys Tyr Asn Val His Gln Pro Ser Glu Glu Arg Leu
                885                 890                 895

Glu Arg Met Gly Asp Thr Gly Ile His Ala Asp Leu Gln Lys Gly Thr
            900                 905                 910

Val Glu Lys Trp Pro Ala Leu Asn Leu Phe Ser Val Asp His Val Lys
        915                 920                 925

Asn Ile Glu Asn Leu His Gly Ser Arg Leu Asn Asp Arg Ile Ala Gly
    930                 935                 940

Asp Asp Gln Asp Asn Glu Leu Trp Gly His Asp Gly Asn Asp Thr Ile
945                 950                 955                 960

Arg Gly Arg Gly Gly Asp Asp Ile Leu Arg Gly Gly Leu Gly Leu Asp
                965                 970                 975

Thr Leu Tyr Gly Glu Asp Gly Asn Asp Ile Phe Leu Gln Asp Asp Glu
            980                 985                 990

Thr Val Ser Asp Asp Ile Asp Gly Gly Ala Gly Leu Asp Thr Val Asp
        995                 1000                1005

Tyr Ser Ala Met Ile His Pro Gly Arg Ile Val Ala Pro His Glu
    1010                1015                1020

Tyr Gly Phe Gly Ile Glu Ala Asp Leu Ser Arg Glu Trp Val Arg
    1025                1030                1035

Lys Ala Ser Ala Leu Gly Val Asp Tyr Tyr Asp Asn Val Arg Asn
    1040                1045                1050

Val Glu Asn Val Ile Gly Thr Ser Met Lys Asp Val Leu Ile Gly
    1055                1060                1065

Asp Ala Gln Ala Asn Thr Leu Met Gly Gln Gly Gly Asp Asp Thr
    1070                1075                1080

Val Arg Gly Gly Asp Gly Asp Asp Leu Leu Phe Gly Gly Asp Gly
    1085                1090                1095

Asn Asp Met Leu Tyr Gly Asp Ala Gly Asn Asp Thr Leu Tyr Gly
    1100                1105                1110

Gly Leu Gly Asp Asp Thr Leu Glu Gly Gly Ala Gly Asn Asp Trp
    1115                1120                1125

Phe Gly Gln Thr Gln Ala Arg Glu His Asp Val Leu Arg Gly Gly
    1130                1135                1140

Asp Gly Val Asp Thr Val Asp Tyr Ser Gln Thr Gly Ala His Ala
    1145                1150                1155

Gly Ile Ala Ala Gly Arg Ile Gly Leu Gly Ile Leu Ala Asp Leu
    1160                1165                1170

Gly Ala Gly Arg Val Asp Lys Leu Gly Glu Ala Gly Ser Ser Ala
    1175                1180                1185

Tyr Asp Thr Val Ser Gly Ile Glu Asn Val Val Gly Thr Glu Leu
    1190                1195                1200

Ala Asp Arg Ile Thr Gly Asp Ala Gln Ala Asn Val Leu Arg Gly
    1205                1210                1215

Ala Gly Gly Ala Asp Val Leu Ala Gly Gly Glu Gly Asp Asp Val
    1220                1225                1230

Leu Leu Gly Gly Asp Gly Asp Asp Gln Leu Ser Gly Asp Ala Gly
    1235                1240                1245

Arg Asp Arg Leu Tyr Gly Glu Ala Gly Asp Asp Trp Phe Phe Gln
    1250                1255                1260

Asp Ala Ala Asn Ala Gly Asn Leu Leu Asp Gly Gly Asp Gly Arg
    1265                1270                1275

Asp Thr Val Asp Phe Ser Gly Pro Gly Arg Gly Leu Asp Ala Gly
    1280                1285                1290

Ala Lys Gly Val Phe Leu Ser Leu Gly Lys Gly Phe Ala Ser Leu
    1295                1300                1305

Met Asp Glu Pro Glu Thr Ser Asn Val Leu Arg Asn Ile Glu Asn
    1310                1315                1320

Ala Val Gly Ser Ala Arg Asp Asp Val Leu Ile Gly Asp Ala Gly
    1325                1330                1335

Ala Asn Val Leu Asn Gly Leu Ala Gly Asn Asp Val Leu Ser Gly
    1340                1345                1350

Gly Ala Gly Asp Asp Val Leu Leu Gly Asp Glu Gly Ser Asp Leu

1355                1360                1365

Leu Ser  Gly Asp Ala Gly Asn  Asp Asp Leu Phe Gly  Gly Gln Gly
    1370                1375                1380

Asp Asp  Thr Tyr Leu Phe Gly  Val Gly Tyr Gly His  Asp Thr Ile
    1385                1390                1395

Tyr Glu  Ser Gly Gly Gly His  Asp Thr Ile Arg Ile  Asn Ala Gly
    1400                1405                1410

Ala Asp  Gln Leu Trp Phe Ala  Arg Gln Gly Asn Asp  Leu Glu Ile
    1415                1420                1425

Arg Ile  Leu Gly Thr Asp Asp  Ala Leu Thr Val His  Asp Trp Tyr
    1430                1435                1440

Arg Asp  Ala Asp His Arg Val  Glu Ile Ile His Ala  Ala Asn Gln
    1445                1450                1455

Ala Val  Asp Gln Ala Gly Ile  Glu Lys Leu Val Glu  Ala Met Ala
    1460                1465                1470

Gln Tyr  Pro Asp Pro Gly Ala  Ala Ala Ala Ala Pro  Pro Ala Ala
    1475                1480                1485

Arg Val  Pro Asp Thr Leu Met  Gln Ser Leu Ala Val  Asn Trp Arg
    1490                1495                1500

<210>  15
<211>  1613
<212>  PRT
<213>  Artificial Sequence

<220>
<223>  Polypeptide 1-227 + 321-1706 of SEQ ID NO:6

<400>  15

Met Gln Gln Ser His Gln Ala Gly Tyr Ala Asn Ala Ala Asp Arg Glu
1               5                   10                  15

Ser Gly Ile Pro Ala Ala Val Leu Asp Gly Ile Lys Ala Val Ala Lys
            20                  25                  30

Glu Lys Asn Ala Thr Leu Met Phe Arg Leu Val Asn Pro His Ser Thr
        35                  40                  45

Ser Leu Ile Ala Glu Gly Val Ala Thr Lys Gly Leu Gly Val His Ala
    50                  55                  60

Lys Ser Ser Asp Trp Gly Leu Gln Ala Gly Tyr Ile Pro Val Asn Pro
65                  70                  75                  80

Asn Leu Ser Lys Leu Phe Gly Arg Ala Pro Glu Val Ile Ala Arg Ala
                85                  90                  95

Asp Asn Asp Val Asn Ser Ser Leu Ala His Gly His Thr Ala Val Asp
            100                 105                 110

Leu Thr Leu Ser Lys Glu Arg Leu Asp Tyr Leu Arg Gln Ala Gly Leu
        115                 120                 125

Val Thr Gly Met Ala Asp Gly Val Val Ala Ser Asn His Ala Gly Tyr
    130                 135                 140

Glu Gln Phe Glu Phe Arg Val Lys Glu Thr Ser Asp Gly Arg Tyr Ala
145                 150                 155                 160

Val Gln Tyr Arg Arg Lys Gly Gly Asp Asp Phe Glu Ala Val Lys Val
                165                 170                 175

Ile Gly Asn Ala Ala Gly Ile Pro Leu Thr Ala Asp Ile Asp Met Phe
            180                 185                 190

Ala Ile Met Pro His Leu Ser Asn Phe Arg Asp Ser Ala Arg Ser Ser
        195                 200                 205

Val Thr Ser Gly Asp Ser Val Thr Asp Tyr Leu Ala Arg Thr Arg Arg
    210                 215                 220

Ala Ala Ser Glu Ser Gln Met Leu Thr Arg Gly Gln Leu Lys Glu Tyr
225                 230                 235                 240

Ile Gly Gln Gln Arg Gly Glu Gly Tyr Val Phe Tyr Glu Asn Arg Ala
                245                 250                 255

Tyr Gly Val Ala Gly Lys Ser Leu Phe Asp Asp Gly Leu Gly Ala Ala
            260                 265                 270

Pro Gly Val Pro Gly Gly Arg Ser Lys Ser Ser Pro Asp Val Leu Glu
        275                 280                 285

Thr Val Pro Ala Ser Pro Gly Leu Arg Arg Pro Ser Leu Gly Ala Val
    290                 295                 300

Glu Arg Gln Asp Ser Gly Tyr Asp Ser Leu Asp Gly Val Gly Ser Arg

305                 310                 315                 320

Ser Phe Ser Leu Gly Glu Val Ser Asp Met Ala Ala Val Glu Ala Ala
                325                 330                 335

Glu Leu Glu Met Thr Arg Gln Val Leu His Ala Gly Ala Arg Gln Asp
            340                 345                 350

Asp Ala Glu Pro Gly Val Ser Gly Ala Ser Ala His Trp Gly Gln Arg
        355                 360                 365

Ala Leu Gln Gly Ala Gln Ala Val Ala Ala Ala Gln Arg Leu Val His
    370                 375                 380

Ala Ile Ala Leu Met Thr Gln Phe Gly Arg Ala Gly Ser Thr Asn Thr
385                 390                 395                 400

Pro Gln Glu Ala Ala Ser Leu Ser Ala Ala Val Phe Gly Leu Gly Glu
                405                 410                 415

Ala Ser Ser Ala Val Ala Glu Thr Val Ser Gly Phe Phe Arg Gly Ser
            420                 425                 430

Ser Arg Trp Ala Gly Gly Phe Gly Val Ala Gly Gly Ala Met Ala Leu
        435                 440                 445

Gly Gly Gly Ile Ala Ala Ala Val Gly Ala Gly Met Ser Leu Thr Asp
    450                 455                 460

Asp Ala Pro Ala Gly Gln Lys Ala Ala Val Gly Ala Glu Ile Ala Leu
465                 470                 475                 480

Gln Leu Thr Gly Gly Thr Val Glu Leu Ala Ser Ser Ile Ala Leu Ala
                485                 490                 495

Leu Ala Ala Ala Arg Gly Val Thr Ser Gly Leu Gln Val Ala Gly Ala
            500                 505                 510

Ser Ala Gly Ala Ala Ala Gly Ala Leu Ala Ala Ala Leu Ser Pro Met
        515                 520                 525

Glu Ile Tyr Gly Leu Val Gln Gln Ser His Tyr Ala Asp Gln Leu Asp
    530                 535                 540

Lys Leu Ala Gln Glu Ser Ser Ala Tyr Gly Tyr Glu Gly Asp Ala Leu
545                 550                 555                 560

Leu Ala Gln Leu Tyr Arg Asp Lys Thr Ala Ala Glu Gly Ala Val Ala
                565                 570                 575

Gly Val Ser Ala Val Leu Ser Thr Val Gly Ala Ala Val Ser Ile Ala
            580                 585                 590

Ala Ala Ala Ser Val Val Gly Ala Pro Val Ala Val Val Thr Ser Leu
        595                 600                 605

Leu Thr Gly Ala Leu Asn Gly Ile Leu Arg Gly Val Gln Gln Pro Ile
    610                 615                 620

Ile Glu Lys Leu Ala Asn Asp Tyr Ala Arg Lys Ile Asp Glu Leu Gly
625                 630                 635                 640

Gly Pro Gln Ala Tyr Phe Glu Lys Asn Leu Gln Ala Arg His Glu Gln
                645                 650                 655

Leu Ala Asn Ser Asp Gly Leu Arg Lys Met Leu Ala Asp Leu Gln Ala
            660                 665                 670

Gly Trp Asn Ala Ser Ser Val Ile Gly Val Gln Thr Thr Glu Ile Ser
        675                 680                 685

Lys Ser Ala Leu Glu Leu Ala Ala Ile Thr Gly Asn Ala Asp Asn Leu
    690                 695                 700

Lys Ser Ala Asp Val Phe Val Asp Arg Phe Ile Gln Gly Glu Arg Val
705                 710                 715                 720

Ala Gly Gln Pro Val Val Leu Asp Val Ala Ala Gly Gly Ile Asp Ile
                725                 730                 735

Ala Ser Arg Lys Gly Glu Arg Pro Ala Leu Thr Phe Ile Thr Pro Leu
            740                 745                 750

Ala Ala Pro Gly Glu Glu Gln Arg Arg Arg Thr Lys Thr Gly Lys Ser
        755                 760                 765

Glu Phe Thr Thr Phe Val Glu Ile Val Gly Lys Gln Asp Arg Trp Arg
    770                 775                 780

Ile Arg Asp Gly Ala Ala Asp Thr Thr Ile Asp Leu Ala Lys Val Val
785                 790                 795                 800

Ser Gln Leu Val Asp Ala Asn Gly Val Leu Lys His Ser Ile Lys Leu
                805                 810                 815

Glu Val Ile Gly Gly Asp Gly Asp Asp Val Val Leu Ala Asn Ala Ser
            820                 825                 830

Arg Ile His Tyr Asp Gly Gly Ala Gly Thr Asn Thr Val Ser Tyr Ala
        835                 840                 845

Ala Leu Gly Arg Gln Asp Ser Ile Thr Val Ser Ala Asp Gly Glu Arg
    850                 855                 860

Phe Asn Val Arg Lys Gln Leu Asn Asn Ala Asn Val Tyr Arg Glu Gly
865                 870                 875                 880

Val Ala Thr Gln Lys Thr Ala Tyr Gly Lys Arg Thr Glu Asn Val Gln
                885                 890                 895

Tyr Arg His Val Glu Leu Ala Arg Val Gly Gln Leu Val Glu Val Asp
            900                 905                 910

Thr Leu Glu His Val Gln His Ile Ile Gly Gly Ala Gly Asn Asp Ser
        915                 920                 925

Ile Thr Gly Asn Ala His Asp Asn Phe Leu Ala Gly Gly Ala Gly Asp
    930                 935                 940

Asp Arg Leu Asp Gly Gly Ala Gly Asn Asp Thr Leu Val Gly Gly Glu
945                 950                 955                 960

Gly His Asn Thr Val Val Gly Gly Ala Gly Asp Asp Val Phe Leu Gln
                965                 970                 975

Asp Leu Gly Val Trp Ser Asn Gln Leu Asp Gly Gly Ala Gly Val Asp
            980                 985                 990

Thr Val Lys Tyr Asn Val His Gln Pro Ser Glu Glu Arg Leu Glu Arg
        995                 1000                1005

Met Gly Asp Thr Gly Ile His Ala Asp Leu Gln Lys Gly Thr Val
    1010                1015                1020

Glu Lys Trp Pro Ala Leu Asn Leu Phe Ser Val Asp His Val Lys
    1025                1030                1035

Asn Ile Glu Asn Leu His Gly Ser Ser Leu Asn Asp Ser Ile Ala
    1040                1045                1050

Gly Asp Asp Arg Asp Asn Glu Leu Trp Gly Asp Asp Gly Asn Asp
    1055                1060                1065

Thr Ile His Gly Arg Gly Gly Asp Asp Ile Leu Arg Gly Gly Leu
    1070                1075                1080

Gly Leu Asp Thr Leu Tyr Gly Glu Asp Gly Asn Asp Ile Phe Leu
    1085                1090                1095

Gln Asp Asp Glu Thr Val Ser Asp Asp Ile Asp Gly Gly Ala Gly
    1100                1105                1110

Leu Asp Thr Val Asp Tyr Ser Ala Met Ile His Ala Gly Lys Ile
    1115                1120                1125

Val Ala Pro His Glu Tyr Gly Phe Gly Ile Glu Ala Asp Leu Ser
    1130                1135                1140

Glu Gly Trp Val Arg Lys Ala Ala Arg Arg Gly Met Gly Tyr Tyr
    1145                1150                1155

Asp Ser Val Arg Ser Val Glu Asn Val Ile Gly Thr Ser Met Lys
    1160                1165                1170

Asp Val Leu Ile Gly Asp Ala Gln Ala Asn Thr Leu Met Gly Gln
    1175                1180                1185

Gly Gly Asp Asp Thr Val Arg Gly Gly Asp Gly Asp Asp Leu Leu
    1190                1195                1200

Phe Gly Gly Asp Gly Asn Asp Met Leu Tyr Gly Asp Ala Gly Asn
    1205                1210                1215

Asp Thr Leu Tyr Gly Gly Leu Gly Asp Asp Thr Leu Glu Gly Gly
    1220                1225                1230

Ala Gly Asn Asp Trp Phe Gly Gln Thr Pro Ala Arg Glu His Asp
    1235                1240                1245

Val Leu Arg Gly Gly Ala Gly Val Asp Thr Val Asp Tyr Ser Gln
    1250                1255                1260

Ala Gly Ala His Ala Gly Val Ala Thr Gly Arg Ile Gly Leu Gly
    1265                1270                1275

Ile Leu Ala Asp Leu Gly Ala Gly Arg Val Asp Lys Leu Gly Glu
    1280                1285                1290

Ala Gly Ser Ser Ala Tyr Asp Thr Val Ser Gly Ile Glu Asn Val

1295                1300                1305

Val Gly  Thr Glu Leu Ala Asp  Arg Ile Thr Gly Asp  Ala Gln Ala
    1310                1315                1320

Asn Val  Leu Arg Gly Ala Gly  Gly Ala Asp Val Leu  Ala Gly Gly
    1325                1330                1335

Glu Gly  Asp Asp Val Leu Leu  Gly Gly Glu Gly Asp  Asp Gln Leu
    1340                1345                1350

Ser Gly  Asp Ala Gly Arg Asp  Arg Leu Tyr Gly Glu  Ala Gly Asp
    1355                1360                1365

Asp Trp  Phe Phe Gln Asp Ala  Ala Asn Ala Gly Asn  Leu Leu Asp
    1370                1375                1380

Gly Gly  Asp Gly Asn Asp Thr  Val Asp Phe Ser Gly  Pro Gly Arg
    1385                1390                1395

Gly Leu  Asp Ala Gly Ala Lys  Gly Val Phe Leu Ser  Leu Gly Lys
    1400                1405                1410

Gly Phe  Ala Ser Leu Met Asp  Glu Pro Glu Thr Ser  Asn Val Leu
    1415                1420                1425

Arg His  Ile Glu Asn Ala Val  Gly Ser Val Arg Asp  Asp Val Leu
    1430                1435                1440

Ile Gly  Asp Ala Gly Ala Asn  Val Leu Asn Gly Leu  Ala Gly Asn
    1445                1450                1455

Asp Val  Leu Ser Gly Gly Ala  Gly Asp Asp Val Leu  Leu Gly Asp
    1460                1465                1470

Glu Gly  Ser Asp Leu Leu Ser  Gly Asp Ala Gly Asn  Asp Asp Leu
    1475                1480                1485

Phe Gly  Gly Gln Gly Asp Asp  Thr Tyr Leu Phe Gly  Ala Gly Tyr
    1490                1495                1500

Gly His  Asp Thr Ile Tyr Glu  Ser Gly Gly Gly His  Asp Thr Ile
    1505                1510                1515

Arg Ile  Asn Ala Gly Ala Asp  Gln Leu Trp Phe Ala  Arg Gln Gly
    1520                1525                1530

Asn Asp Leu Glu Ile Arg Ile Leu Gly Thr Asp Asp Ala Leu Thr
    1535                1540                1545

Val His Asp Trp Tyr Arg Asp Ala Asp His Arg Val Glu Ala Ile
    1550                1555                1560

His Ala Ala Asn Gln Ala Ile Asp Pro Ala Gly Ile Glu Lys Leu
    1565                1570                1575

Val Glu Ala Met Ala Gln Tyr Pro Asp Pro Gly Ala Ala Ala Ala
    1580                1585                1590

Ala Pro Pro Ala Ala Arg Val Pro Asp Thr Leu Met Gln Ser Leu
    1595                1600                1605

Ala Val Asn Trp Arg
    1610

<210>  16
<211>  1503
<212>  PRT
<213>  Artificial Sequence

<220>
<223>  Polypeptide 1-183 + 387-1706 of SEQ ID NO:6

<400>  16

Met Gln Gln Ser His Gln Ala Gly Tyr Ala Asn Ala Ala Asp Arg Glu
1               5                   10                  15

Ser Gly Ile Pro Ala Ala Val Leu Asp Gly Ile Lys Ala Val Ala Lys
            20                  25                  30

Glu Lys Asn Ala Thr Leu Met Phe Arg Leu Val Asn Pro His Ser Thr
        35                  40                  45

Ser Leu Ile Ala Glu Gly Val Ala Thr Lys Gly Leu Gly Val His Ala
    50                  55                  60

Lys Ser Ser Asp Trp Gly Leu Gln Ala Gly Tyr Ile Pro Val Asn Pro
65                  70                  75                  80

Asn Leu Ser Lys Leu Phe Gly Arg Ala Pro Glu Val Ile Ala Arg Ala
                85                  90                  95

Asp Asn Asp Val Asn Ser Ser Leu Ala His Gly His Thr Ala Val Asp
            100                 105                 110

Leu Thr Leu Ser Lys Glu Arg Leu Asp Tyr Leu Arg Gln Ala Gly Leu

        115                 120                 125

Val Thr Gly Met Ala Asp Gly Val Val Ala Ser Asn His Ala Gly Tyr
    130                 135                 140

Glu Gln Phe Glu Phe Arg Val Lys Glu Thr Ser Asp Gly Arg Tyr Ala
145                 150                 155                 160

Val Gln Tyr Arg Arg Lys Gly Gly Asp Asp Phe Glu Ala Val Lys Val
                165                 170                 175

Ile Gly Asn Ala Ala Gly Ile Pro Gly Leu Arg Arg Pro Ser Leu Gly
            180                 185                 190

Ala Val Glu Arg Gln Asp Ser Gly Tyr Asp Ser Leu Asp Gly Val Gly
        195                 200                 205

Ser Arg Ser Phe Ser Leu Gly Glu Val Ser Asp Met Ala Ala Val Glu
    210                 215                 220

Ala Ala Glu Leu Glu Met Thr Arg Gln Val Leu His Ala Gly Ala Arg
225                 230                 235                 240

Gln Asp Asp Ala Glu Pro Gly Val Ser Gly Ala Ser Ala His Trp Gly
                245                 250                 255

Gln Arg Ala Leu Gln Gly Ala Gln Ala Val Ala Ala Ala Gln Arg Leu
            260                 265                 270

Val His Ala Ile Ala Leu Met Thr Gln Phe Gly Arg Ala Gly Ser Thr
        275                 280                 285

Asn Thr Pro Gln Glu Ala Ala Ser Leu Ser Ala Ala Val Phe Gly Leu
    290                 295                 300

Gly Glu Ala Ser Ser Ala Val Ala Glu Thr Val Ser Gly Phe Phe Arg
305                 310                 315                 320

Gly Ser Ser Arg Trp Ala Gly Gly Phe Gly Val Ala Gly Gly Ala Met
                325                 330                 335

Ala Leu Gly Gly Gly Ile Ala Ala Ala Val Gly Ala Gly Met Ser Leu
            340                 345                 350

Thr Asp Asp Ala Pro Ala Gly Gln Lys Ala Ala Val Gly Ala Glu Ile
        355                 360                 365

Ala Leu Gln Leu Thr Gly Gly Thr Val Glu Leu Ala Ser Ser Ile Ala
    370                 375                 380

Leu Ala Leu Ala Ala Ala Arg Gly Val Thr Ser Gly Leu Gln Val Ala
385                 390                 395                 400

Gly Ala Ser Ala Gly Ala Ala Ala Gly Ala Leu Ala Ala Ala Leu Ser
                405                 410                 415

Pro Met Glu Ile Tyr Gly Leu Val Gln Gln Ser His Tyr Ala Asp Gln
            420                 425                 430

Leu Asp Lys Leu Ala Gln Glu Ser Ser Ala Tyr Gly Tyr Glu Gly Asp
        435                 440                 445

Ala Leu Leu Ala Gln Leu Tyr Arg Asp Lys Thr Ala Ala Glu Gly Ala
    450                 455                 460

Val Ala Gly Val Ser Ala Val Leu Ser Thr Val Gly Ala Ala Val Ser
465                 470                 475                 480

Ile Ala Ala Ala Ala Ser Val Val Gly Ala Pro Val Ala Val Val Thr
                485                 490                 495

Ser Leu Leu Thr Gly Ala Leu Asn Gly Ile Leu Arg Gly Val Gln Gln
            500                 505                 510

Pro Ile Ile Glu Lys Leu Ala Asn Asp Tyr Ala Arg Lys Ile Asp Glu
        515                 520                 525

Leu Gly Gly Pro Gln Ala Tyr Phe Glu Lys Asn Leu Gln Ala Arg His
    530                 535                 540

Glu Gln Leu Ala Asn Ser Asp Gly Leu Arg Lys Met Leu Ala Asp Leu
545                 550                 555                 560

Gln Ala Gly Trp Asn Ala Ser Ser Val Ile Gly Val Gln Thr Thr Glu
                565                 570                 575

Ile Ser Lys Ser Ala Leu Glu Leu Ala Ala Ile Thr Gly Asn Ala Asp
            580                 585                 590

Asn Leu Lys Ser Ala Asp Val Phe Val Asp Arg Phe Ile Gln Gly Glu
        595                 600                 605

Arg Val Ala Gly Gln Pro Val Val Leu Asp Val Ala Ala Gly Gly Ile
    610                 615                 620

Asp Ile Ala Ser Arg Lys Gly Glu Arg Pro Ala Leu Thr Phe Ile Thr
625                 630                 635                 640

Pro Leu Ala Ala Pro Gly Glu Glu Gln Arg Arg Arg Thr Lys Thr Gly
                645                 650                 655

Lys Ser Glu Phe Thr Thr Phe Val Glu Ile Val Gly Lys Gln Asp Arg
            660                 665                 670

Trp Arg Ile Arg Asp Gly Ala Ala Asp Thr Thr Ile Asp Leu Ala Lys
        675                 680                 685

Val Val Ser Gln Leu Val Asp Ala Asn Gly Val Leu Lys His Ser Ile
    690                 695                 700

Lys Leu Glu Val Ile Gly Gly Asp Gly Asp Asp Val Val Leu Ala Asn
705                 710                 715                 720

Ala Ser Arg Ile His Tyr Asp Gly Gly Ala Gly Thr Asn Thr Val Ser
                725                 730                 735

Tyr Ala Ala Leu Gly Arg Gln Asp Ser Ile Thr Val Ser Ala Asp Gly
            740                 745                 750

Glu Arg Phe Asn Val Arg Lys Gln Leu Asn Asn Ala Asn Val Tyr Arg
        755                 760                 765

Glu Gly Val Ala Thr Gln Lys Thr Ala Tyr Gly Lys Arg Thr Glu Asn
    770                 775                 780

Val Gln Tyr Arg His Val Glu Leu Ala Arg Val Gly Gln Leu Val Glu
785                 790                 795                 800

Val Asp Thr Leu Glu His Val Gln His Ile Ile Gly Gly Ala Gly Asn
                805                 810                 815

Asp Ser Ile Thr Gly Asn Ala His Asp Asn Phe Leu Ala Gly Gly Ala
            820                 825                 830

Gly Asp Asp Arg Leu Asp Gly Gly Ala Gly Asn Asp Thr Leu Val Gly
        835                 840                 845

Gly Glu Gly His Asn Thr Val Val Gly Gly Ala Gly Asp Asp Val Phe
    850                 855                 860

Leu Gln Asp Leu Gly Val Trp Ser Asn Gln Leu Asp Gly Gly Ala Gly
865                 870                 875                 880

Val Asp Thr Val Lys Tyr Asn Val His Gln Pro Ser Glu Glu Arg Leu
                885                 890                 895

Glu Arg Met Gly Asp Thr Gly Ile His Ala Asp Leu Gln Lys Gly Thr
            900                 905                 910

Val Glu Lys Trp Pro Ala Leu Asn Leu Phe Ser Val Asp His Val Lys
        915                 920                 925

Asn Ile Glu Asn Leu His Gly Ser Ser Leu Asn Asp Ser Ile Ala Gly
    930                 935                 940

Asp Asp Arg Asp Asn Glu Leu Trp Gly Asp Asp Gly Asn Asp Thr Ile
945                 950                 955                 960

His Gly Arg Gly Gly Asp Asp Ile Leu Arg Gly Gly Leu Gly Leu Asp
                965                 970                 975

Thr Leu Tyr Gly Glu Asp Gly Asn Asp Ile Phe Leu Gln Asp Asp Glu
            980                 985                 990

Thr Val Ser Asp Asp Ile Asp Gly  Gly Ala Gly Leu Asp  Thr Val Asp
        995                 1000                1005

Tyr Ser  Ala Met Ile His Ala  Gly Lys Ile Val Ala  Pro His Glu
    1010                 1015                 1020

Tyr Gly  Phe Gly Ile Glu Ala  Asp Leu Ser Glu Gly  Trp Val Arg
    1025                 1030                 1035

Lys Ala  Ala Arg Arg Gly Met  Gly Tyr Tyr Asp Ser  Val Arg Ser
    1040                 1045                 1050

Val Glu  Asn Val Ile Gly Thr  Ser Met Lys Asp Val  Leu Ile Gly
    1055                 1060                 1065

Asp Ala  Gln Ala Asn Thr Leu  Met Gly Gln Gly Gly  Asp Asp Thr
    1070                 1075                 1080

Val Arg  Gly Gly Asp Gly Asp  Asp Leu Leu Phe Gly  Gly Asp Gly
    1085                 1090                 1095

Asn Asp  Met Leu Tyr Gly Asp  Ala Gly Asn Asp Thr  Leu Tyr Gly
    1100                 1105                 1110

Gly Leu  Gly Asp Asp Thr Leu  Glu Gly Gly Ala Gly  Asn Asp Trp

1115                1120                1125

Phe Gly Gln Thr Pro Ala Arg Glu His Asp Val Leu Arg Gly Gly
    1130                1135                1140

Ala Gly Val Asp Thr Val Asp Tyr Ser Gln Ala Gly Ala His Ala
    1145                1150                1155

Gly Val Ala Thr Gly Arg Ile Gly Leu Gly Ile Leu Ala Asp Leu
    1160                1165                1170

Gly Ala Gly Arg Val Asp Lys Leu Gly Glu Ala Gly Ser Ser Ala
    1175                1180                1185

Tyr Asp Thr Val Ser Gly Ile Glu Asn Val Val Gly Thr Glu Leu
    1190                1195                1200

Ala Asp Arg Ile Thr Gly Asp Ala Gln Ala Asn Val Leu Arg Gly
    1205                1210                1215

Ala Gly Gly Ala Asp Val Leu Ala Gly Gly Glu Gly Asp Asp Val
    1220                1225                1230

Leu Leu Gly Gly Glu Gly Asp Asp Gln Leu Ser Gly Asp Ala Gly
    1235                1240                1245

Arg Asp Arg Leu Tyr Gly Glu Ala Gly Asp Asp Trp Phe Phe Gln
    1250                1255                1260

Asp Ala Ala Asn Ala Gly Asn Leu Leu Asp Gly Gly Asp Gly Asn
    1265                1270                1275

Asp Thr Val Asp Phe Ser Gly Pro Gly Arg Gly Leu Asp Ala Gly
    1280                1285                1290

Ala Lys Gly Val Phe Leu Ser Leu Gly Lys Gly Phe Ala Ser Leu
    1295                1300                1305

Met Asp Glu Pro Glu Thr Ser Asn Val Leu Arg His Ile Glu Asn
    1310                1315                1320

Ala Val Gly Ser Val Arg Asp Asp Val Leu Ile Gly Asp Ala Gly
    1325                1330                1335

Ala Asn Val Leu Asn Gly Leu Ala Gly Asn Asp Val Leu Ser Gly
    1340                1345                1350

Gly Ala Gly Asp Asp Val Leu Leu Gly Asp Glu Gly Ser Asp Leu
    1355                1360                1365

Leu Ser Gly Asp Ala Gly Asn Asp Asp Leu Phe Gly Gly Gln Gly
    1370                1375                1380

Asp Asp Thr Tyr Leu Phe Gly Ala Gly Tyr Gly His Asp Thr Ile
    1385                1390                1395

Tyr Glu Ser Gly Gly Gly His Asp Thr Ile Arg Ile Asn Ala Gly
    1400                1405                1410

Ala Asp Gln Leu Trp Phe Ala Arg Gln Gly Asn Asp Leu Glu Ile
    1415                1420                1425

Arg Ile Leu Gly Thr Asp Asp Ala Leu Thr Val His Asp Trp Tyr
    1430                1435                1440

Arg Asp Ala Asp His Arg Val Glu Ala Ile His Ala Ala Asn Gln
    1445                1450                1455

Ala Ile Asp Pro Ala Gly Ile Glu Lys Leu Val Glu Ala Met Ala
    1460                1465                1470

Gln Tyr Pro Asp Pro Gly Ala Ala Ala Ala Ala Pro Pro Ala Ala
    1475                1480                1485

Arg Val Pro Asp Thr Leu Met Gln Ser Leu Ala Val Asn Trp Arg
    1490                1495                1500

<210> 17
<211> 1612
<212> PRT
<213> Artificial Sequence

<220>
<223> Polypeptide 1-227 + 321-1705 of SEQ ID NO:8

<400> 17

Met Gln Gln Ser His Gln Ala Gly Tyr Ala Asn Ala Ala Asp Arg Glu
1               5                   10                  15

Ser Gly Ile Pro Ala Ala Val Leu Asp Gly Ile Lys Ala Val Ala Lys
            20                  25                  30

Glu Lys Asn Ala Thr Leu Met Phe Arg Leu Val Asn Pro His Ser Thr
        35                  40                  45

Ser Leu Ile Ala Glu Gly Val Ala Thr Lys Gly Leu Gly Val His Ala

50                  55                  60

Lys Ser Ser Asp Trp Gly Leu Gln Ala Gly Tyr Ile Pro Val Asn Pro
65                  70                  75                  80

Asn Leu Ser Lys Leu Phe Gly Arg Ala Pro Glu Val Ile Ala Arg Ala
                85                  90                  95

Asp Asn Asp Val Asn Ser Ser Leu Ala His Gly His Thr Ala Val Asp
            100                 105                 110

Leu Thr Leu Ser Lys Glu Arg Leu Asp Tyr Leu Arg Gln Ala Gly Leu
        115                 120                 125

Val Thr Gly Met Ala Asp Gly Val Val Ala Ser Asn His Ala Gly Tyr
    130                 135                 140

Glu Gln Phe Glu Phe Arg Val Lys Glu Thr Ser Asp Gly Arg Tyr Ala
145                 150                 155                 160

Val Gln Tyr Arg Arg Lys Gly Gly Asp Asp Phe Glu Ala Val Lys Val
                165                 170                 175

Ile Gly Asn Ala Ala Gly Ile Pro Leu Thr Ala Asp Ile Asp Met Phe
            180                 185                 190

Ala Ile Met Pro His Leu Ser Asn Phe Arg Asp Ser Ala Arg Ser Ser
        195                 200                 205

Val Thr Ser Gly Asp Ser Val Thr Asp Tyr Leu Ala Arg Thr Arg Arg
    210                 215                 220

Ala Ala Ser Glu Ser Gln Met Leu Thr Arg Gly Gln Leu Lys Glu Tyr
225                 230                 235                 240

Ile Gly Gln Gln Arg Gly Glu Gly Tyr Val Phe Tyr Glu Asn Arg Ala
                245                 250                 255

Tyr Gly Val Ala Gly Lys Ser Leu Phe Asp Asp Gly Leu Gly Ala Ala
            260                 265                 270

Pro Gly Val Pro Gly Arg Arg Ser Lys Ser Ser Pro Asp Val Leu Glu
        275                 280                 285

Thr Val Pro Ala Ser Pro Gly Leu Arg Arg Pro Ser Leu Gly Ala Val
    290                 295                 300

Glu Arg Gln Asp Ser Gly Tyr Asp Ser Leu Asp Gly Val Gly Ser Arg
305                 310                 315                 320

Ser Phe Ser Leu Gly Glu Val Ser Asp Met Ala Ala Val Glu Ala Ala
                325                 330                 335

Glu Leu Glu Met Thr Arg Gln Val Leu His Ala Gly Ala Arg Gln Asp
            340                 345                 350

Asp Ala Glu Pro Gly Val Ser Gly Ala Ser Ala His Trp Gly Gln Arg
        355                 360                 365

Ala Leu Gln Gly Ala Gln Ala Val Ala Ala Ala Gln Arg Leu Val His
    370                 375                 380

Ala Ile Ala Leu Met Thr Gln Phe Gly Arg Ala Gly Ser Thr Asn Thr
385                 390                 395                 400

Pro Gln Glu Ala Ala Ser Leu Ser Ala Ala Val Phe Gly Leu Gly Glu
                405                 410                 415

Ala Ser Ser Ala Val Ala Glu Thr Val Ser Gly Phe Phe Arg Gly Ser
            420                 425                 430

Ser Arg Trp Ala Gly Gly Phe Gly Val Ala Gly Gly Ala Met Ala Leu
        435                 440                 445

Gly Gly Gly Ile Gly Ala Val Gly Ala Gly Met Ser Leu Thr Asp Asp
    450                 455                 460

Ala Pro Ala Gly Gln Lys Ala Ala Ala Gly Ala Glu Ile Ala Leu Gln
465                 470                 475                 480

Leu Thr Gly Gly Thr Val Glu Leu Ala Ser Ser Ile Ala Leu Ala Leu
                485                 490                 495

Ala Ala Ala Arg Gly Val Thr Ser Gly Leu Gln Val Ala Gly Ala Ser
            500                 505                 510

Ala Gly Ala Ala Ala Gly Ala Leu Ala Ala Ala Leu Ser Pro Met Glu
        515                 520                 525

Ile Tyr Gly Leu Val Gln Gln Ser His Tyr Ala Asp Gln Leu Asp Lys
    530                 535                 540

Leu Ala Gln Glu Ser Ser Ala Tyr Gly Tyr Glu Gly Asp Ala Leu Leu
545                 550                 555                 560

Ala Gln Leu Tyr Arg Asp Lys Thr Ala Ala Glu Gly Ala Val Ala Gly
                565                 570                 575

Val Ser Ala Val Leu Ser Thr Val Gly Ala Ala Val Ser Ile Ala Ala
            580                 585                 590

Ala Ala Ser Val Val Gly Ala Pro Val Ala Val Val Thr Ser Leu Leu
        595                 600                 605

Thr Gly Ala Leu Asn Gly Ile Leu Arg Gly Val Gln Gln Pro Ile Ile
    610                 615                 620

Glu Lys Leu Ala Asn Asp Tyr Ala Arg Lys Ile Asp Glu Leu Gly Gly
625                 630                 635                 640

Pro Gln Ala Tyr Phe Glu Lys Asn Leu Gln Ala Arg His Glu Gln Leu
                645                 650                 655

Ala Asn Ser Asp Gly Leu Arg Lys Met Leu Ala Asp Leu Gln Ala Gly
            660                 665                 670

Trp Asn Ala Ser Ser Val Ile Gly Val Gln Thr Thr Glu Ile Ser Lys
        675                 680                 685

Ser Ala Leu Glu Leu Ala Ala Ile Thr Gly Asn Ala Asp Asn Leu Lys
    690                 695                 700

Ser Ala Asp Val Phe Val Asp Arg Phe Ile Gln Gly Glu Arg Val Ala
705                 710                 715                 720

Gly Gln Pro Val Val Leu Asp Val Ala Ala Gly Gly Ile Asp Ile Ala
                725                 730                 735

Ser Arg Lys Gly Glu Arg Pro Ala Leu Thr Phe Ile Thr Pro Leu Ala
            740                 745                 750

Ala Pro Gly Glu Glu Gln Arg Arg Arg Thr Lys Thr Gly Lys Ser Glu
        755                 760                 765

Phe Thr Thr Phe Val Glu Ile Val Gly Lys Gln Asp Arg Trp Arg Ile
    770                 775                 780

Arg Asp Gly Ala Ala Asp Thr Thr Ile Asp Leu Ala Lys Val Val Ser
785                 790                 795                 800

Gln Leu Val Asp Ala Asn Gly Val Leu Lys His Ser Ile Lys Leu Glu
                805                 810                 815

Val Ile Gly Gly Asp Gly Asp Asp Val Val Leu Ala Asn Ala Ser Arg
            820                 825                 830

Ile His Tyr Asp Gly Gly Ala Gly Thr Asn Thr Val Ser Tyr Ala Ala
        835                 840                 845

Leu Gly Arg Gln Asp Ser Ile Thr Val Ser Ala Asp Gly Glu Arg Phe
    850                 855                 860

Asn Val Arg Lys Gln Leu Asn Asn Ala Asn Val Tyr Arg Glu Gly Val
865                 870                 875                 880

Ala Thr Gln Lys Thr Ala Tyr Gly Lys Arg Thr Glu Asn Val Gln Tyr
                885                 890                 895

Arg His Val Glu Leu Ala Arg Val Gly Gln Leu Val Glu Val Asp Thr
            900                 905                 910

Leu Glu His Val Gln His Ile Ile Gly Gly Ala Gly Asn Asp Ser Ile
        915                 920                 925

Thr Gly Asn Ala His Asp Asn Phe Leu Ala Gly Gly Ala Gly Asp Asp
    930                 935                 940

Arg Leu Asp Gly Gly Ala Gly Asn Asp Thr Leu Val Gly Gly Glu Gly
945                 950                 955                 960

His Asn Thr Val Val Gly Gly Ala Gly Asp Asp Val Phe Leu Gln Asp
                965                 970                 975

Leu Gly Val Trp Ser Asn Gln Leu Asp Gly Gly Ala Gly Val Asp Thr
            980                 985                 990

Val Lys Tyr Asn Val His Gln Pro  Ser Glu Glu Arg Leu  Glu Arg Met
        995                 1000                1005

Gly Asp  Thr Gly Ile His Ala  Asp Leu Gln Lys Gly  Thr Val Glu
    1010                1015                1020

Lys Trp  Pro Ala Leu Asn Leu  Phe Ser Val Asp His  Val Lys Asn
    1025                1030                1035

Ile Glu  Asn Leu His Gly Ser  Ser Leu Asn Asp Ser  Ile Ala Gly
    1040                1045                1050

Asp Asp  Arg Asp Asn Glu Leu  Trp Gly Asp Asp Gly  Asn Asp Thr

1055                 1060                 1065

Ile His Gly Arg Gly Gly Asp Asp Ile Leu Arg Gly Gly Leu Gly
    1070                 1075                 1080

Leu Asp Thr Leu Tyr Gly Glu Asp Gly Asn Asp Ile Phe Leu Gln
    1085                 1090                 1095

Asp Asp Glu Thr Val Ser Asp Asp Ile Asp Gly Gly Ala Gly Leu
    1100                 1105                 1110

Asp Thr Val Asp Tyr Ser Ala Met Ile His Ala Gly Lys Ile Val
    1115                 1120                 1125

Ala Pro His Glu Tyr Gly Phe Gly Ile Glu Ala Asp Leu Ser Glu
    1130                 1135                 1140

Gly Trp Val Arg Lys Ala Ala Arg Arg Gly Met Asp Tyr Tyr Asp
    1145                 1150                 1155

Ser Val Arg Ser Val Glu Asn Val Ile Gly Thr Ser Met Lys Asp
    1160                 1165                 1170

Val Leu Ile Gly Asp Ala Gln Ala Asn Thr Leu Met Gly Gln Gly
    1175                 1180                 1185

Gly Asp Asp Thr Val Arg Gly Gly Asp Gly Asp Asp Leu Leu Phe
    1190                 1195                 1200

Gly Gly Asp Gly Asn Asp Met Leu Tyr Gly Asp Ala Gly Asn Asp
    1205                 1210                 1215

Thr Leu Tyr Gly Gly Leu Gly Asp Asp Thr Leu Glu Gly Gly Ala
    1220                 1225                 1230

Gly Asn Asp Trp Phe Gly Gln Thr Pro Ala Arg Glu His Asp Val
    1235                 1240                 1245

Leu Arg Gly Gly Ala Gly Val Asp Thr Val Asp Tyr Ser Gln Ala
    1250                 1255                 1260

Gly Ala His Ala Gly Val Ala Thr Gly Arg Ile Gly Leu Gly Ile
    1265                 1270                 1275

Leu Ala Asp Leu Gly Ala Gly Arg Val Asp Lys Leu Gly Glu Ala
    1280                 1285                 1290

Gly Ser Ser Ala Tyr Asp Thr Val Ser Gly Ile Glu Asn Val Val
    1295                1300                1305

Gly Thr Glu Leu Ala Asp Arg Ile Thr Gly Asp Ala Gln Ala Asn
    1310                1315                1320

Val Leu Arg Gly Ala Gly Gly Ala Asp Val Leu Ala Gly Gly Glu
    1325                1330                1335

Gly Asp Asp Val Leu Leu Gly Gly Asp Gly Asp Asp Gln Leu Ser
    1340                1345                1350

Gly Asp Ala Gly Arg Asp Arg Leu Tyr Gly Glu Ala Gly Asp Asp
    1355                1360                1365

Trp Phe Phe Gln Asp Ala Ala Asn Ala Gly Asn Leu Leu Asp Gly
    1370                1375                1380

Gly Asp Gly Asn Asp Thr Val Asp Phe Ser Gly Pro Gly Arg Gly
    1385                1390                1395

Leu Asp Ala Gly Ala Lys Gly Val Phe Leu Ser Leu Gly Lys Gly
    1400                1405                1410

Phe Ala Ser Leu Met Asp Glu Pro Glu Thr Ser Asn Val Leu Arg
    1415                1420                1425

His Ile Glu Asn Ala Val Gly Ser Val Arg Asp Asp Val Leu Ile
    1430                1435                1440

Gly Asp Ala Gly Ala Asn Val Leu Asn Gly Leu Ala Gly Asn Asp
    1445                1450                1455

Val Leu Ser Gly Gly Ala Gly Asp Asp Val Leu Leu Gly Asp Glu
    1460                1465                1470

Gly Ser Asp Leu Leu Ser Gly Asp Ala Gly Asn Asp Asp Leu Phe
    1475                1480                1485

Gly Gly Gln Gly Asp Asp Thr Tyr Leu Phe Gly Ala Gly Tyr Gly
    1490                1495                1500

His Asp Thr Ile Tyr Glu Ser Gly Gly Gly His Asp Thr Ile Arg
    1505                1510                1515

Ile Asn Ala Gly Ala Asp Gln Leu Trp Phe Ala Arg Gln Gly Asn
    1520                1525                1530

Asp Leu Glu Ile Arg Ile Leu Gly Thr Asp Asp Ala Leu Thr Val
    1535                1540                1545

His Asp Trp Tyr Arg Asp Ala Asp His Arg Val Glu Ala Ile His
    1550                1555                1560

Ala Ala Asn Gln Ala Ile Asp Pro Ala Gly Ile Glu Lys Leu Val
    1565                1570                1575

Glu Ala Met Ala Gln Tyr Pro Asp Pro Gly Ala Ala Ala Ala Ala
    1580                1585                1590

Pro Pro Ala Ala Arg Val Pro Asp Thr Leu Met Gln Ser Leu Ala
    1595                1600                1605

Val Asn Trp Arg
    1610

<210> 18
<211> 1502
<212> PRT
<213> Artificial Sequence

<220>
<223> Polypeptide 1-183 + 387-1705 of SEQ ID NO:8

<400> 18

Met Gln Gln Ser His Gln Ala Gly Tyr Ala Asn Ala Ala Asp Arg Glu
1               5                   10                  15

Ser Gly Ile Pro Ala Ala Val Leu Asp Gly Ile Lys Ala Val Ala Lys
            20                  25                  30

Glu Lys Asn Ala Thr Leu Met Phe Arg Leu Val Asn Pro His Ser Thr
        35                  40                  45

Ser Leu Ile Ala Glu Gly Val Ala Thr Lys Gly Leu Gly Val His Ala
    50                  55                  60

Lys Ser Ser Asp Trp Gly Leu Gln Ala Gly Tyr Ile Pro Val Asn Pro
65                  70                  75                  80

Asn Leu Ser Lys Leu Phe Gly Arg Ala Pro Glu Val Ile Ala Arg Ala
                85                  90                  95

Asp Asn Asp Val Asn Ser Ser Leu Ala His Gly His Thr Ala Val Asp
            100                 105                 110

Leu Thr Leu Ser Lys Glu Arg Leu Asp Tyr Leu Arg Gln Ala Gly Leu
        115                 120                 125

Val Thr Gly Met Ala Asp Gly Val Val Ala Ser Asn His Ala Gly Tyr
    130                 135                 140

Glu Gln Phe Glu Phe Arg Val Lys Glu Thr Ser Asp Gly Arg Tyr Ala
145                 150                 155                 160

Val Gln Tyr Arg Arg Lys Gly Gly Asp Asp Phe Glu Ala Val Lys Val
                165                 170                 175

Ile Gly Asn Ala Ala Gly Ile Pro Gly Leu Arg Arg Pro Ser Leu Gly
            180                 185                 190

Ala Val Glu Arg Gln Asp Ser Gly Tyr Asp Ser Leu Asp Gly Val Gly
        195                 200                 205

Ser Arg Ser Phe Ser Leu Gly Glu Val Ser Asp Met Ala Ala Val Glu
    210                 215                 220

Ala Ala Glu Leu Glu Met Thr Arg Gln Val Leu His Ala Gly Ala Arg
225                 230                 235                 240

Gln Asp Asp Ala Glu Pro Gly Val Ser Gly Ala Ser Ala His Trp Gly
                245                 250                 255

Gln Arg Ala Leu Gln Gly Ala Gln Ala Val Ala Ala Ala Gln Arg Leu
            260                 265                 270

Val His Ala Ile Ala Leu Met Thr Gln Phe Gly Arg Ala Gly Ser Thr
        275                 280                 285

Asn Thr Pro Gln Glu Ala Ala Ser Leu Ser Ala Ala Val Phe Gly Leu
    290                 295                 300

Gly Glu Ala Ser Ser Ala Val Ala Glu Thr Val Ser Gly Phe Phe Arg
305                 310                 315                 320

Gly Ser Ser Arg Trp Ala Gly Gly Phe Gly Val Ala Gly Gly Ala Met
                325                 330                 335

Ala Leu Gly Gly Gly Ile Gly Ala Val Gly Ala Gly Met Ser Leu Thr
            340                 345                 350

Asp Asp Ala Pro Ala Gly Gln Lys Ala Ala Ala Gly Ala Glu Ile Ala
        355                 360                 365

Leu Gln Leu Thr Gly Gly Thr Val Glu Leu Ala Ser Ser Ile Ala Leu
    370                 375                 380

Ala Leu Ala Ala Ala Arg Gly Val Thr Ser Gly Leu Gln Val Ala Gly
385                 390                 395                 400

Ala Ser Ala Gly Ala Ala Ala Gly Ala Leu Ala Ala Ala Leu Ser Pro
                405                 410                 415

Met Glu Ile Tyr Gly Leu Val Gln Gln Ser His Tyr Ala Asp Gln Leu
            420                 425                 430

Asp Lys Leu Ala Gln Glu Ser Ser Ala Tyr Gly Tyr Glu Gly Asp Ala
        435                 440                 445

Leu Leu Ala Gln Leu Tyr Arg Asp Lys Thr Ala Ala Glu Gly Ala Val
    450                 455                 460

Ala Gly Val Ser Ala Val Leu Ser Thr Val Gly Ala Ala Val Ser Ile
465                 470                 475                 480

Ala Ala Ala Ala Ser Val Val Gly Ala Pro Val Ala Val Val Thr Ser
                485                 490                 495

Leu Leu Thr Gly Ala Leu Asn Gly Ile Leu Arg Gly Val Gln Gln Pro
            500                 505                 510

Ile Ile Glu Lys Leu Ala Asn Asp Tyr Ala Arg Lys Ile Asp Glu Leu
        515                 520                 525

Gly Gly Pro Gln Ala Tyr Phe Glu Lys Asn Leu Gln Ala Arg His Glu
    530                 535                 540

Gln Leu Ala Asn Ser Asp Gly Leu Arg Lys Met Leu Ala Asp Leu Gln
545                 550                 555                 560

Ala Gly Trp Asn Ala Ser Ser Val Ile Gly Val Gln Thr Thr Glu Ile
                565                 570                 575

Ser Lys Ser Ala Leu Glu Leu Ala Ala Ile Thr Gly Asn Ala Asp Asn
            580                 585                 590

Leu Lys Ser Ala Asp Val Phe Val Asp Arg Phe Ile Gln Gly Glu Arg
        595                 600                 605

Val Ala Gly Gln Pro Val Val Leu Asp Val Ala Ala Gly Gly Ile Asp
    610                 615                 620

Ile Ala Ser Arg Lys Gly Glu Arg Pro Ala Leu Thr Phe Ile Thr Pro
625                 630                 635                 640

Leu Ala Ala Pro Gly Glu Glu Gln Arg Arg Arg Thr Lys Thr Gly Lys
                645                 650                 655

Ser Glu Phe Thr Thr Phe Val Glu Ile Val Gly Lys Gln Asp Arg Trp
            660                 665                 670

Arg Ile Arg Asp Gly Ala Ala Asp Thr Thr Ile Asp Leu Ala Lys Val
        675                 680                 685

Val Ser Gln Leu Val Asp Ala Asn Gly Val Leu Lys His Ser Ile Lys
    690                 695                 700

Leu Glu Val Ile Gly Gly Asp Gly Asp Asp Val Val Leu Ala Asn Ala
705                 710                 715                 720

Ser Arg Ile His Tyr Asp Gly Gly Ala Gly Thr Asn Thr Val Ser Tyr
                725                 730                 735

Ala Ala Leu Gly Arg Gln Asp Ser Ile Thr Val Ser Ala Asp Gly Glu
            740                 745                 750

Arg Phe Asn Val Arg Lys Gln Leu Asn Asn Ala Asn Val Tyr Arg Glu
        755                 760                 765

Gly Val Ala Thr Gln Lys Thr Ala Tyr Gly Lys Arg Thr Glu Asn Val
    770                 775                 780

Gln Tyr Arg His Val Glu Leu Ala Arg Val Gly Gln Leu Val Glu Val
785                 790                 795                 800

Asp Thr Leu Glu His Val Gln His Ile Ile Gly Gly Ala Gly Asn Asp
                805                 810                 815

Ser Ile Thr Gly Asn Ala His Asp Asn Phe Leu Ala Gly Gly Ala Gly
            820                 825                 830

Asp Asp Arg Leu Asp Gly Gly Ala Gly Asn Asp Thr Leu Val Gly Gly
        835                 840                 845

Glu Gly His Asn Thr Val Val Gly Gly Ala Gly Asp Asp Val Phe Leu
    850                 855                 860

Gln Asp Leu Gly Val Trp Ser Asn Gln Leu Asp Gly Gly Ala Gly Val

865                 870                 875                 880

Asp Thr Val Lys Tyr Asn Val His Gln Pro Ser Glu Glu Arg Leu Glu
                885                 890                 895

Arg Met Gly Asp Thr Gly Ile His Ala Asp Leu Gln Lys Gly Thr Val
            900                 905                 910

Glu Lys Trp Pro Ala Leu Asn Leu Phe Ser Val Asp His Val Lys Asn
        915                 920                 925

Ile Glu Asn Leu His Gly Ser Ser Leu Asn Asp Ser Ile Ala Gly Asp
    930                 935                 940

Asp Arg Asp Asn Glu Leu Trp Gly Asp Asp Gly Asn Asp Thr Ile His
945                 950                 955                 960

Gly Arg Gly Gly Asp Asp Ile Leu Arg Gly Gly Leu Gly Leu Asp Thr
                965                 970                 975

Leu Tyr Gly Glu Asp Gly Asn Asp Ile Phe Leu Gln Asp Asp Glu Thr
            980                 985                 990

Val Ser Asp Asp Ile Asp Gly Gly  Ala Gly Leu Asp Thr  Val Asp Tyr
        995                 1000                1005

Ser Ala  Met Ile His Ala Gly  Lys Ile Val Ala Pro  His Glu Tyr
    1010                1015                1020

Gly Phe  Gly Ile Glu Ala Asp  Leu Ser Glu Gly Trp  Val Arg Lys
    1025                1030                1035

Ala Ala  Arg Arg Gly Met Asp  Tyr Tyr Asp Ser Val  Arg Ser Val
    1040                1045                1050

Glu Asn  Val Ile Gly Thr Ser  Met Lys Asp Val Leu  Ile Gly Asp
    1055                1060                1065

Ala Gln  Ala Asn Thr Leu Met  Gly Gln Gly Gly Asp  Asp Thr Val
    1070                1075                1080

Arg Gly  Gly Asp Gly Asp Asp  Leu Leu Phe Gly Gly  Asp Gly Asn
    1085                1090                1095

Asp Met  Leu Tyr Gly Asp Ala  Gly Asn Asp Thr Leu  Tyr Gly Gly
    1100                1105                1110

Leu Gly Asp Asp Thr Leu Glu Gly Gly Ala Gly Asn Asp Trp Phe
    1115                1120                1125

Gly Gln Thr Pro Ala Arg Glu His Asp Val Leu Arg Gly Gly Ala
    1130                1135                1140

Gly Val Asp Thr Val Asp Tyr Ser Gln Ala Gly Ala His Ala Gly
    1145                1150                1155

Val Ala Thr Gly Arg Ile Gly Leu Gly Ile Leu Ala Asp Leu Gly
    1160                1165                1170

Ala Gly Arg Val Asp Lys Leu Gly Glu Ala Gly Ser Ser Ala Tyr
    1175                1180                1185

Asp Thr Val Ser Gly Ile Glu Asn Val Val Gly Thr Glu Leu Ala
    1190                1195                1200

Asp Arg Ile Thr Gly Asp Ala Gln Ala Asn Val Leu Arg Gly Ala
    1205                1210                1215

Gly Gly Ala Asp Val Leu Ala Gly Gly Glu Gly Asp Asp Val Leu
    1220                1225                1230

Leu Gly Gly Asp Gly Asp Asp Gln Leu Ser Gly Asp Ala Gly Arg
    1235                1240                1245

Asp Arg Leu Tyr Gly Glu Ala Gly Asp Asp Trp Phe Phe Gln Asp
    1250                1255                1260

Ala Ala Asn Ala Gly Asn Leu Leu Asp Gly Gly Asp Gly Asn Asp
    1265                1270                1275

Thr Val Asp Phe Ser Gly Pro Gly Arg Gly Leu Asp Ala Gly Ala
    1280                1285                1290

Lys Gly Val Phe Leu Ser Leu Gly Lys Gly Phe Ala Ser Leu Met
    1295                1300                1305

Asp Glu Pro Glu Thr Ser Asn Val Leu Arg His Ile Glu Asn Ala
    1310                1315                1320

Val Gly Ser Val Arg Asp Asp Val Leu Ile Gly Asp Ala Gly Ala
    1325                1330                1335

Asn Val Leu Asn Gly Leu Ala Gly Asn Asp Val Leu Ser Gly Gly
    1340                1345                1350

Ala Gly Asp Asp Val Leu Leu Gly Asp Glu Gly Ser Asp Leu Leu
    1355                1360                1365

Ser Gly Asp Ala Gly Asn Asp Asp Leu Phe Gly Gly Gln Gly Asp
    1370                1375                1380

Asp Thr Tyr Leu Phe Gly Ala Gly Tyr Gly His Asp Thr Ile Tyr
    1385                1390                1395

Glu Ser Gly Gly Gly His Asp Thr Ile Arg Ile Asn Ala Gly Ala
    1400                1405                1410

Asp Gln Leu Trp Phe Ala Arg Gln Gly Asn Asp Leu Glu Ile Arg
    1415                1420                1425

Ile Leu Gly Thr Asp Asp Ala Leu Thr Val His Asp Trp Tyr Arg
    1430                1435                1440

Asp Ala Asp His Arg Val Glu Ala Ile His Ala Ala Asn Gln Ala
    1445                1450                1455

Ile Asp Pro Ala Gly Ile Glu Lys Leu Val Glu Ala Met Ala Gln
    1460                1465                1470

Tyr Pro Asp Pro Gly Ala Ala Ala Ala Ala Pro Pro Ala Ala Arg
    1475                1480                1485

Val Pro Asp Thr Leu Met Gln Ser Leu Ala Val Asn Trp Arg
    1490                1495                1500

<210> 19
<211> 1547
<212> PRT
<213> Artificial Sequence

<220>
<223> Polypeptide 1-227 + 387-1706 of SEQ ID NO:2

<400> 19

Met Gln Gln Ser His Gln Ala Gly Tyr Ala Asn Ala Ala Asp Arg Glu
1               5                   10                  15

Ser Gly Ile Pro Ala Ala Val Leu Asp Gly Ile Lys Ala Val Ala Lys
            20                  25                  30

Glu Lys Asn Ala Thr Leu Met Phe Arg Leu Val Asn Pro His Ser Thr
        35                  40                  45

Ser Leu Ile Ala Glu Gly Val Ala Thr Lys Gly Leu Gly Val His Ala
    50                  55                  60

Lys Ser Ser Asp Trp Gly Leu Gln Ala Gly Tyr Ile Pro Val Asn Pro
65                  70                  75                  80

Asn Leu Ser Lys Leu Phe Gly Arg Ala Pro Glu Val Ile Ala Arg Ala
                85                  90                  95

Asp Asn Asp Val Asn Ser Ser Leu Ala His Gly His Thr Ala Val Asp
            100                 105                 110

Leu Thr Leu Ser Lys Glu Arg Leu Asp Tyr Leu Arg Gln Ala Gly Leu
        115                 120                 125

Val Thr Gly Met Ala Asp Gly Val Val Ala Ser Asn His Ala Gly Tyr
    130                 135                 140

Glu Gln Phe Glu Phe Arg Val Lys Glu Thr Ser Asp Gly Arg Tyr Ala
145                 150                 155                 160

Val Gln Tyr Arg Arg Lys Gly Gly Asp Asp Phe Glu Ala Val Lys Val
                165                 170                 175

Ile Gly Asn Ala Ala Gly Ile Pro Leu Thr Ala Asp Ile Asp Met Phe
            180                 185                 190

Ala Ile Met Pro His Leu Ser Asn Phe Arg Asp Ser Ala Arg Ser Ser
        195                 200                 205

Val Thr Ser Gly Asp Ser Val Thr Asp Tyr Leu Ala Arg Thr Arg Arg
    210                 215                 220

Ala Ala Ser Pro Gly Leu Arg Arg Pro Ser Leu Gly Ala Val Glu Arg
225                 230                 235                 240

Gln Asp Ser Gly Tyr Asp Ser Leu Asp Gly Val Gly Ser Arg Ser Phe
                245                 250                 255

Ser Leu Gly Glu Val Ser Asp Met Ala Ala Val Glu Ala Ala Glu Leu
            260                 265                 270

Glu Met Thr Arg Gln Val Leu His Ala Gly Ala Arg Gln Asp Asp Ala
        275                 280                 285

Glu Pro Gly Val Ser Gly Ala Ser Ala His Trp Gly Gln Arg Ala Leu
    290                 295                 300

Gln Gly Ala Gln Ala Val Ala Ala Ala Gln Arg Leu Val His Ala Ile
305                 310                 315                 320

Ala Leu Met Thr Gln Phe Gly Arg Ala Gly Ser Thr Asn Thr Pro Gln
                325                 330                 335

Glu Ala Ala Ser Leu Ser Ala Ala Val Phe Gly Leu Gly Glu Ala Ser
            340                 345                 350

Ser Ala Val Ala Glu Thr Val Ser Gly Phe Phe Arg Gly Ser Ser Arg
        355                 360                 365

Trp Ala Gly Gly Phe Gly Val Ala Gly Gly Ala Met Ala Leu Gly Gly
    370                 375                 380

Gly Ile Ala Ala Ala Val Gly Ala Gly Met Ser Leu Thr Asp Asp Ala
385                 390                 395                 400

Pro Ala Gly Gln Lys Ala Ala Ala Gly Ala Glu Ile Ala Leu Gln Leu
                405                 410                 415

Thr Gly Gly Thr Val Glu Leu Ala Ser Ser Ile Ala Leu Ala Leu Ala
            420                 425                 430

Ala Ala Arg Gly Val Thr Ser Gly Leu Gln Val Ala Gly Ala Ser Ala
        435                 440                 445

Gly Ala Ala Ala Gly Ala Leu Ala Ala Ala Leu Ser Pro Met Glu Ile
    450                 455                 460

Tyr Gly Leu Val Gln Gln Ser His Tyr Ala Asp Gln Leu Asp Lys Leu
465                 470                 475                 480

Ala Gln Glu Ser Ser Ala Tyr Gly Tyr Glu Gly Asp Ala Leu Leu Ala
                485                 490                 495

Gln Leu Tyr Arg Asp Lys Thr Ala Ala Glu Gly Ala Val Ala Gly Val
            500                 505                 510

Ser Ala Val Leu Ser Thr Val Gly Ala Ala Val Ser Ile Ala Ala Ala
        515                 520                 525

Ala Ser Val Val Gly Ala Pro Val Ala Val Val Thr Ser Leu Leu Thr
    530                 535                 540

Gly Ala Leu Asn Gly Ile Leu Arg Gly Val Gln Gln Pro Ile Ile Glu
545                 550                 555                 560

Lys Leu Ala Asn Asp Tyr Ala Arg Lys Ile Asp Glu Leu Gly Gly Pro
                565                 570                 575

Gln Ala Tyr Phe Glu Lys Asn Leu Gln Ala Arg His Glu Gln Leu Ala
            580                 585                 590

Asn Ser Asp Gly Leu Arg Lys Met Leu Ala Asp Leu Gln Ala Gly Trp
        595                 600                 605

Asn Ala Ser Ser Val Ile Gly Val Gln Thr Thr Glu Ile Ser Lys Ser
    610                 615                 620

Ala Leu Glu Leu Ala Ala Ile Thr Gly Asn Ala Asp Asn Leu Lys Ser
625                 630                 635                 640

Val Asp Val Phe Val Asp Arg Phe Val Gln Gly Glu Arg Val Ala Gly
                645                 650                 655

Gln Pro Val Val Leu Asp Val Ala Ala Gly Gly Ile Asp Ile Ala Ser
            660                 665                 670

Arg Lys Gly Glu Arg Pro Ala Leu Thr Phe Ile Thr Pro Leu Ala Ala
        675                 680                 685

Pro Gly Glu Glu Gln Arg Arg Arg Thr Lys Thr Gly Lys Ser Glu Phe
    690                 695                 700

Thr Thr Phe Val Glu Ile Val Gly Lys Gln Asp Arg Trp Arg Ile Arg
705                 710                 715                 720

Asp Gly Ala Ala Asp Thr Thr Ile Asp Leu Ala Lys Val Val Ser Gln
                725                 730                 735

Leu Val Asp Ala Asn Gly Val Leu Lys His Ser Ile Lys Leu Asp Val
            740                 745                 750

Ile Gly Gly Asp Gly Asp Asp Val Val Leu Ala Asn Ala Ser Arg Ile
        755                 760                 765

His Tyr Asp Gly Gly Ala Gly Thr Asn Thr Val Ser Tyr Ala Ala Leu
    770                 775                 780

Gly Arg Gln Asp Ser Ile Thr Val Ser Ala Asp Gly Glu Arg Phe Asn
785                 790                 795                 800

Val Arg Lys Gln Leu Asn Asn Ala Asn Val Tyr Arg Glu Gly Val Ala

805                 810                 815

Thr Gln Thr Thr Ala Tyr Gly Lys Arg Thr Glu Asn Val Gln Tyr Arg
            820                 825                 830

His Val Glu Leu Ala Arg Val Gly Gln Leu Val Glu Val Asp Thr Leu
        835                 840                 845

Glu His Val Gln His Ile Ile Gly Gly Ala Gly Asn Asp Ser Ile Thr
    850                 855                 860

Gly Asn Ala His Asp Asn Phe Leu Ala Gly Gly Ser Gly Asp Asp Arg
865                 870                 875                 880

Leu Asp Gly Gly Ala Gly Asn Asp Thr Leu Val Gly Gly Glu Gly Gln
                885                 890                 895

Asn Thr Val Ile Gly Gly Ala Gly Asp Asp Val Phe Leu Gln Asp Leu
            900                 905                 910

Gly Val Trp Ser Asn Gln Leu Asp Gly Gly Ala Gly Val Asp Thr Val
        915                 920                 925

Lys Tyr Asn Val His Gln Pro Ser Glu Glu Arg Leu Glu Arg Met Gly
    930                 935                 940

Asp Thr Gly Ile His Ala Asp Leu Gln Lys Gly Thr Val Glu Lys Trp
945                 950                 955                 960

Pro Ala Leu Asn Leu Phe Ser Val Asp His Val Lys Asn Ile Glu Asn
                965                 970                 975

Leu His Gly Ser Arg Leu Asn Asp Arg Ile Ala Gly Asp Asp Gln Asp
            980                 985                 990

Asn Glu Leu Trp Gly His Asp Gly  Asn Asp Thr Ile Arg  Gly Arg Gly
        995                 1000                 1005

Gly Asp  Asp Ile Leu Arg Gly  Gly Leu Gly Leu Asp  Thr Leu Tyr
    1010                 1015                 1020

Gly Glu  Asp Gly Asn Asp Ile  Phe Leu Gln Asp Asp  Glu Thr Val
    1025                 1030                 1035

Ser Asp  Asp Ile Asp Gly Gly  Ala Gly Leu Asp Thr  Val Asp Tyr
    1040                 1045                 1050

Ser Ala Met Ile His Pro Gly Arg Ile Val Ala Pro His Glu Tyr
    1055                1060                1065

Gly Phe Gly Ile Glu Ala Asp Leu Ser Arg Glu Trp Val Arg Lys
    1070                1075                1080

Ala Ser Ala Leu Gly Val Asp Tyr Tyr Asp Asn Val Arg Asn Val
    1085                1090                1095

Glu Asn Val Ile Gly Thr Ser Met Lys Asp Val Leu Ile Gly Asp
    1100                1105                1110

Ala Gln Ala Asn Thr Leu Met Gly Gln Gly Gly Asp Asp Thr Val
    1115                1120                1125

Arg Gly Gly Asp Gly Asp Asp Leu Leu Phe Gly Gly Asp Gly Asn
    1130                1135                1140

Asp Met Leu Tyr Gly Asp Ala Gly Asn Asp Thr Leu Tyr Gly Gly
    1145                1150                1155

Leu Gly Asp Asp Thr Leu Glu Gly Gly Ala Gly Asn Asp Trp Phe
    1160                1165                1170

Gly Gln Thr Gln Ala Arg Glu His Asp Val Leu Arg Gly Gly Asp
    1175                1180                1185

Gly Val Asp Thr Val Asp Tyr Ser Gln Thr Gly Ala His Ala Gly
    1190                1195                1200

Ile Ala Ala Gly Arg Ile Gly Leu Gly Ile Leu Ala Asp Leu Gly
    1205                1210                1215

Ala Gly Arg Val Asp Lys Leu Gly Glu Ala Gly Ser Ser Ala Tyr
    1220                1225                1230

Asp Thr Val Ser Gly Ile Glu Asn Val Val Gly Thr Glu Leu Ala
    1235                1240                1245

Asp Arg Ile Thr Gly Asp Ala Gln Ala Asn Val Leu Arg Gly Ala
    1250                1255                1260

Gly Gly Ala Asp Val Leu Ala Gly Gly Glu Gly Asp Asp Val Leu
    1265                1270                1275

Leu Gly Gly Asp Gly Asp Asp Gln Leu Ser Gly Asp Ala Gly Arg
    1280                1285                1290

Asp Arg Leu Tyr Gly Glu Ala Gly Asp Asp Trp Phe Phe Gln Asp
    1295                1300                1305

Ala Ala Asn Ala Gly Asn Leu Leu Asp Gly Gly Asp Gly Arg Asp
    1310                1315                1320

Thr Val Asp Phe Ser Gly Pro Gly Arg Gly Leu Asp Ala Gly Ala
    1325                1330                1335

Lys Gly Val Phe Leu Ser Leu Gly Lys Gly Phe Ala Ser Leu Met
    1340                1345                1350

Asp Glu Pro Glu Thr Ser Asn Val Leu Arg Asn Ile Glu Asn Ala
    1355                1360                1365

Val Gly Ser Ala Arg Asp Asp Val Leu Ile Gly Asp Ala Gly Ala
    1370                1375                1380

Asn Val Leu Asn Gly Leu Ala Gly Asn Asp Val Leu Ser Gly Gly
    1385                1390                1395

Ala Gly Asp Asp Val Leu Leu Gly Asp Glu Gly Ser Asp Leu Leu
    1400                1405                1410

Ser Gly Asp Ala Gly Asn Asp Asp Leu Phe Gly Gly Gln Gly Asp
    1415                1420                1425

Asp Thr Tyr Leu Phe Gly Val Gly Tyr Gly His Asp Thr Ile Tyr
    1430                1435                1440

Glu Ser Gly Gly Gly His Asp Thr Ile Arg Ile Asn Ala Gly Ala
    1445                1450                1455

Asp Gln Leu Trp Phe Ala Arg Gln Gly Asn Asp Leu Glu Ile Arg
    1460                1465                1470

Ile Leu Gly Thr Asp Asp Ala Leu Thr Val His Asp Trp Tyr Arg
    1475                1480                1485

Asp Ala Asp His Arg Val Glu Ile Ile His Ala Ala Asn Gln Ala
    1490                1495                1500

Val Asp Gln Ala Gly Ile Glu Lys Leu Val Glu Ala Met Ala Gln
    1505                1510                1515

Tyr Pro Asp Pro Gly Ala Ala Ala Ala Ala Pro Pro Ala Ala Arg
    1520                1525                1530

Val Pro Asp Thr Leu Met Gln Ser Leu Ala Val Asn Trp Arg
    1535                1540                1545

<210> 20
<211> 1569
<212> PRT
<213> Artificial Sequence

<220>
<223> polypeptide 1-183 + 321-1706 of SEQ ID NO:2

<400> 20

Met Gln Gln Ser His Gln Ala Gly Tyr Ala Asn Ala Ala Asp Arg Glu
1               5                   10                  15

Ser Gly Ile Pro Ala Ala Val Leu Asp Gly Ile Lys Ala Val Ala Lys
            20                  25                  30

Glu Lys Asn Ala Thr Leu Met Phe Arg Leu Val Asn Pro His Ser Thr
        35                  40                  45

Ser Leu Ile Ala Glu Gly Val Ala Thr Lys Gly Leu Gly Val His Ala
    50                  55                  60

Lys Ser Ser Asp Trp Gly Leu Gln Ala Gly Tyr Ile Pro Val Asn Pro
65                  70                  75                  80

Asn Leu Ser Lys Leu Phe Gly Arg Ala Pro Glu Val Ile Ala Arg Ala
                85                  90                  95

Asp Asn Asp Val Asn Ser Ser Leu Ala His Gly His Thr Ala Val Asp
            100                 105                 110

Leu Thr Leu Ser Lys Glu Arg Leu Asp Tyr Leu Arg Gln Ala Gly Leu
        115                 120                 125

Val Thr Gly Met Ala Asp Gly Val Val Ala Ser Asn His Ala Gly Tyr
    130                 135                 140

Glu Gln Phe Glu Phe Arg Val Lys Glu Thr Ser Asp Gly Arg Tyr Ala
145                 150                 155                 160

Val Gln Tyr Arg Arg Lys Gly Gly Asp Asp Phe Glu Ala Val Lys Val
                165                 170                 175

Ile Gly Asn Ala Ala Gly Ile Glu Ser Gln Met Leu Thr Arg Gly Gln
            180                 185                 190

Leu Lys Glu Tyr Ile Gly Gln Gln Arg Gly Glu Gly Tyr Val Phe Tyr
        195                 200                 205

Glu Asn Arg Ala Tyr Gly Val Ala Gly Lys Ser Leu Phe Asp Asp Gly
    210                 215                 220

Leu Gly Ala Ala Pro Gly Val Pro Ser Gly Arg Ser Lys Phe Ser Pro
225                 230                 235                 240

Asp Val Leu Glu Thr Val Pro Ala Ser Pro Gly Leu Arg Arg Pro Ser
                245                 250                 255

Leu Gly Ala Val Glu Arg Gln Asp Ser Gly Tyr Asp Ser Leu Asp Gly
            260                 265                 270

Val Gly Ser Arg Ser Phe Ser Leu Gly Glu Val Ser Asp Met Ala Ala
        275                 280                 285

Val Glu Ala Ala Glu Leu Glu Met Thr Arg Gln Val Leu His Ala Gly
    290                 295                 300

Ala Arg Gln Asp Asp Ala Glu Pro Gly Val Ser Gly Ala Ser Ala His
305                 310                 315                 320

Trp Gly Gln Arg Ala Leu Gln Gly Ala Gln Ala Val Ala Ala Ala Gln
                325                 330                 335

Arg Leu Val His Ala Ile Ala Leu Met Thr Gln Phe Gly Arg Ala Gly
            340                 345                 350

Ser Thr Asn Thr Pro Gln Glu Ala Ala Ser Leu Ser Ala Ala Val Phe
        355                 360                 365

Gly Leu Gly Glu Ala Ser Ser Ala Val Ala Glu Thr Val Ser Gly Phe
    370                 375                 380

Phe Arg Gly Ser Ser Arg Trp Ala Gly Gly Phe Gly Val Ala Gly Gly
385                 390                 395                 400

Ala Met Ala Leu Gly Gly Gly Ile Ala Ala Ala Val Gly Ala Gly Met
                405                 410                 415

Ser Leu Thr Asp Asp Ala Pro Ala Gly Gln Lys Ala Ala Ala Gly Ala
            420                 425                 430

Glu Ile Ala Leu Gln Leu Thr Gly Gly Thr Val Glu Leu Ala Ser Ser
        435                 440                 445

Ile Ala Leu Ala Leu Ala Ala Ala Arg Gly Val Thr Ser Gly Leu Gln
    450                 455                 460

Val Ala Gly Ala Ser Ala Gly Ala Ala Ala Gly Ala Leu Ala Ala Ala
465                 470                 475                 480

Leu Ser Pro Met Glu Ile Tyr Gly Leu Val Gln Gln Ser His Tyr Ala
                485                 490                 495

Asp Gln Leu Asp Lys Leu Ala Gln Glu Ser Ser Ala Tyr Gly Tyr Glu
            500                 505                 510

Gly Asp Ala Leu Leu Ala Gln Leu Tyr Arg Asp Lys Thr Ala Ala Glu
        515                 520                 525

Gly Ala Val Ala Gly Val Ser Ala Val Leu Ser Thr Val Gly Ala Ala
    530                 535                 540

Val Ser Ile Ala Ala Ala Ala Ser Val Val Gly Ala Pro Val Ala Val
545                 550                 555                 560

Val Thr Ser Leu Leu Thr Gly Ala Leu Asn Gly Ile Leu Arg Gly Val
                565                 570                 575

Gln Gln Pro Ile Ile Glu Lys Leu Ala Asn Asp Tyr Ala Arg Lys Ile
            580                 585                 590

Asp Glu Leu Gly Gly Pro Gln Ala Tyr Phe Glu Lys Asn Leu Gln Ala
        595                 600                 605

Arg His Glu Gln Leu Ala Asn Ser Asp Gly Leu Arg Lys Met Leu Ala
    610                 615                 620

Asp Leu Gln Ala Gly Trp Asn Ala Ser Ser Val Ile Gly Val Gln Thr
625                 630                 635                 640

Thr Glu Ile Ser Lys Ser Ala Leu Glu Leu Ala Ala Ile Thr Gly Asn
                645                 650                 655

Ala Asp Asn Leu Lys Ser Val Asp Val Phe Val Asp Arg Phe Val Gln
            660                 665                 670

Gly Glu Arg Val Ala Gly Gln Pro Val Val Leu Asp Val Ala Ala Gly
        675                 680                 685

Gly Ile Asp Ile Ala Ser Arg Lys Gly Glu Arg Pro Ala Leu Thr Phe

690                 695                 700

Ile Thr Pro Leu Ala Ala Pro Gly Glu Glu Gln Arg Arg Arg Thr Lys
705                 710                 715                 720

Thr Gly Lys Ser Glu Phe Thr Thr Phe Val Glu Ile Val Gly Lys Gln
                725                 730                 735

Asp Arg Trp Arg Ile Arg Asp Gly Ala Ala Asp Thr Thr Ile Asp Leu
            740                 745                 750

Ala Lys Val Val Ser Gln Leu Val Asp Ala Asn Gly Val Leu Lys His
        755                 760                 765

Ser Ile Lys Leu Asp Val Ile Gly Gly Asp Gly Asp Asp Val Val Leu
    770                 775                 780

Ala Asn Ala Ser Arg Ile His Tyr Asp Gly Gly Ala Gly Thr Asn Thr
785                 790                 795                 800

Val Ser Tyr Ala Ala Leu Gly Arg Gln Asp Ser Ile Thr Val Ser Ala
                805                 810                 815

Asp Gly Glu Arg Phe Asn Val Arg Lys Gln Leu Asn Asn Ala Asn Val
            820                 825                 830

Tyr Arg Glu Gly Val Ala Thr Gln Thr Thr Ala Tyr Gly Lys Arg Thr
        835                 840                 845

Glu Asn Val Gln Tyr Arg His Val Glu Leu Ala Arg Val Gly Gln Leu
    850                 855                 860

Val Glu Val Asp Thr Leu Glu His Val Gln His Ile Ile Gly Gly Ala
865                 870                 875                 880

Gly Asn Asp Ser Ile Thr Gly Asn Ala His Asp Asn Phe Leu Ala Gly
                885                 890                 895

Gly Ser Gly Asp Asp Arg Leu Asp Gly Gly Ala Gly Asn Asp Thr Leu
            900                 905                 910

Val Gly Gly Glu Gly Gln Asn Thr Val Ile Gly Gly Ala Gly Asp Asp
        915                 920                 925

Val Phe Leu Gln Asp Leu Gly Val Trp Ser Asn Gln Leu Asp Gly Gly
    930                 935                 940

Ala Gly Val Asp Thr Val Lys Tyr Asn Val His Gln Pro Ser Glu Glu
945                 950                 955                 960

Arg Leu Glu Arg Met Gly Asp Thr Gly Ile His Ala Asp Leu Gln Lys
                965                 970                 975

Gly Thr Val Glu Lys Trp Pro Ala Leu Asn Leu Phe Ser Val Asp His
            980                 985                 990

Val Lys Asn Ile Glu Asn Leu His  Gly Ser Arg Leu Asn  Asp Arg Ile
        995                 1000                 1005

Ala Gly  Asp Asp Gln Asp Asn  Glu Leu Trp Gly His  Asp Gly Asn
    1010                 1015                 1020

Asp Thr  Ile Arg Gly Arg Gly  Gly Asp Asp Ile Leu  Arg Gly Gly
    1025                 1030                 1035

Leu Gly  Leu Asp Thr Leu Tyr  Gly Glu Asp Gly Asn  Asp Ile Phe
    1040                 1045                 1050

Leu Gln  Asp Asp Glu Thr Val  Ser Asp Asp Ile Asp  Gly Gly Ala
    1055                 1060                 1065

Gly Leu  Asp Thr Val Asp Tyr  Ser Ala Met Ile His  Pro Gly Arg
    1070                 1075                 1080

Ile Val  Ala Pro His Glu Tyr  Gly Phe Gly Ile Glu  Ala Asp Leu
    1085                 1090                 1095

Ser Arg  Glu Trp Val Arg Lys  Ala Ser Ala Leu Gly  Val Asp Tyr
    1100                 1105                 1110

Tyr Asp  Asn Val Arg Asn Val  Glu Asn Val Ile Gly  Thr Ser Met
    1115                 1120                 1125

Lys Asp  Val Leu Ile Gly Asp  Ala Gln Ala Asn Thr  Leu Met Gly
    1130                 1135                 1140

Gln Gly  Gly Asp Asp Thr Val  Arg Gly Gly Asp Gly  Asp Asp Leu
    1145                 1150                 1155

Leu Phe  Gly Gly Asp Gly Asn  Asp Met Leu Tyr Gly  Asp Ala Gly
    1160                 1165                 1170

Asn Asp  Thr Leu Tyr Gly Gly  Leu Gly Asp Asp Thr  Leu Glu Gly
    1175                 1180                 1185

Gly Ala Gly Asn Asp Trp Phe Gly Gln Thr Gln Ala Arg Glu His
    1190                1195                1200

Asp Val Leu Arg Gly Gly Asp Gly Val Asp Thr Val Asp Tyr Ser
    1205                1210                1215

Gln Thr Gly Ala His Ala Gly Ile Ala Ala Gly Arg Ile Gly Leu
    1220                1225                1230

Gly Ile Leu Ala Asp Leu Gly Ala Gly Arg Val Asp Lys Leu Gly
    1235                1240                1245

Glu Ala Gly Ser Ser Ala Tyr Asp Thr Val Ser Gly Ile Glu Asn
    1250                1255                1260

Val Val Gly Thr Glu Leu Ala Asp Arg Ile Thr Gly Asp Ala Gln
    1265                1270                1275

Ala Asn Val Leu Arg Gly Ala Gly Gly Ala Asp Val Leu Ala Gly
    1280                1285                1290

Gly Glu Gly Asp Asp Val Leu Leu Gly Gly Asp Gly Asp Asp Gln
    1295                1300                1305

Leu Ser Gly Asp Ala Gly Arg Asp Arg Leu Tyr Gly Glu Ala Gly
    1310                1315                1320

Asp Asp Trp Phe Phe Gln Asp Ala Ala Asn Ala Gly Asn Leu Leu
    1325                1330                1335

Asp Gly Gly Asp Gly Arg Asp Thr Val Asp Phe Ser Gly Pro Gly
    1340                1345                1350

Arg Gly Leu Asp Ala Gly Ala Lys Gly Val Phe Leu Ser Leu Gly
    1355                1360                1365

Lys Gly Phe Ala Ser Leu Met Asp Glu Pro Glu Thr Ser Asn Val
    1370                1375                1380

Leu Arg Asn Ile Glu Asn Ala Val Gly Ser Ala Arg Asp Asp Val
    1385                1390                1395

Leu Ile Gly Asp Ala Gly Ala Asn Val Leu Asn Gly Leu Ala Gly
    1400                1405                1410

Asn Asp Val Leu Ser Gly Gly Ala Gly Asp Asp Val Leu Leu Gly
    1415                1420                1425

Asp Glu Gly Ser Asp Leu Leu Ser Gly Asp Ala Gly Asn Asp Asp
    1430                1435                1440

Leu Phe Gly Gly Gln Gly Asp Asp Thr Tyr Leu Phe Gly Val Gly
    1445                1450                1455

Tyr Gly His Asp Thr Ile Tyr Glu Ser Gly Gly Gly His Asp Thr
    1460                1465                1470

Ile Arg Ile Asn Ala Gly Ala Asp Gln Leu Trp Phe Ala Arg Gln
    1475                1480                1485

Gly Asn Asp Leu Glu Ile Arg Ile Leu Gly Thr Asp Asp Ala Leu
    1490                1495                1500

Thr Val His Asp Trp Tyr Arg Asp Ala Asp His Arg Val Glu Ile
    1505                1510                1515

Ile His Ala Ala Asn Gln Ala Val Asp Gln Ala Gly Ile Glu Lys
    1520                1525                1530

Leu Val Glu Ala Met Ala Gln Tyr Pro Asp Pro Gly Ala Ala Ala
    1535                1540                1545

Ala Ala Pro Pro Ala Ala Arg Val Pro Asp Thr Leu Met Gln Ser
    1550                1555                1560

Leu Ala Val Asn Trp Arg
    1565

<210>  21
<211>  558
<212>  DNA
<213>  Bordetella pertussis

<400>  21
atgcttccgt ccgcccaagc gccctccctc ctcaatccca ccgacgactt cgcggcactg      60

ggcaatattg cctggctgtg gatgaactct cccatgcacc gcgactggcc ggtgcatctg     120

ctcgcacgca acacgctcgc gccgattcaa ctgggccaat acattctgct gcgatgcaat     180

gacgtgccgg ttgcatactg cagctgggcc ctaatggacg ccgacaccga actctcctat     240

gtcatggcgc cctcgtcgct gggcgggaat gcctggaact gcggcgaccg actgtggatc     300

atcgactgga tcgcgccatt ctcgcgcgac gacaatcgtg cgctgcgccg cgcgctggcc     360

gaacggcacc ccgacagcgt gggccgttcg ctgcgcgttc ggcgcggcgg cgacaccgcg     420

cgcgtcaagg agtaccgagg ccgcgcgctg gacgcggccg ccgctcgcgc gcagctggac     480

cgctaccatg ccgaactgat cgcaggactg cgcgcgagca acggcggata cgcgccgcga 540

ggccggggca ccgcctga 558

<210> 22
<211> 558
<212> DNA
<213> Artificial Sequence

<220>
<223> Optimized version of CyaC nucleotide coding sequence

<220>
<221> CDS
<222> (1)..(558)

<400> 22
atg ctg ccg tct gca cag gcg ccg tct ctg ctg aat cca act gat gac 48
Met Leu Pro Ser Ala Gln Ala Pro Ser Leu Leu Asn Pro Thr Asp Asp
1               5                   10                  15

ttc gct gcg ctg ggt aac atc gcg tgg ctg tgg atg aac agc ccg atg 96
Phe Ala Ala Leu Gly Asn Ile Ala Trp Leu Trp Met Asn Ser Pro Met
            20                  25                  30

cac cgt gac tgg ccg gtt cac ctg ctg gca cgt aac acg ctg gcg ccg 144
His Arg Asp Trp Pro Val His Leu Leu Ala Arg Asn Thr Leu Ala Pro
        35                  40                  45

atc cag ctg ggt caa tac atc ctg ctg cgt tgt aac gac gtg cct gtg 192
Ile Gln Leu Gly Gln Tyr Ile Leu Leu Arg Cys Asn Asp Val Pro Val
    50                  55                  60

gcg tat tgc tcc tgg gca ctg atg gat gcg gat acc gag ctg tcc tac 240
Ala Tyr Cys Ser Trp Ala Leu Met Asp Ala Asp Thr Glu Leu Ser Tyr
65                  70                  75                  80

gtt atg gcc cca tct agc ctg ggc ggt aac gct tgg aac tgc ggc gat 288
Val Met Ala Pro Ser Ser Leu Gly Gly Asn Ala Trp Asn Cys Gly Asp
                85                  90                  95

cgt ctg tgg atc att gat tgg att gct ccg ttt agc cgt gac gac aac 336
Arg Leu Trp Ile Ile Asp Trp Ile Ala Pro Phe Ser Arg Asp Asp Asn
            100                 105                 110

cgt gct ctg cgt cgc gcg ctg gcc gaa cgt cat ccg gat tct gtt ggc 384
Arg Ala Leu Arg Arg Ala Leu Ala Glu Arg His Pro Asp Ser Val Gly
        115                 120                 125

cgt tcc ctg cgc gta cgt cgc ggt ggc gac acc gct cgc gtc aaa gaa 432
Arg Ser Leu Arg Val Arg Arg Gly Gly Asp Thr Ala Arg Val Lys Glu
    130                 135                 140

tac cgt ggt cgt gct ctg gac gca gcg gcg gcc cgc gct cag ctg gac 480
Tyr Arg Gly Arg Ala Leu Asp Ala Ala Ala Ala Arg Ala Gln Leu Asp
145                 150                 155                 160

cgc tac cac gca gaa ctg att gcc ggt ctg cgt gca tcc aat ggc ggt 528
Arg Tyr His Ala Glu Leu Ile Ala Gly Leu Arg Ala Ser Asn Gly Gly
                165                 170                 175

tat gct ccg cgt ggc cgt ggc acc gca taa                                    558
Tyr Ala Pro Arg Gly Arg Gly Thr Ala
            180                 185

<210>  23
<211>  185
<212>  PRT
<213>  Artificial Sequence

<220>
<223>  Synthetic Construct

<400>  23

Met Leu Pro Ser Ala Gln Ala Pro Ser Leu Leu Asn Pro Thr Asp Asp
1               5                   10                  15

Phe Ala Ala Leu Gly Asn Ile Ala Trp Leu Trp Met Asn Ser Pro Met
            20                  25                  30

His Arg Asp Trp Pro Val His Leu Leu Ala Arg Asn Thr Leu Ala Pro
        35                  40                  45

Ile Gln Leu Gly Gln Tyr Ile Leu Leu Arg Cys Asn Asp Val Pro Val
    50                  55                  60

Ala Tyr Cys Ser Trp Ala Leu Met Asp Ala Asp Thr Glu Leu Ser Tyr
65                  70                  75                  80

Val Met Ala Pro Ser Ser Leu Gly Gly Asn Ala Trp Asn Cys Gly Asp
                85                  90                  95

Arg Leu Trp Ile Ile Asp Trp Ile Ala Pro Phe Ser Arg Asp Asp Asn
            100                 105                 110

Arg Ala Leu Arg Arg Ala Leu Ala Glu Arg His Pro Asp Ser Val Gly
        115                 120                 125

Arg Ser Leu Arg Val Arg Arg Gly Gly Asp Thr Ala Arg Val Lys Glu
    130                 135                 140

Tyr Arg Gly Arg Ala Leu Asp Ala Ala Ala Ala Arg Ala Gln Leu Asp
145                 150                 155                 160

Arg Tyr His Ala Glu Leu Ile Ala Gly Leu Arg Ala Ser Asn Gly Gly
                165                 170                 175

Tyr Ala Pro Arg Gly Arg Gly Thr Ala
            180                 185

<210>  24
<211>  5121
<212>  DNA
<213>  Artificial Sequence

<220>
<223>  Nucleotide sequence encoding the wild type CyaA protein of
       Bordetella pertussis, optimized for E. coli expression

<400>  24
atgcagcagt cccatcaggc cggttacgca aacgcagccg atcgtgaatc tggtatcccg      60

gcagcggttc tggatggtat taaggcagtt gccaaagaga agaacgctac cctgatgttc     120

cgtctggtca acccacactc tacttccctg atcgcggaag gtgtggcgac caaaggcctg     180

ggtgtgcacg ccaaaagcag cgactggggt ctgcaggcag gttatattcc ggtgaacccg     240

aatctgtcta aactgttcgg tcgtgccccg gaggtgatcg cacgtgcaga caacgatgtc     300

aactcttctc tggcgcatgg tcacaccgcg gttgatctga ccctgtccaa agaacgcctg     360

gattacctgc gtcaggcggg cctggtgacg ggcatggccg acggtgttgt agcgagcaac     420

cacgcgggtt atgaacaatt tgagtttcgc gttaaagaga cctctgacgg tcgttacgcg     480

gtgcagtacc gccgcaaagg tggcgatgac tttgaagctg tcaaagtgat cggtaacgct     540

gctggaattc cgctgaccgc ggatatcgac atgtttgcca tcatgcctca cctgtccaac     600

tttcgcgatt ccgcacgcag ctctgtgacg tctggtgatt ccgtcactga ctacctggct     660

cgtacccgtc gtgcggcctc tgaagccact ggtggtctgg atcgtgaacg cattgacctg     720

ctgtggaaaa tcgcccgtgc cggtgcgcgc tctgctgttg gtaccgaagc acgccgccaa     780

tttcgttacg acggcgatat gaacatcggt gtgatcacgg attttgaact ggaagttcgc     840

aatgccctga accgtcgtgc acacgcggtc ggcgcgcagg acgttgttca gcacggtacg     900

gagcagaata acccgttccc agaagcggac gaaaagatct ttgttgtgtc tgccaccggt     960

gaatcccaga tgctgactcg tggccagctg aaagaataca tcggccaaca gcgtggcgaa    1020

ggctacgttt tctacgagaa tcgtgcttac ggcgttgcgg gcaaatccct gttcgacgat    1080

ggtctgggcg ctgcaccggg cgttccgagc ggccgttcta aattcagccc ggacgtactg    1140

gaaactgtgc cggcttcccc gggcctgcgc cgtccgtccc tgggcgcagt cgaacgtcag    1200

gactccggct acgattccct ggatggcgtt ggctcccgct ccttctccct gggcgaggtt    1260

tctgacatgg ctgcggtgga agcggcagag ctggaaatga cccgtcaagt gctgcacgcg    1320

ggcgcacgtc aggacgatgc tgagccgggc gtgtccggtg cgtctgcaca ctggggtcaa    1380

cgtgccctgc agggtgctca agctgtcgcg gcagcgcagc gtctggtaca tgcgatcgca    1440

ctgatgaccc agttcggtcg cgcaggttct accaacactc ctcaggaagc agctagcctg    1500

tctgctgctg ttttcggtct gggcgaagcg tcttctgcgg ttgcagaaac cgtgagcggt    1560

ttcttccgtg gttctagccg ctgggcgggt ggttttggtg tagcaggtgg cgctatggcc 1620

ctgggtggcg gtatcgcggc tgctgtcggc gcaggtatga gcctgaccga cgatgctcct 1680

gcgggccaga aagccgcagc tggcgctgaa atcgcgctgc agctgactgg tggtaccgtt 1740

gaactggcta gctctatcgc gctggctctg gcagcggcac gtggcgtgac ttctggcctg 1800

caagtcgccg gtgcctctgc gggcgctgct gcgggcgctc tggcagctgc actgtccccc 1860

atggaaatct acggtctggt acagcagtct cactacgcag accagctgga taaactggcg 1920

caggagtctt ctgcatacgg ttacgaaggc gacgcactgc tggcgcagct gtatcgtgac 1980

aaaactgctg ctgaaggtgc tgtggcaggc gtttctgcgg tactgtctac cgttggcgct 2040

gcggtttcta ttgcggctgc cgcatccgtt gtaggtgcgc cggttgctgt agttacctct 2100

ctgctgactg gtgcactgaa cggtatcctg cgtggtgttc agcagcctat tatcgaaaaa 2160

ctggcgaatg attatgcccg taaaatcgac gaactgggcg gtccgcaggc ttattttgaa 2220

aaaaacctgc aggcgcgcca cgaacagctg gcaaacagcg acggcctgcg caaaatgctg 2280

gcagacctgc aagctggttg gaacgcgtct tctgtgatcg gcgtgcagac caccgaaatt 2340

agcaaatctg cgctggaact ggctgcaatt actggcaacg cggataacct gaaaagcgtt 2400

gatgtttttg tcgatcgctt cgttcagggc gagcgcgttg ctggtcagcc ggttgttctg 2460

gacgttgcgg caggcggcat cgacatcgct agccgcaagg gcgagcgtcc ggctctgact 2520

ttcattaccc cgctggcagc tccgggtgaa gaacagcgtc gccgtaccaa gactggtaaa 2580

agcgagttca ccaccttcgt ggaaatcgta ggtaaacagg accgctggcg tattcgcgac 2640

ggtgcggcgg acaccaccat cgacctggca aaagttgttt cccaactggt agatgctaac 2700

ggcgtgctga aacattctat taaactggat gtaatcggcg gtgacggcga tgacgttgta 2760

ctggcgaacg cgtctcgtat ccactatgac ggcggcgcag gtaccaacac ggtttcctac 2820

gcagcgctgg gccgccagga ttccatcact gttagcgccg atggcgaacg tttcaacgtg 2880

cgtaaacagc tgaataacgc aaacgtctat cgtgagggtg tagctaccca gaccaccgcg 2940

tacggtaagc gtaccgaaaa cgttcagtat cgtcacgtgg aactggcacg tgtgggtcag 3000

ctggtagagg tggatacgct ggaacacgtg cagcacatca tcggtggcgc cggtaacgat 3060

tccattactg gtaacgctca cgacaacttc ctggcaggtg gttctggtga tgatcgtctg 3120

gacggtggtg cgggtaacga tacgctggta ggcggcgaag gtcagaacac cgtaattggt 3180

ggtgccggtg acgacgtttt cctgcaggat ctgggcgttt ggagcaacca gctggatggc 3240

ggtgccggtg ttgacacggt taagtataac gttcaccagc cgtctgaaga gcgcctggaa 3300

cgtatgggtg acactggtat tcatgccgat ctgcagaaag gcactgttga aaaatggcct 3360

gcgctgaacc tgttctctgt ggaccatgtt aagaacattg aaaacctgca tggctctcgc 3420

ctgaacgacc gtatcgctgg tgacgaccag gataacgaac tgtggggtca tgacggcaat 3480

gataccattc gcggtcgtgg cggcgacgat attctgcgtg gcggtctggg cctggatacc 3540

ctgtatggtg aagatggtaa cgatattttc ctgcaagacg atgaaacggt atctgatgac 3600

atcgacggtg gtgcaggcct ggacaccgta gattacagcg ctatgatcca tccgggtcgt 3660

atcgtagctc cgcacgagta cggcttcggt atcgaggcgg acctgtctcg tgaatgggtt 3720

cgtaaagcgt ccgcgctggg tgtggactac tacgataacg ttcgtaacgt ggaaaacgtg 3780

atcggtacct ccatgaaaga cgtgctgatt ggcgacgcac aggccaacac tctgatgggc 3840

cagggcggtg atgacacggt tcgcggcggc gacggtgatg acctgctgtt cggtggtgac 3900

ggcaacgaca tgctgtacgg cgacgcgggc aacgacaccc tgtacggcgg tctgggtgac 3960

gacactctgg aaggtggtgc aggtaacgac tggttcggcc aaacccaggc acgcgaacac 4020

gacgtgctgc gtggtggcga cggcgtagac accgtggatt actcccaaac tggcgctcac 4080

gcgggtatcg cggccggtcg tatcggtctg ggcattctgg ccgatctggg tgccggccgt 4140

gtcgacaaac tgggtgaggc tggctctagc gcctatgata ctgtttccgg catcgaaaac 4200

gtggtaggca ctgaactggc agatcgtatc acgggtgacg cgcaagcgaa cgttctgcgt 4260

ggtgctggcg gtgcagacgt gctggcgggt ggtgaaggcg acgacgtact gctgggcggt 4320

gacggtgacg atcagctgag cggtgatgcg ggtcgtgacc gtctgtacgg tgaagcaggt 4380

gacgattggt tcttccagga cgctgctaac gctggcaacc tgctggacgg cggcgatggc 4440

cgtgacactg ttgacttcag cggtccgggt cgcggtctgg acgcaggcgc gaaaggcgtt 4500

ttcctgagcc tgggtaaggg tttcgcatct ctgatggacg aaccagaaac cagcaatgtc 4560

ctgcgtaaca tcgaaaacgc tgtcggttcc gcacgcgatg atgttctgat tggcgatgct 4620

ggtgccaatg ttctgaacgg tctggcgggt aatgatgtac tgtctggtgg cgctggtgat 4680

gatgtcctgc tgggtgatga gggcagcgat ctgctgtccg gcgatgccgg caacgacgat 4740

ctgttcggtg gccaaggtga tgatacttac ctgttcggcg ttggctatgg tcatgacacc 4800

atctatgagt ctggtggcgg ccacgatacg attcgtatta atgcaggtgc tgaccaactg 4860

tggtttgcac gtcagggtaa cgatctggaa attcgcatcc tgggtactga tgacgctctg 4920

accgtacacg actggtaccg cgatgcggat caccgcgtag aaatcatcca tgcggctaac 4980

caagctgtag accaggctgg tattgaaaaa ctggtagaag cgatggcaca gtatccggac 5040

ccaggtgcgg ctgctgcagc acctccagcg gctcgtgtgc cggataccct gatgcagagc 5100

ctggccgtca attggcgtta a 5121

<210> 25
<211> 11172
<212> DNA
<213> Artificial Sequence

<220>
<223>  pGTP-gtCyaAd93-CyaCopt

<400>  25
atcgagatct gtatggctgt gcaggtcgta aatcactgca taattcgtgt cgctcaaggc      60

gcactcccgt tctggataat gttttttgcg ccgacatcat aacggttctg gcaaatattc     120

tgaaatgagc tgttgacaat taatcatcgg ctcgtataat gtgtggaatt gtgagcggat     180

aacaattata atttgtttta actttaagaa ggagatatac atatgcagca gtcccatcag     240

gccggttacg caaacgcagc cgatcgtgaa tctggtatcc cggcagcggt tctggatggt     300

attaaggcag ttgccaaaga gaagaacgct accctgatgt tccgtctggt caacccacac     360

tctacttccc tgatcgcgga aggtgtggcg accaaaggcc tgggtgtgca cgccaaaagc     420

agcgactggg gtctgcaggc aggttatatt ccggtgaacc cgaatctgtc taaactgttc     480

ggtcgtgccc cggaggtgat cgcacgtgca gacaacgatg tcaactcttc tctggcgcat     540

ggtcacaccg cggttgatct gaccctgtcc aaagaacgcc tggattacct gcgtcaggcg     600

ggcctggtga cgggcatggc cgacggtgtt gtagcgagca accacgcggg ttatgaacaa     660

tttgagtttc gcgttaaaga gacctctgac ggtcgttacg cggtgcagta ccgccgcaaa     720

ggtggcgatg actttgaagc tgtcaaagtg atcggtaacg ctgctggaat tccgctgacc     780

gcggatatcg acatgtttgc catcatgcct cacctgtcca actttcgcga ttccgcacgc     840

agctctgtga cgtctggtga ttccgtcact gactacctgg ctcgtacccg tcgtgcggcc     900

tctgaatccc agatgctgac tcgtggccag cttaaggaat acatcggcca acagcgtggc     960

gaaggctacg ttttctacga gaatcgtgct tacggcgttg cgggcaaatc cctgttcgac    1020

gatggtctgg gcgctgcacc gggcgttccg agcggccgtt ctaaattcag cccggacgta    1080

ctggaaactg tgccggcttc cccgggcctg cgccgtccgt ccctgggcgc agtcgaacgt    1140

caggactccg gctacgattc cctggatggc gttggctccc gctccttctc cctgggcgag    1200

gtttctgaca tggctgcggt ggaagcggca gagctggaaa tgacccgtca agtgctgcac    1260

gcgggcgcac gtcaggacga tgctgagccg ggcgtgtccg gtgcgtctgc acactggggt    1320

caacgtgccc tgcagggtgc tcaagctgtc gcggcagcgc agcgtctggt acatgcgatc    1380

gcactgatga cccagttcgg tcgcgcaggt tctaccaaca ctcctcagga agcagctagc    1440

ctgtctgctg ctgttttcgg tctgggcgaa gcgtcttctg cggttgcaga aaccgtgagc    1500

ggtttcttcc gtggttctag ccgctgggcg ggtggttttg gtgtagcagg tggcgctatg    1560

gccctgggtg gcggtatcgc ggctgctgtc ggcgcaggta tgagcctgac cgacgatgct    1620

cctgcgggcc agaaagccgc agctggcgct gaaatcgcgc tgcagctgac tggtggtacc    1680

gttgaactgg ctagctctat cgcgctggct ctggcagcgg cacgtggcgt gacttctggc    1740

ctgcaagtcg ccggtgcctc tgcgggcgct gctgcgggcg ctctggcagc tgcactgtcc    1800

cccatggaaa tctacggtct ggtacagcag tctcactacg cagaccagct ggataaactg 1860

gcgcaggagt cttctgcata cggttacgaa ggcgacgcac tgctggcgca gctgtatcgt 1920

gacaaaactg ctgctgaagg tgctgtggca ggcgtttctg cggtactgtc taccgttggc 1980

gctgcggttt ctattgcggc tgccgcatcc gttgtaggtg cgccggttgc tgtagttacc 2040

tctctgctga ctggtgcact gaacggtatc ctgcgtggtg ttcagcagcc tattatcgaa 2100

aaactggcga atgattatgc ccgtaaaatc gacgaactgg gcggtccgca ggcttatttt 2160

gaaaaaaacc tgcaggcgcg ccacgaacag ctggcaaaca gcgacggcct gcgcaaaatg 2220

ctggcagacc tgcaagctgg ttggaacgcg tcttctgtga tcggcgtgca gaccaccgaa 2280

attagcaaat ctgcgctgga actggctgca attactggca acgcggataa cctgaaaagc 2340

gttgatgttt ttgtcgatcg cttcgttcag ggcgagcgcg ttgctggtca gccggttgtt 2400

ctggacgttg cggcaggcgg catcgacatc gctagccgca agggcgagcg tccggctctg 2460

actttcatta ccccgctggc agctccgggt gaagaacagc gtcgccgtac caagactggt 2520

aaaagcgagt tcaccacctt cgtggaaatc gtaggtaaac aggaccgctg gcgtattcgc 2580

gacggtgcgg cggacaccac catcgacctg gcaaaagttg tttcccaact ggtagatgct 2640

aacggcgtgc tgaaacattc tattaaactg gatgtaatcg gcggtgacgg cgatgacgtt 2700

gtactggcga acgcgtctcg tatccactat gacggcggcg caggtaccaa cacggtttcc 2760

tacgcagcgc tgggccgcca ggattccatc actgttagcg ccgatggcga acgtttcaac 2820

gtgcgtaaac agctgaataa cgcaaacgtc tatcgtgagg gtgtagctac ccagaccacc 2880

gcgtacggta agcgtaccga aaacgttcag tatcgtcacg tggaactggc acgtgtgggt 2940

cagctggtag aggtggatac gctggaacac gtgcagcaca tcatcggtgg cgccggtaac 3000

gattccatta ctggtaacgc tcacgacaac ttcctggcag gtggttctgg tgatgatcgt 3060

ctggacggtg gtgcgggtaa cgatacgctg gtaggcggcg aaggtcagaa caccgtaatt 3120

ggtggtgccg gtgacgacgt tttcctgcag gatctgggcg tttggagcaa ccagctggat 3180

ggcggtgccg gtgttgacac ggttaagtat aacgttcacc agccgtctga agagcgcctg 3240

gaacgtatgg gtgacactgg tattcatgcc gatctgcaga aaggcactgt tgaaaaatgg 3300

cctgcgctga acctgttctc tgtggaccat gttaagaaca ttgaaaacct gcatggctct 3360

cgcctgaacg accgtatcgc tggtgacgac caggataacg aactgtgggg tcatgacggc 3420

aatgatacca ttcgcggtcg tggcggcgac gatattctgc gtggcggtct gggcctggat 3480

accctgtatg gtgaagatgg taacgatatt ttcctgcaag acgatgaaac ggtatctgat 3540

gacatcgacg gtggtgcagg cctggacacc gtagattaca gcgctatgat ccatccgggt 3600

cgtatcgtag ctccgcacga gtacggcttc ggtatcgagg cggacctgtc tcgtgaatgg 3660

gttcgtaaag cgtccgcgct gggtgtggac tactacgata acgttcgtaa cgtggaaaac 3720

gtgatcggta cctccatgaa agacgtgctg attggcgacg cacaggccaa cactctgatg 3780

ggccagggcg gtgatgacac ggttcgcggc ggcgacggtg atgacctgct gttcggtggt 3840

gacggcaacg acatgctgta cggcgacgcg ggcaacgaca ccctgtacgg cggtctgggt 3900

gacgacactc tggaaggtgg tgcaggtaac gactggttcg gccaaaccca ggcacgcgaa 3960

cacgacgtgc tgcgtggtgg cgacggcgta gacaccgtgg attactccca aactggcgct 4020

cacgcgggta tcgcggccgg tcgtatcggt ctgggcattc tggccgatct gggtgccggc 4080

cgtgtcgaca aactgggtga ggctggctct agcgcctatg atactgtttc cggcatcgaa 4140

aacgtggtag gcactgaact ggcagatcgt atcacgggtg acgcgcaagc gaacgttctg 4200

cgtggtgctg gcggtgcaga cgtgctggcg ggtggtgaag gcgacgacgt actgctgggc 4260

ggtgacggtg acgatcagct gagcggtgat gcgggtcgtg accgtctgta cggtgaagca 4320

ggtgacgatt ggttcttcca ggacgctgct aacgctggca acctgctgga cggcggcgat 4380

ggccgtgaca ctgttgactt cagcggtccg ggtcgcggtc tggacgcagg cgcgaaaggc 4440

gttttcctga gcctgggtaa gggtttcgca tctctgatgg acgaaccaga aaccagcaat 4500

gtcctgcgta acatcgaaaa cgctgtcggt tccgcacgcg atgatgttct gattggcgat 4560

gctggtgcca atgttctgaa cggtctggcg ggtaatgatg tactgtctgg tggcgctggt 4620

gatgatgtcc tgctgggtga tgagggcagc gatctgctgt ccggcgatgc cggcaacgac 4680

gatctgttcg gtggccaagg tgatgatact tacctgttcg gcgttggcta tggtcatgac 4740

accatctatg agtctggtgg cggccacgat acgattcgta ttaatgcagg tgctgaccaa 4800

ctgtggtttg cacgtcaggg taacgatctg gaaattcgca tcctgggtac tgatgacgct 4860

ctgaccgtac acgactggta ccgcgatgcg gatcaccgcg tagaaatcat ccatgcggct 4920

aaccaagctg tagaccaggc tggtattgaa aaactggtag aagcgatggc acagtatccg 4980

gacccaggtg cggctgctgc agcacctcca gcggctcgtg tgccggatac cctgatgcag 5040

agcctggccg tcaattggcg ttaatggatc caggcctctt tagaaggaga tataccatgc 5100

tgccgtctgc acaggcgccg tctctgctga atccaactga tgacttcgct gcgctgggta 5160

acatcgcgtg gctgtggatg aacagcccga tgcaccgtga ctggccggtt cacctgctgg 5220

cacgtaacac gctggcgccg atccagctgg gtcaatacat cctgctgcgt tgtaacgacg 5280

tgcctgtggc gtattgctcc tgggcactga tggatgcgga taccgagctg tcctacgtta 5340

tggccccatc tagcctgggc ggtaacgctt ggaactgcgg cgatcgtctg tggatcattg 5400

attggattgc tccgtttagc cgtgacgaca accgtgctct gcgtcgcgcg ctggccgaac 5460

gtcatccgga ttctgttggc cgttccctgc gcgtacgtcg cggtggcgac accgctcgcg 5520

tcaaagaata ccgtggtcgt gctctggacg cagcggcggc ccgcgctcag ctggaccgct 5580

accacgcaga actgattgcc ggtctgcgtg catccaatgg cggttatgct ccgcgtggcc 5640

gtggcaccgc ataatctaga attctttaat taaagatccg gctgctaaca aagcccgaaa 5700

ggaagctgag ttggctgctg ccaccgctga gcaatccagg catcaaataa aacgaaaggc 5760

tcagtcgaaa gactgggcct ttcgttttat ctgttgtttg tcggtgaacg ctctcctgag 5820

taggacaaat ccgccgggag cggatttgaa cgttgcgaag caacggcccg gagggtggcg 5880

ggcaggacgc ccgccataaa ctgccaggca tcaaattaag cagaaggcca tcctgacgga 5940

tggccttttt gctagcagca cgccatagtg actggcgatg ctgtcggaat ggacgatatc 6000

ccgcaagagg cccggcagta ccggcataac caagcctatg cctacagcat ccagggtgac 6060

ggtgccgagg atgacgatga gcgcattgtt agatttcata cacggtgcct gactgcgtta 6120

gcaatttaac tgtgataaac taccgcatta aagcttatcg atgataagct gtcaaacatg 6180

agaattctta gaaaaactca tcgagcatca aatgaaactg caatttattc atatcaggat 6240

tatcaatacc atatttttga aaaagccgtt tctgtaatga aggagaaaac tcaccgaggc 6300

agttccatag gatggcaaga tcctggtatc ggtctgcgat tccgactcgt ccaacatcaa 6360

tacaacctat taatttcccc tcgtcaaaaa taaggttatc aagtgagaaa tcaccatgag 6420

tgacgactga atccggtgag aatggcaaaa gcttatgcat ttctttccag acttgttcaa 6480

caggccagcc attacgctcg tcatcaaaat cactcgcatc aaccaaaccg ttattcattc 6540

gtgattgcgc ctgagcgaga cgaaatacgc gatcgctgtt aaaaggacaa ttacaaacag 6600

gaatcgaatg caaccggcgc aggaacactg ccagcgcatc aacaatattt tcacctgaat 6660

caggatattc ttctaatacc tggaatgctg ttttcccggg gatcgcagtg gtgagtaacc 6720

atgcatcatc aggagtacgg ataaaatgct tgatggtcgg aagaggcata aattccgtca 6780

gccagtttag tctgaccatc tcatctgtaa catcattggc aacgctacct ttgccatgtt 6840

tcagaaacaa ctctggcgca tcgggcttcc catacaatcg atagattgtc gcacctgatt 6900

gcccgacatt atcgcgagcc catttatacc catataaatc agcatccatg ttggaattta 6960

atcgcggcct cgagcaagac gtttcccgtt gaatatggct cataacaccc cttgtattac 7020

tgtttatgta agcagacagt tttattgttc atgaccaaaa tcccttaacg tgagttttcg 7080

ttccactgag cgtcagaccc cgtagaaaag atcaaaggat cttcttgaga tccttttttt 7140

ctgcgcgtaa tctgctgctt gcaaacaaaa aaaccaccgc taccagcggt ggtttgtttg 7200

ccggatcaag agctaccaac tctttttccg aaggtaactg gcttcagcag agcgcagata 7260

ccaaatactg tccttctagt gtagccgtag ttaggccacc acttcaagaa ctctgtagca 7320

ccgcctacat acctcgctct gctaatcctg ttaccagtgg ctgctgccag tggcgataag 7380

tcgtgtctta ccgggttgga ctcaagacga tagttaccgg ataaggcgca gcggtcgggc 7440

tgaacggggg gttcgtgcac acagcccagc ttggagcgaa cgacctacac cgaactgaga 7500

tacctacagc gtgagctatg agaaagcgcc acgcttcccg aagggagaaa ggcggacagg 7560

tatccggtaa gcggcagggt cggaacagga gagcgcacga gggagcttcc agggggaaac 7620

gcctggtatc tttatagtcc tgtcgggttt cgccacctct gacttgagcg tcgatttttg 7680

tgatgctcgt caggggggcg gagcctatgg aaaaacgcca gcaacgcggc ctttttacgg 7740

ttcctggcct tttgctggcc ttttgctcac atgttctttc ctgcgttatc ccctgattct 7800

gtggataacc gtattaccgc ctttgagtga gctgataccg ctcgccgcag ccgaacgacc 7860

gagcgcagcg agtcagtgag cgaggaagcg gaagagcgcc tgatgcggta ttttctcctt 7920

acgcatctgt gcggtatttc acaccgcaat ggtgcactct cagtacaatc tgctctgatg 7980

ccgcatagtt aagccagtat acactccgct atcgctacgt gactgggtca tggctgcgcc 8040

ccgacacccg ccaacacccg ctgacgcgcc ctgacgggct tgtctgctcc cggcatccgc 8100

ttacagacaa gctgtgaccg tctccgggag ctgcatgtgt cagaggtttt caccgtcatc 8160

accgaaacgc gcgaggcagc tgcggtaaag ctcatcagcg tggtcgtgaa gcgattcaca 8220

gatgtctgcc tgttcatccg cgtccagctc gttgagtttc tccagaagcg ttaatgtctg 8280

gcttctgata aagcgggcca tgttaagggc ggttttttcc tgtttggtca ctgatgcctc 8340

cgtgtaaggg ggatttctgt tcatgggggt aatgataccg atgaaacgag agaggatgct 8400

cacgatacgg gttactgatg atgaacatgc ccggttactg gaacgttgtg agggtaaaca 8460

actggcggta tggatgcggc gggaccagag aaaaatcact cagggtcaat gccagcgctt 8520

cgttaataca gatgtaggtg ttccacaggg tagccagcag catcctgcga tgcagatccg 8580

gaacataatg gtgcagggcg ctgacttccg cgtttccaga ctttacgaaa cacggaaacc 8640

gaagaccatt catgttgttg ctcaggtcgc agacgttttg cagcagcagt cgcttcacgt 8700

tcgctcgcgt atcggtgatt cattctgcta accagtaagg caaccccgcc agcctagccg 8760

ggtcctcaac gacaggagca cgatcatgcg cacccgtggc caggacccaa cgctgcccga 8820

gatgcgccgc gtgcggctgc tggagatggc ggacgcgatg gatatgttct gccaagggtt 8880

ggtttgcgca ttcacagttc tccgcaagaa ttgattggct ccaattcttg gagtggtgaa 8940

tccgttagcg aggtgccgcc ggcttccatt caggtcgagg tggcccggct ccatgcaccg 9000

cgacgcaacg cggggaggca gacaaggtat agggcggcgc ctacaatcca tgccaacccg 9060

ttccatgtgc tcgccgaggc ggcataaatc gccgtgacga tcagcggtcc aatgatcgaa 9120

gttaggctgg taagagccgc gagcgatcct tgaagctgtc cctgatggtc gtcatctacc 9180

tgcctggaca gcatggcctg caacgcgggc atcccgatgc cgccggaagc gagaagaatc 9240

ataatgggga aggccatcca gcctcgcgtc gcgaacgcca gcaagacgta gcccagcgcg 9300

tcggccgcca tgccggcgat aatggcctgc ttctcgccga aacgtttggt ggcgggacca 9360

gtgacgaagg cttgagcgag ggcgtgcaag attccgaata ccgcaagcga caggccgatc 9420

atcgtcgcgc tccagcgaaa gcggtcctcg ccgaaaatga cccagagcgc tgccggcacc 9480

tgtcctacga gttgcatgat aaagaagaca gtcataagtg cggcgacgat agtcatgccc 9540

cgcgcccacc ggaaggagct gactgggttg aaggctctca agggcatcgg tcgactcata 9600

gctgtttcct gtgtgaaatt gttatccgct cacaattcca cacaacatac gagccggaag 9660

cataaagtgt aaagcctggg gtgcctaatg agtgagctaa cttacattaa ttgcgttgcg 9720

ctcactgccc gctttccagt cgggaaacct gtcgtgccag ctgcattaat gaatcggcca 9780

acgcgcgggg agaggcggtt tgcgtattgg gcgctagcgt ggtttttctt ttcaccagtg 9840

agacgggcaa cagctgattg cccttcaccg cctggccctg agagagttgc agcaagcggt 9900

ccacgctggt ttgccccagc aggcgaaaat cctgtttgat ggtggttaac ggcgggatat 9960

aacatgagct gtcttcggta tcgtcgtatc ccactaccga gatatccgca ccaacgcgca 10020

gcccggactc ggtaatggcg cgcattgcgc ccagcgccat ctgatcgttg gcaaccagca 10080

tcgcagtggg aacgatgccc tcattcagca tttgcatggt ttgttgaaaa ccggacatgg 10140

cactccagtc gccttcccgt tccgctatcg gctgaatttg attgcgagtg agatatttat 10200

gccagccagc cagacgcaga cgcgccgaga cagaacttaa tgggcccgct aacagcgcga 10260

tttgctggtg acccaatgcg accagatgct ccacgcccag tcgcgtaccg tcttcatggg 10320

agaaaataat actgttgatg ggtgtctggt cagagacatc aagaaataac gccggaacat 10380

tagtgcaggc agcttccaca gcaatggcat cctggtcatc cagcggatag ttaatgatca 10440

gcccactgac gcgttgcgcg agaagattgt gcaccgccgc tttacaggct tcgacgccgc 10500

ttcgttctac catcgacacc accacgctgg cacccagttg atcggcgcga gatttaatcg 10560

ccgcgacaat ttgcgacggc gcgtgcaggg ccagactgga ggtggcaacg ccaatcagca 10620

acgactgttt gcccgccagt tgttgtgcca cgcggttggg aatgtaattc agctccgcca 10680

tcgccgcttc cactttttcc cgcgttttcg cagaaacgtg gctggcctgg ttcaccacgc 10740

gggaaacggt ctgataagag acaccggcat actctgcgac atcgtataac gttactggtt 10800

tcacattcac caccctgaat tgactctctt ccgggcgcta tcatgccata ccgcgaaagg 10860

ttttgcgcca ttcgatggtg tcggaattta tgcggtgtga aataccgcac agatgcgtaa 10920

ggagaaaata ccgcatcagg cgctcttccg cttcctcgct cactgactcg ctgccacccc 10980

gtggcatgca aggagatggc gcccaacagt cccccggcca cggggcctgc caccataccc 11040

acgccgaaac aagcgctcat gagcccgaag tggcgagccc gatcttcccc atcggtgatg 11100

tcggcgatat aggcgccagc aaccgcacct gtggcgccgg tgatgccggc cacgatgcgt 11160

ccggcgtaga gg 11172